# EUROPEAN PATENT SPECIFICATION

(11) **EP 0 979 823 B1**
(45) Date of publication and mention of the grant of the patent: **29.03.2006**
(21) Application number: 98953063.9
(22) Date of filing: 16.11.1998
(51) Int. Cl.: C07D 403/12, C07D 413/12, C07D 417/12, A61K 31/44, A61K 31/505, C07D 401/12, C07D 235/00, C07D 213/00, C07D 263/00, C07D 277/00

(54) **NOVEL AMIDE COMPOUNDS AND DRUGS CONTAINING THE SAME**
NEUE AMIDVERBINDUNGEN UND MEDIKAMENTE DIE DIESE ENTHALTEN
NOUVEAUX COMPOSES D'AMIDE ET MEDICAMENTS CONTENANT CES DERNIERS

(30) Priority: 14.11.1997 JP 33087797
(43) Date of publication of application: 16.02.2000
(73) Proprietor: Kowa Company Ltd., Nagoya-shi Aichi-ken, 460-8625 (JP)
(72) Inventor: SHIBUYA, Kimiyuki, 403 Lions-Hills-Nishitokorozawa, Tokorozawa-shi, Saitama 359-1142 (JP); MIURA, Toru, Omiya-shi, Saitama 331-0062 (JP); KAWAMINE, Katsumi, Kowa-Higashimurayama-ryo, Higashimurayama-shi, Tokyo 189-0022 (JP); SATO, Yukihiro, Kowa-Higashimurayama-ryo, Higashimurayama-shi, Tokyo 189-0022 (JP); OHGIYA, Tadaaki, Tokorozawa-shi, Saitama 359-0038 (JP); KITAMURA, Takahiro, 202, Kowa-Higashimurayama-so, Higashimurayama-shi, Tokyo 189-0022 (JP); OZAKI, Chiyoka, Tokyo 177-0045 (JP); EDANO, Toshiyuki, 304, Itaya-Kawagoe-Park-Hitsu, Kawagoe-shi, Saitama 350-1124 (JP); HIRATA, Mitsuteru, Tsurugashima-shi, Saitama 350-2211 (JP)
(74) Representative: Cresswell, Thomas Anthony
(86) International application number: PCT/JP1998/005149
(87) International publication number: WO 1999/025712

(56) References cited:
- EP-A- 0 807 627
- JP-A- 4 139 172
- JP-A- 10 029 986
- JP-A- 62 042 989
- JP-B- 51 016 433
- US-A- 5 358 946
- WILDE R G ET AL: "Acyl CoA:cholesterol acyltransferase (ACAT) inhibitors: ureas bearing heterocyclic groups bioisosteric for an imidazole" BIOORGANIC & MEDICINAL CHEMISTRY LETTERS, OXFORD, GB, vol. 5, no. 2, 19 January 1995 (1995-01-19), pages 167-172, XP004135810 ISSN: 0960-894X
- WILDE R G ET AL: "Acyl CoA:cholesterol acyltransferase (ACAT) inhibitors: ureas bearing two heterocyclic head groups" BIOORGANIC & MEDICINAL CHEMISTRY LETTERS, OXFORD, GB, vol. 5, no. 2, 19 January 1995 (1995-01-19), pages 173-176, XP004135811 ISSN: 0960-894X
- MATSUDA K: "ACAT INHIBITORS AS ANTIATHEROSCLEROTIC AGENTS: COMPOUNDS AND MECHANISMS" MEDICINAL RESEARCH REVIEWS, NEW YORK, NY, US, vol. 14, no. 3, 1994, pages 271-305, XP000567458 ISSN: 0198-6325
- R BELLEMIN, J DECERPRIT, D FESTAL: "New indole derivativesas ACAT inhibitors: synthesis and structure-activity relationships" EUR J MED CHEM, vol. 31, 1996, pages 123-132, XP002211604
- MAHMOUD ABDALLA M. et al., "Synthesis of some New Aryl- and Aralkyl- Mercaptobenzoxazoles,-Benzimidazoles and -Benzothioazoles of Potential Biological Interest", GAZZ. CHIM. ITAL., Vol. 112, No. 1-2, 1982, pages 55-56, XP002918407
- MAHMOUD ABDALLA M. et al., "Synthesis and Biological Activities of some New 2-(N-Heterocyclic Carboxamidomethyl Thio) Benzoxazoles, Benzthiazoles and Benzimidazoles. Part VIII", EUR. J. MED. CHEM.-CHIM. THER., Vol. 16, No. 4, (1981), p. 383-384, XP002918408
- ABDEL RAHMAN A.E. et al., "Synthesis and Biological Activities of some New 2-(N-Heterocyclo Carboxamidomethyl Thio) Naphth[1,2-d]Oxazoles. Part VI", J. INDIAN CHEM. SOC., Vol. 58, No. 2, 1981, pages 171-173, XP002918409
- EL-EZBAWY S.R. et al., "Synthesis and Antibacterial Activity of some New Pyridyl DiarylSulfide and DiarylSulfones", PHOSPHORUS, SULFUR SILICON RELAT. ELEM., Vol. 48, No. 1-4, (1990), p. 111-116, XP002918410
- TURAN-ZITOUNI G. et al., "Synthesis of some 5-Methoxy-3-[(Benzazole-2-yl)Thioacetylamin o]-2,3-Dihydrobenzofuran Derivatives and Investigation of Antihypertensive Activities", ANN. PHARM. FR., Vol. 54, No. 3, 1996, p. 109-111, XP002918411
- TRIVEDI BHAVIN et al., "Synthesis and Antimicrobial Activity of 2-Aryl-3- Benzothiazol-2'-Yl-Thioacetamido)-5-H/ Methyl/carboxymethyl-4-Thiazolidinones", PROC. INDIAN ACAD. SCI. (CHEM. SCI.), Vol. 104, No. 4, 1992, p. 489-496, XP002918412

## Description

The present invention relates to novel amide compounds and medications containing the same. More specifically the present invention relates to ACAT inhibitor compounds represented by the formula (I) 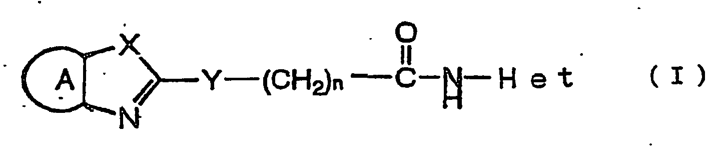 wherein 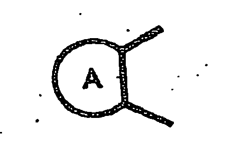 represents an optionally substituted benzene, pyridine, cyclohexane or naphthalene.

Het represents a substituted or unsubstituted pyridyl or pyrimidyl group as further defined below,
X represents -NH-, an oxygen atom or a sulfur atom,
Y represents -NR₄-, an oxygen atom, a sulfur atom, a sulfoxide or a sulfone,
R₄ represents a hydrogen atom, a lower alkyl group, an aryl group or an optionally substituted silyl lower alkyl group, and
n is an integer of from 1 to 15,
or salts or solvates thereof, and a pharmaceutical composition containing these compounds.

In one aspect the present invention relates to compounds represented by the the formula (IA) 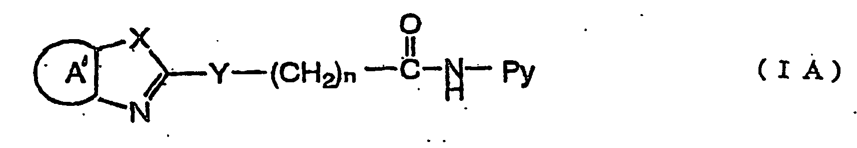 wherein 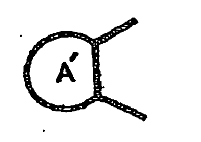 represents an optionallysubstituted benzene or pyridine,
Py represents an optionally substituted pyridyl or pyrimidyl group as further defined below,
X represents -NN-, an oxygen atom or a sulfur atom,
Y represents -NR₄-, an oxygen atom, a sulfur atom, a sulfoxide or a sulfone,
R₄ represents a hydrogen atom, a lower alkyl group, an aryl group or an optionally substituted silyl lower alkyl group, and n is an integer of from 1 to 15,
or salts or solvates thereof, and a pharmaceutical composition containing these compounds.

Preferably, the present invention relates to compounds represented by the formula (II) 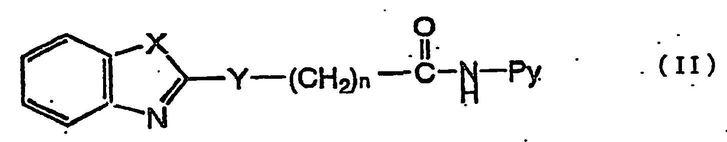 wherein
X represents -NH-, an oxygen atom or a sulfur atom,
Y represents -NR₄-, an oxygen atom, a sulfur atom, a sulfoxide or a sulfone,
R₄ represents a hydrogen atom, a lower alkyl group, an aryl group or an optionally substituted silyl lower alkyl group,
Py represents an optionally substituted pyridyl or pyrimidyl group as further defined below,
and n is an integer of from 1 to 15,
or salts or solvates thereof, and a pharmaceutical composition containing these compounds.

### BACKGROUND ART :

In recent years, hyperlipemia and arteriosclerosis derived therefrom have been rapidly increased with the change to western eating habits with high-calory and high-cholesterol foods based on the higher level of life and with the advance of age of the population, and this has been one of social problems. The conventional pharmacotherapy of hyperlipemia and arteriosclerosis has mainly put stress on the decrease in blood lipid that causes these diseases, and the lesion of the arteriosclerosis itself has not been treated as a target. Acyl coenzyme A cholesterol acyltransferase (ACAT) is an enzyme that catalyzes synthesis from cholesterol to cholesterol ester, and plays a vital role in metabolism of cholesterol and absorption thereof in digestive organs. Inhibition of the ACAT enzyme that catalyzes esterification of free cholesterol in epithelial cells of the small intestine results in inhibition of absorption of cholesterol from the intestine, and inhibition of synthesis of cholesterol ester in the liver based on the ACAT inhibition results in suppression of secretion of VLDL from the liver to the blood. These results are considered to lead to an activity of decreasing blood cholesterol. Most of conventional ACAT inhibitors have been expected to exhibit an activity of decreasing blood cholesterol as an antihyperlipemic agent by acting on the ACAT enzymes in the small intestine and the liver.

For example, as an ACAT inhibitor, the specification of U. S. Patent No. 4,716,175 describes 2,2-dimethyl-N-(2,4,6-trimethosyphenyl) dodecanamide, and European Patent No. 372,445 describes N'-(2,4-difluorophenyl)-N-[5-(4,5-diphenyl-1H-imidazol-2-ylthio)pentyl]-N-heptylurea. However, most of the conventional ACAT inhibitors have put stress on an activity of decreasing blood cholesterol as an antihyperlipemic agent, and the administration thereof at a high dose for exhibiting its activity has often caused side effects such as intestinal bleeding, intestinal disorders, diarrhea, bepatopathy and the like at the stage of a clinical test, making difficult the clinical development thereof.

The arteriosclerosis is inherently a characteristic lesion such as intima hypertrophy and lipidosis of the blood vessel. According to the recent studies, suppression of foamation of macrophages that play a main role in formation of the arteriosclerosis lesion has been expected to lead to regression of the arteriosclerosis lesion itself. Foam cells derived from macrophages (cholesterol ester is stored in cells as fat droplets) have been observed in the gruel arteriosclerosis lesion, and the foamation of macrophages is deemed to deeply participate in the progression of the lesion. Further, it has been reported that the ACAT activity in the blood vessel wall in the arteriosclerosis lesion site is increased and cholesterol ester is stored in the blood vessel wall [refer to Gillease, J. et al., Exp. Mole. Pathol., 44, 329 - 339 (1986)].

The inhibition of esterification of cholesterol with an ACAT inhibitor results in formation of free cholesterol in cells, and this free cholesterol is removed with high-density lipoprotein (HDL), transferred to the liver (inversely transferred with HDL), and metabolized. Accordingly, suppression of storage of cholesterol ester in the lesion site is expected. As a result, it is considered to provide a direct anti-arteriosclerotic activity. There is a report that ACAT includes two types, a type present in the small intestine and a type present in the blood vessel wall [Kinunen M. et al., Biochemistry, 27, 7344-7350 (1988)]. However, many of the past researches on the ACAT inhibitor have been conducted using an enzyme of a type present in the small intestine and the liver [Tomoda Eiichi et al., J. Antibiotics, 47, 148 - 153 (1994)].

The present inventors considered that medications which selectively inhibit an ACAT enzyme of a type present in the blood vessel wall can be those for treating arteriosclerosis that give less side effects, and have conducted synthesis and researches of such inhibitors.

The present inventors continued studies for achieving this object, and found in advance that compounds represented by the formula (IV) 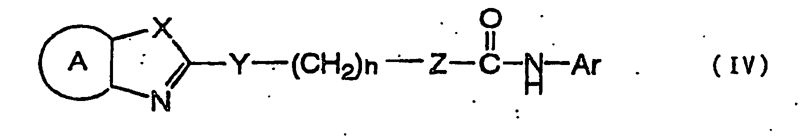 wherein 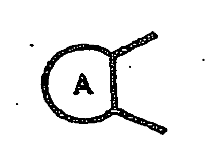 represents an optionally substituted divalent residue such as benzene, pyridine, cyclohexane or naphthalene or a group, 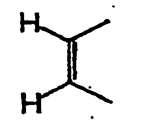 Ar represents an optionally substituted aryl group
X represents -NH-, an oxygen atom or a sulfur atom,
Y represents -NR₄-, an oxygen atom, a sulfur atom, a sulfoxide or a sulfone,
Z represents a single bond or -NR₅-,
R₄ represents a hydrogen atom, a lower alkyl group, an aryl group or an optionally substituted silyl lower alkyl group,
R₅ represents a hydrogen atom, a lower alkyl group, an aryl group or an optionally substituted silyl lower alkyl group, and
n is an integer of from 0 to 15,
or salts or solvates thereof , and compounds represented by the formula (V) 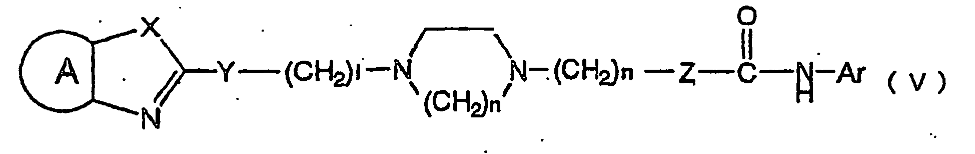 wherein 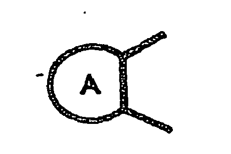 represents an optionally substituted divalent residue such as benzene, pyridine, cyclohexane or naphthalene, or a group, 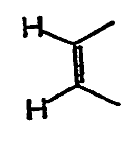 Ar represents an optionally substituted aryl group,
X represents -NH-, an oxygen atom or a sulfur atom,
Y represents -NR₄-, an oxygen atom, a sulfur atom, a sulfoxide or a sulfone,
Z represents a single bond or -NR₅-,
R₄ represents a hydrogen atom, a lower alkyl group, an aryl group or an optionally substituted silyl lower alkyl group,
R₅ represents a hydrogen atom, a lower alkyl group, an aryl group or an optionally substituted silyl lower alkyl group,
l is an integer of from 0 to 15,
m is an integer of 2 or 3, and
n is integer of from 0 to 3,
or salts or solvates thereof have an excellent ACAT inhibitory activity, and they applied the same for patents (Japanese Patent Application Nos. 88,660/1997, 90, 146/1997 and 149, 892/1997).

Further, as compounds similar to the compounds represented by the formula (I), 3-(benzothiazol-2-ylthio)-N-(phenyl)propanamide is disclosed in J. Chem. Eng. Data, 27, 207 (1982), and 3-(benzoxazol-2-ylthio)-N-(phenyl)propanamide in Fungitsidy, Ed. Melnikov, N. N. Izd. FanUzb. SSR: Tashkent, USSR. 82 - 88 (1980). However, these compounds are not only those in which an amide moiety is a phenyl group, but also these documents are totally devoid of the description that the compounds have an ACAT inhibitory activity.

Thus, the present inventors found that the compounds represented by the formula (IV) or (V) have an organ-selective ACAT inhibitory activity and an intracellular cholesterol transfer inhibitory activity, and that these are useful as an antihyperlipemic agent having an activity of decreasing blood cholesterol and as an agent for preventing and treating arteriosclerosis having a macrophage formation inhibitory activity.

However, the compounds represented by these formulas (IV) and (V) did not necessarily have a sufficient activity, nor was the organ-selectivity satisfactory.

Under these circumstances, the present inventors have conducted further investigations to develop an ACAT inhibitor having a superior ACAT inhibitory activity, and have consequently found that the compounds represented by the formula (I) are useful ACAT inhibitors which conquer the above-mentioned defects . This finding has led to the completion of the present invention.

### Disclosure of Invention

The present invention provides ACAT inhibitor compounds represented by the formula (I) as defined above wherein
Het represents a substituted or unsubstituted pyridyl or pyrimidyl group, wherein any substituent on Het is selected from an amino group substituted with 1 or 2 lower alkyl groups each having from 1 to 10 carbon atoms, a lower alkoxy group having from 1 to 10 carbon atoms, a lower alkylthio group having from 1 to 10 carbon atoms, a lower alkylcarbonyl group having from 1 to 10 carbon atoms, a halogen atom an amino group or a lower alkyl group having from 1 to 10 carbon atoms, a hydroxyl group, a phosphate group, a sulfonamide group, a lower alkyl sulphonyl group having 1 to 10 carbon atoms, or two substituents may be bound to form an alkylenedioxy group of 1 to 10 carbon atoms or salts or solvates thereof.

Further, the present invention is to provide a pharmaceutical composition containing at least one type selected from the compounds represented by the formula (I), and the salts and the solvates thereof in a therapeutically effective amount, and a pharmaceutically acceptable carrier.

Still further, the present invention is to provide such a compound, salt or solvate for use in a method of traitement of the human or animal body by therapy, for instance for use as an ACAT inhibitor, an intracellular cholesterol transfer inhibitor, a blood cholesterol depressant or a macrophage formation suppressant The present invention also provides such a compound, salt or solvate for use in treating or preventing diseases such as hyperlipemia, arteriosclerosis, cervical and cerebral arteriosclerosis, cerebrovascular accidents, ischemic heart disease, coronary arteriosclerosis, nephrosclerosis, arteriosclerotic nephrosclerosis, arteriolonephrosclerosis, malignant nephrosclerosis, ischemic intestinal disease, acute occlusion of mesenteric vessel, chronic mesenteric angina, ischemic colitis, aortic aneurysm and arteriosclerosis obliterans (ASO).

### Best Mode for Carrying Out the Invention

Preferable examples of the compounds represented by the the formula (IA) are defined above.

More preferable examples of the compounds represented by the formula (I) in the present invention are the compounds represented by the formula (II) as defined above.

As further preferable examples of the compounds represented by the formula (I) in the present invention, the compounds represented by the formula (III) 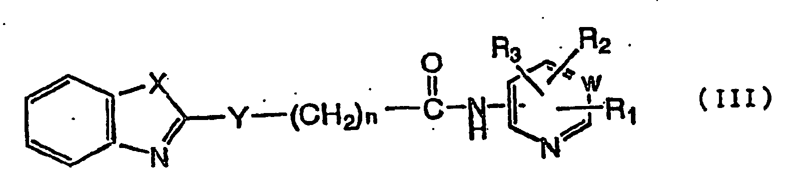 wherein
W represents =CH- or =N-, and
R₁, R₂ and R₃ are the same or different, and each represents a hydrogen atom, a lower alkyl group, a lower alkoxy group, a halogen atom, a hydroxyl group, a phosphate group, a sulfonamide group, a lower alkylthio group or an optionally substituted amino group, or two of R₁, R₂ and R₃ together form an alkylenedioxide group.

These pyridyl groups and pyrimidyl groups may be unsubstituted, but have preferably one or more substituents. Preferable examples the substituents include an amino group substituted with a lower alkyl group, a lower alkoxy group, a lower alkylthio group, a lower alkylcarbonyl group, a halogen atom, an amino group or a lower alkyl group. Further, two substituents may be bound to form an alkylenedioxy group such as a methylenedioxy group.

As the lower alkyl group having from 1 to 10 carbon atoms, a linear or branched alkyl group, preferably from 1 to 6 carbon atoms, is preferable. Especially preferable examples thereof include methyl, ethyl, n-propyl, iso-propyl, n-butyl, iso-butyl, tert-butyl, n-pentyl and n-hexyl groups.

As the lower alkyl group having from 1 to 10 carbon atoms in the lower alkoxy group, the lower alkylthio group and the lower alkylcarbonyl group, the above-mentioned linear or branched alkyl group, preferably from 1 to 6 carbon atoms is preferable. Examples thereof include methoxy, ethoxy, n-propoxy, iso-propoxy, n-butaxy, iso-butoxy, tert-butoxy, n-pentyloxy, n-hexyloxy, methylthio, ethylthio, n-propylthio, iso-propylthio, n-butylthio, iso-butylthio, tert-butylthio, n-pentylthio, n-hexylthio, methylcarbonyl, ethylcarbonyl, n-propylcarbonyl, iso-propylcarbonyl, n-butylcarbonyl, iso-butylcarbonyl, tert-butylcarbonyl, n-pentylcarbonyl and n-hexylcarbonyl groups.

Preferable examples of the halogen atom are fluorine, chlorine, bromine and iodine atoms.

Examples of the substituent in the substituted amino group include the above-mentioned lower alkyl and the number of the substituent in the amino group may be 1 or 2. Preferable examples of the substituted amino group include methylamino, ethylamino, dimethylamino and diethylamino groups.

The alkylene group of the alkylenedioxy group is a linear or branched alkylene group having from 1 to 10 carbon atoms, preferably from 1 to 5 carbon atoms. Preferable examples thereof include methylenedioxy and ethylenedioxy groups.

As the preferable Het group, a group represented by the formula (VI) is mentioned. 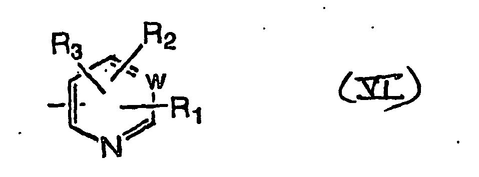 wherein W, R₁, R₂ and R₃ are as defined above.

Preferable examples of the Het group include
2-methylthio-3-pyridyl,
2-ethylthio-3-pyridyl,
2-(iso-propylthio)-3-pyridyl,
2-methoxy-3-pyridyl,
2-chloro-3-pyridyl,
2-methylthio-4-methyl-3-pyridyl,
2-ethylthio-4-methyl-3-pyridyl,
2-(iso-propylthio)-4-methyl-3-pyridyl,
2-methoxy-4-methyl-3-pyridyl,
2,6-bis(methylthio)-3-pyridyl,
2,6-bis(ethylthio)-3-pyridyl,
2,6-bis(iso-propylthio)-3-pyridyl,
2-methylthio-6-methoxy-3-pyridyl,
2-ethylthio-6-methoxy-3-pyridyl,
2-(iso-propylthio)-6-methoxy-3-pyridyl,
2-methylthio-6-methyl-3-pyridyl,
2-ethylthio-6-methyl-3-pyridyl,
2-(iso-propylthio)-6-methyl-3-pyridyl
2,6-dimethoxy-3-pyridyl,
2-methoxy-6-methyl-3-pyridyl,
2-methyl-6-methylthio-3-pyridyl,
2-methyl-6-ethylthio-3-pyridyl,
2-methyl-6-(iso-propylthio)-3-pyridyl,
2-methyl-6-methoxy-3-pyridyl,
2,6-dimehtyl-3-pyridyl,
2,6-diethyl-3-pyridyl,
2,4-bismethylthio-6-methyl-3-pyridyl,
2,4-bisethylthio-6-methyl-3-pyridyl,
2,4-bis(iso-propylthio)-6-methyl-3-pyridyl,
2,4-dimethoxy-6-methyl-3-pyridyl,
2,4,6-trimethyl-3-pyridyl,
4-ethyl-2,6-dimethyl-3-pyridyl,
2,4-dichloro-6-methyl-3-pyridyl,
4,6-bis(methylthio)-5-pyrimidyl,
4,6-bis(ethylthio)-5-pyrimidyl,
4,6-bis(iso-propylthio)-5-pyrimidyl,
4,6-dimethoxy-5-pyrimidyl,
4,6-dichloro-2-methyl-5-pyrimidyl,
4,6-bis(dimethylamino)-5-pyrimidyl,
4,6-bismethylthio-2-methyl-5-pyrimidyl.
2,4,6-trimethoxy-5-pyrimidyl
4-methyl-6-methyltio-3-pyridyl,
5-methylthlo-2-pyridyl,
2,4,6-tris(methylthio)-5-pyrimidyl groups and so on.

The substituent 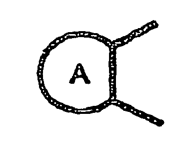 in the compounds represented by the the formula (I) in the present invention is a divalent group adjacent the azole ring which is formed with two carbon atoms constituting the azole ring. It is an optionally substituted benzene, pyridine, cyclohexane or naphthalene.

An optionally substituted benzene or pyridine is preferable. These divalent groups may have a substituent. Examples of the substituent include (a) the above-mentioned lower alkyl group, lower alkoxy group, lower alkylsulfonyl group lower alkylthio group, lower alkylcarbonyl group, halogen atom, amino group, amino group substituted with the lower alkyl group, or (b) substituted or unsubstituted aryl group such as a phenyl group or a naphthyl group, and substituted or unsubstituted aralkyl group such as a benzyl group or a phenethyl group as described below. Further, the two substituents may be bound to form an alkylenedioxy group such as a methylenedioxy group.

The substituent X in the compounds represented by the formula (I) in the present invention represents -NH- , an oxygen atom or a sulfur atom, and forms, together with the above-mentioned substituent, an azole ring such as imidazole, oxazole or thiazole.

Further, the substituent Y in the compounds represented by the formula (I) of the present invention represents -NR₄-, an oxygen atom, a sulfur atom, a sulfoxide or a sulfone, and the substituent R₄ of the nitrogen atom represents a hydrogen atom, a lower alkyl group, an aryl group or an optionally substituted silyl lower alkyl group. The lower alkyl group as the substitutent R₄ is as mentioned above and the aryl group is described below. Examples thereof include methyl, ethyl and phenyl groups. The lower alkyl group of the optionally substituted silyl lower alkyl group as the substituent R₄ may be the above-mentioned group. Examples of the substituent of the silyl lower alkyl group include the above-mentioned lower alkyl, and the following aryl and aralkyl groups.

As the aryl groups mentioned above, aryl groups having from 6 to 20 carbon atoms, preferably from 6 to 10 carbon atoms, are mentioned. These aryl groups may be unsubstituted or substituted with the above-mentioned lower alkyl group, lower alkoxy group, lower alkylthio group, lower alkylcarbonyl group, halogen atom, amino group or amino group substituted with the lower alkyl group. Preferable examples of these aryl groups include phenyl, naphthyl, 2-methoxyphenyl and 4-methylthiophenyl groups.

The aralkyl groups mentioned above are aralkyl groups having from 7 to 20 carbon atoms, preferably from 7 to 12 carbon atoms. These aralkyl groups may be unsubstituted or substituted with the above-mentioned lower alkyl group, lower alkoxy group, lower alkylthio group, lower alkylcarbonyl group, halogen atom, amino group or amino group substituted with the lower alkyl group. Preferable examples of these aralkyl groups include benzyl, phenethyl and 4-methylbenzyl groups.

Preferable examples of the silyl lower alkyl group include trimethylsilylmethyl and dimethylphenylsilylmethyl groups.

As the substituent Y, a sulfur atom is preferable.

The number n of recurring units in the compounds represented by the formula (I) in the present invention is an integer of from 1 to 15, preferably an integer of from 1 to 9. As the recurring unit, a methylene group is mentioned in the formula (I). The methylene group may have a substituent or one or more methylene units may be substituted with a heteroatom such as a nitrogen atom, an oxygen atom or a sulfur atom unless the ACAT inhibitory activity of the present invention is impaired.

The substituents X, Y and the recurring unit in the compounds represented by the formula (II) in the present invention are the above-mentioned ones. The substituent Py represents an optionally substituted pyridyl or pyrimidyl group. The group represented by the formula (VI) is preferable.

The substituents X, Y and the recurring unit in the compounds represented by the formula (III) in the present invention are the above-mentioned ones. The substituent W represents a carbon atom or a nitrogen atom, and forms a pyridine or pyrimidine ring. Further, the substituents R₁, R₂ and R₃ are the same or different, and each represents a hydrogen atom, a lower alkyl group, a lower alkoxy group, a halogen atom, a hydroxyl group, a phosphate group, a sulfonamide group, a lower alkylthio group or an optionally substituted amino group, or two of R₁, R₂ and R₃ together form an alkylenedioxy group. Of these groups, the lower alkyl group, the lower alkoacy group, the halogen atom, the lower alkylthio group, the optionally substituted amino group and the alkylenedioxy group are the above-mentioned ones. Preferable examples of R₁, R₂ and R₃ include methyl, ethyl, iso-propyl, methoxy, ethoxy and iso-propoxy groups, chlorine, and methylthio, ethylthio, iso-propylthio and dimethylamino groups. The site of the pyridine ring or the pyrimidine ring bound to the adjacent nitrogen atom is not particularly limited either unless the ACAT inhibitory activity of the present invention is impaired.

The salts of the compounds represented by the formula (I), (II) or (III) in the present invention are not particularly limited unless the ACAT inhibitory activity of the present invention is impaired. Acid addition salts or base addition salts can be used as required. Preferable examples of the acid addition salts include inorganic acid salts such as a hydrochloride, a sulfate, a nitrate and a phosphate; and organic acid salts such as a methanesulfonate, a maleate, a fumarate and a citrate.

Further, the solvates of the compounds represented by the formula (I), (II) or (III) in the present invention are products to which solvents used in the production, the purification or the like, such as water, alcohol and the like are added, and are not particularly limited unless they have an adverse effect on the ACAT inhibitory activity. As the solvates, hydrates are preferable.

A process for producing the compounds of the present invention is described below.

Compounds (I) can be produced by various known processes, and the process is not particularly limited. For example, compounds (I) can be produced according to the following reaction steps.

Process for producing compounds of the formula (I):

A carboxylic acid represented by the formula (VII) or its reactive derivative, for example, an acidhalide, is reacted with a heterocyclic amine represented by the formula (VIII) according to the following reaction formulae 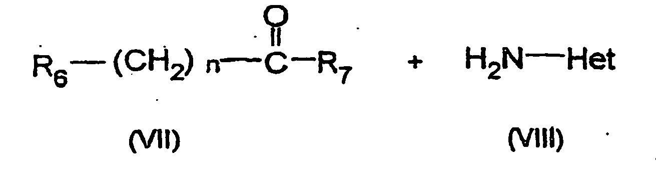 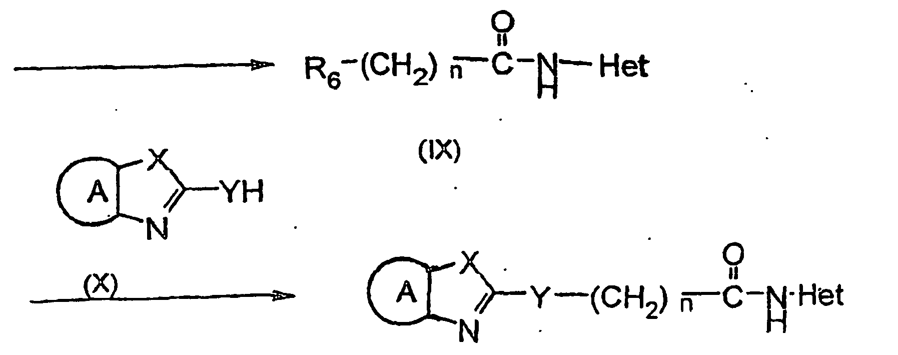 wherein R₆ represents a leaving group, and R₇ represents a reactive derivative residue of a hydroxyl group or a carboxylate group, to form an amide derivative represented by the formula (IX). When the resulting compound of the formula (IX) is reacted with an azole derivative represented by the formula (X), a desired compound (I') can be produced.

An ordinary method used in peptide synthesis can be applied to the reaction between compounds (VII) and (VIII). Examples of the leaving group R₆ in the formula (VII) include halogen atoms such as chlorine and bromine atoms. Preferable examples of the reactive derivative residue R₇ include acid anhydride residues with mesylic acid, tosylic acid, acetic acid, pivaloylic acid and the like. This reaction is described more specifically below. The desired compound can be obtained by reacting both of the compounds in a solvent in the presence of a condensation agent. As the condensation agent, for example, 1-(3'-dimethylaminopropyl)-3-ethylcarbodiimide (WSC) and 1,3-dicyclohexylcarbodiimide (DCC) may be used singly, and a combination of 1-hydroxybenzotriazole (HOBt) and N-hydroxysuccinimide (HOSu) is also available. The solvent is not particularly limited. For example, dimethylformamide, methylene chloride, chloroform, tetrahydrofuran and toluene can be used either singly or in combination. The reaction conditions vary depending on a starting material to be used. Generally, the reaction is conducted at from 0 to 100°C, preferably at a temperature close to room temperature, for from 1 to 30 hours, preferably for from 10 to 20 hours. In this manner, the reaction is completed. Further, when a carbonyl halide having a high reactivity is used as compound (VII), for example, compounds (VII) and (VIII) can be reacted in the presence of a base, for example, triethylamine, 4-dimethylaminopyridine or N-methylmorpholine in a usual manner.

With respect to starting compounds (VII) and (VIII), for example, compound (VII) can be produced by a method in which a haloalkyl alcohol is oxidized into a carboxylic acid with a Jones" reagent or the like, and compound (VIII) by a method in which a nitrated heterocyclic compound is subjected to a reduction reaction such as a catalytic reduction or the like to obtain a corresponding amino -heterocyclic compound, respectively.

The reaction between compounds (IX) and (X) obtained by the above-mentioned methods can be conducted in a solvent in the presence or absence of a base. As the solvent, the above-mentioned various types can be used. The base includes inorganic bases, for example, alkali metal hydroxides such as sodium hydroxide and potassium hydroxide, alkali metal carbonates such as sodium carbonate and potassium carbonate, and alkali metal hydrogencarbonates such as sodium hydrogencarbonate and potassium hydrogencarbonate; and organic bases such as pyridine, triethylamine, N,N-diisopropylethylamine, N-methylmorpholine and N,N-dimethylaniline.

Further, with respect to the desired compound represented by the formula (I'), according to the reaction shown by the following formula 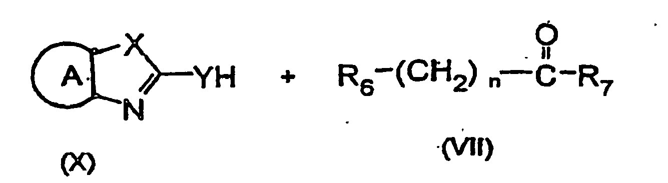 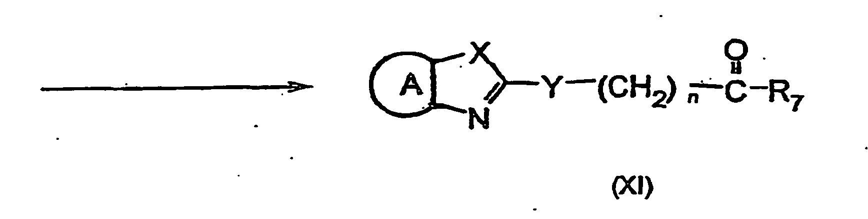 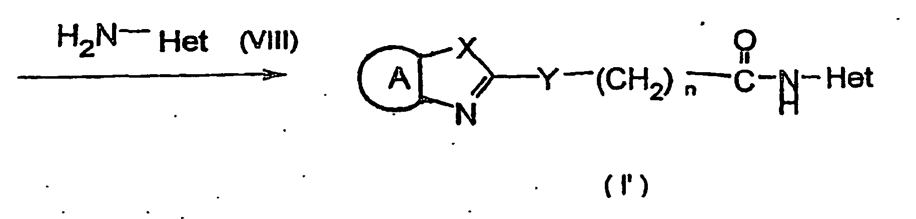 wherein R₆ represents a leaving group, and R₇ represents a reactive derivative residue of a hydroxyl group or a carboxylate group, an azole derivative represented by the formula (X) is reacted with a free carboxylic acid or an inactive substance of a carboxylic acid as the compound represented by the formula (VII) to obtain a carboxylic acid derivative represented by the formula (XI). When the resulting compound represented by the formula (XI) or its reactive derivative, for example, an acid halide, is reacted with a heterocyclic amine derivative represented by the formula (VIII), the desired compound (I') can be produced.

The reaction between compounds (X) and (VII) can be conducted according to the second step of the above-mentioned reaction formula. The reaction in which potassium hydroxide is used as a base and ethanol as a solvent respectively is especially preferable. The reaction between the resulting compounds (XI) and (VIII) can be conducted according to the first step of the above-mentioned reaction formula.

The intermediate and the desired compound obtained in each of the above-mentioned reactions can be isolated and purified by a purification method which is ordinarily used in the synthetic organic chemistry, such as filtration, extraction, washing, drying, concentration, recrystallization and various chromatographies. Further, each intermediate is subjected to the subsequent step without any purification unless any trouble is caused, which is well known to those skilled in the art.

The resulting compounds (I) can be formed into salts of the present invention in a usual manner.

Further, compounds (I) can be formed into solvates with solvents such as a reaction solvent, a recrystallization solvent and the like, especially hydrates in a usual manner, which is well known to those skilled in the art.

The compounds represented by the formula (I), (II) or (III), which are obtained by the process of the present invention are shown in Tables 1 to 63 below.

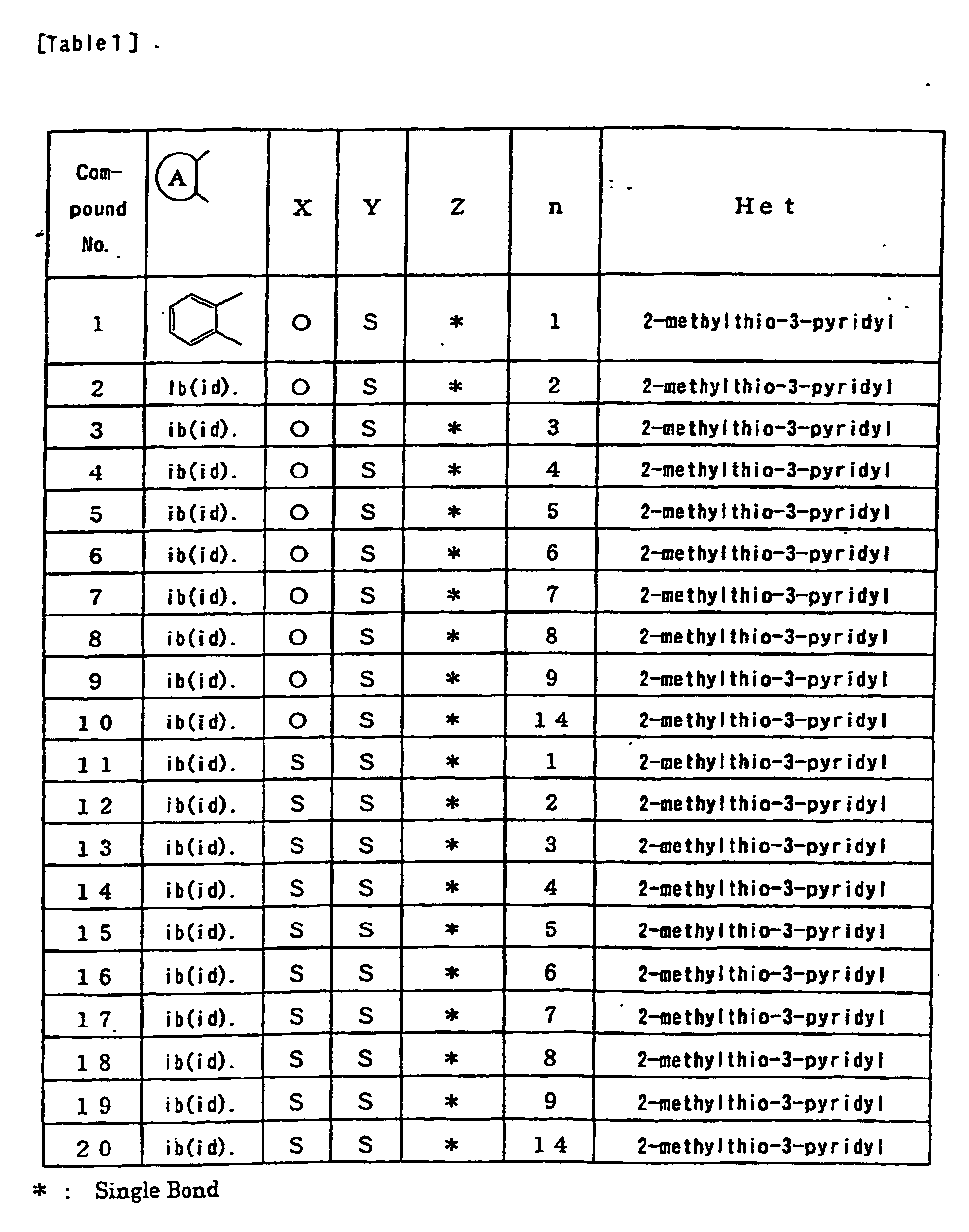

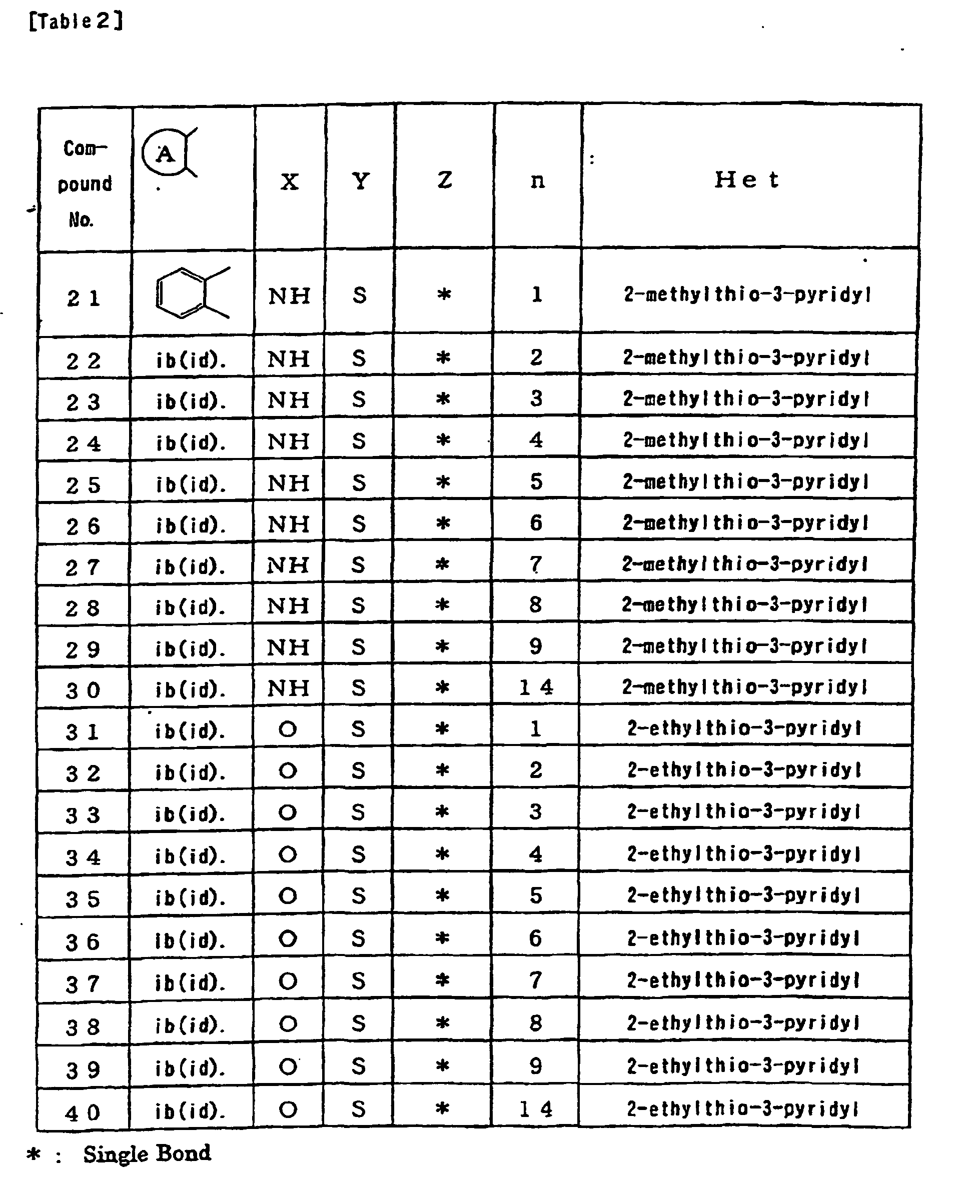

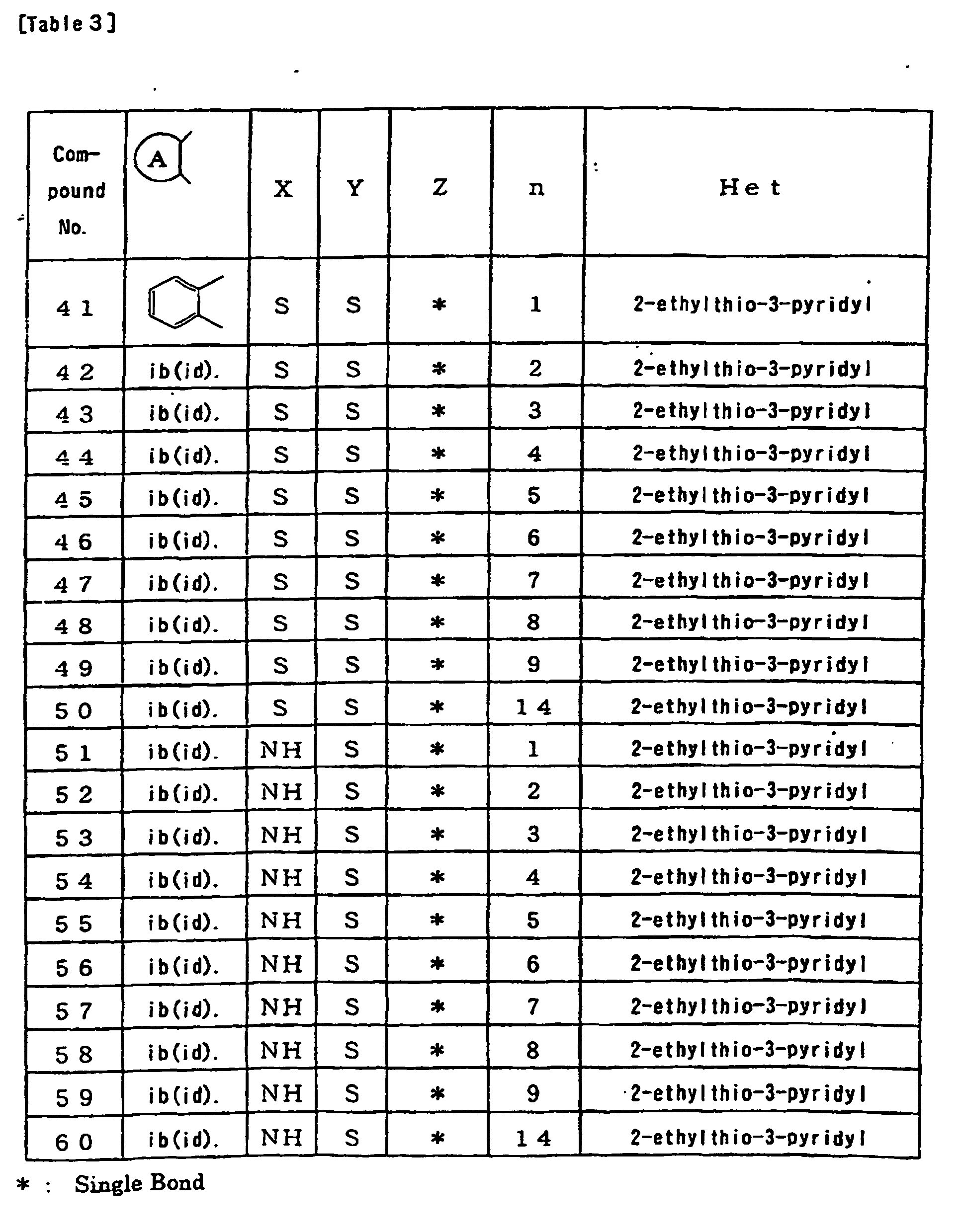

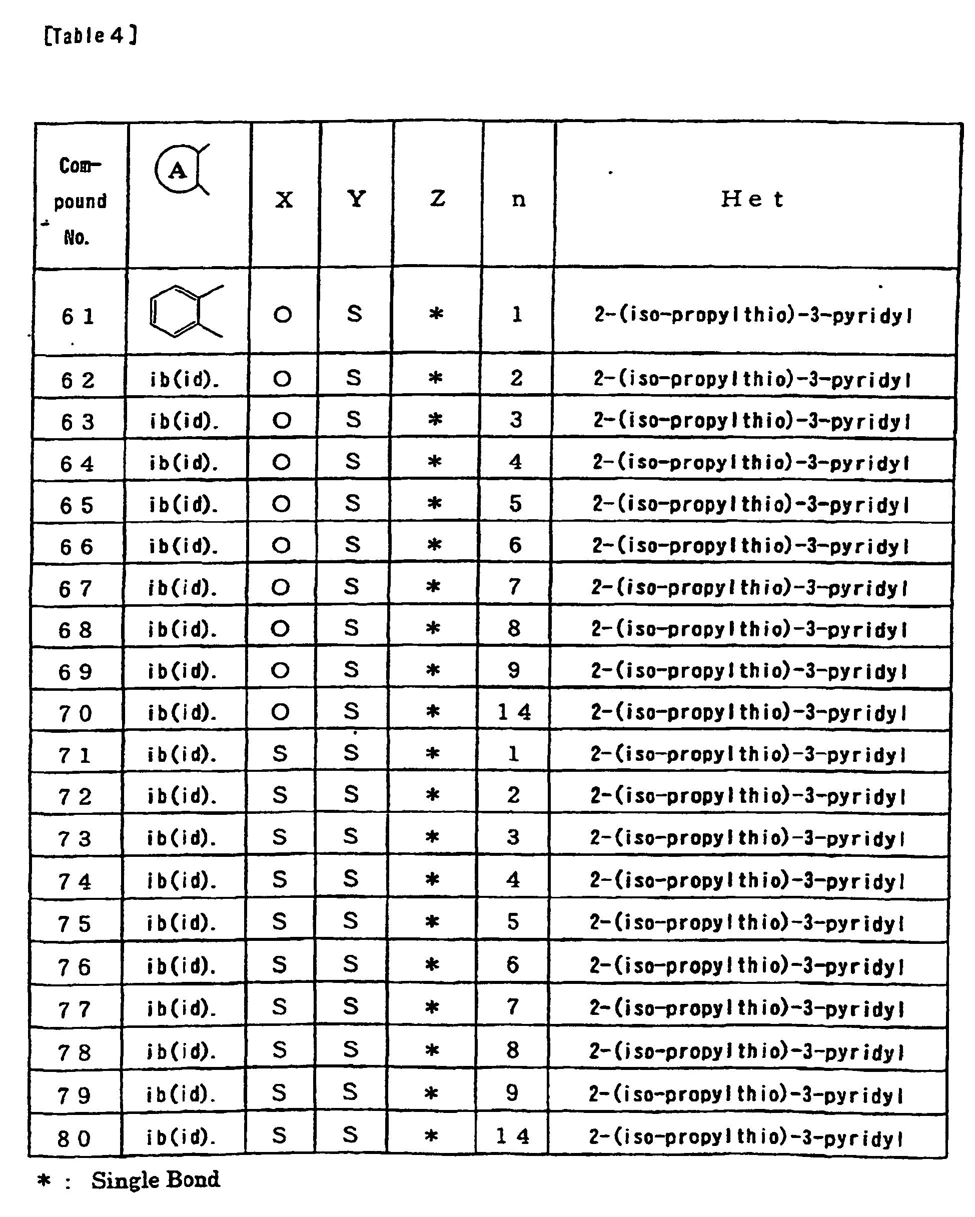

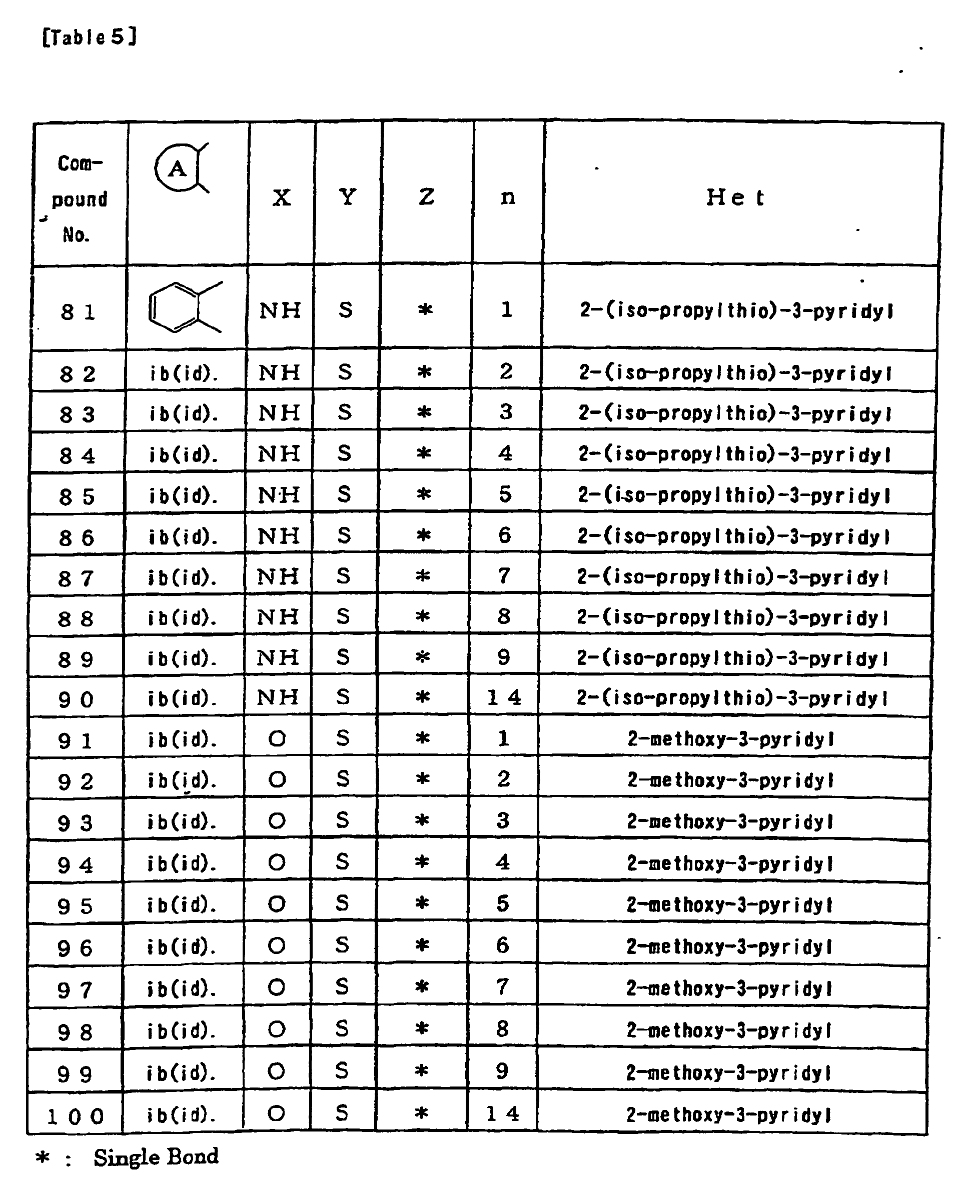

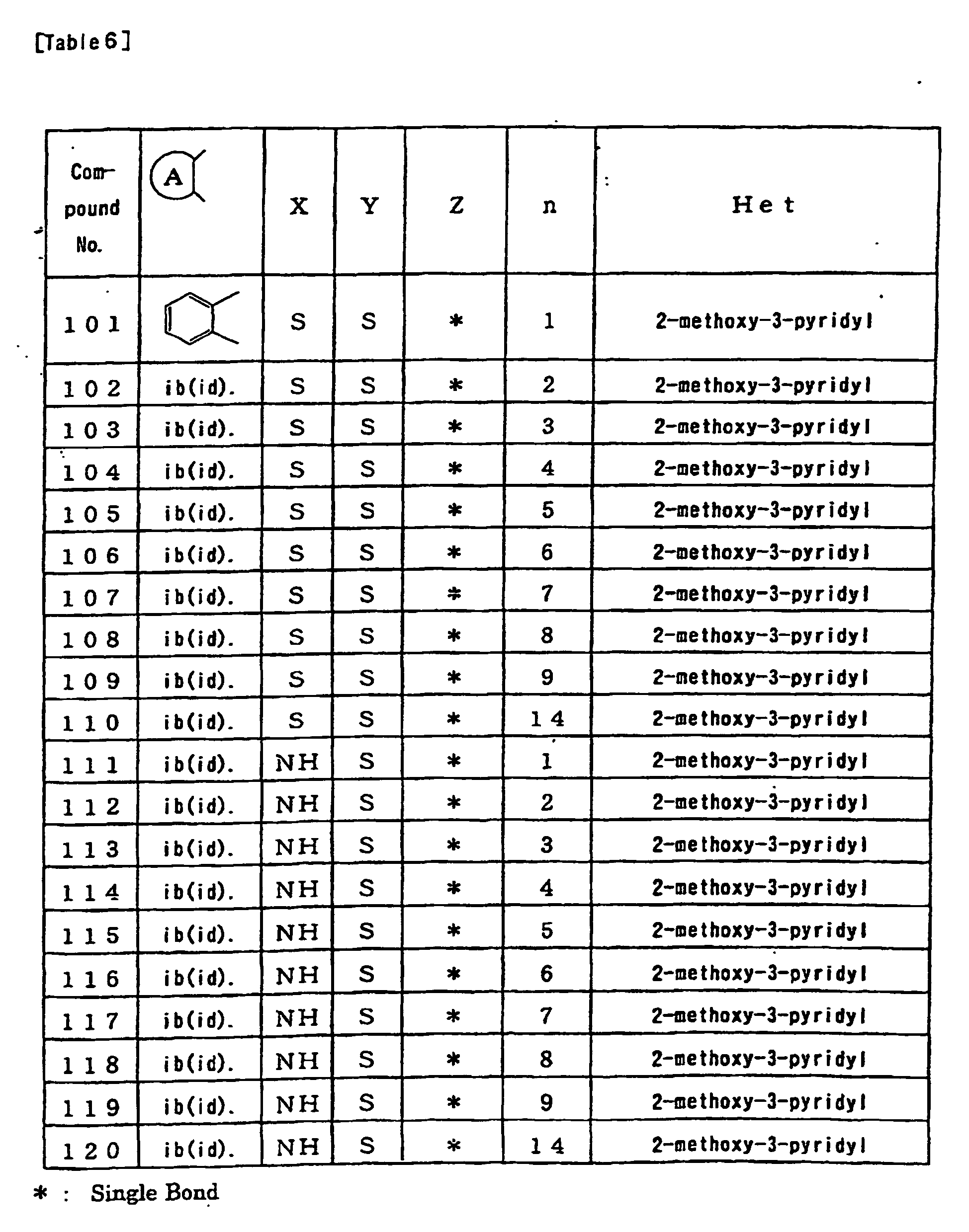

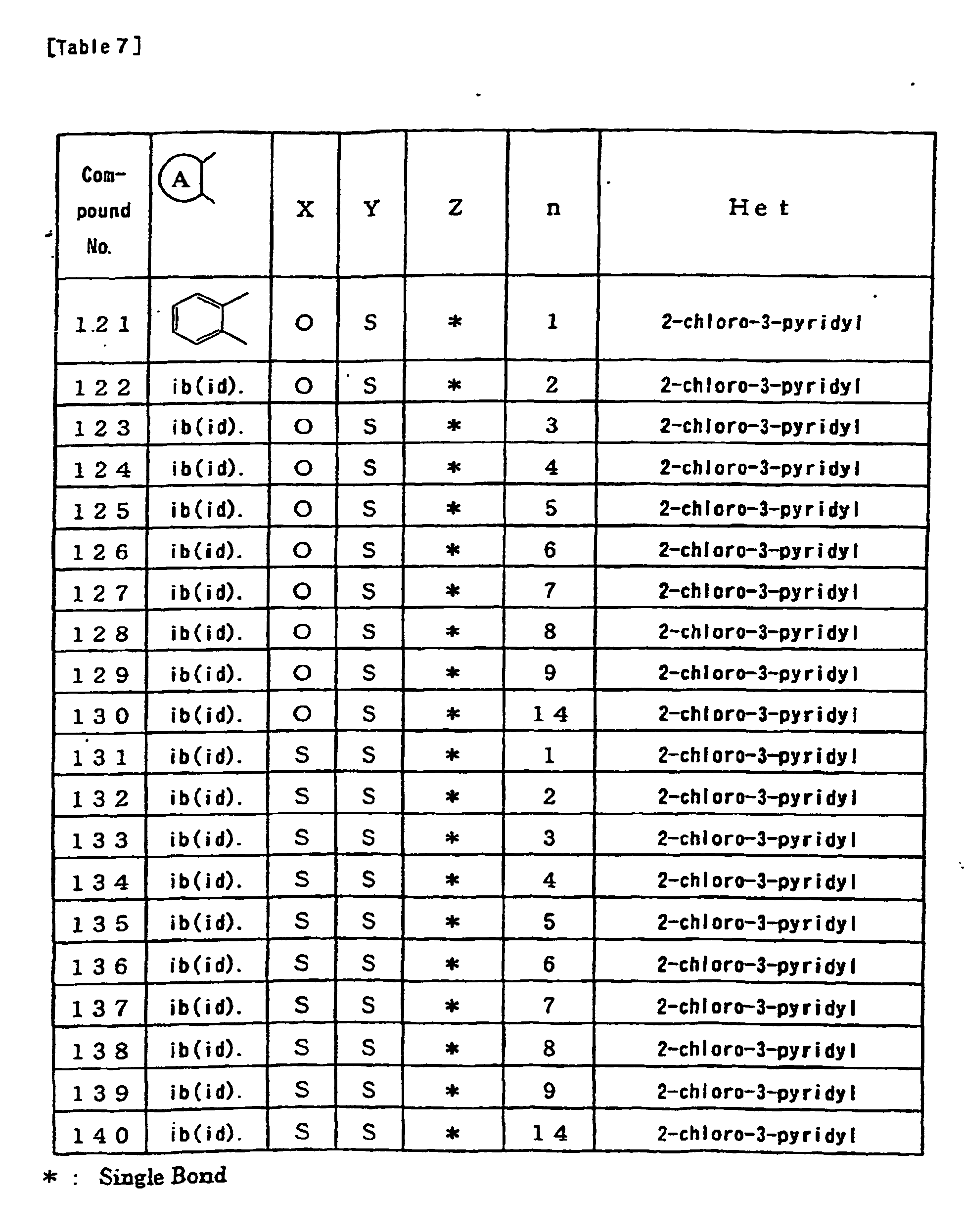

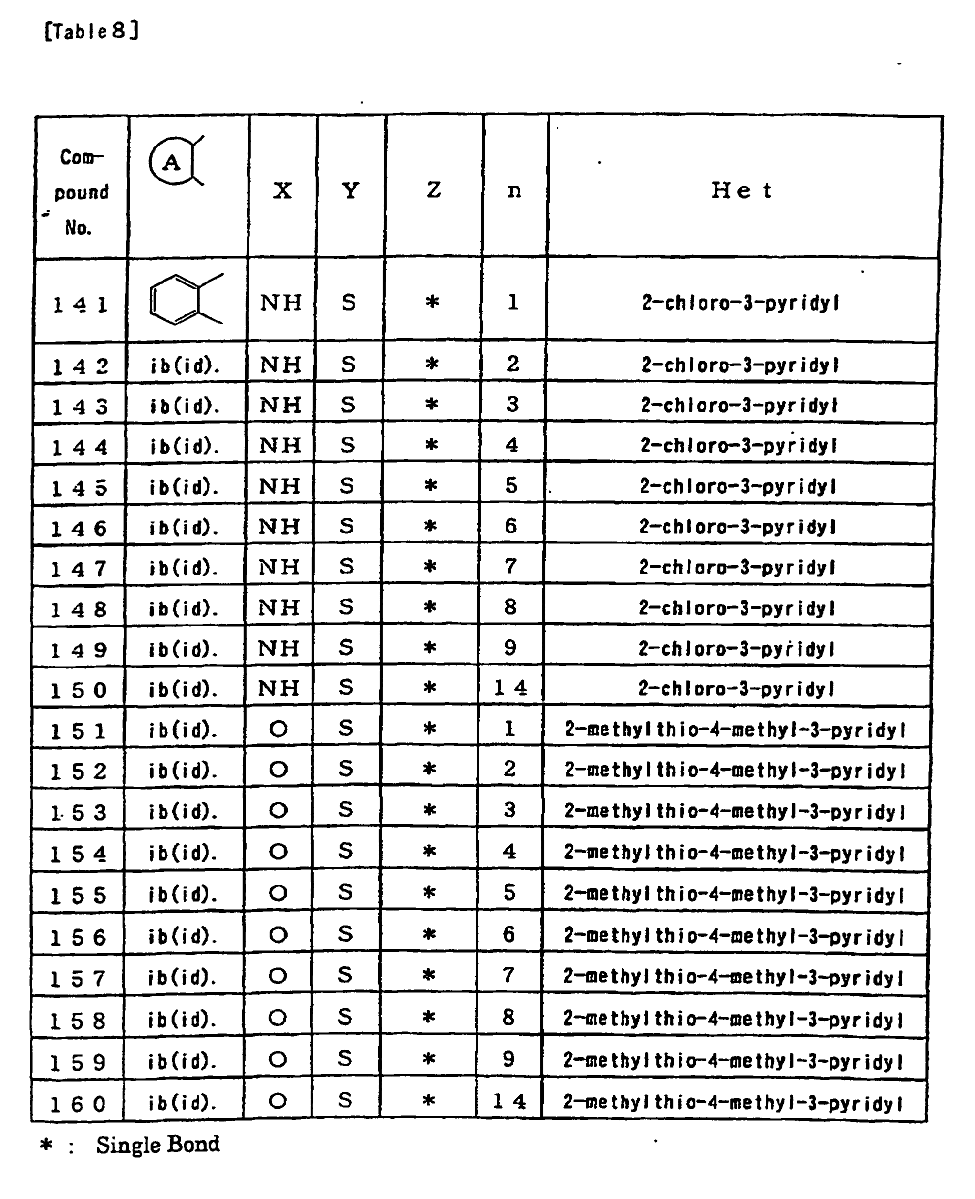

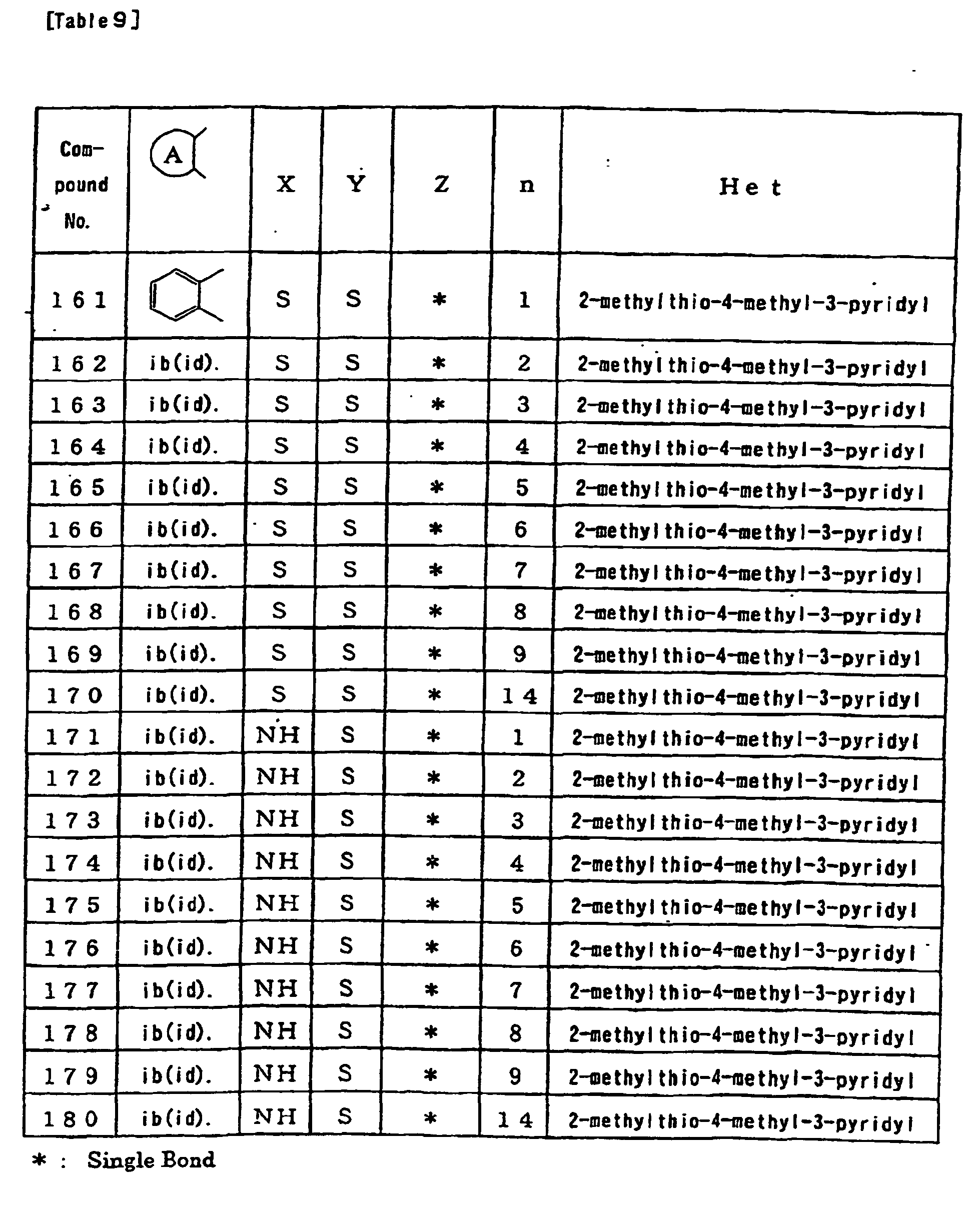

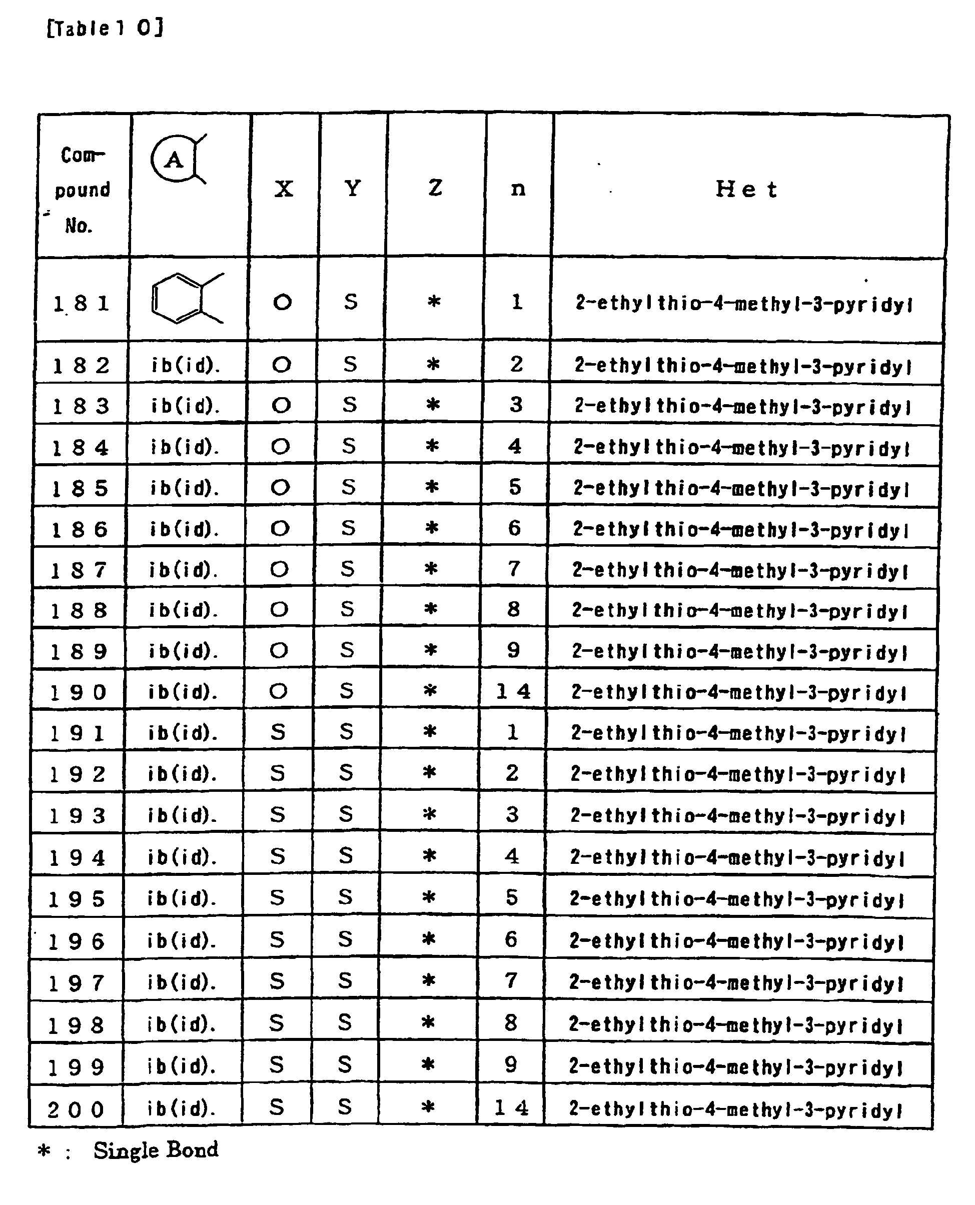

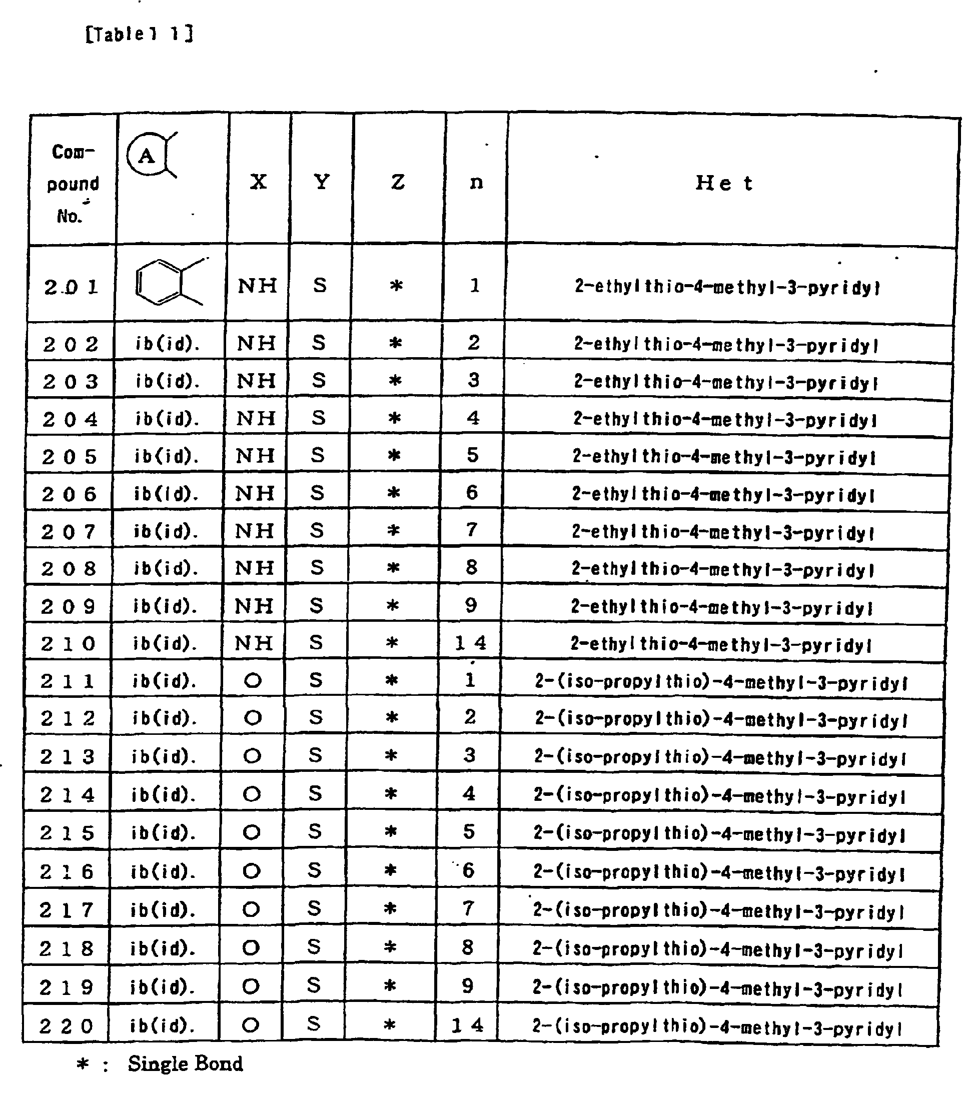

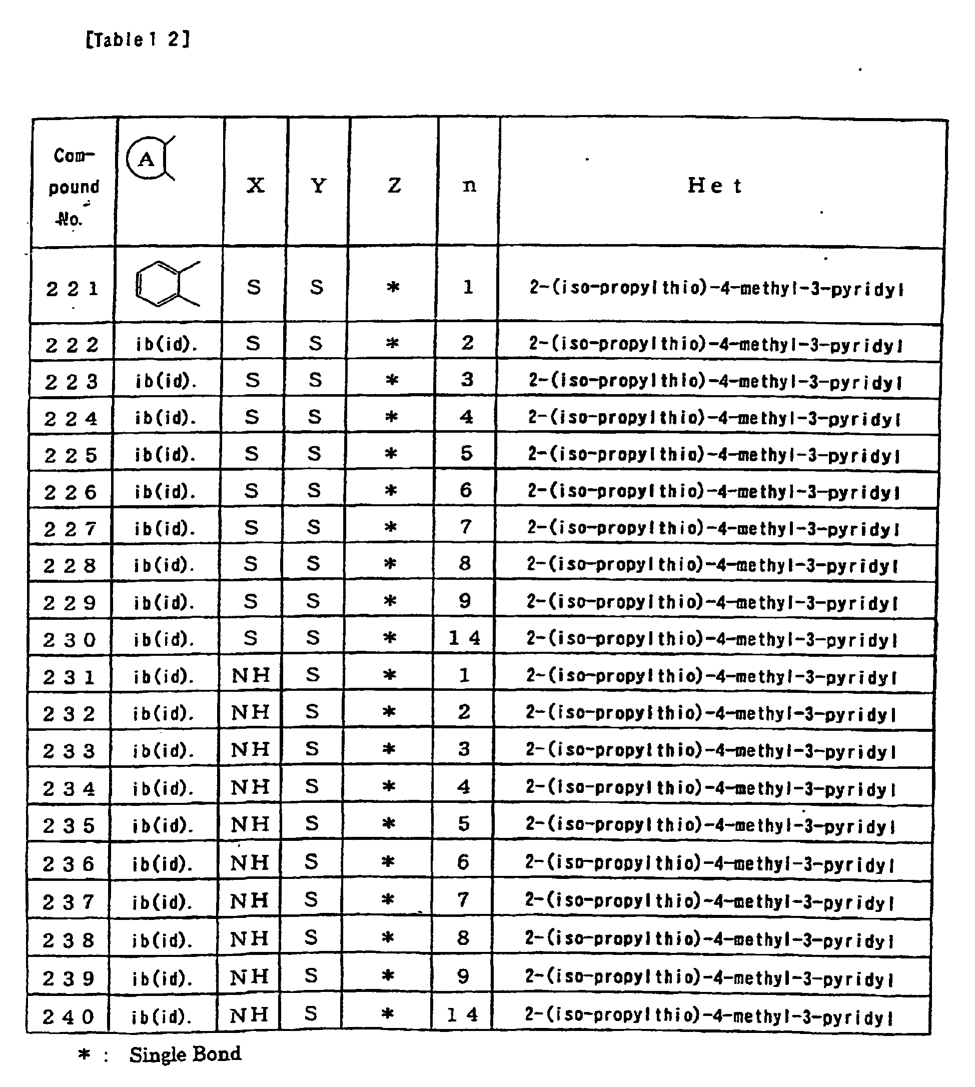

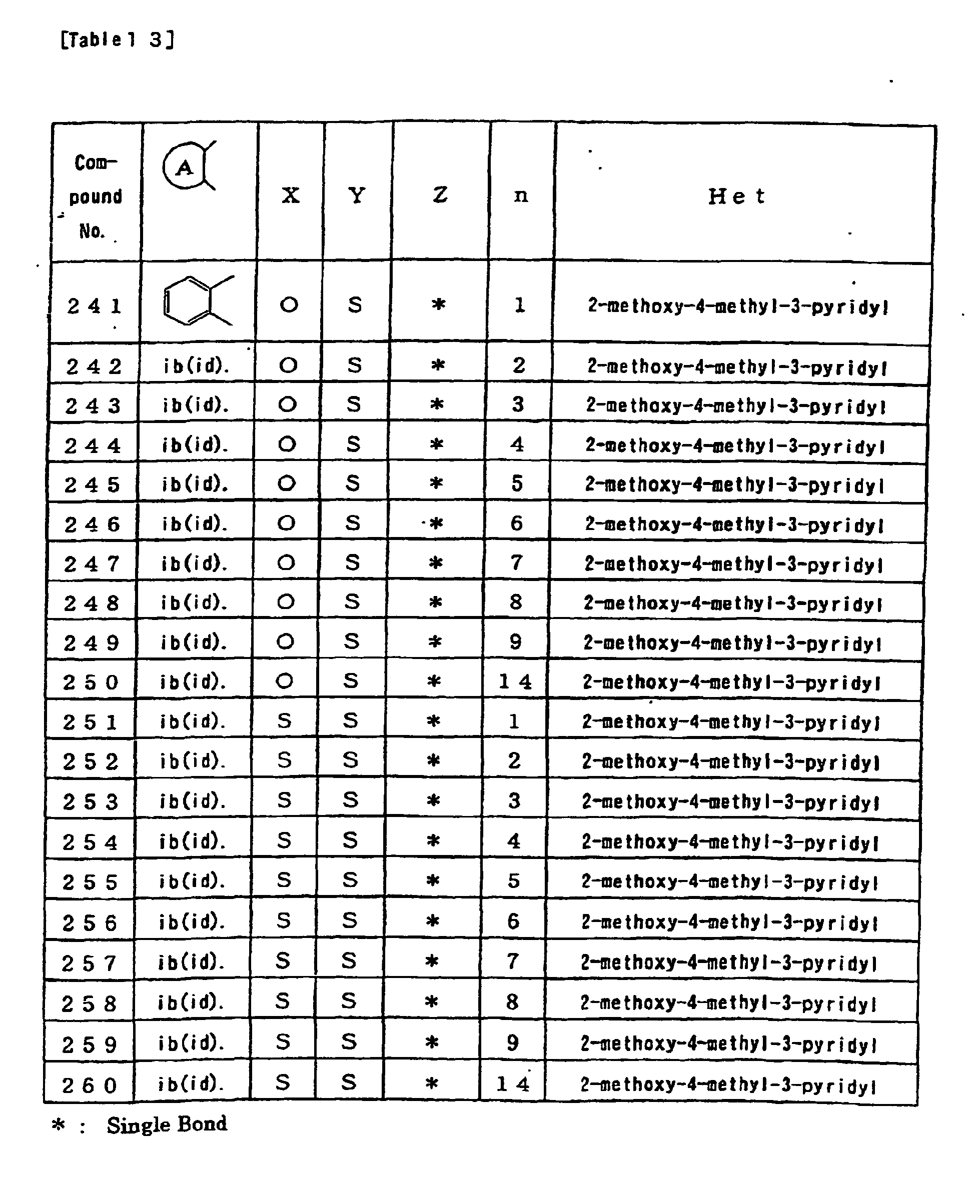

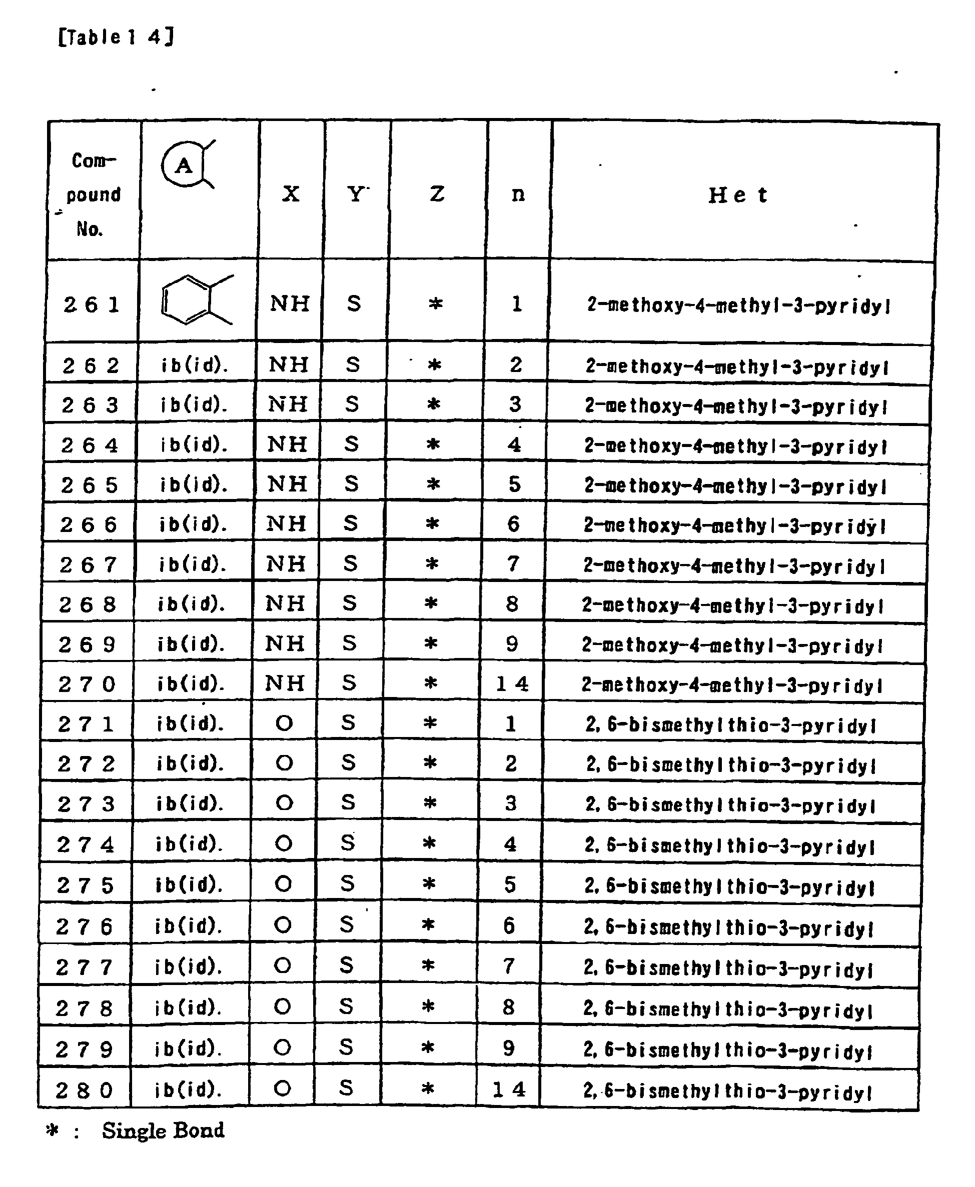

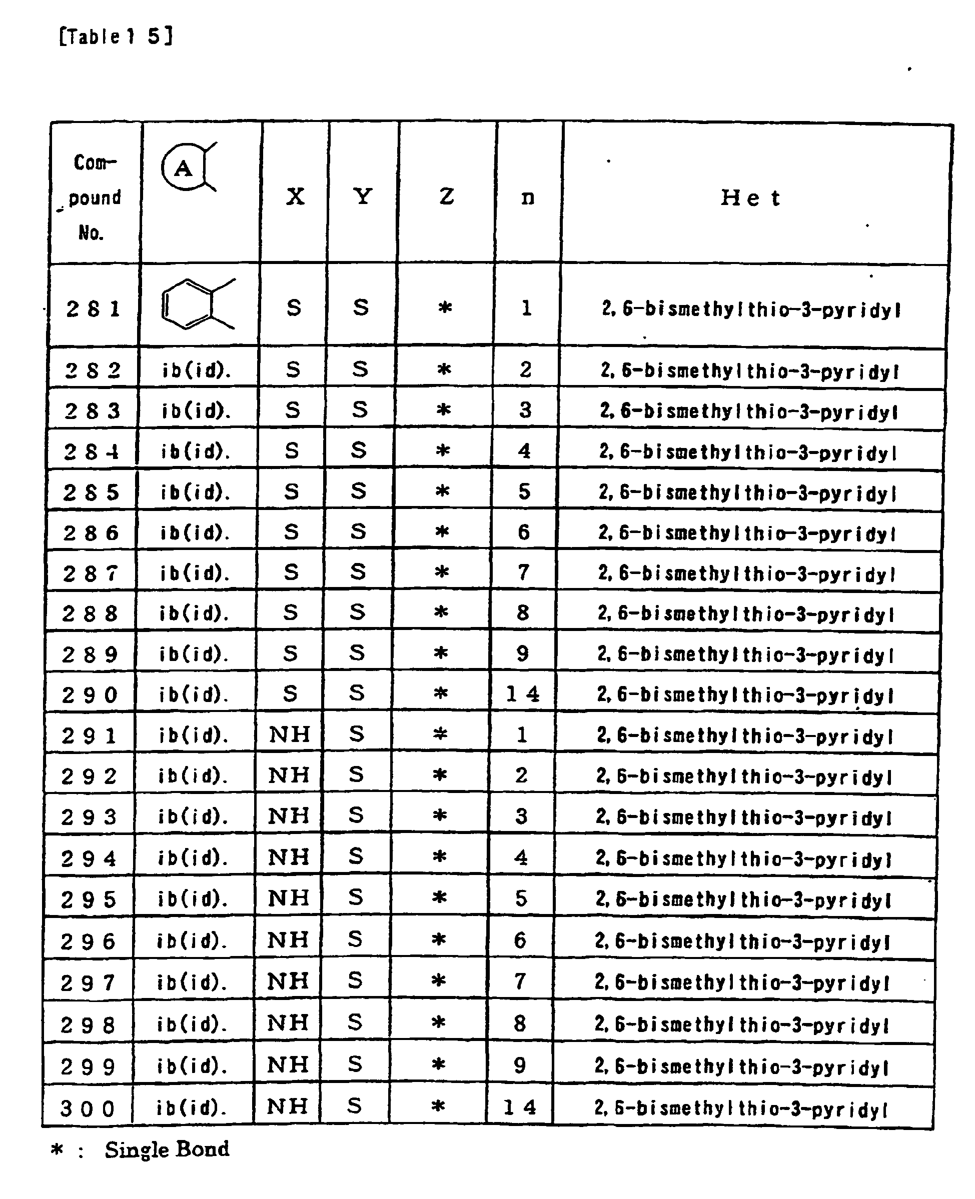

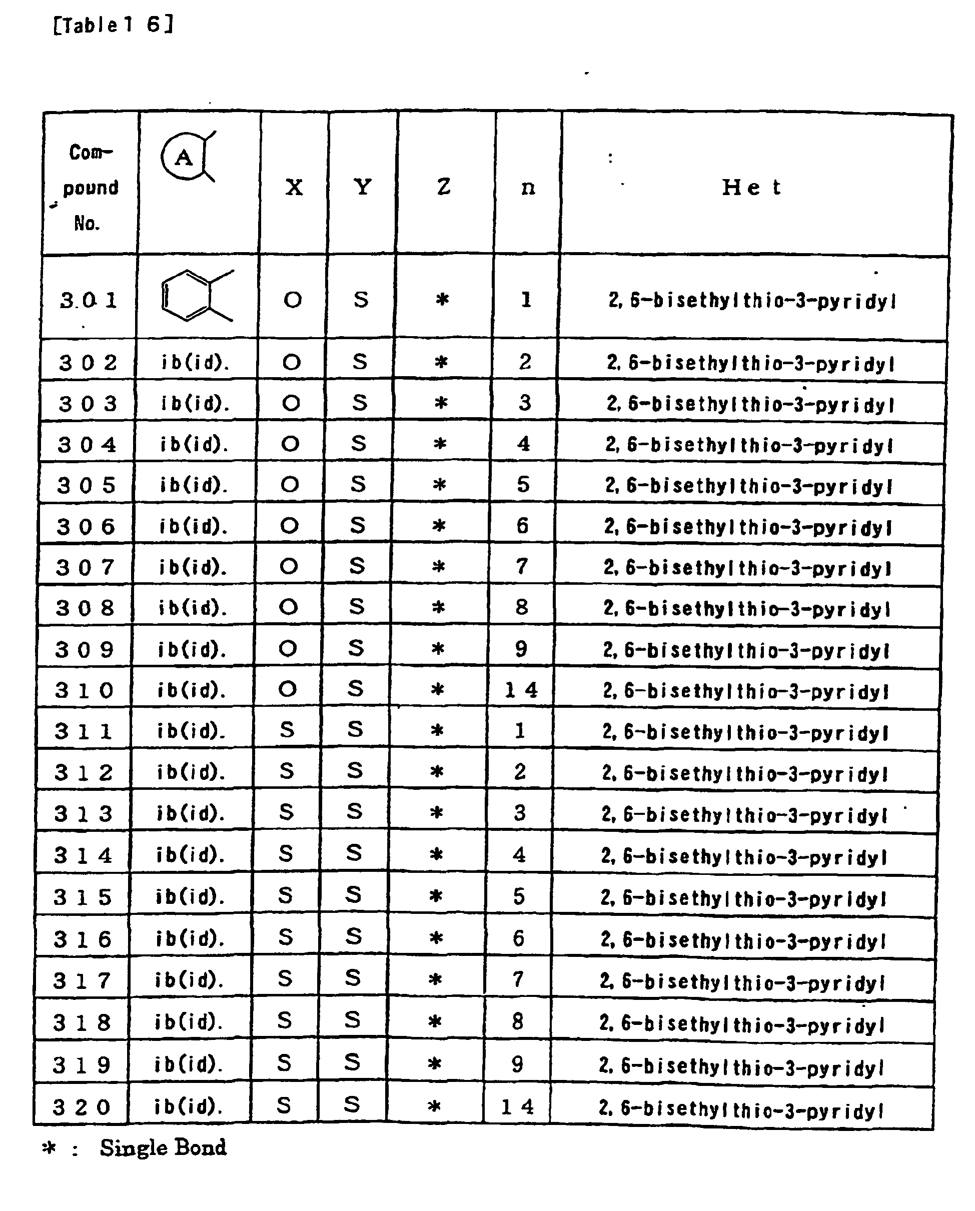

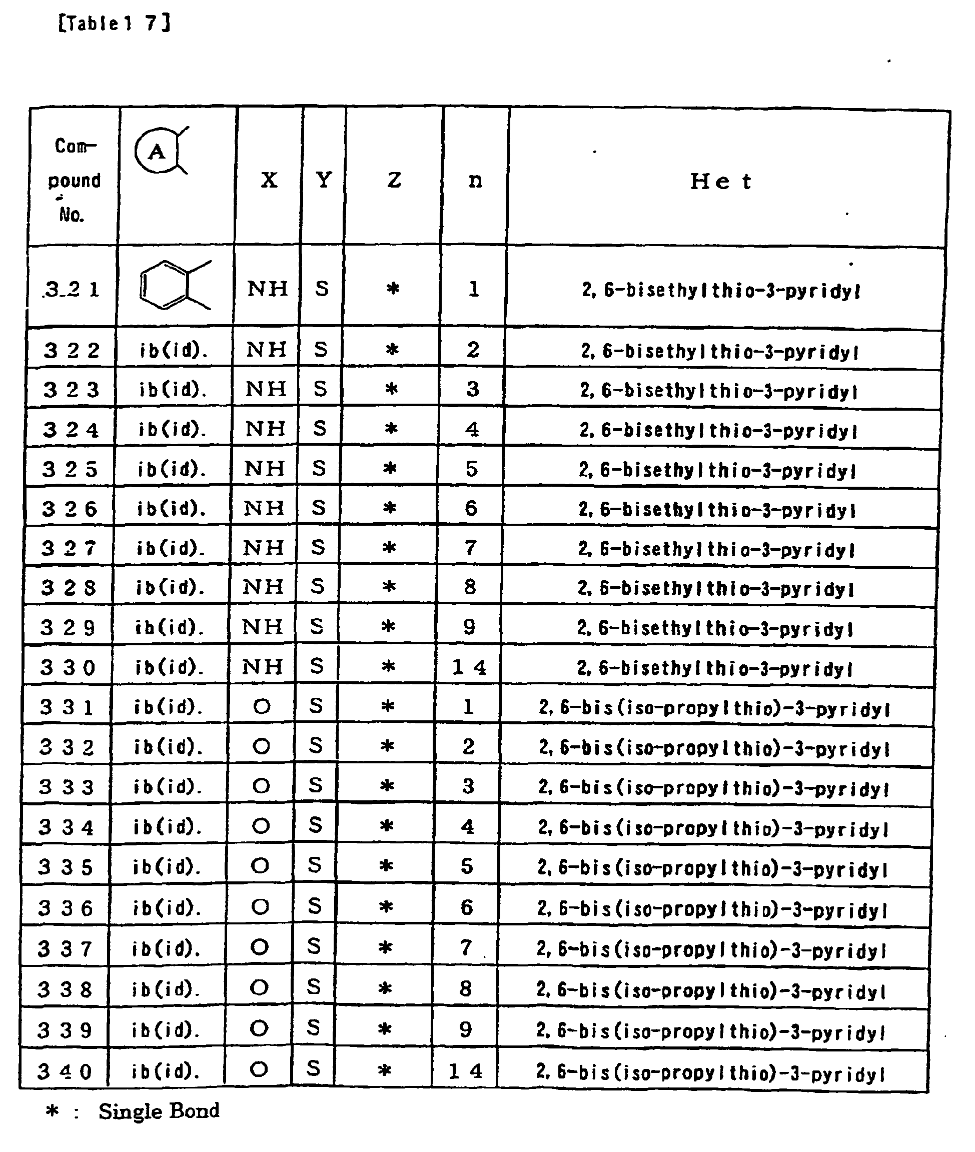

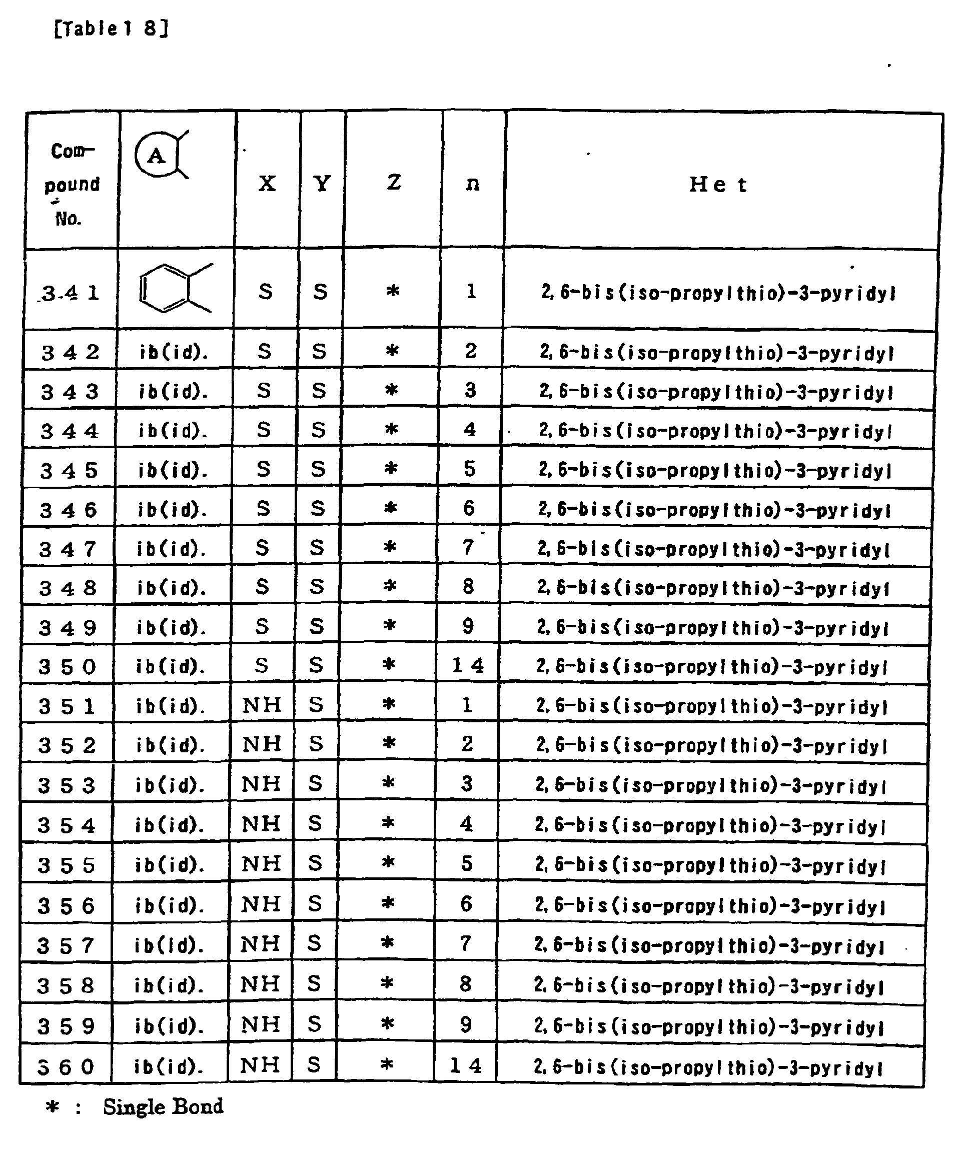

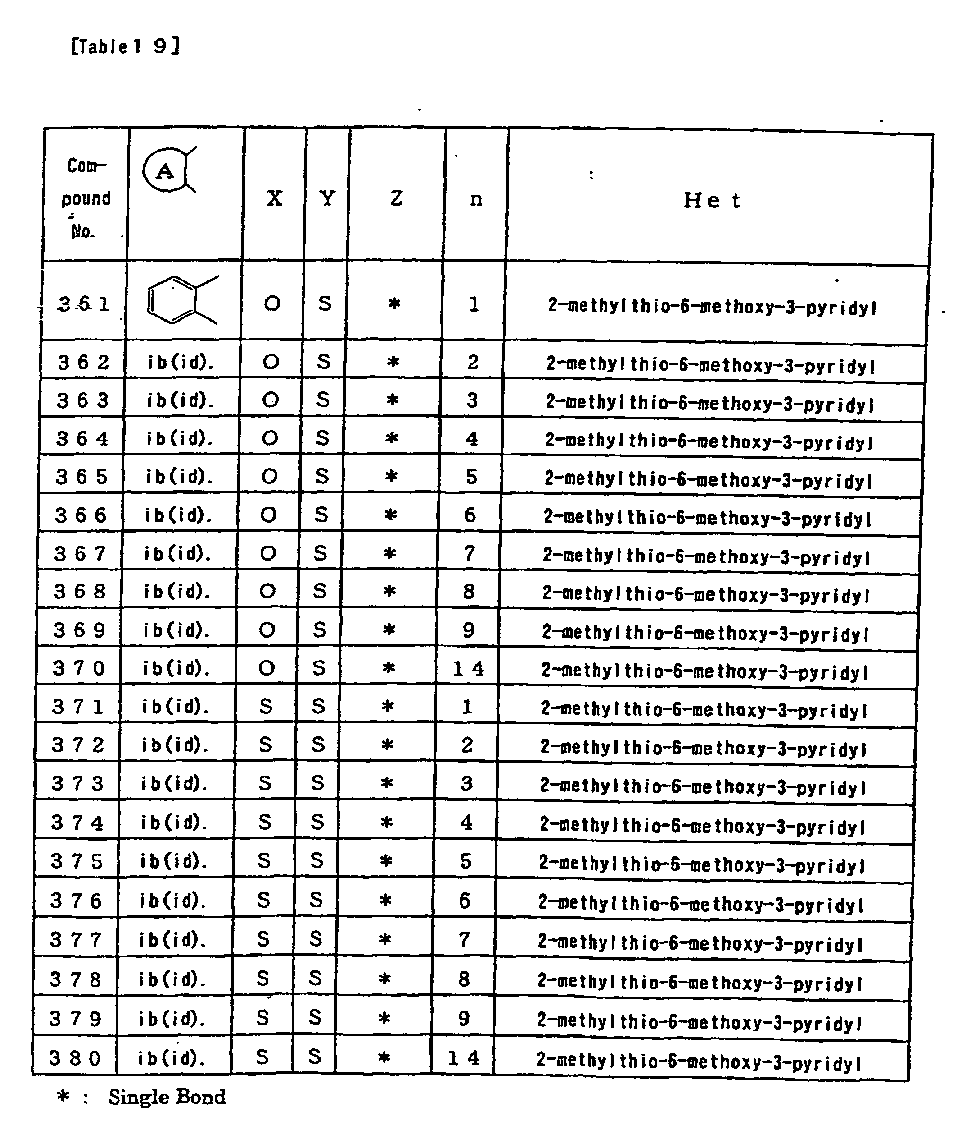

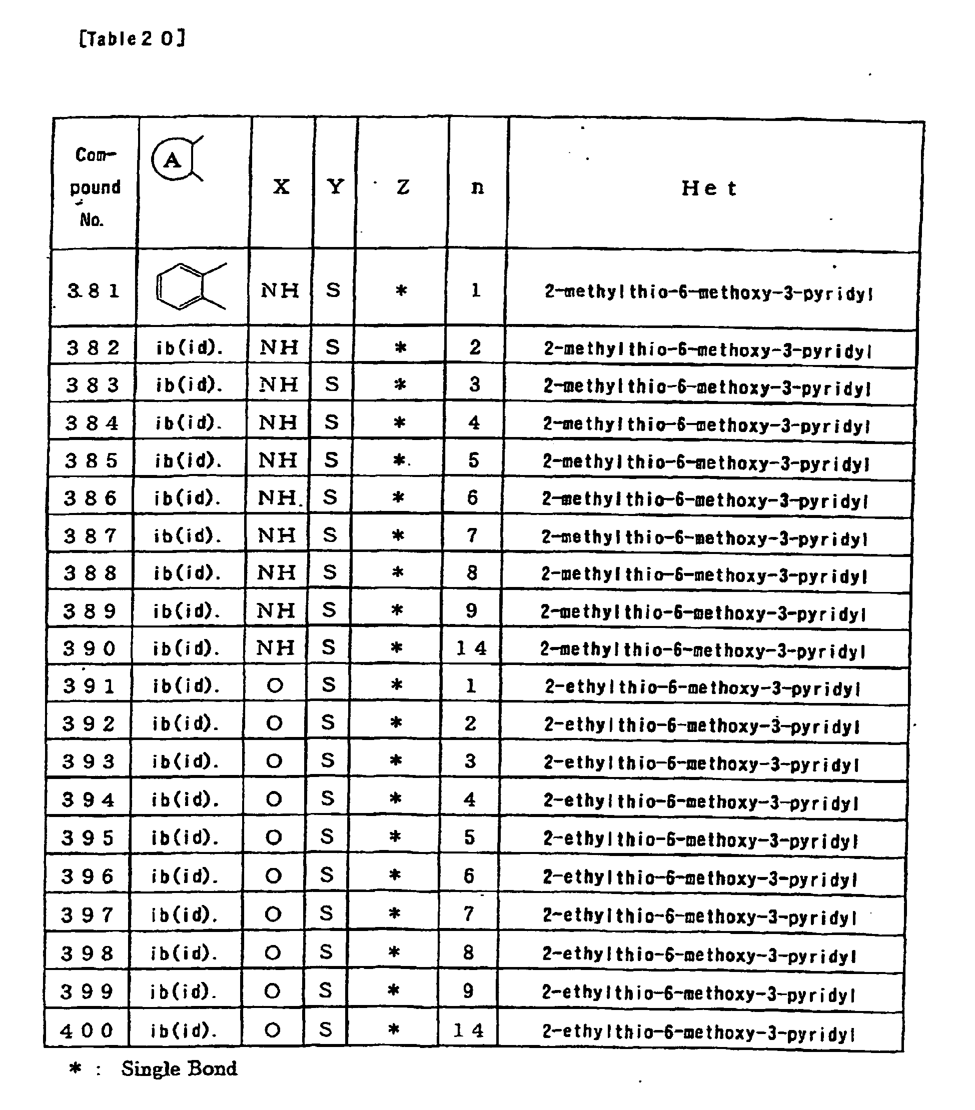

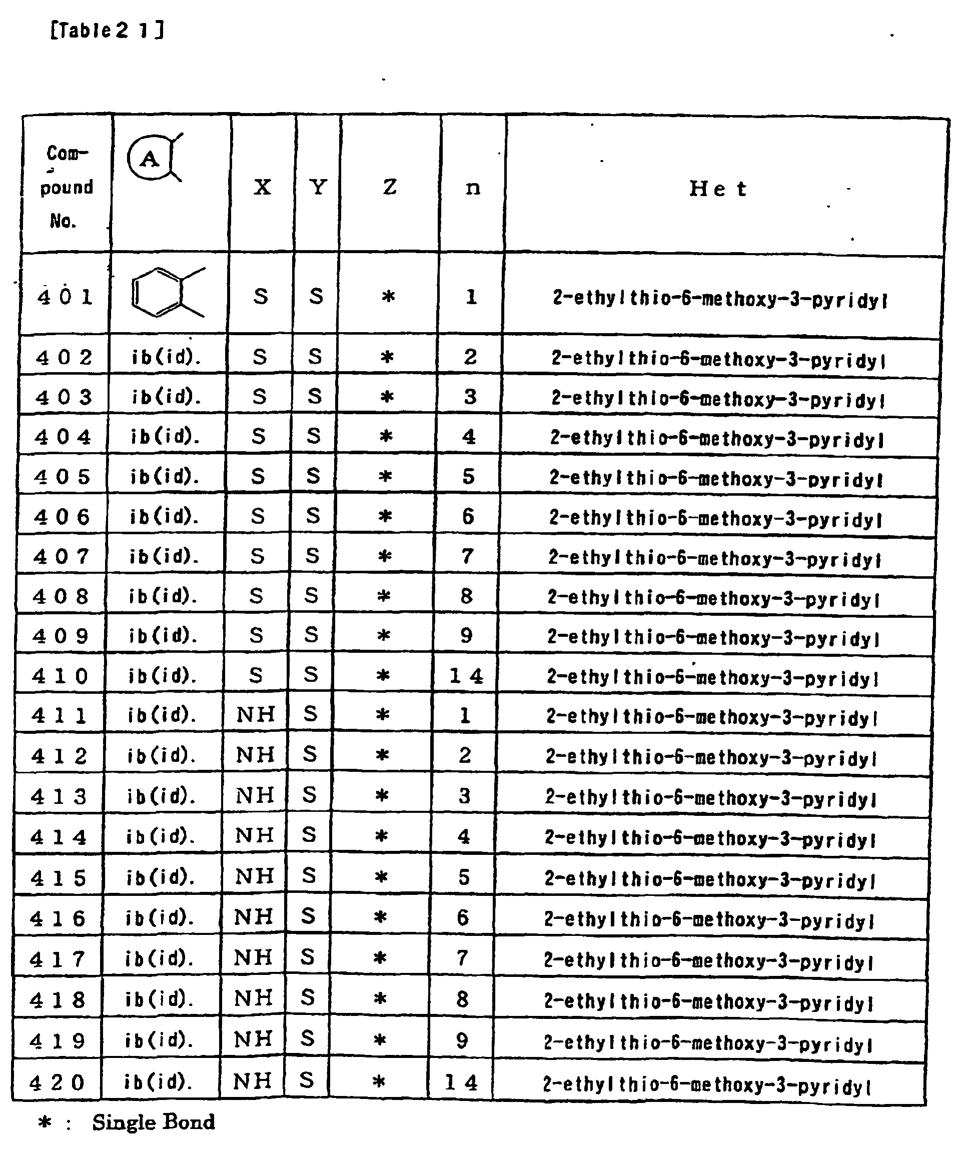

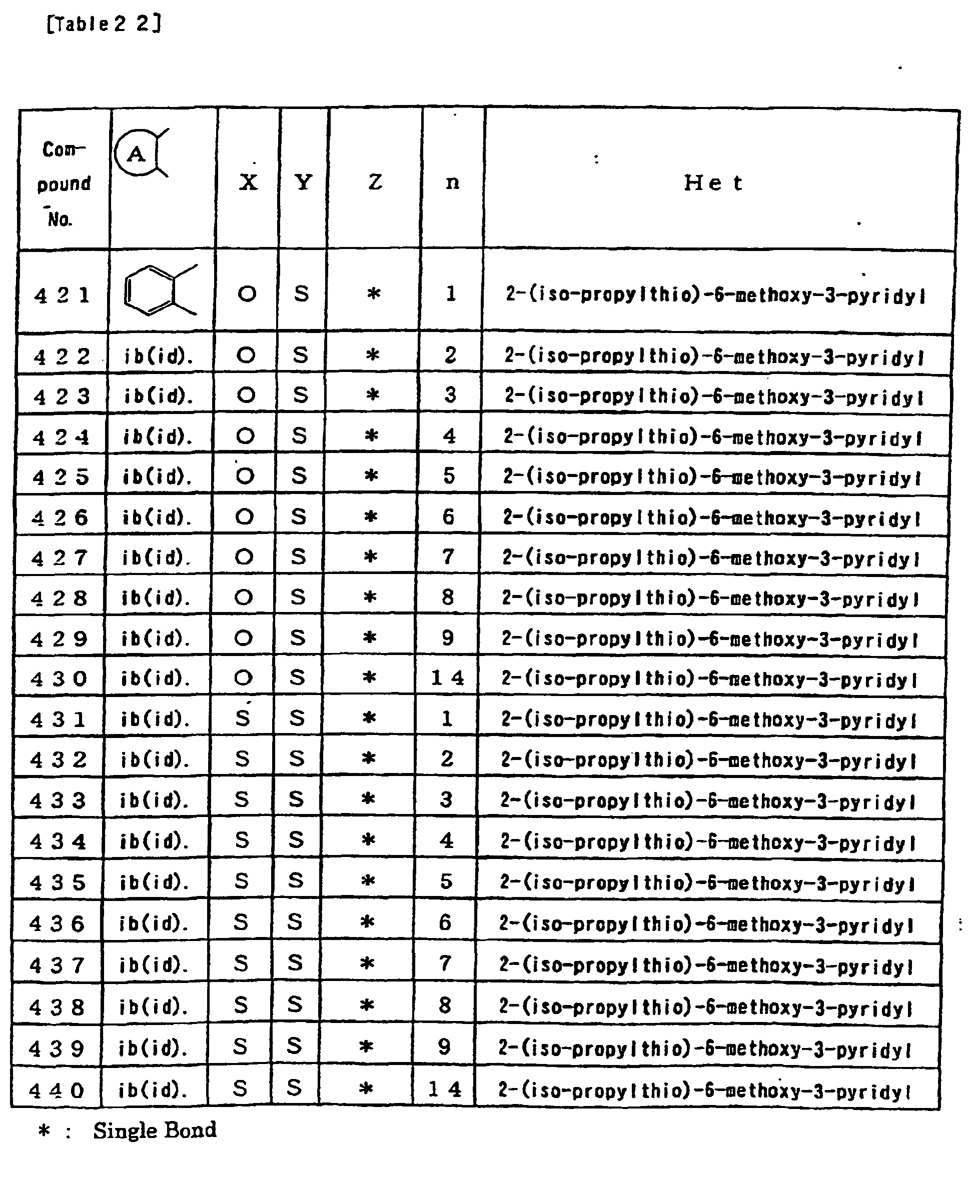

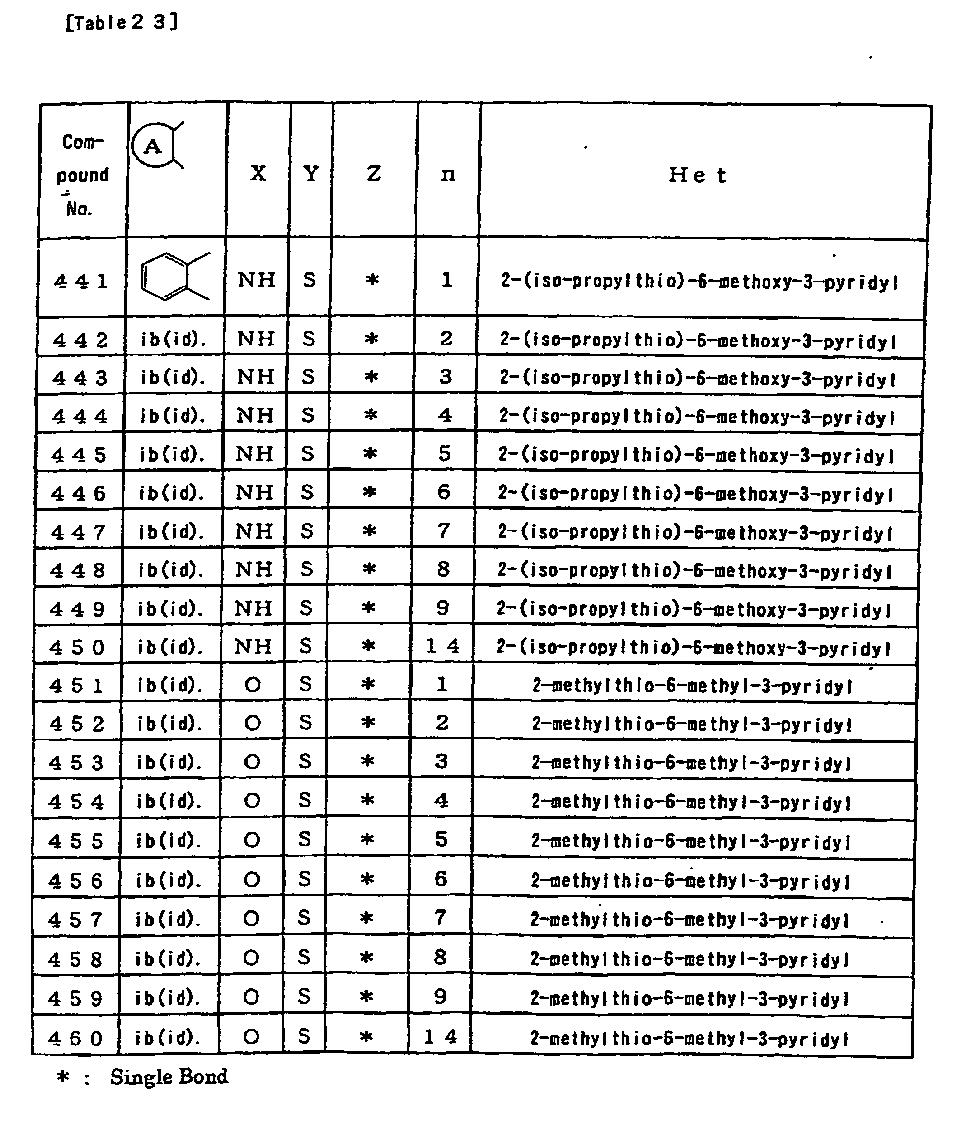

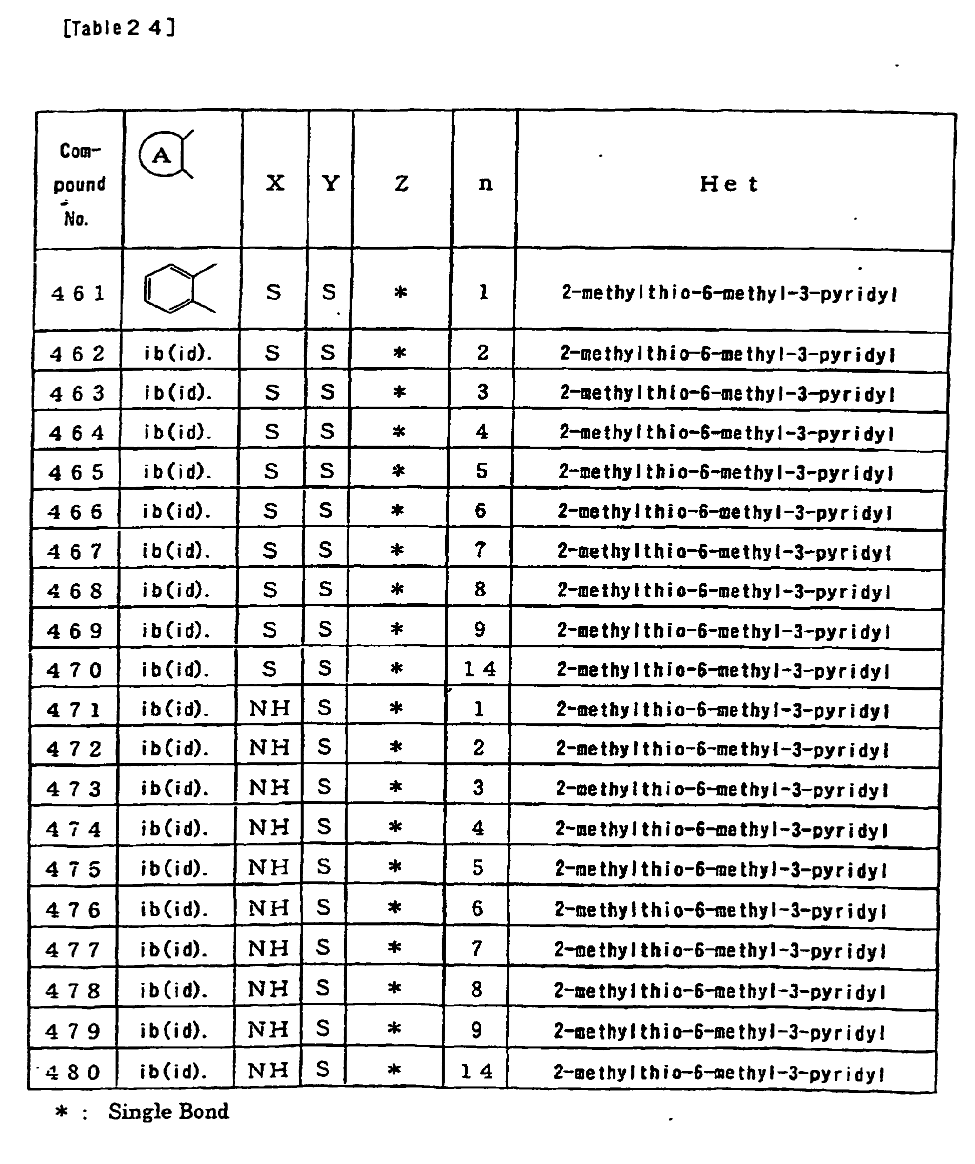

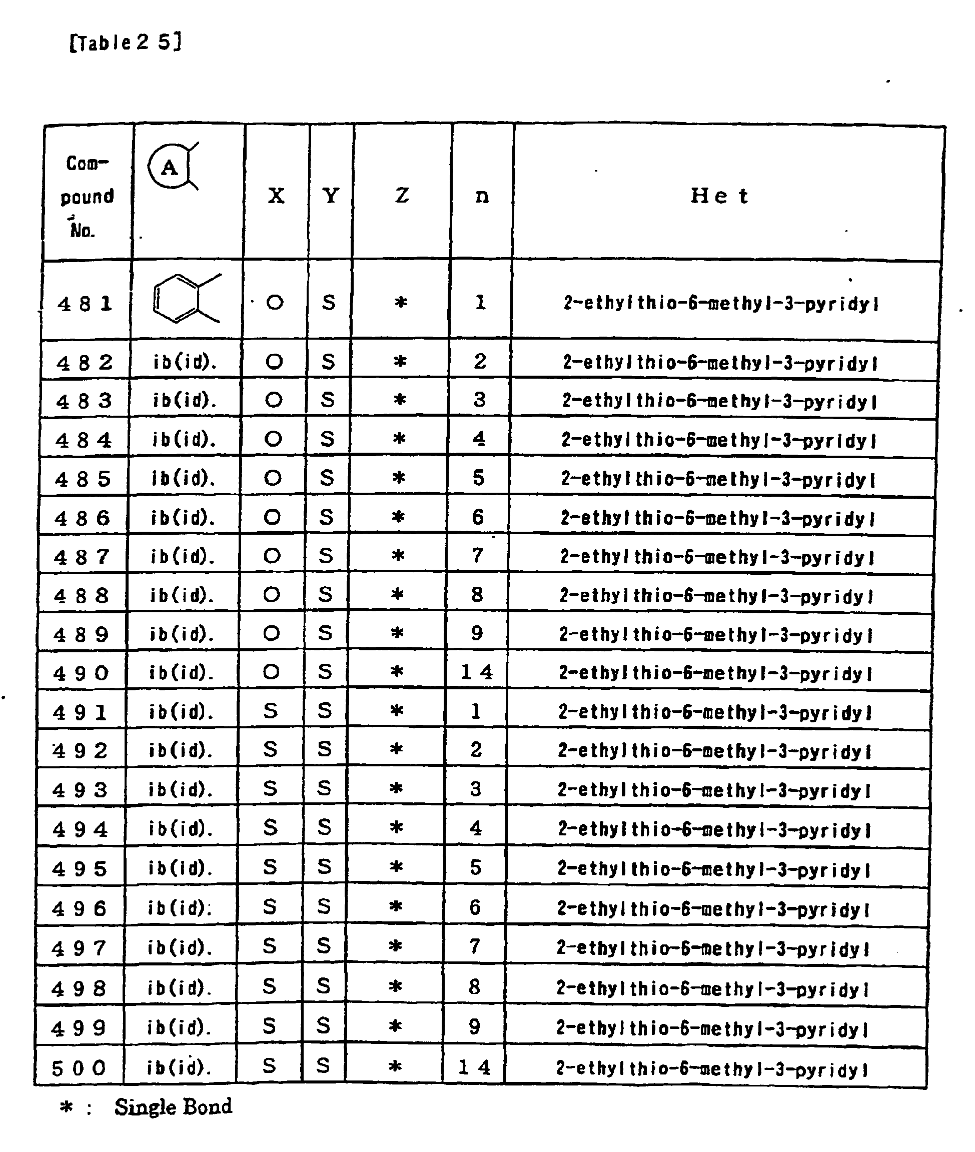

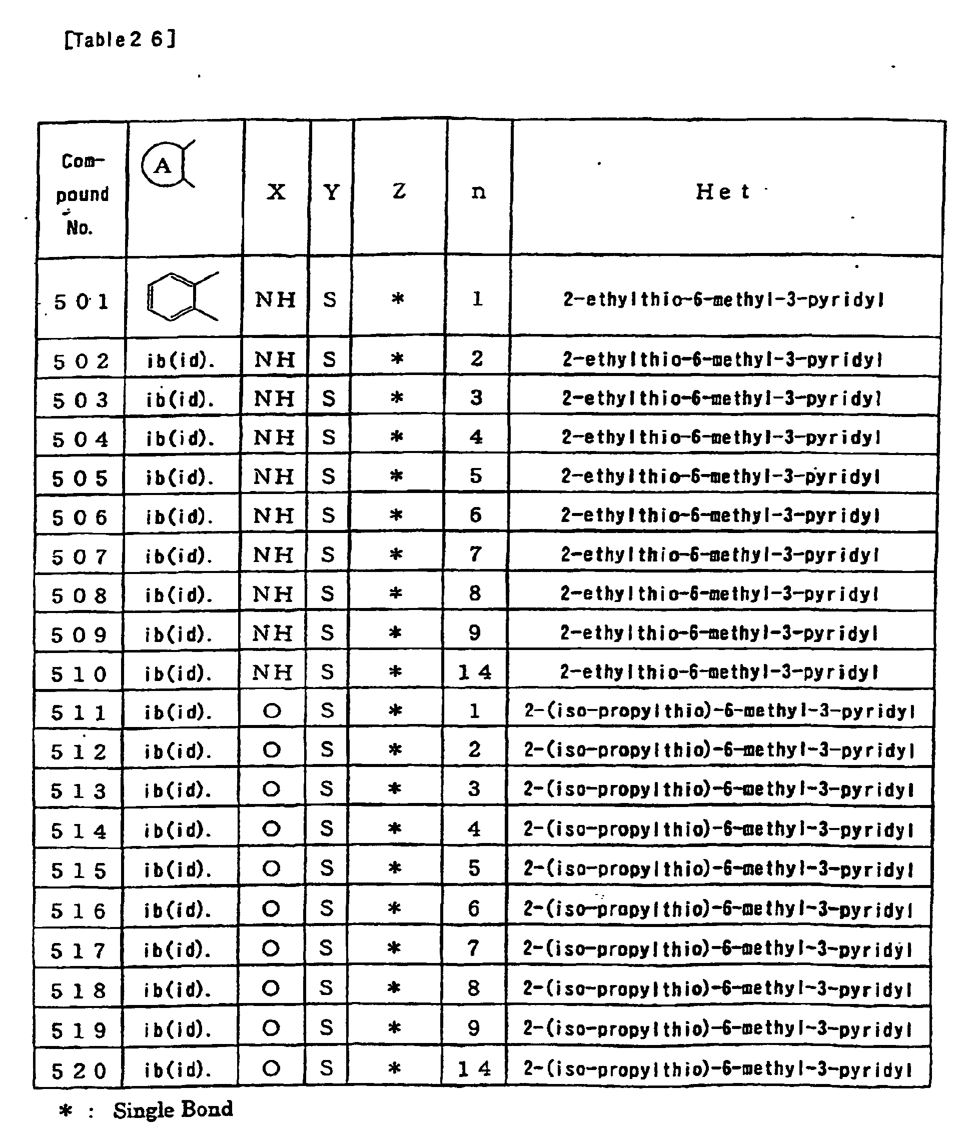

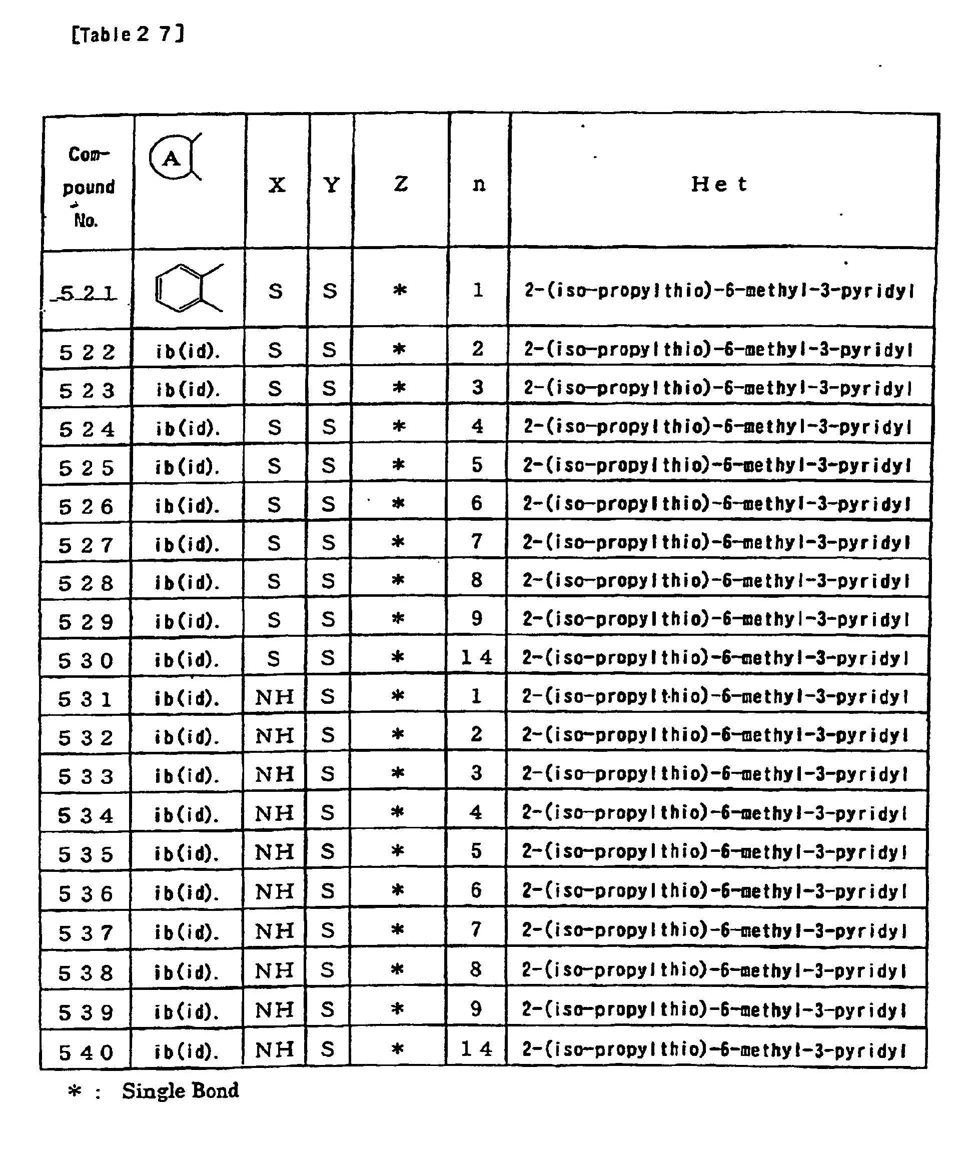

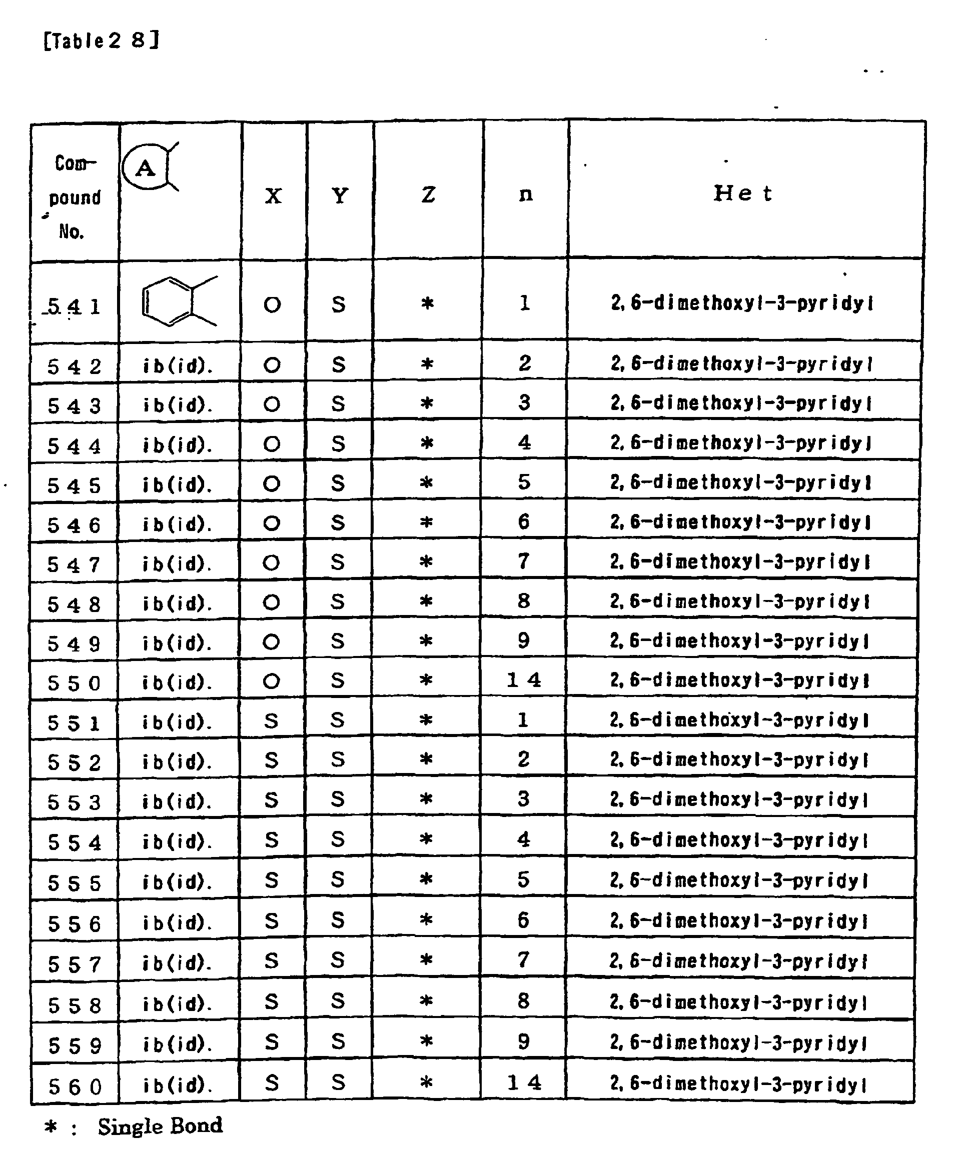

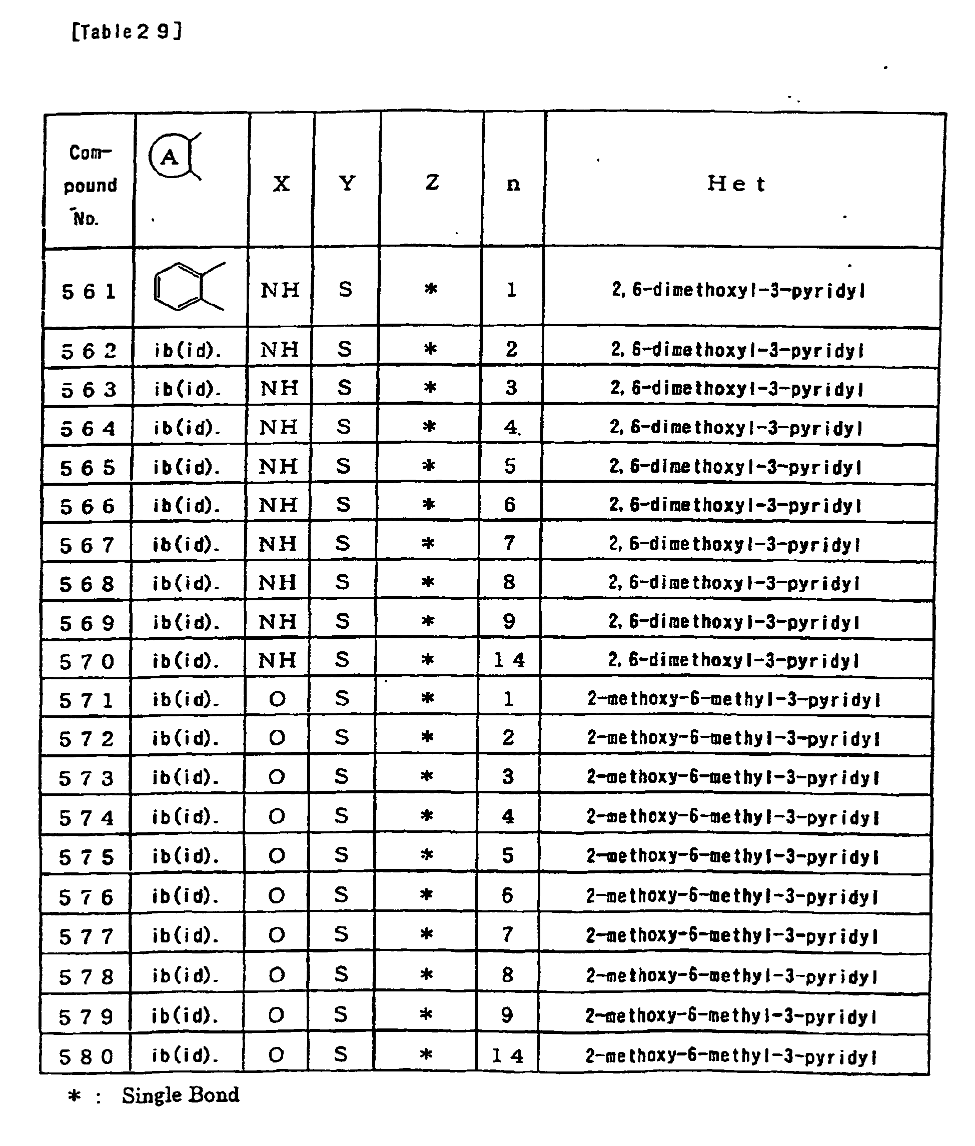

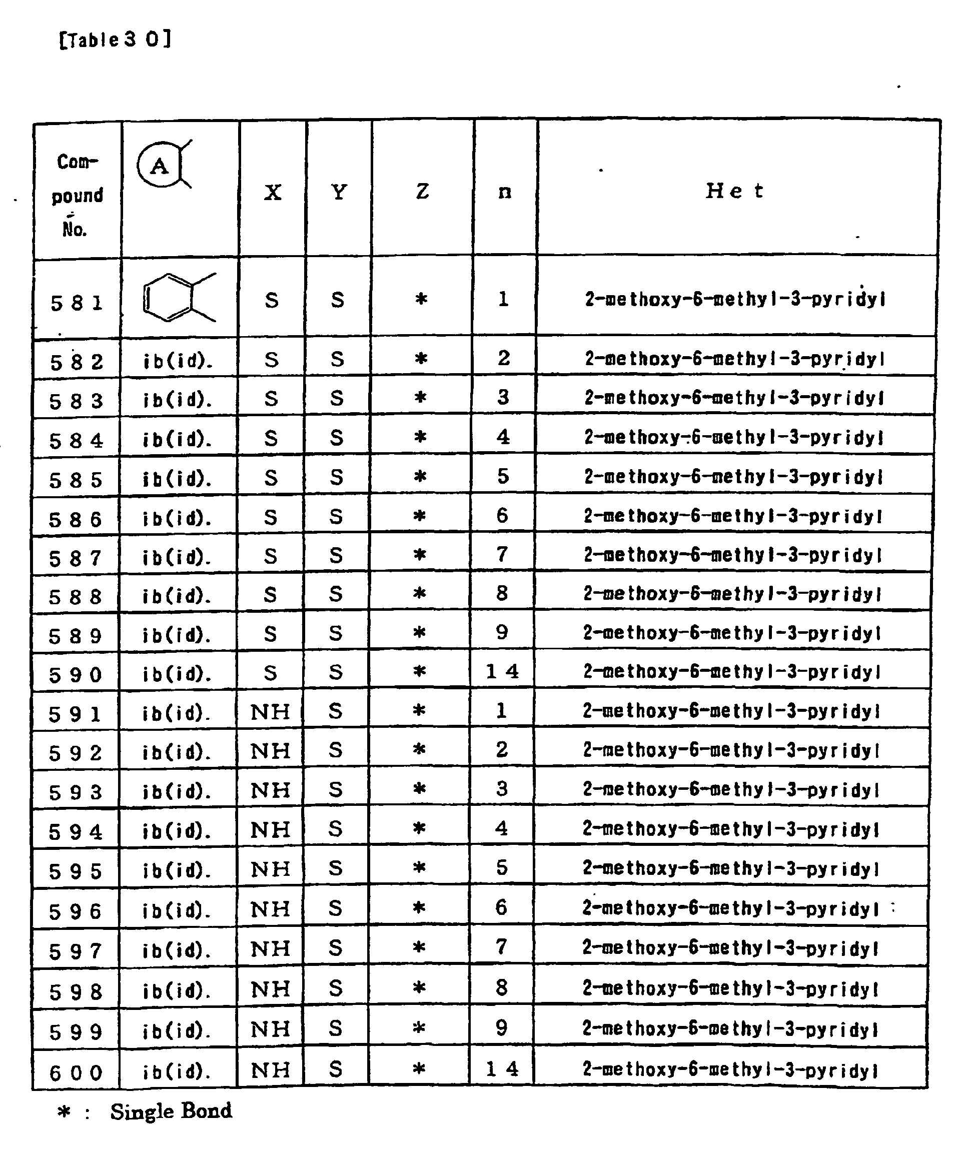

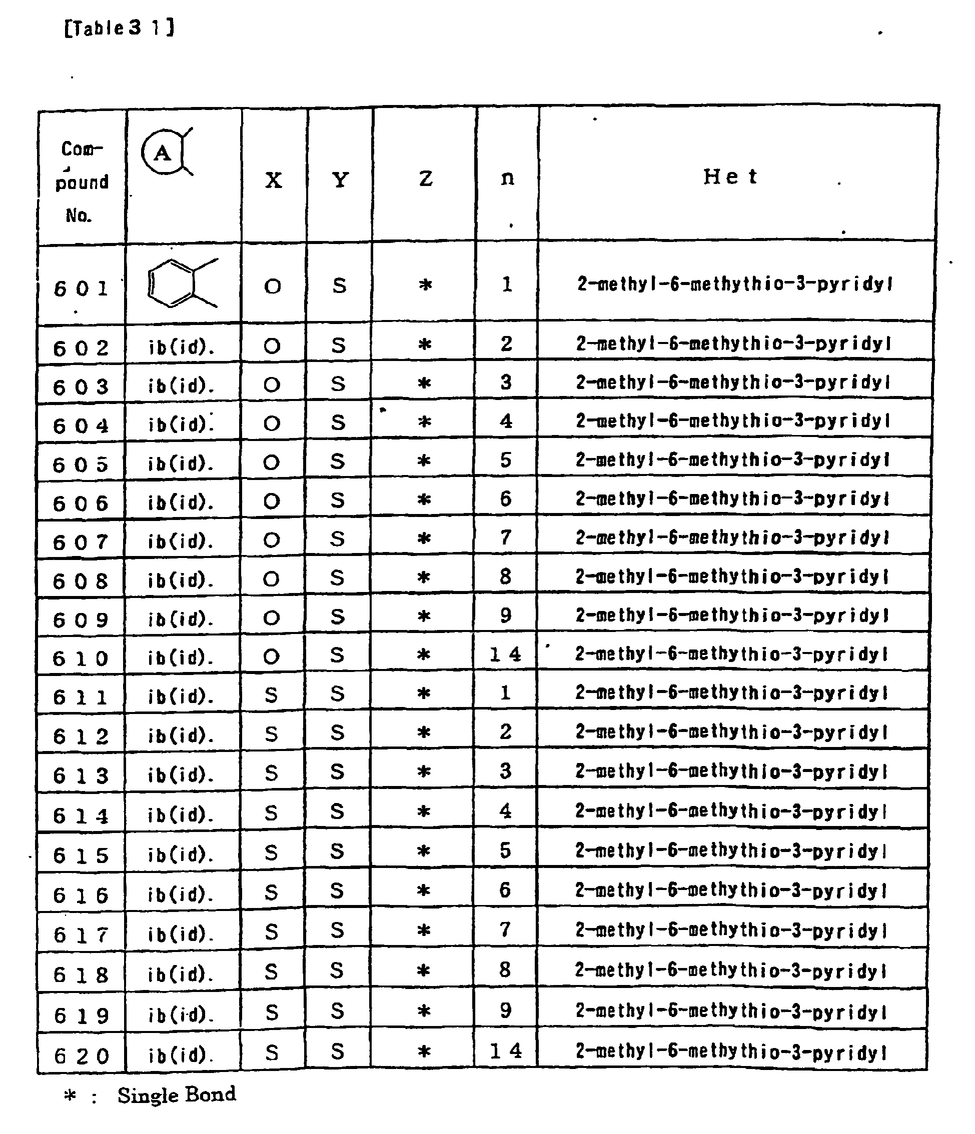

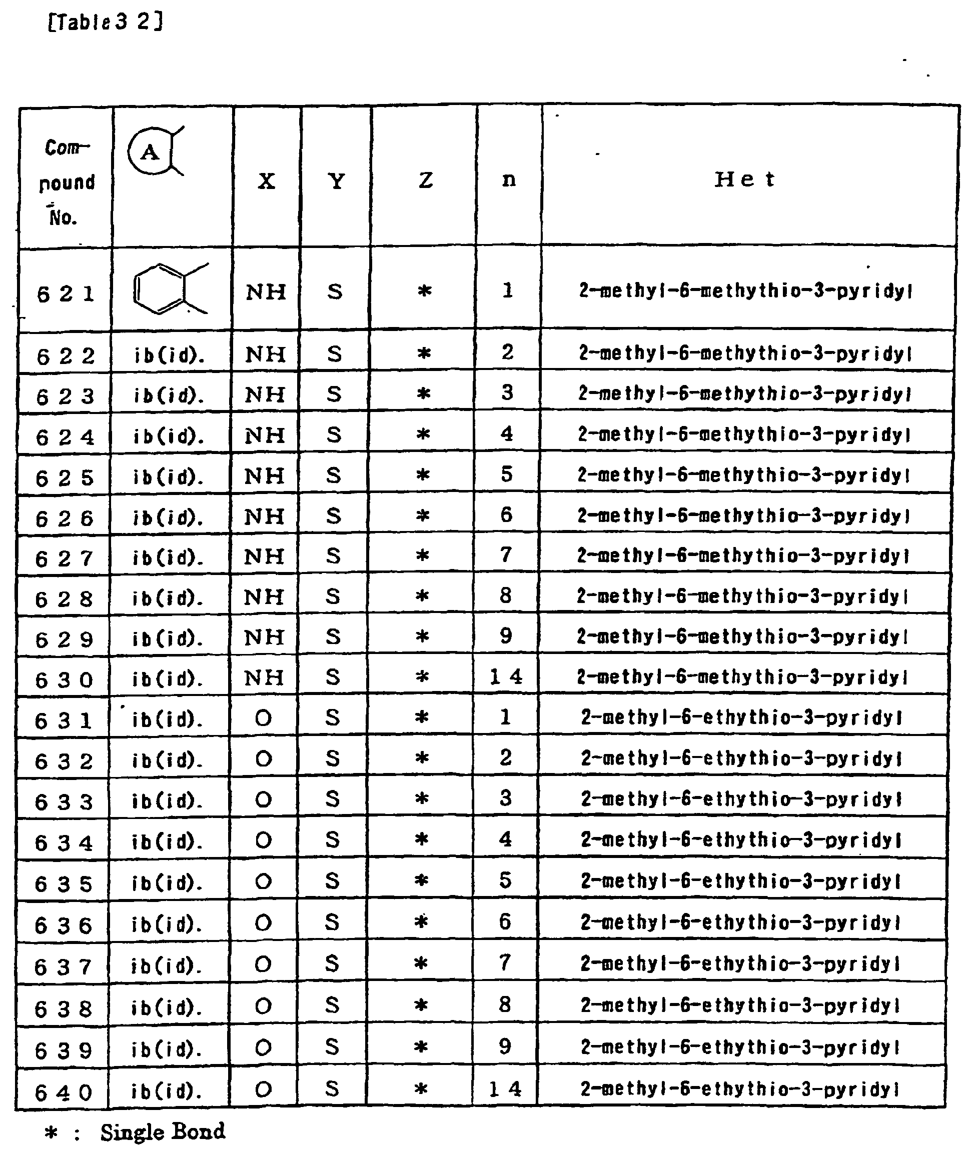

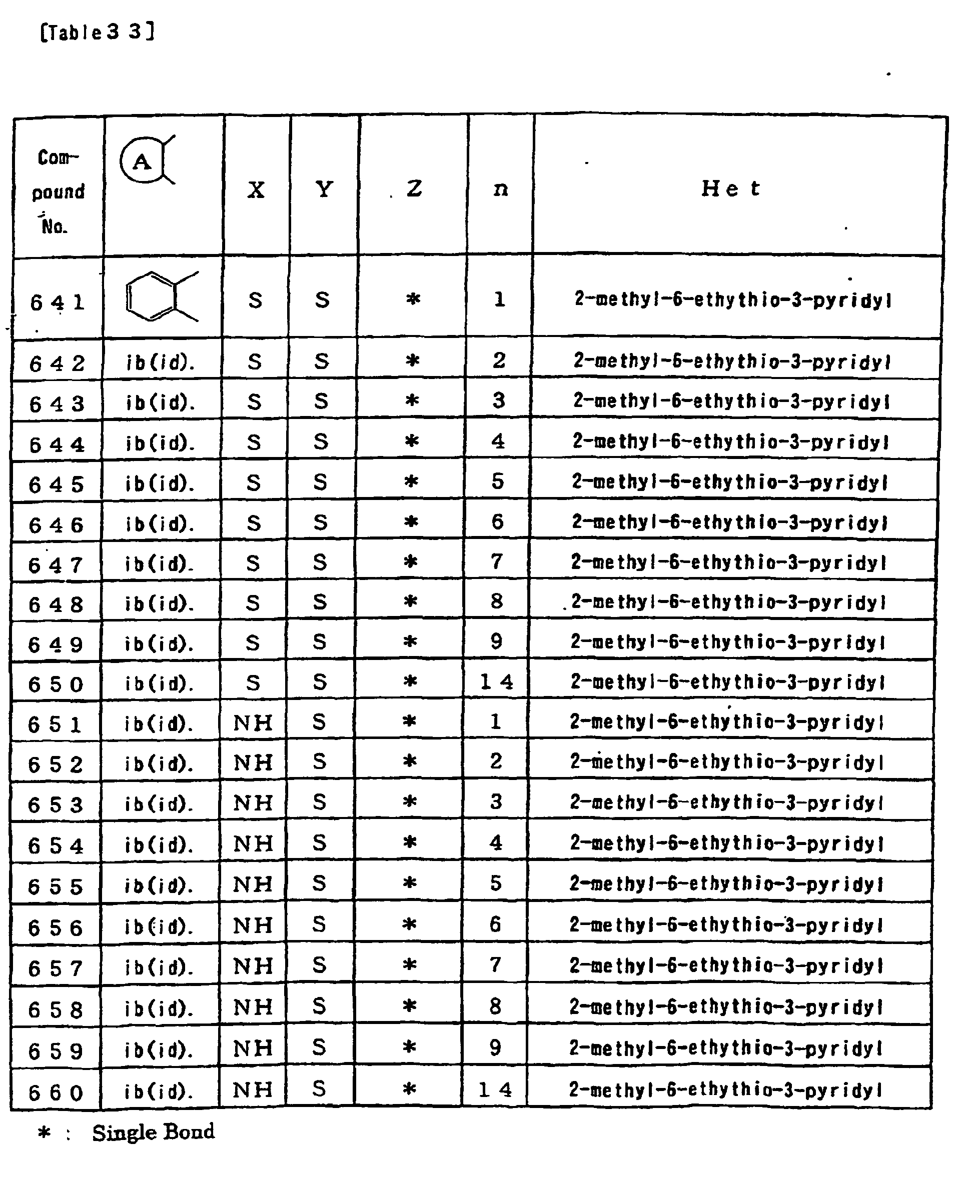

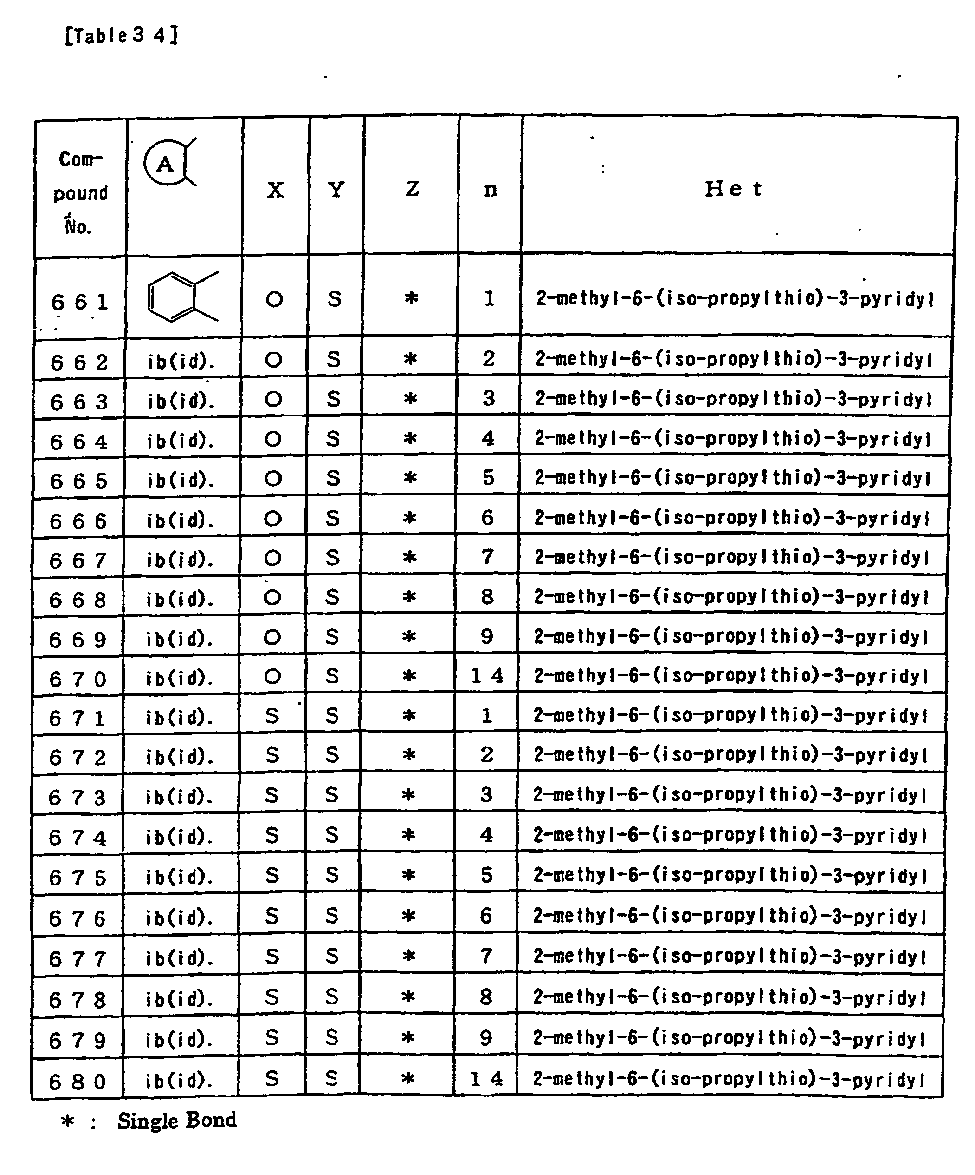

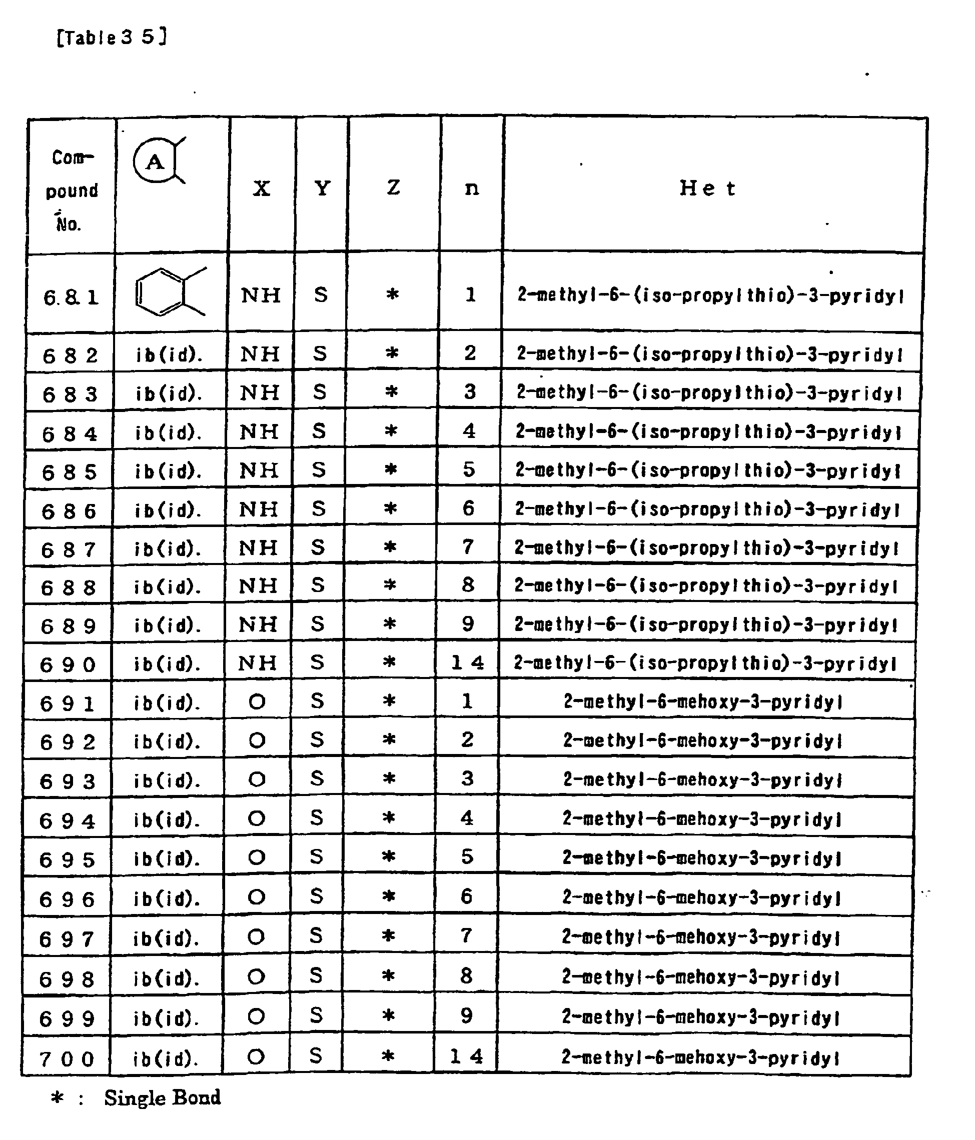

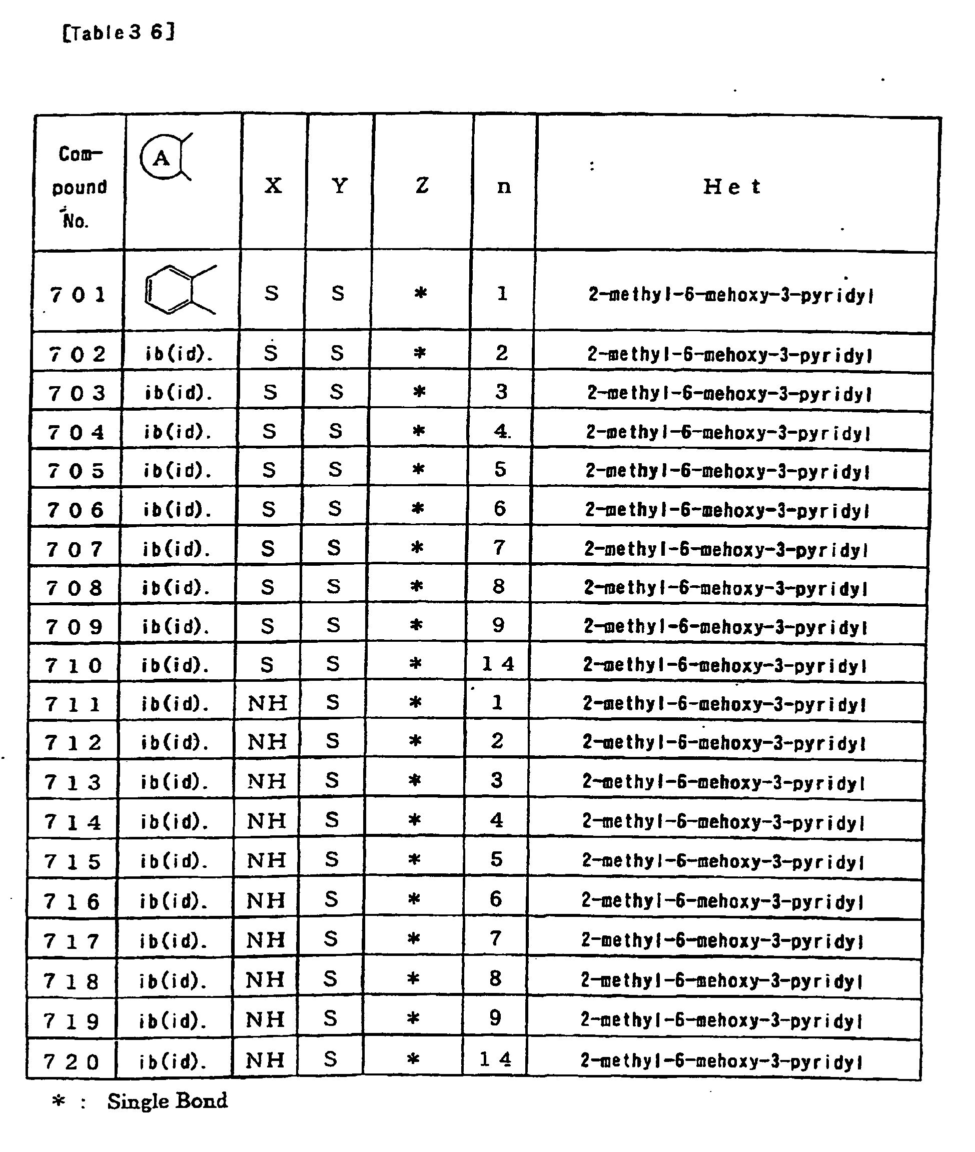

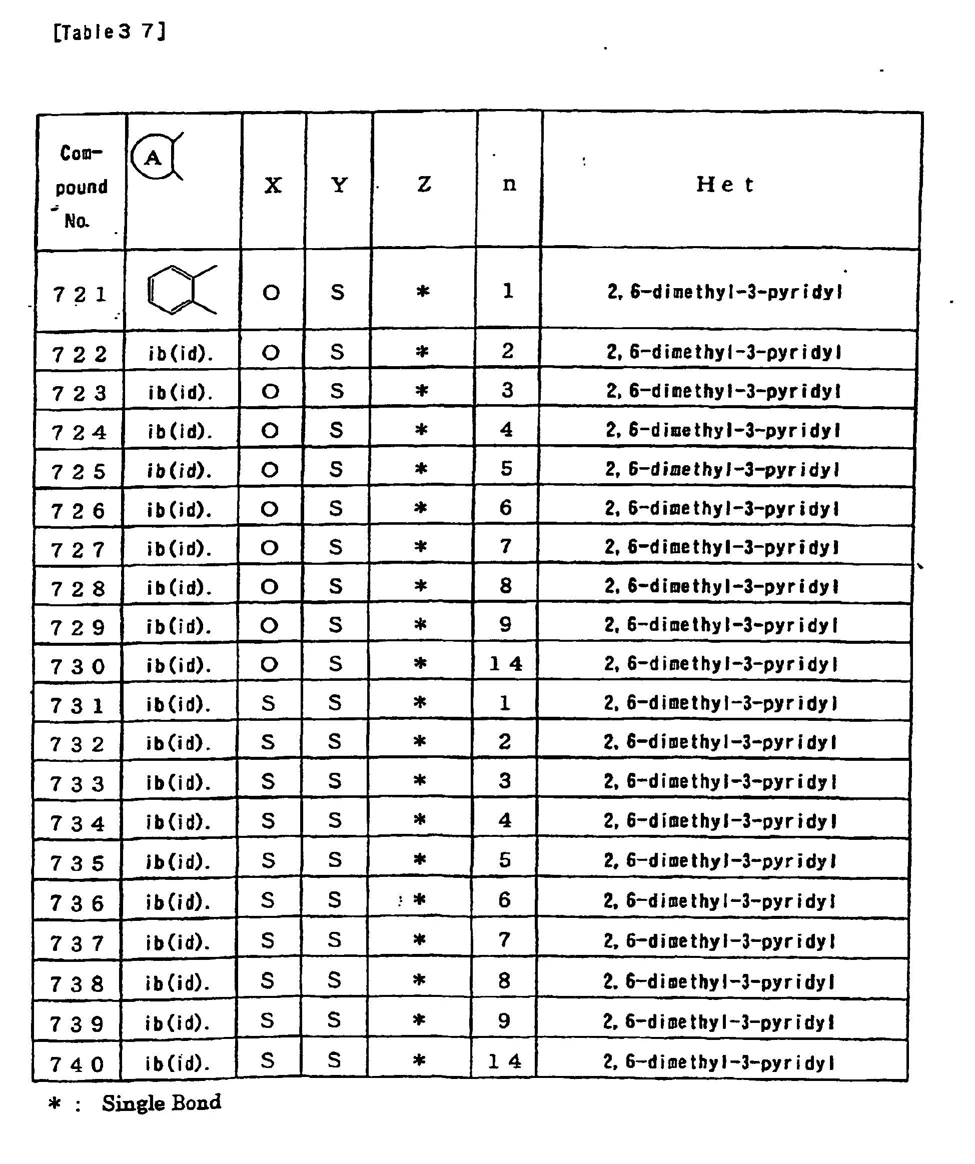

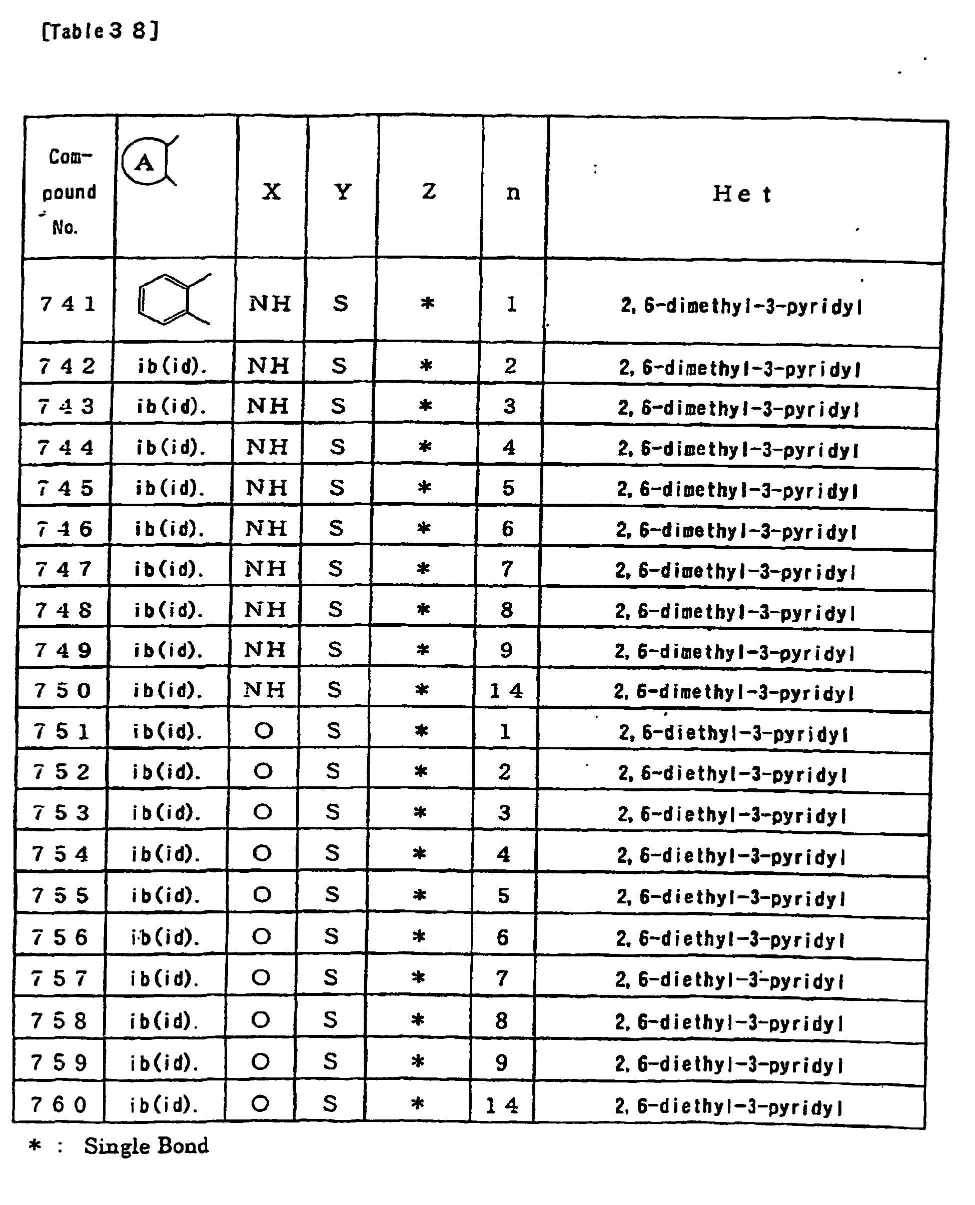

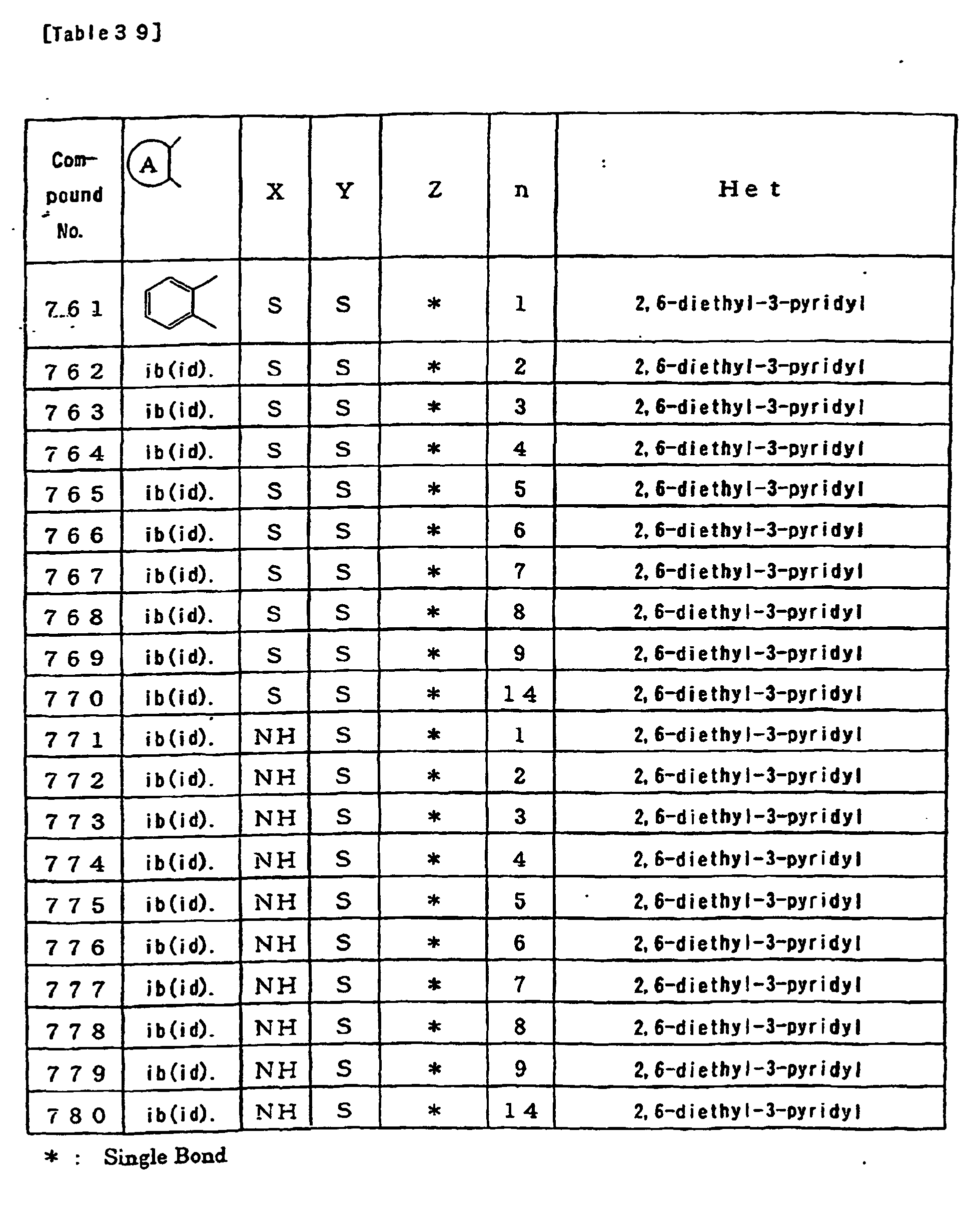

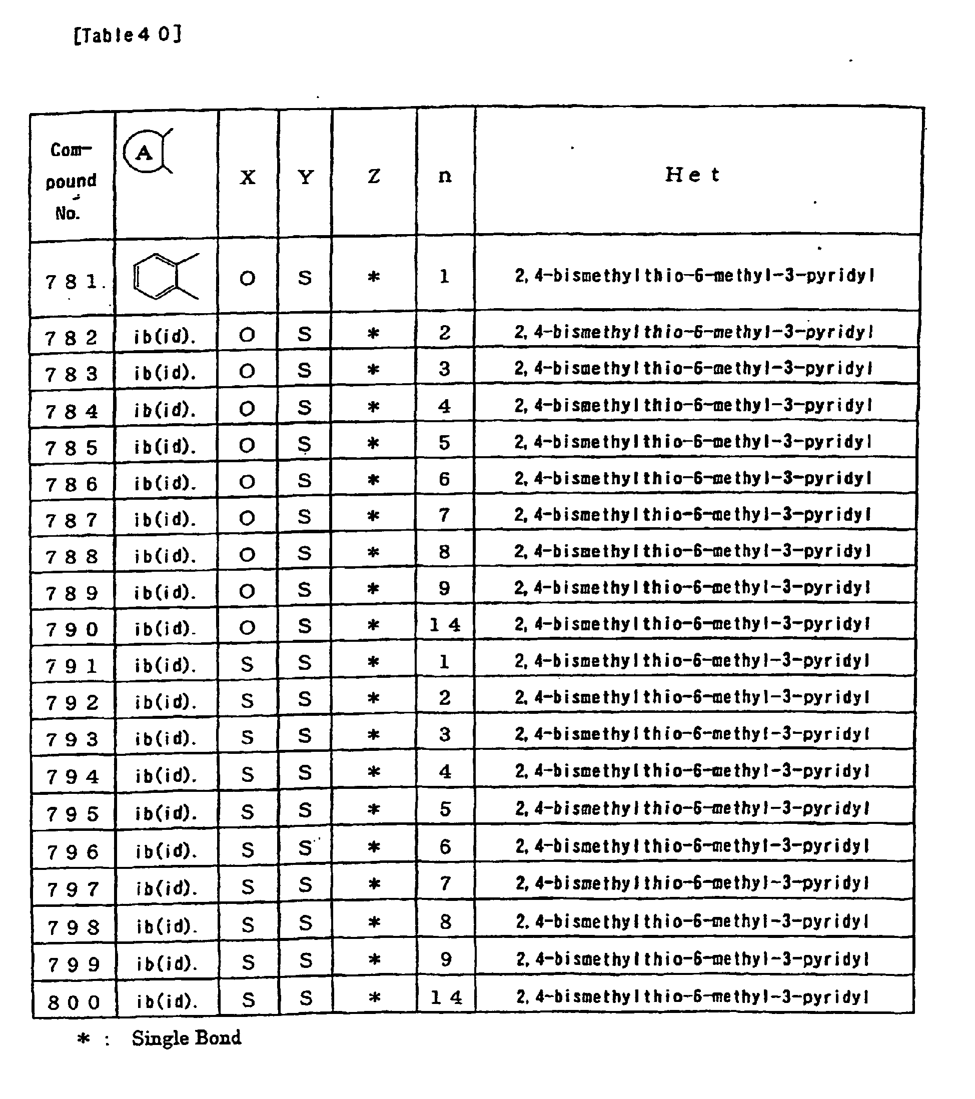

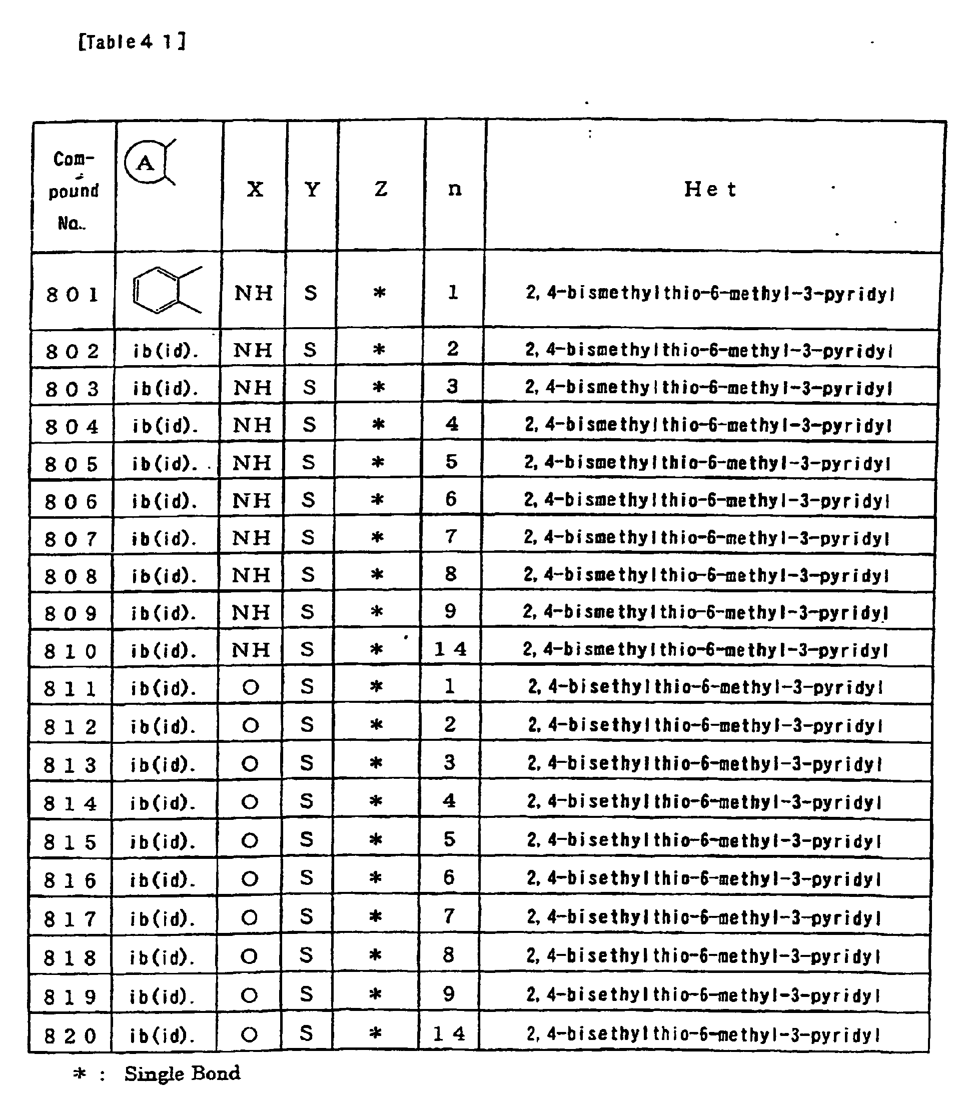

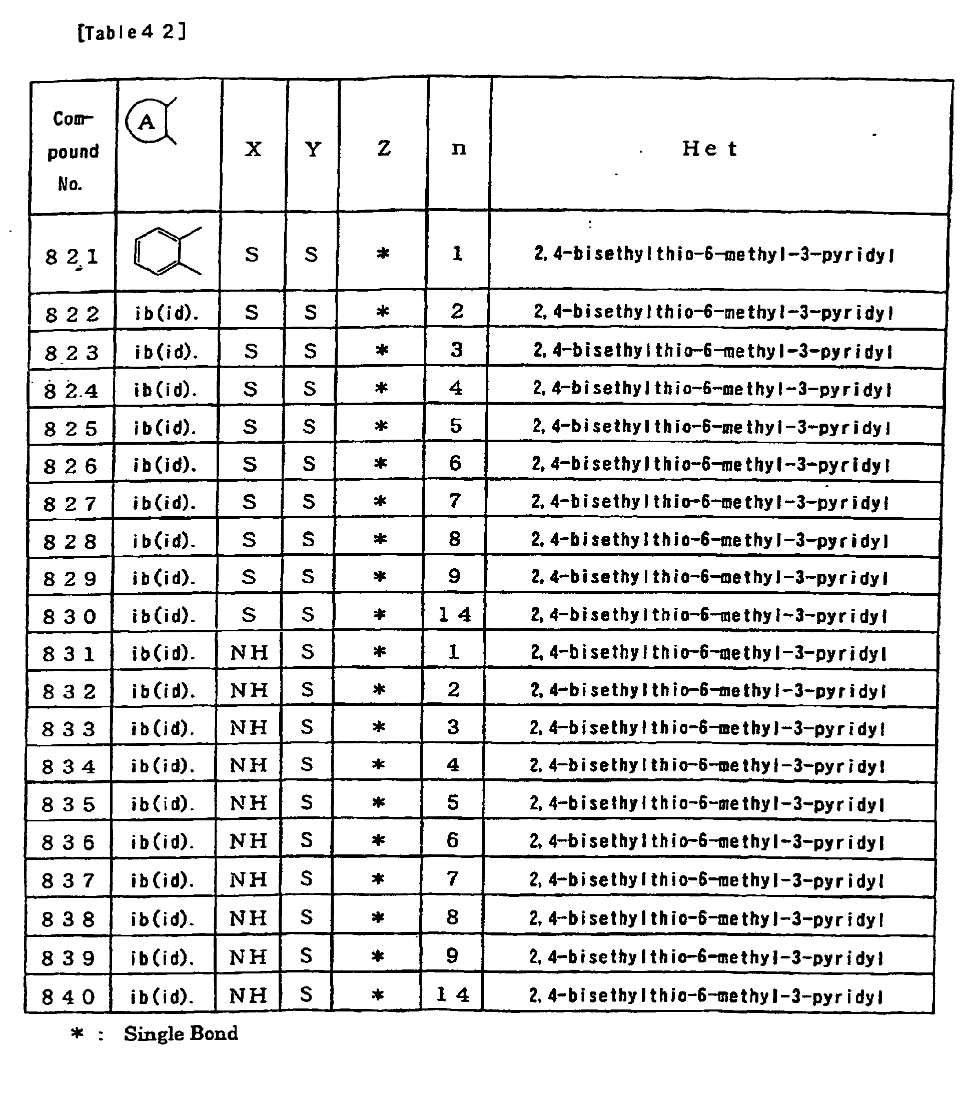

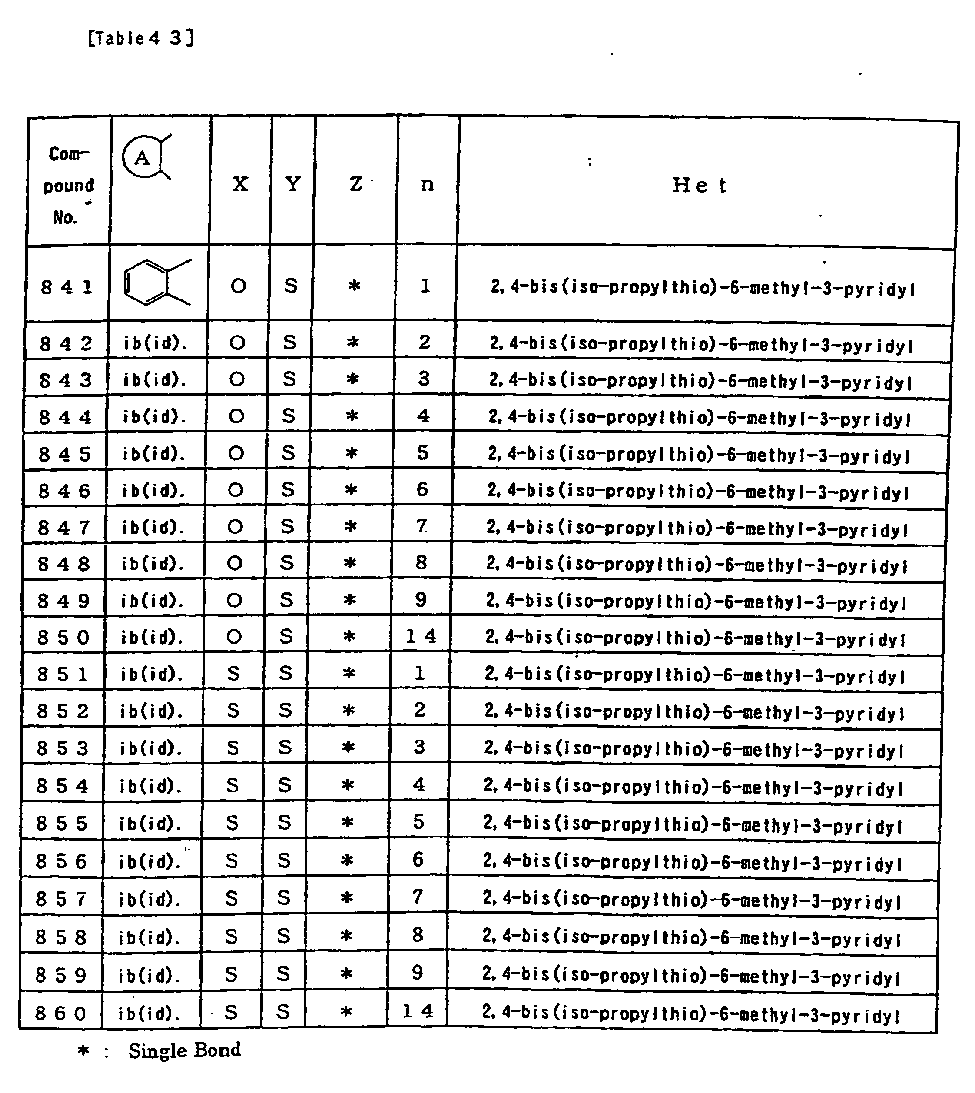

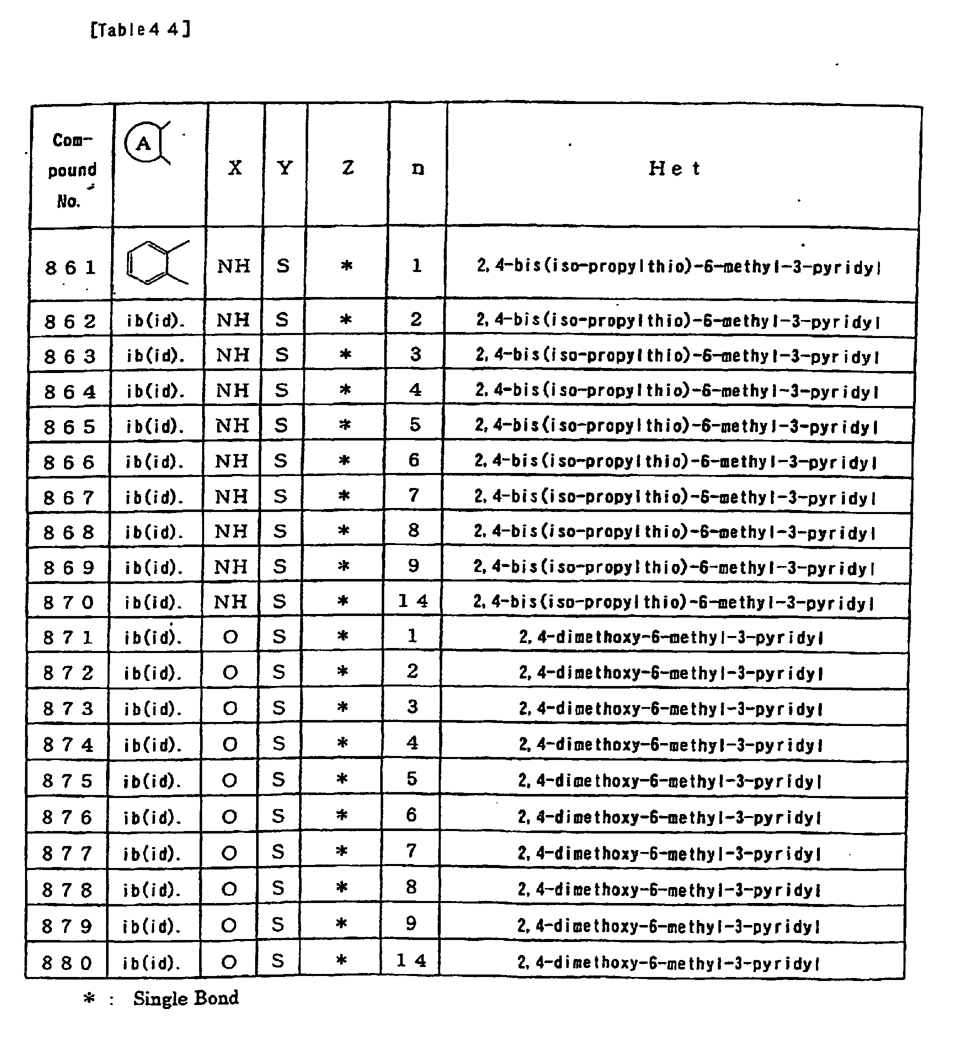

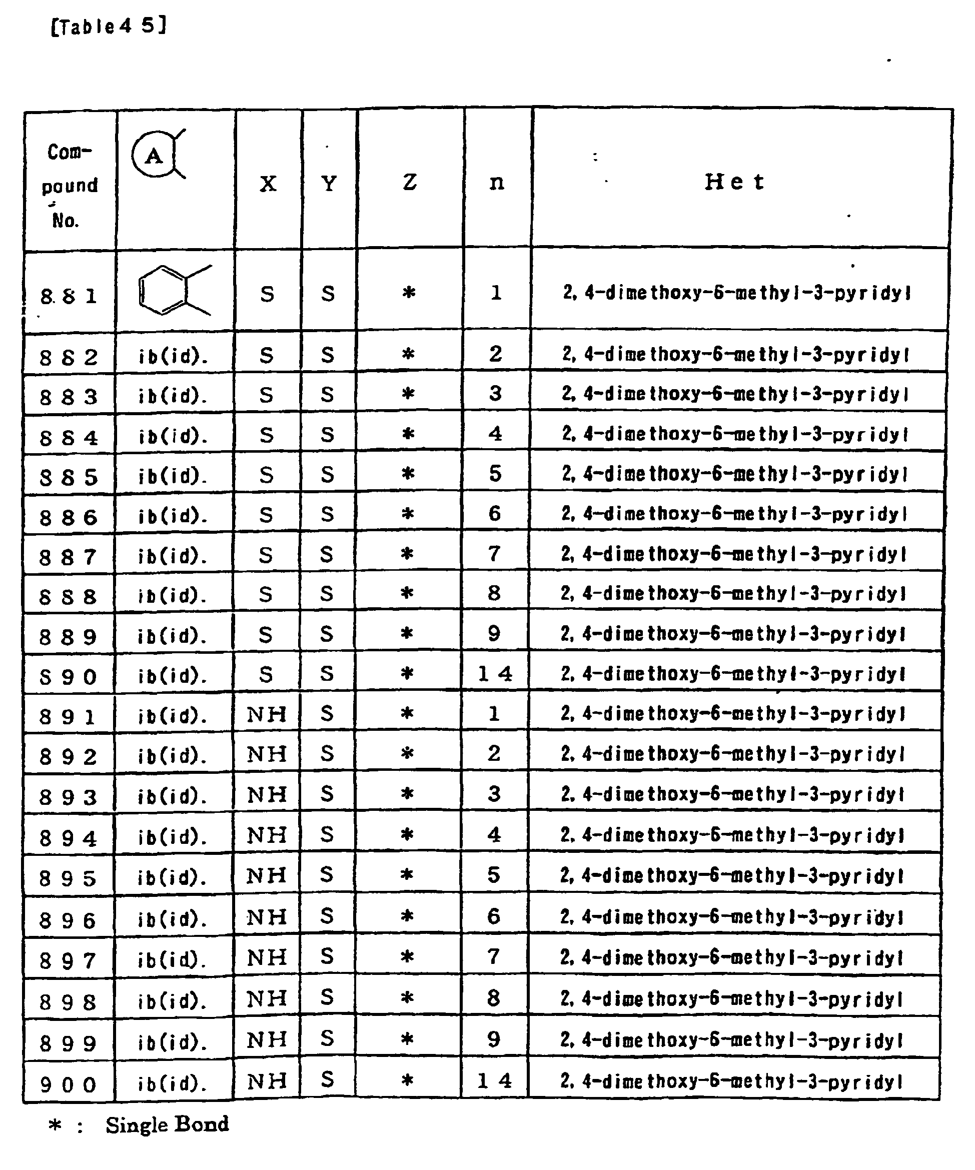

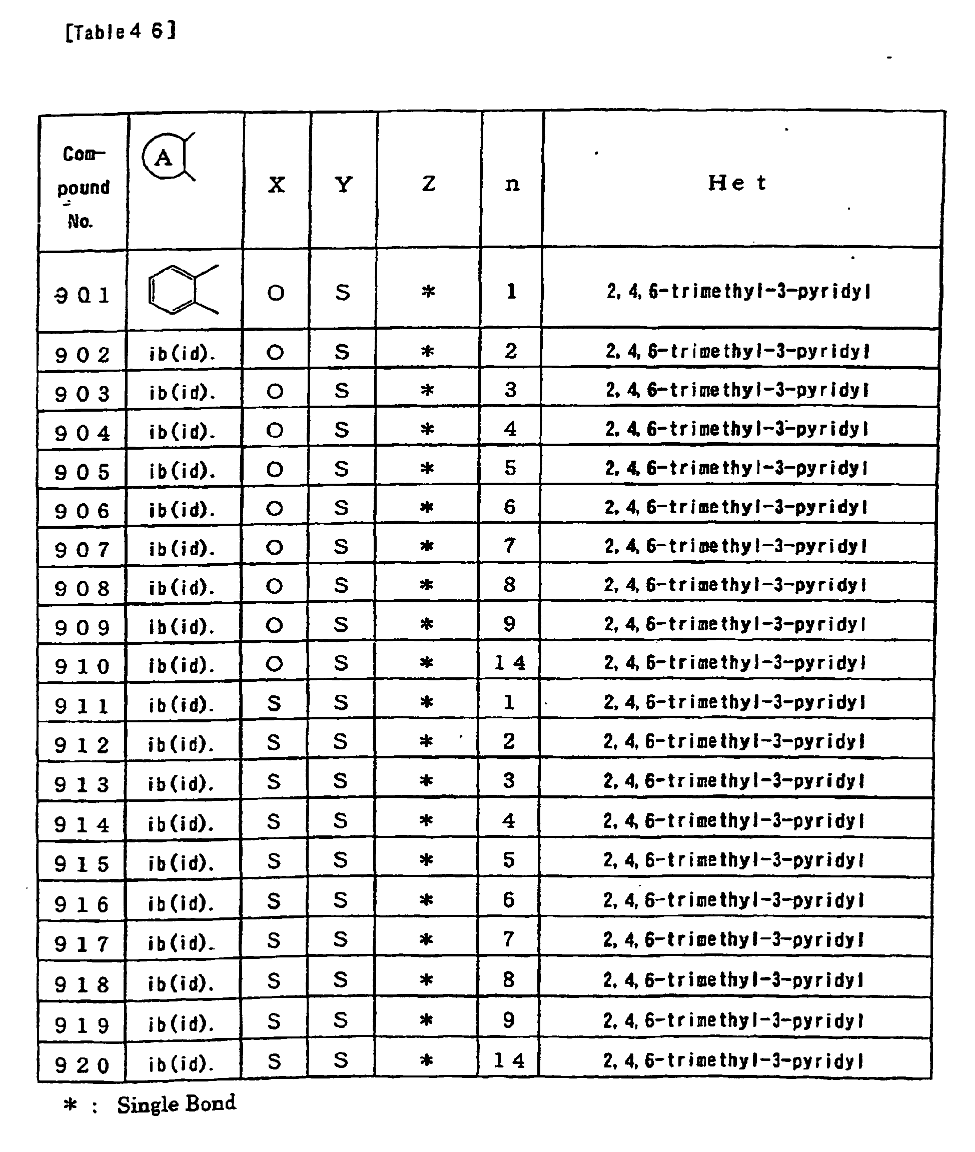

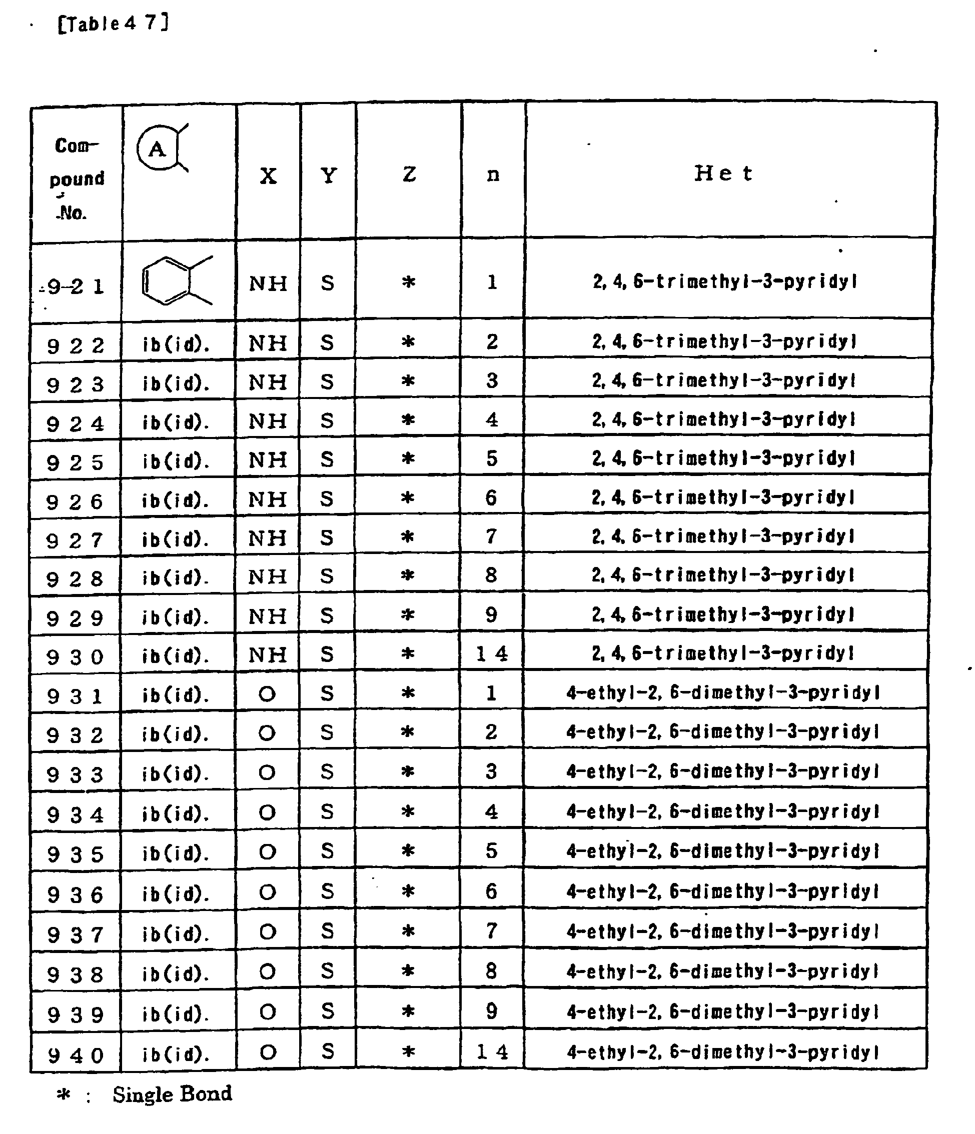

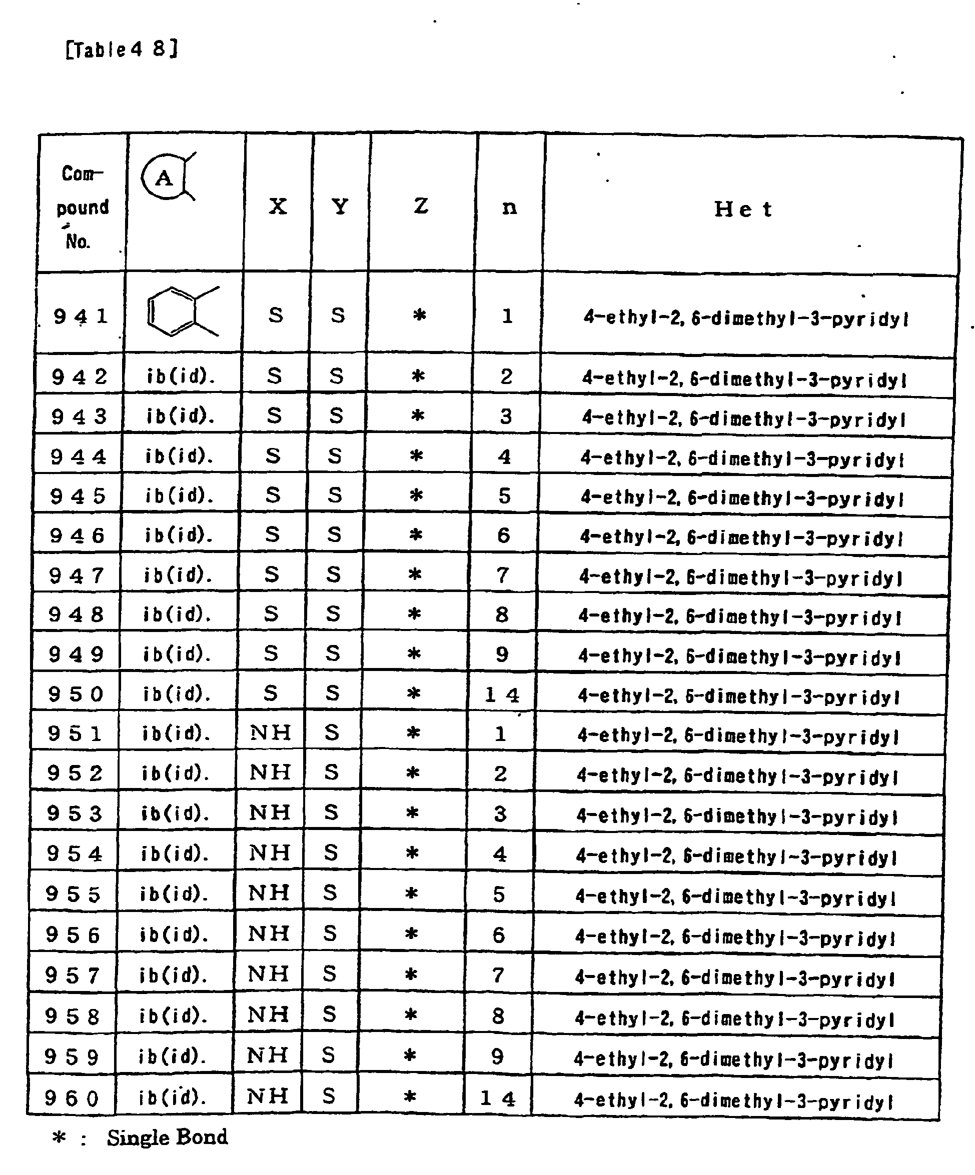

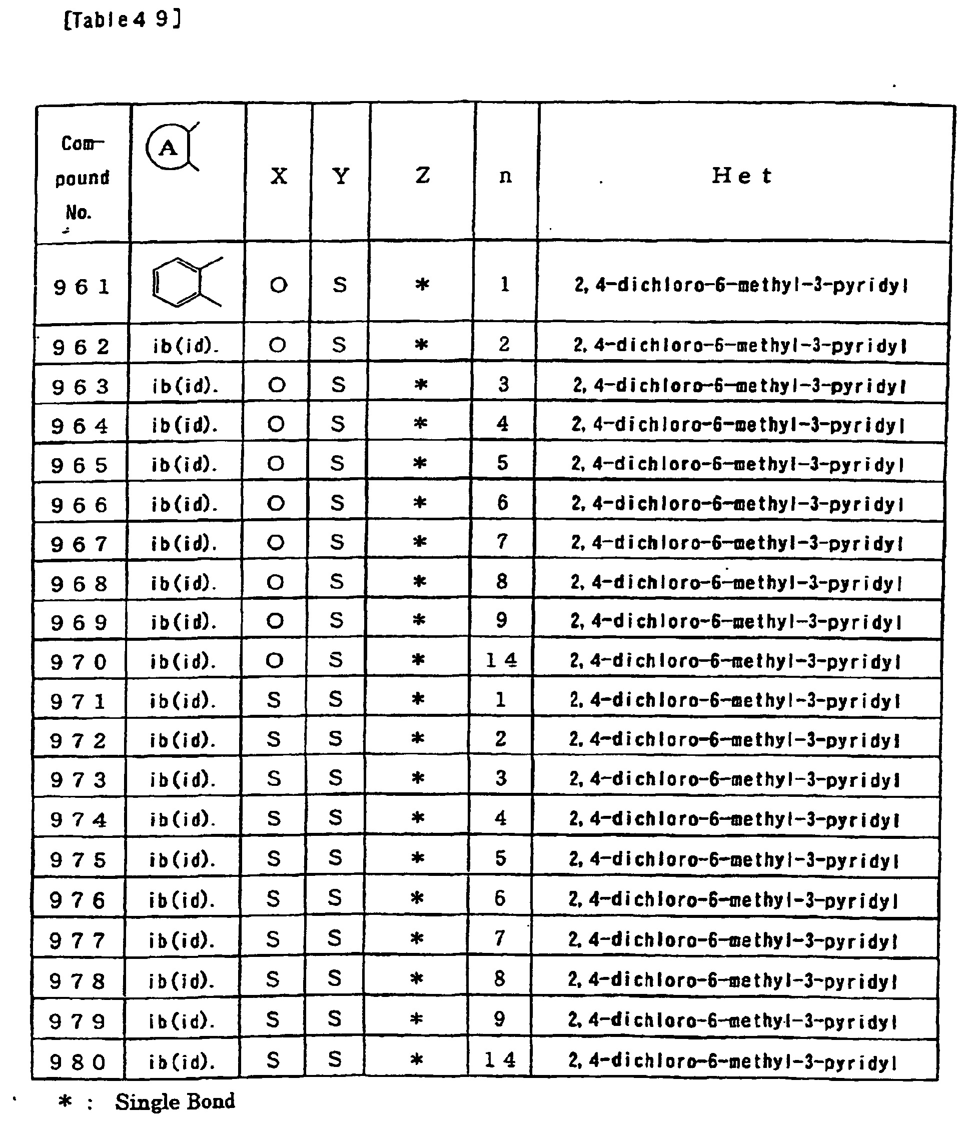

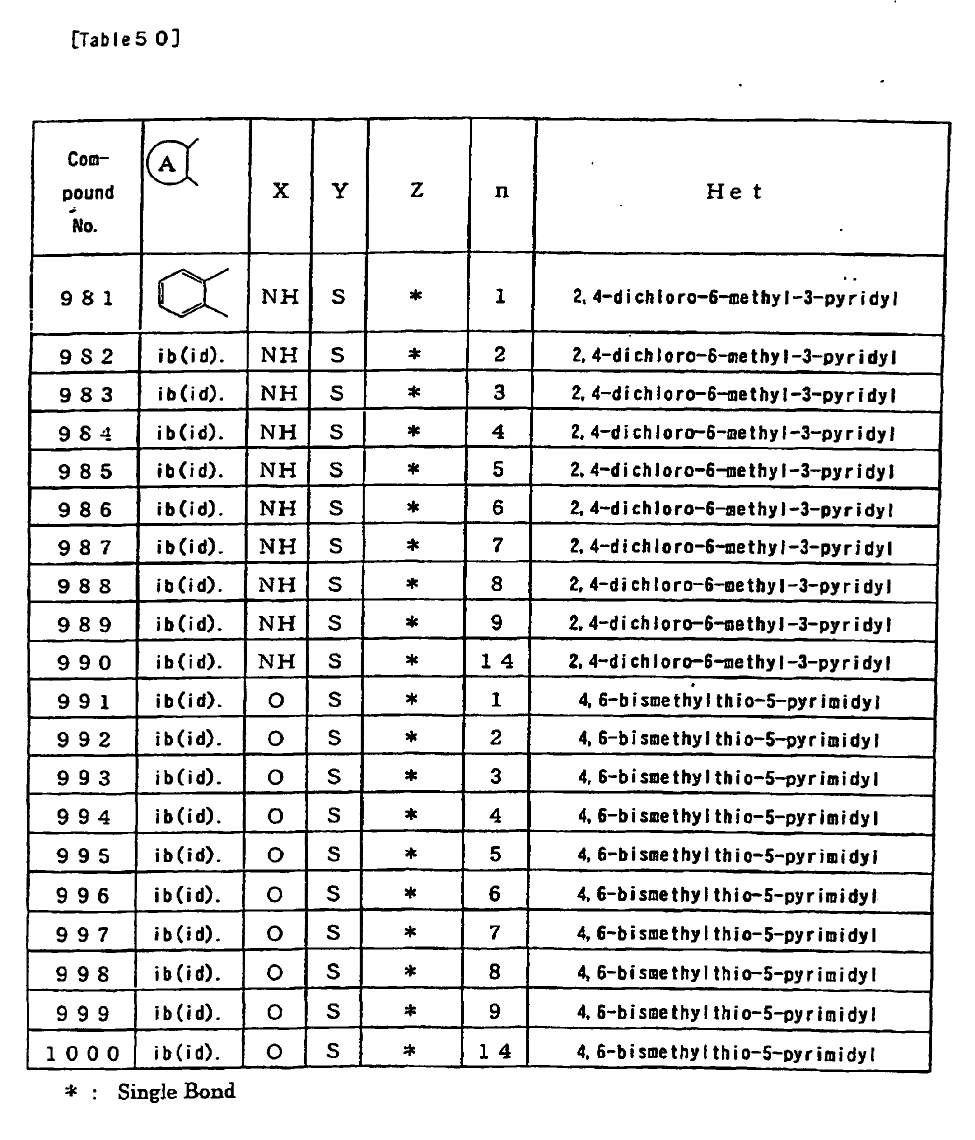

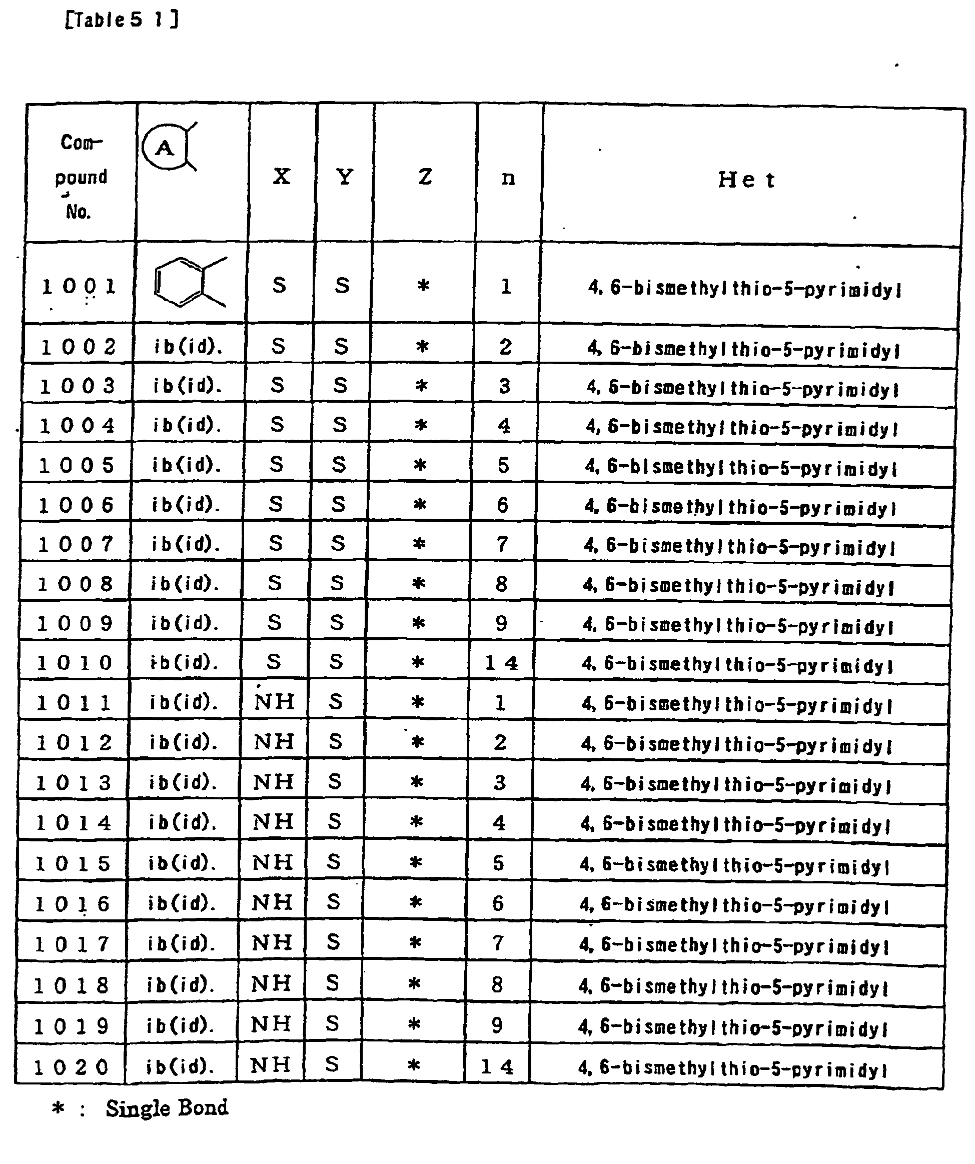

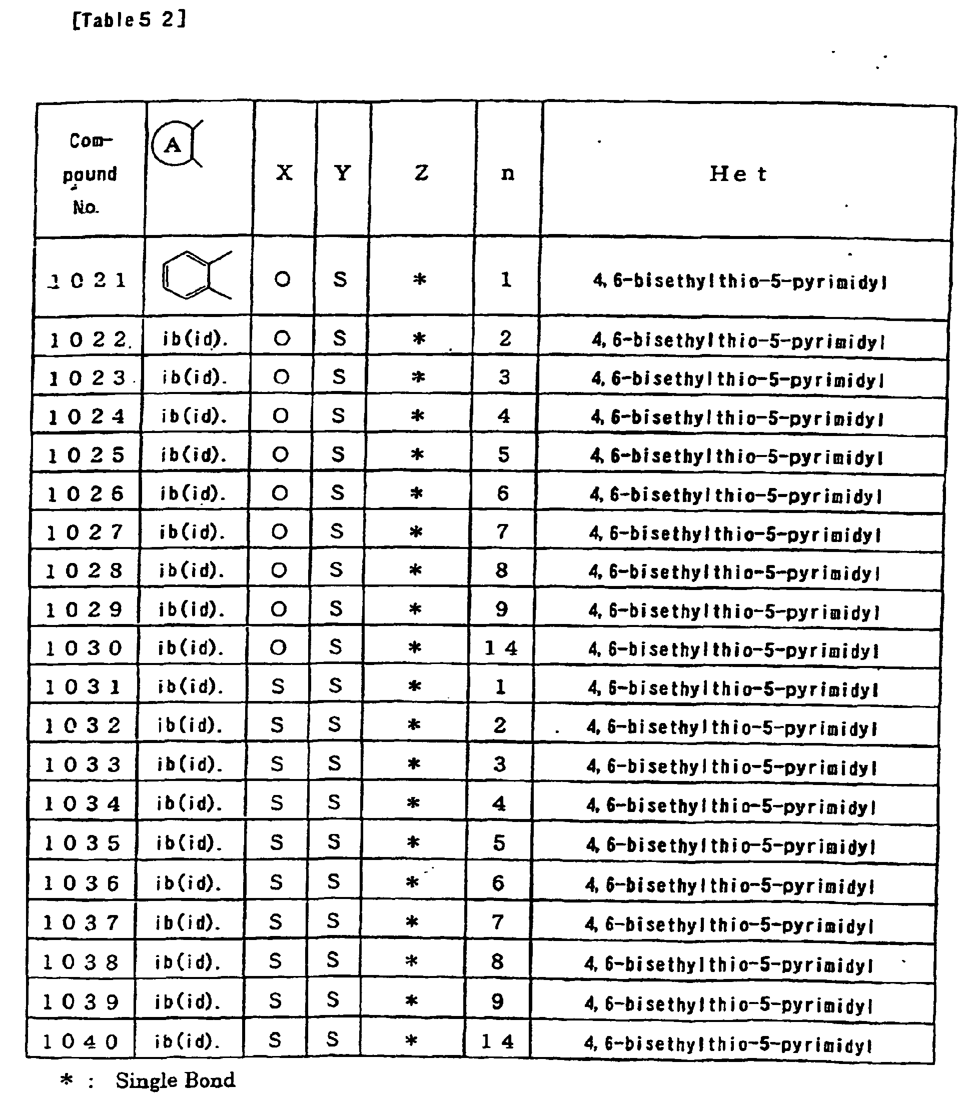

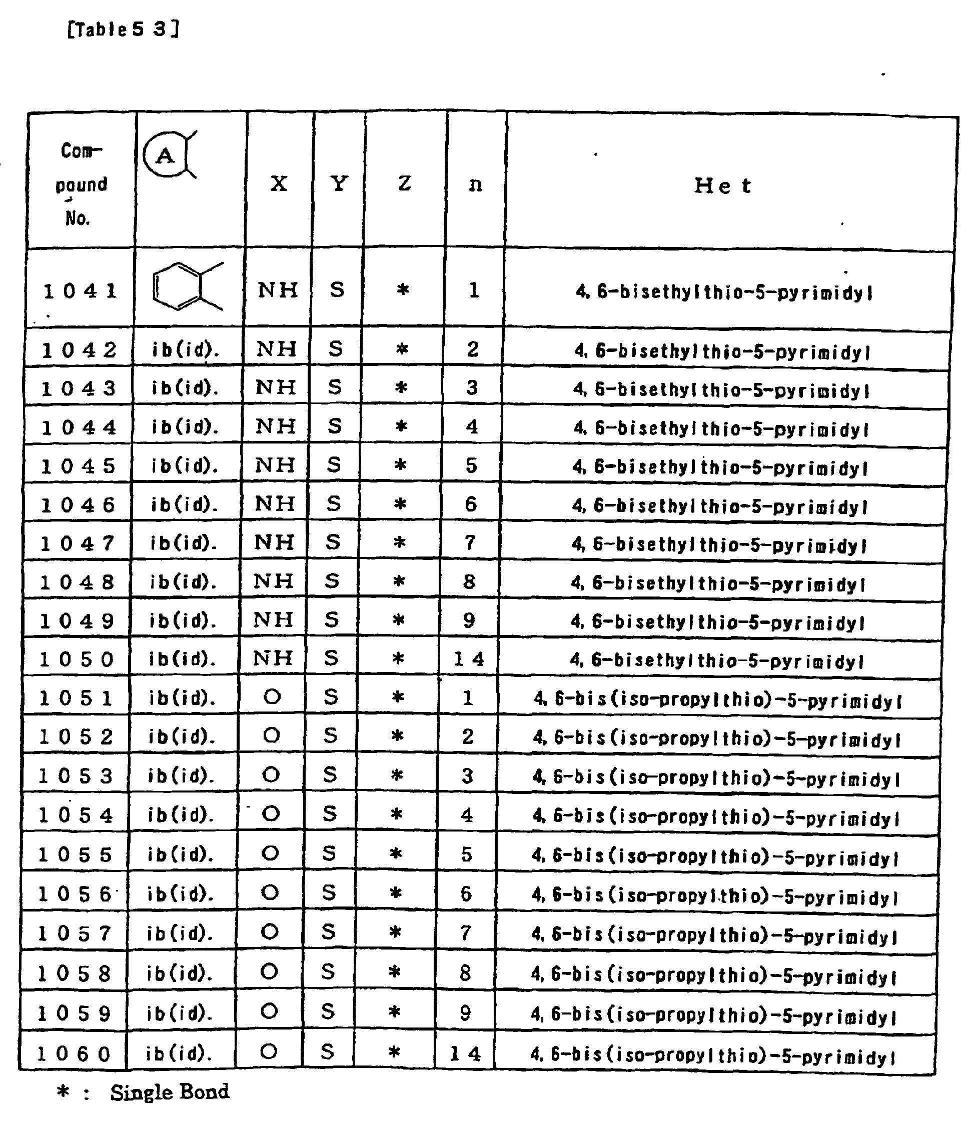

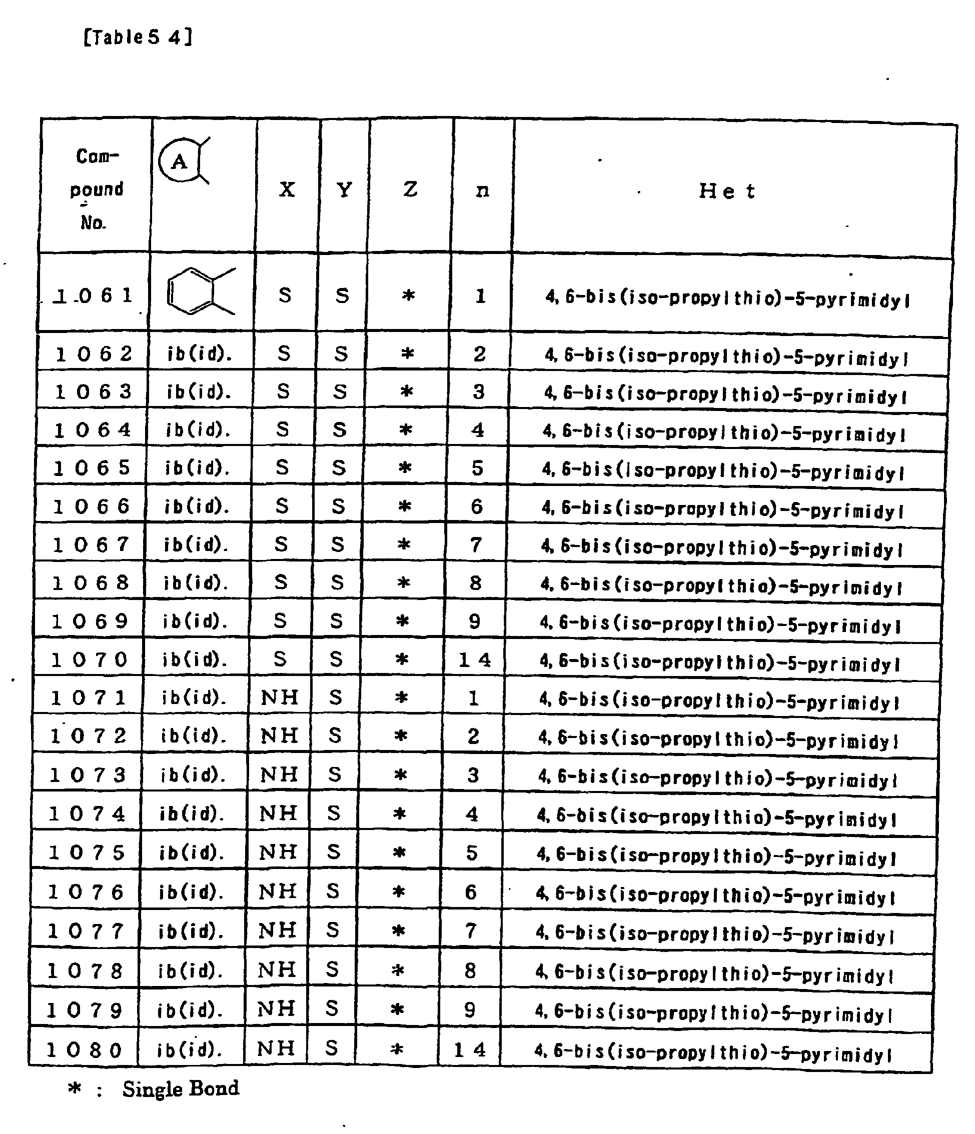

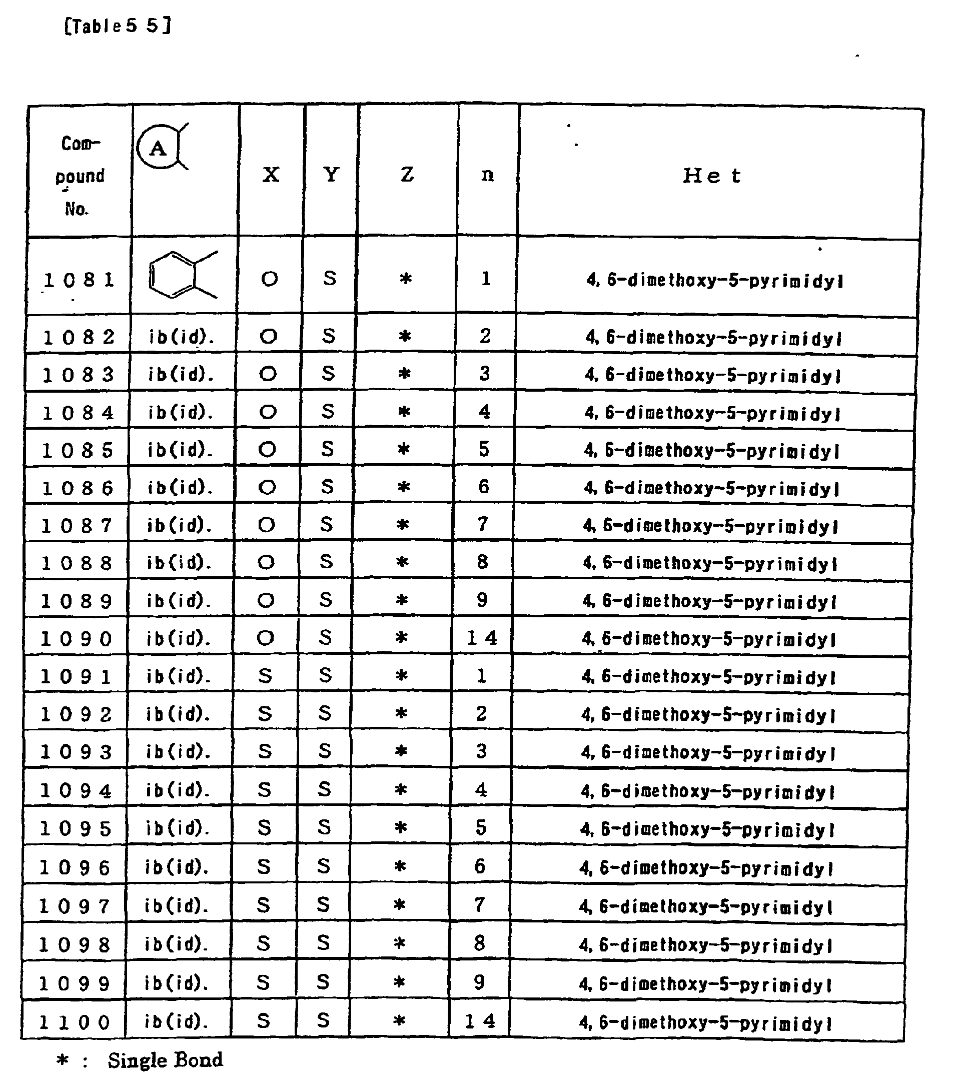

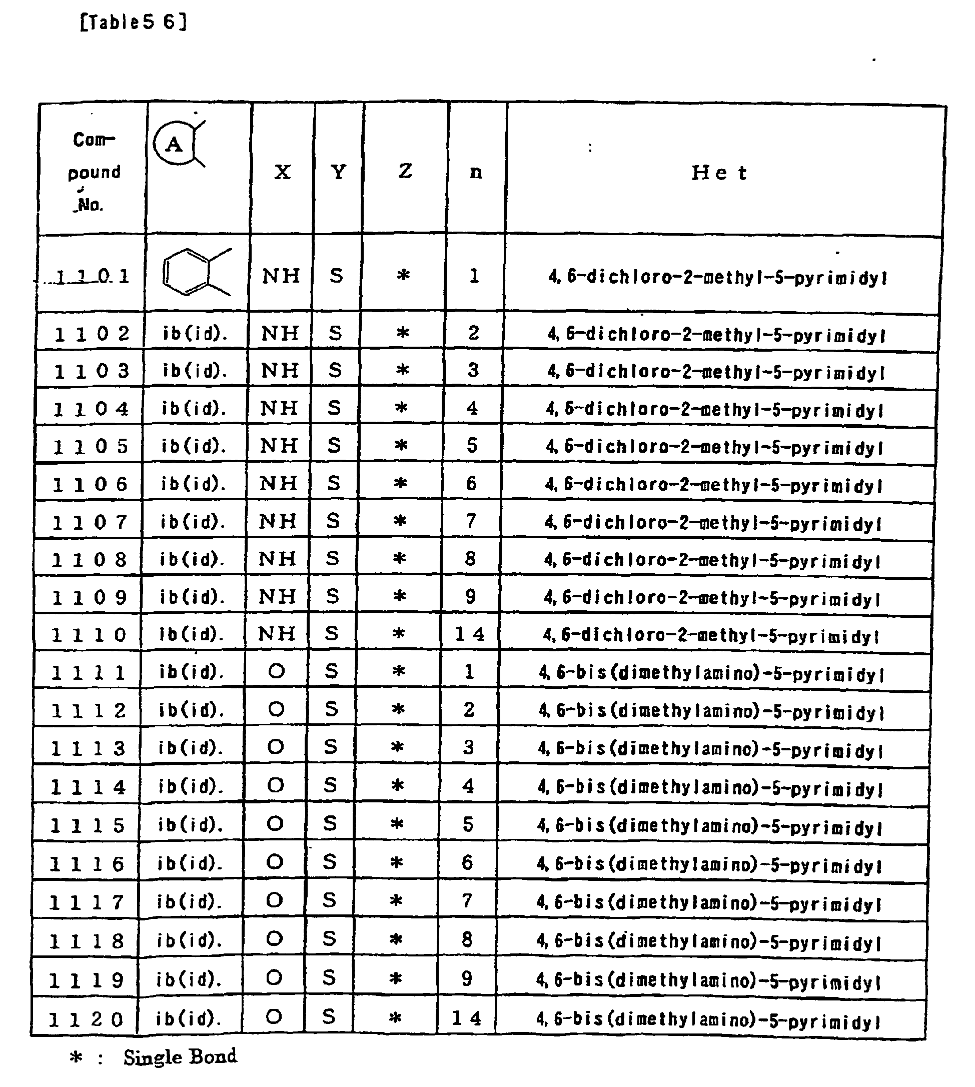

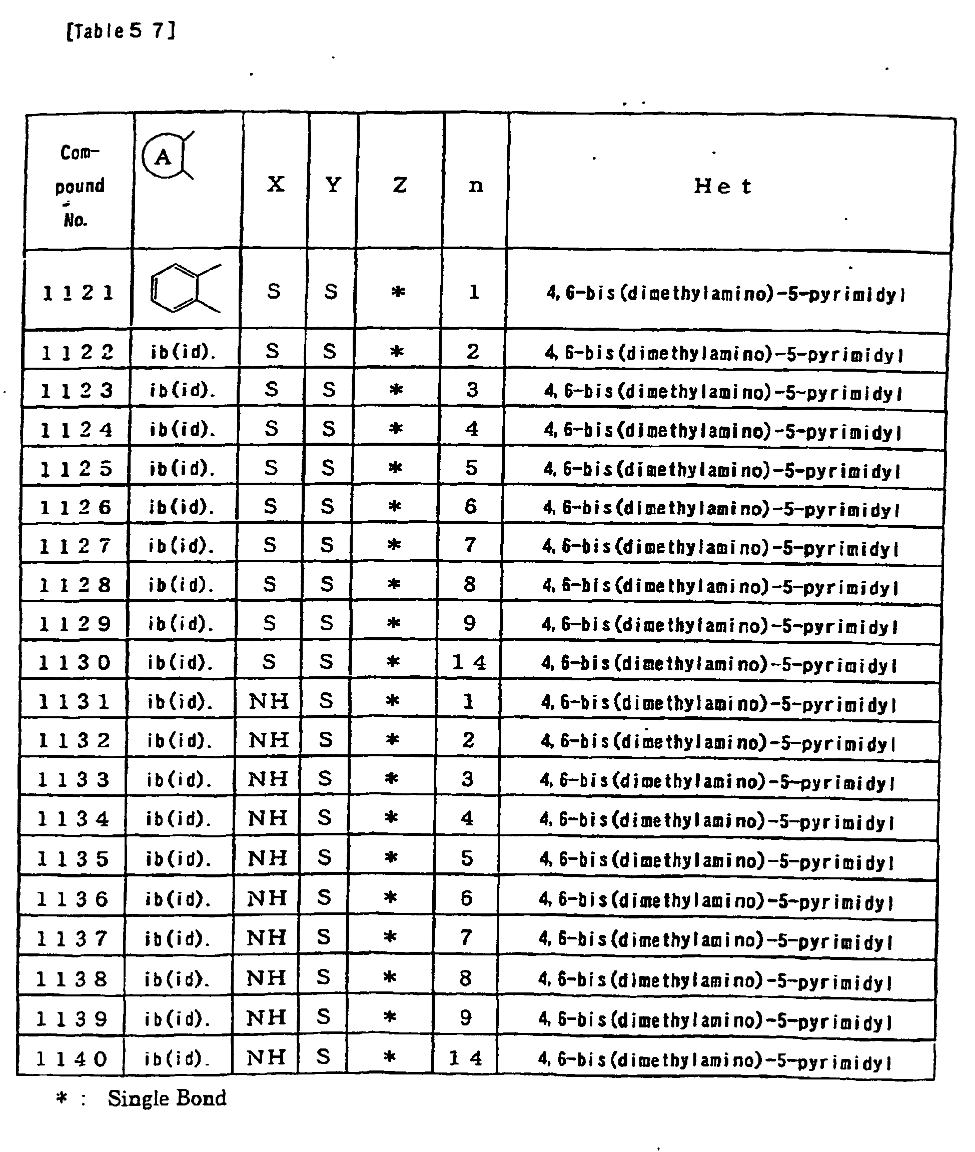

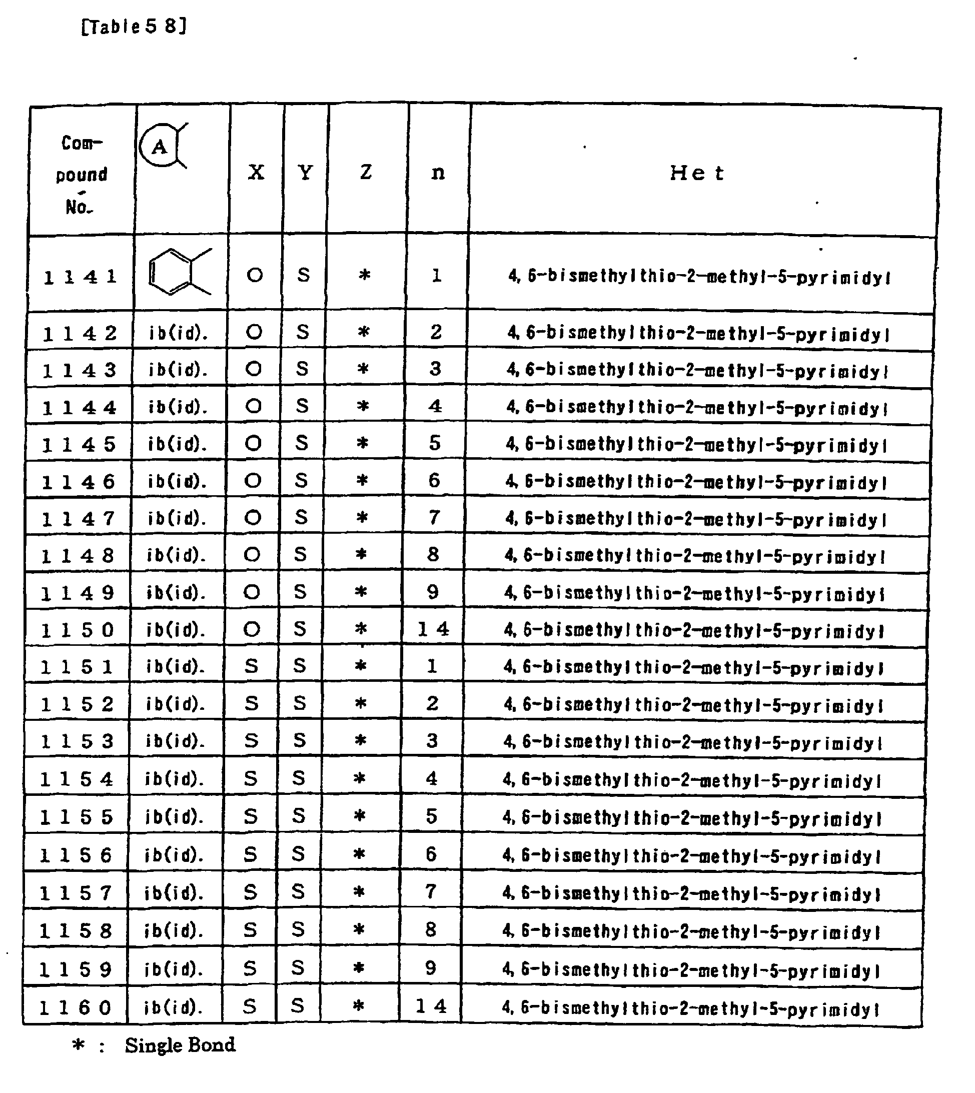

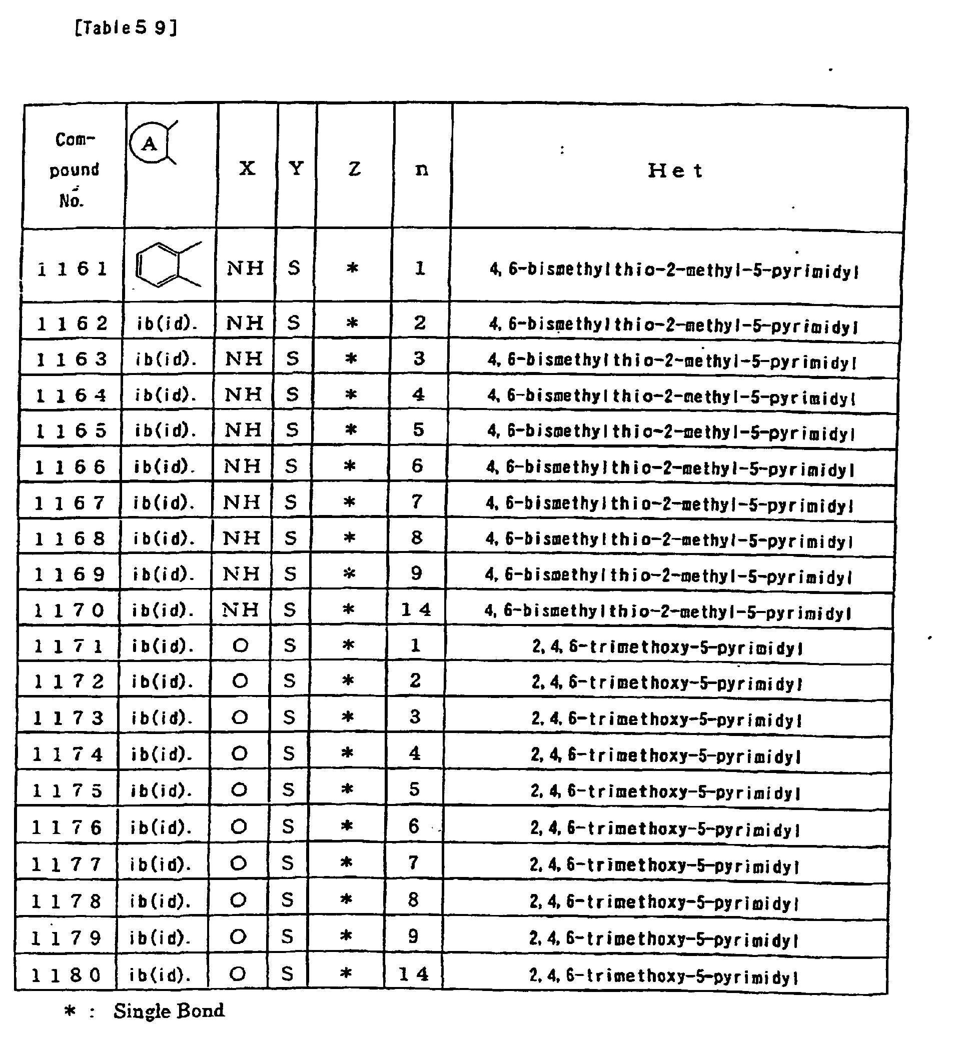

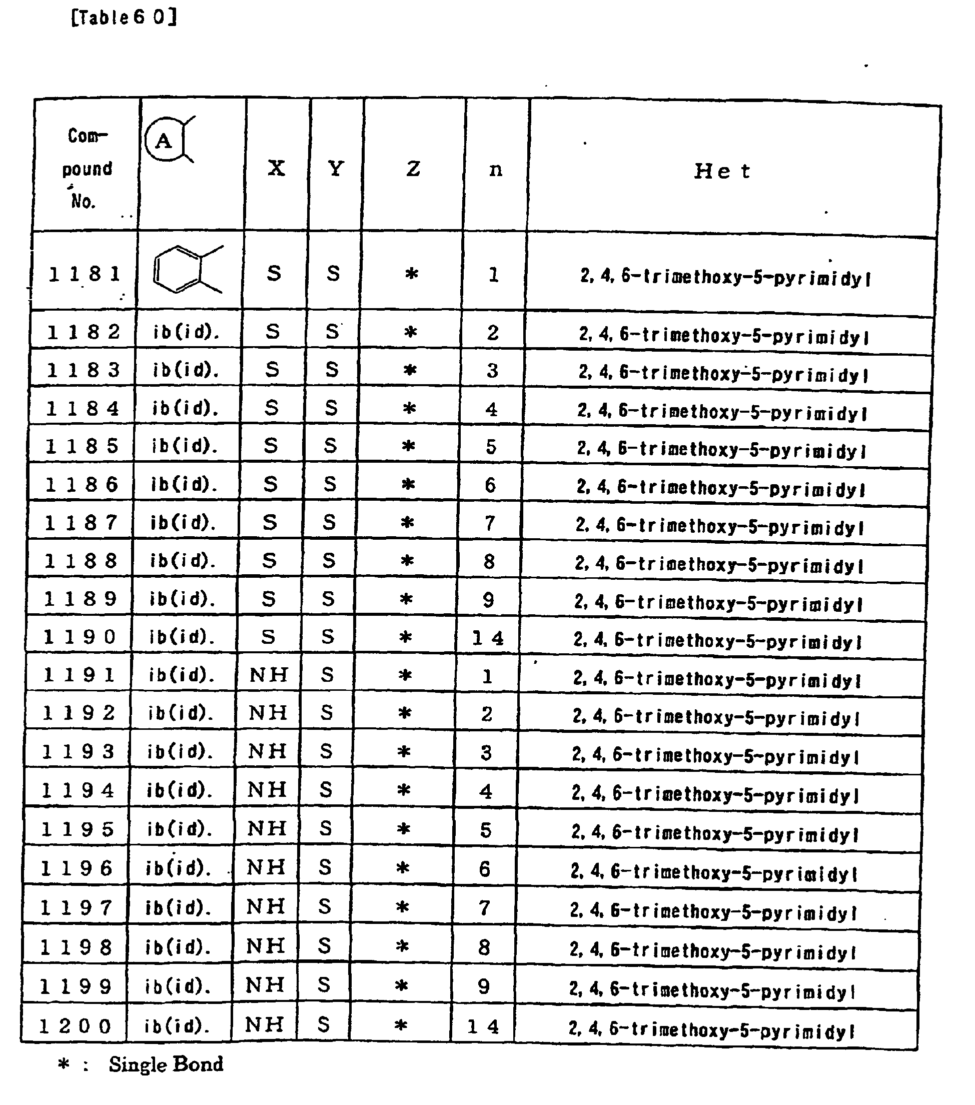

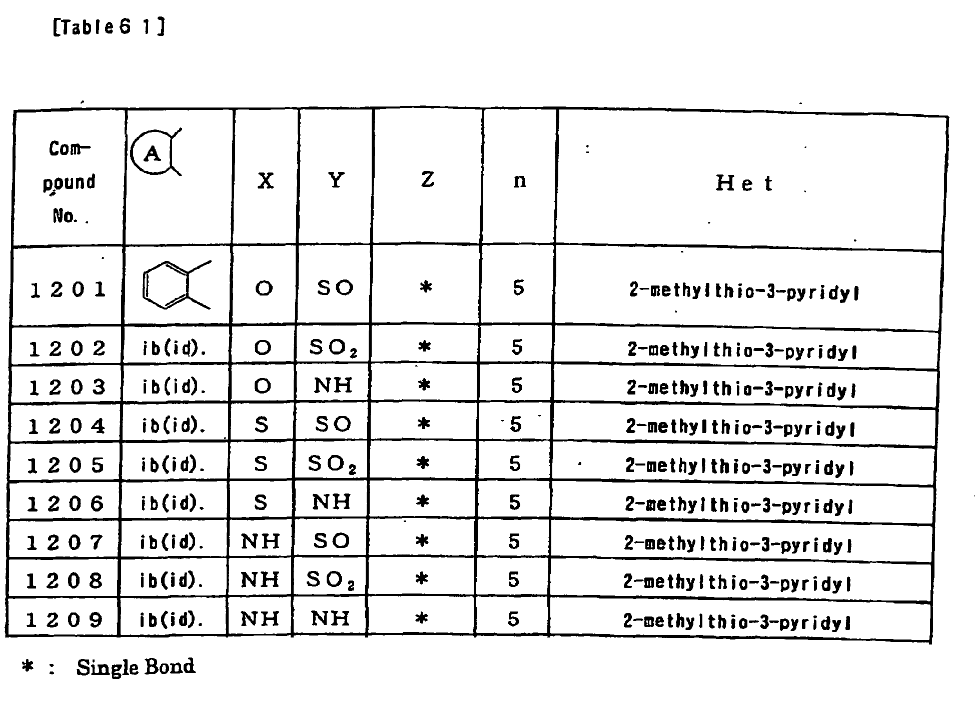

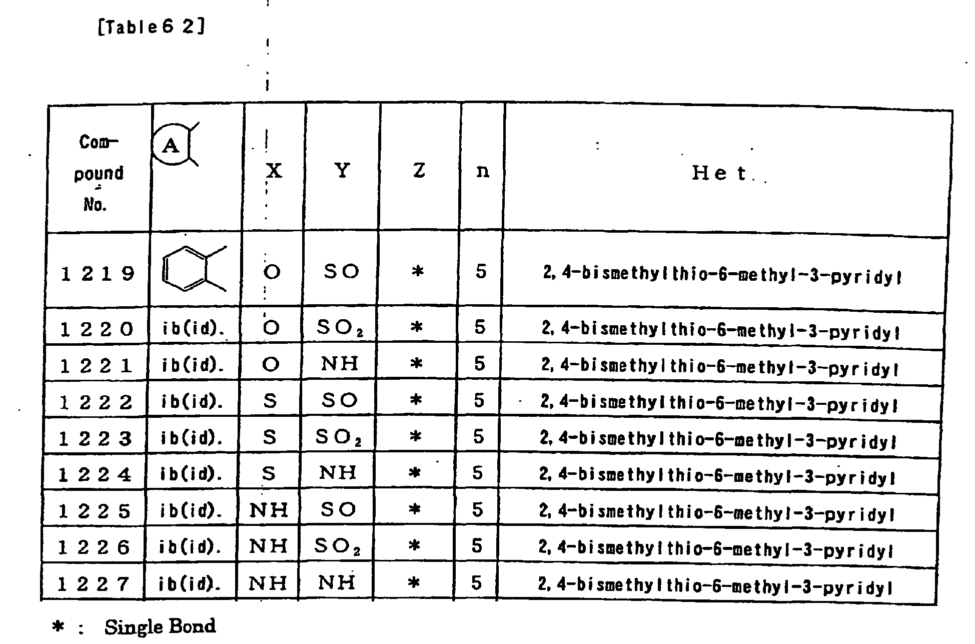

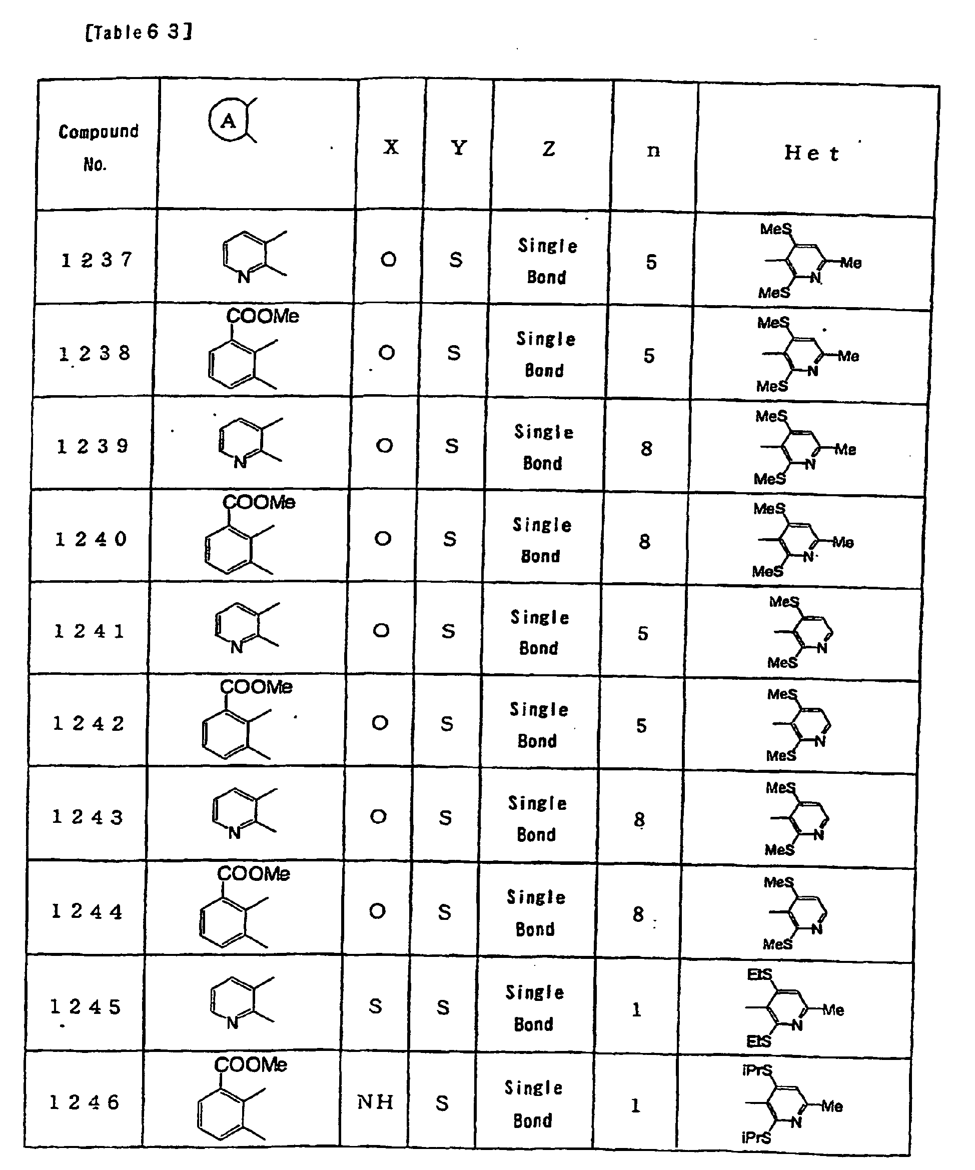

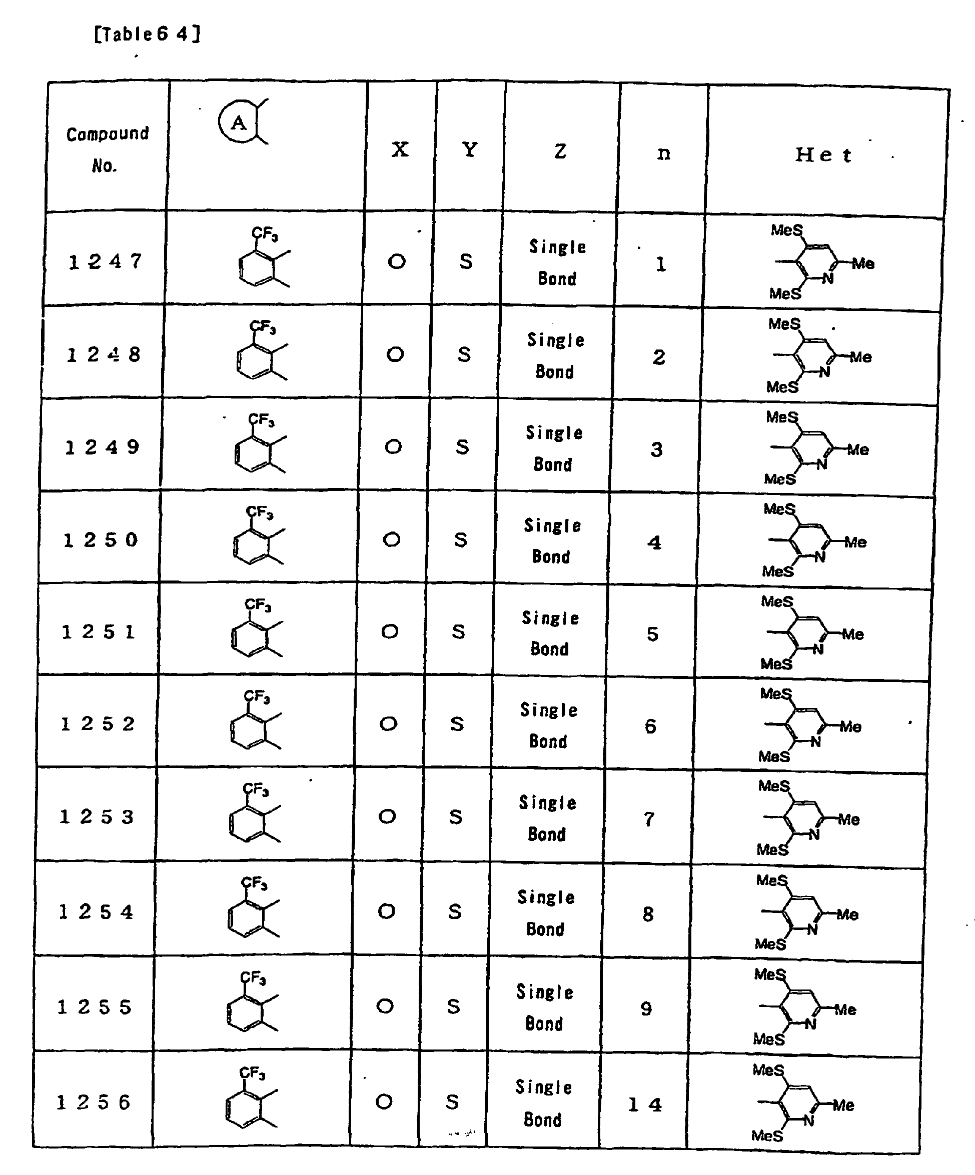

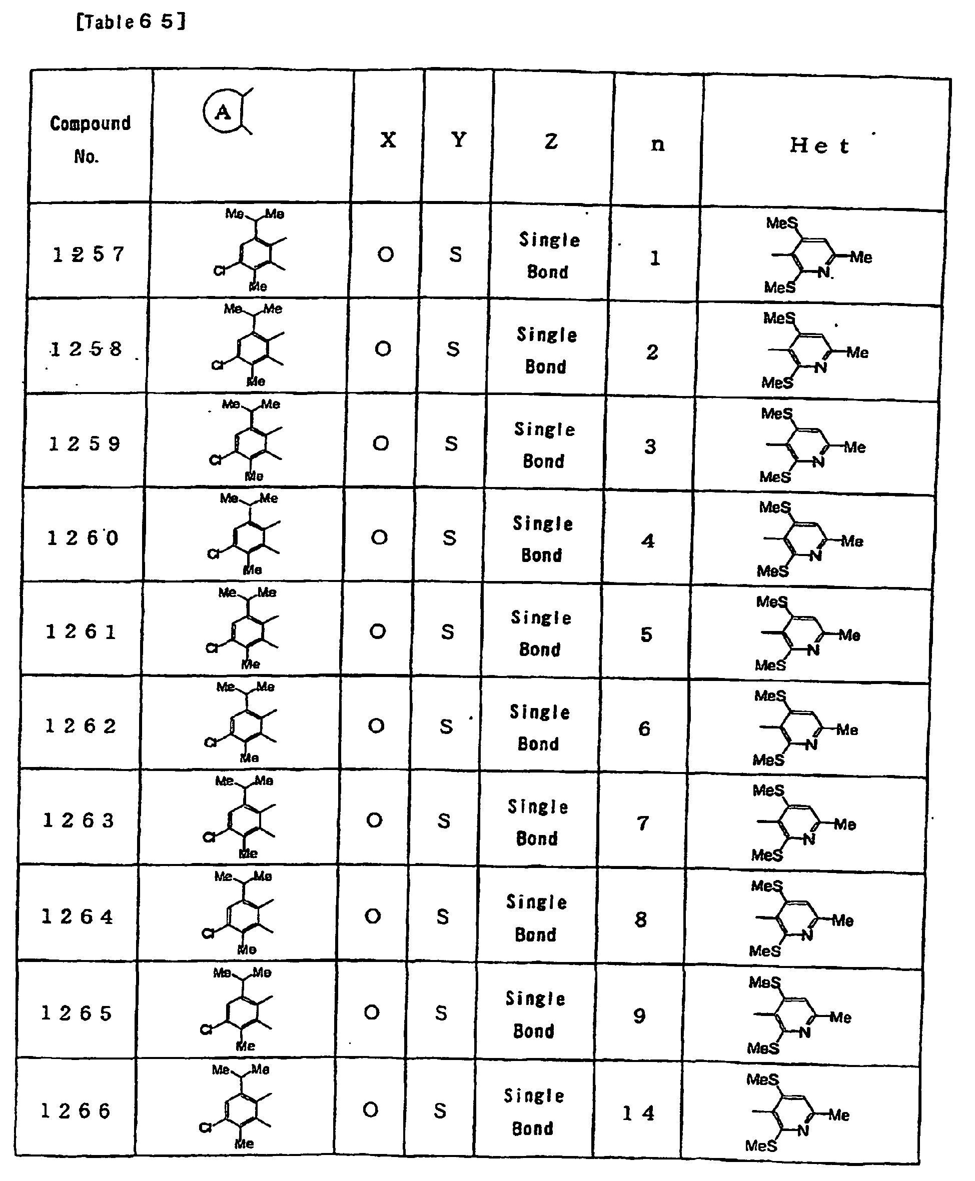

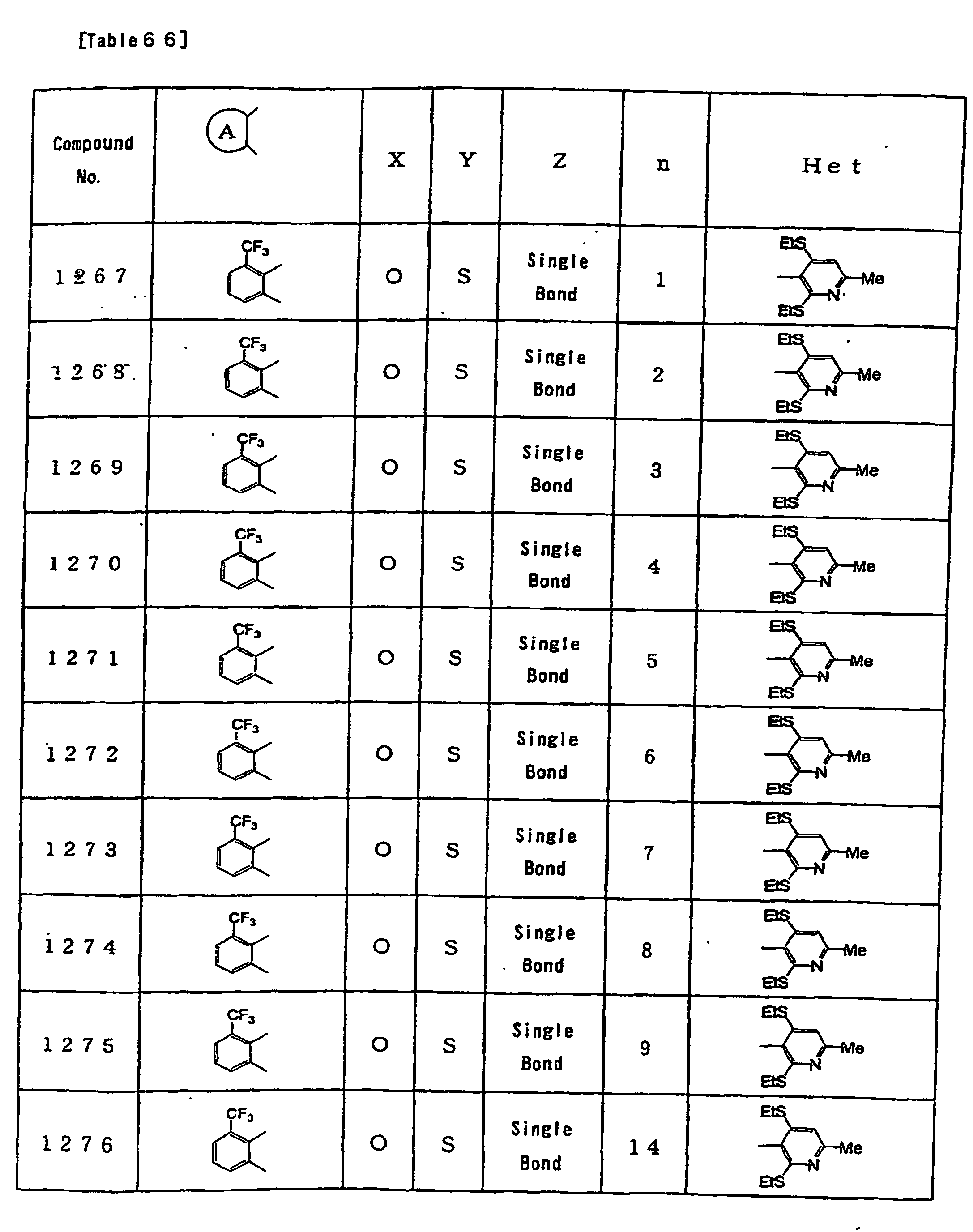

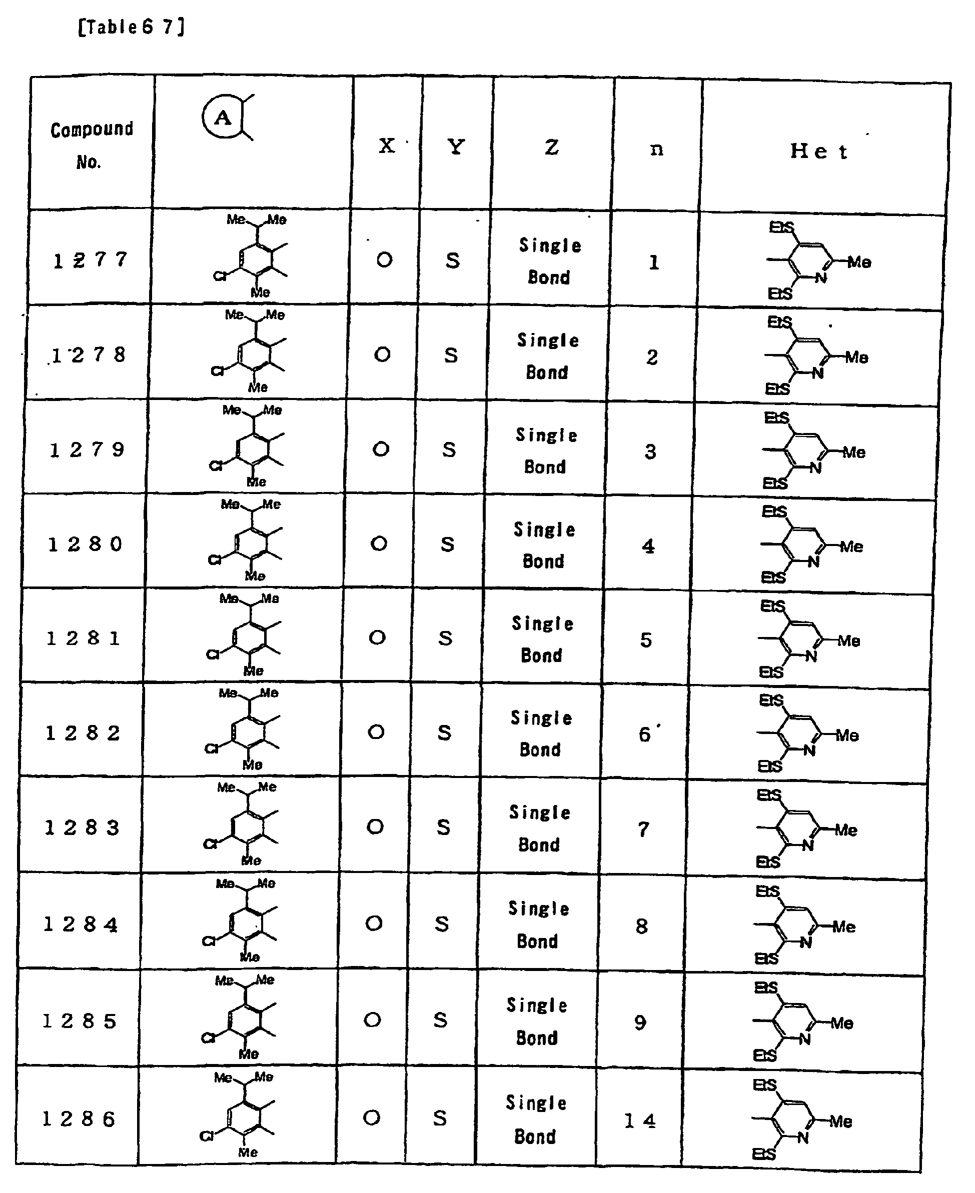

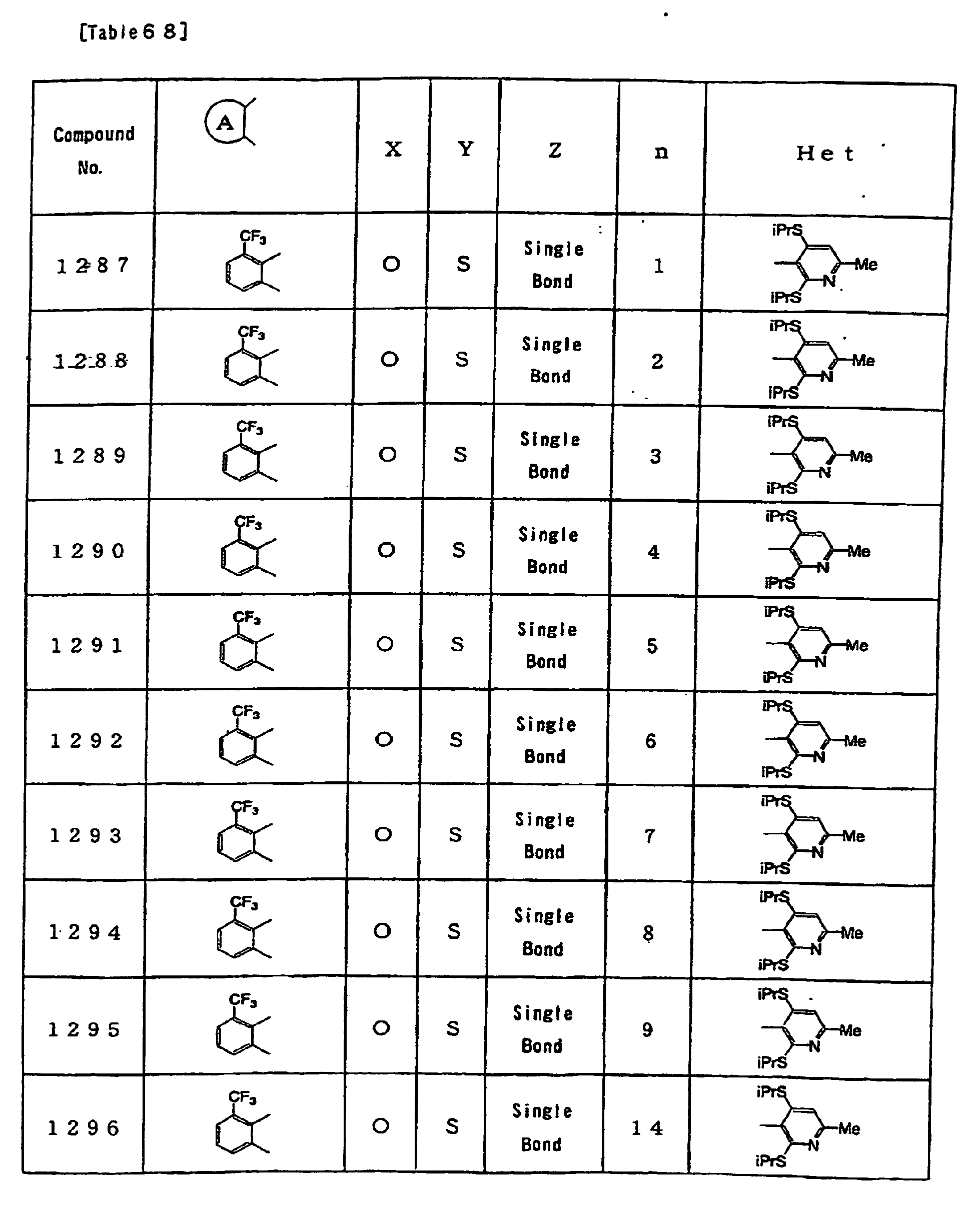

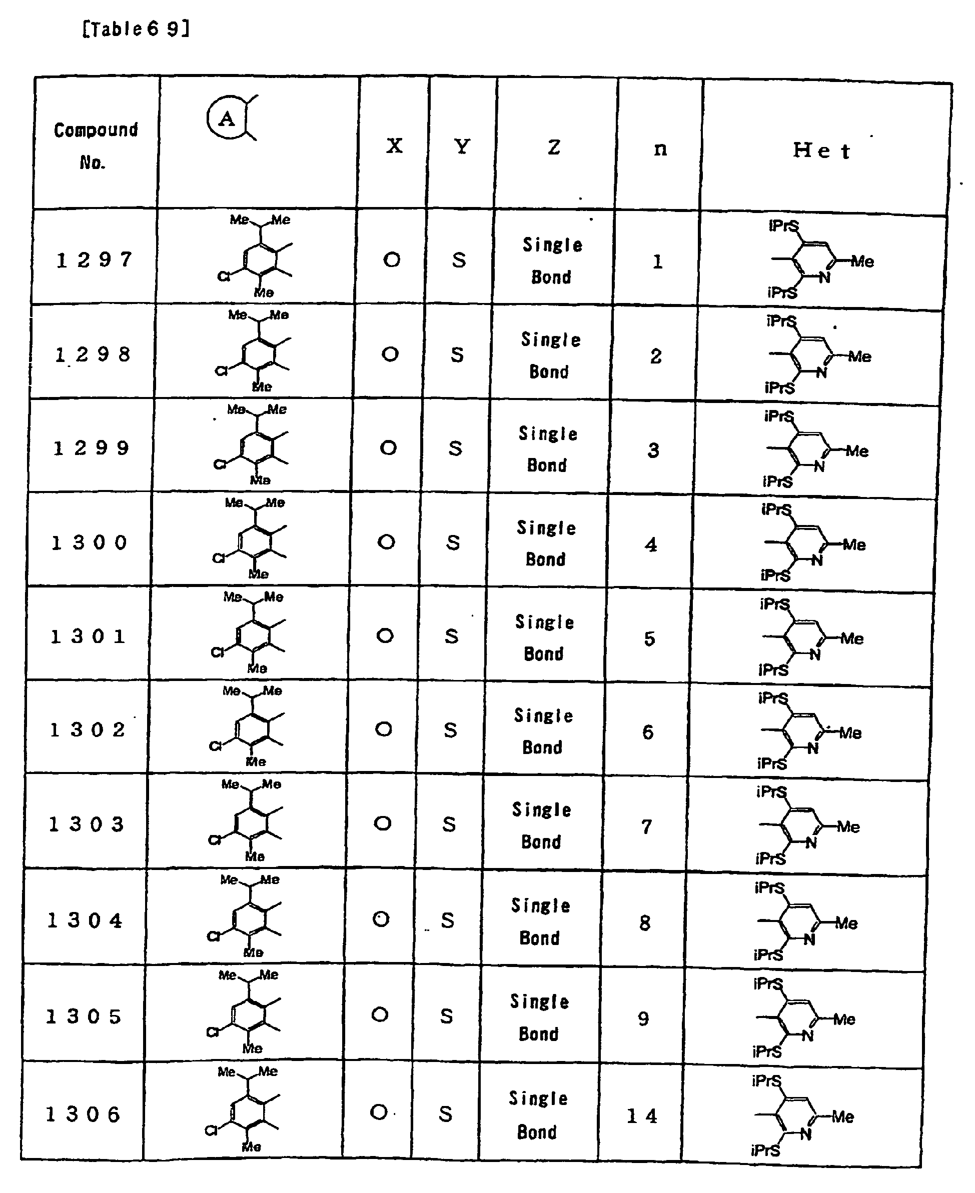

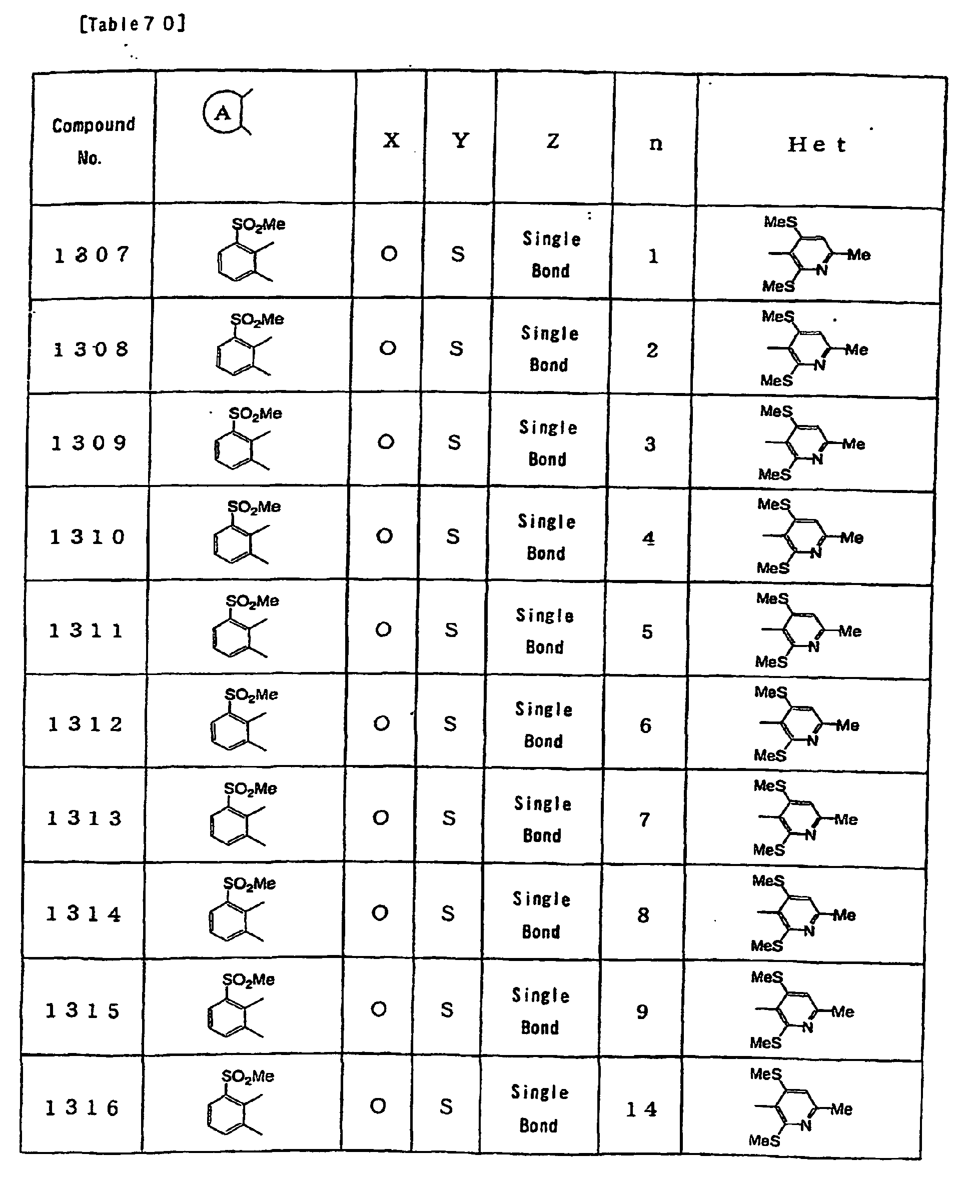

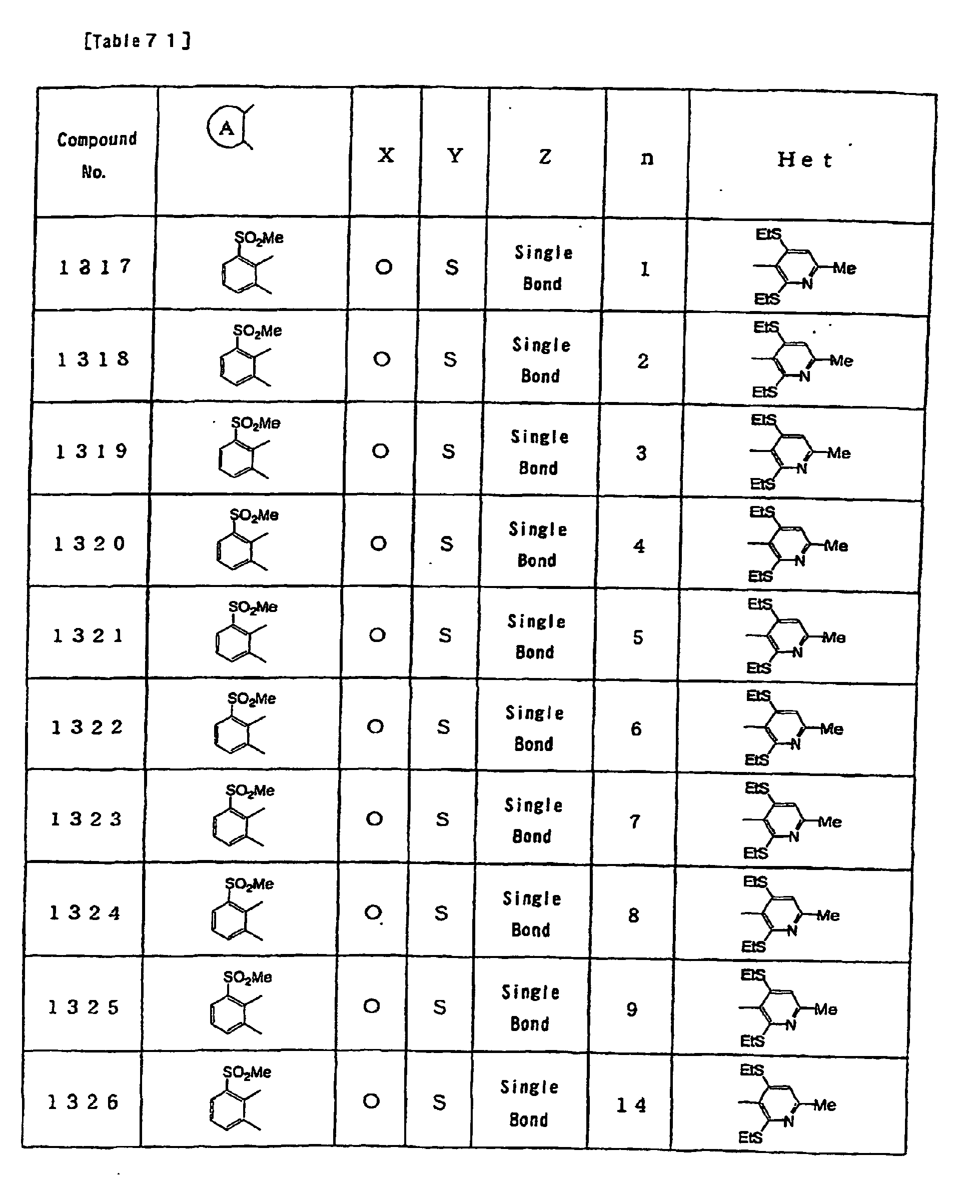

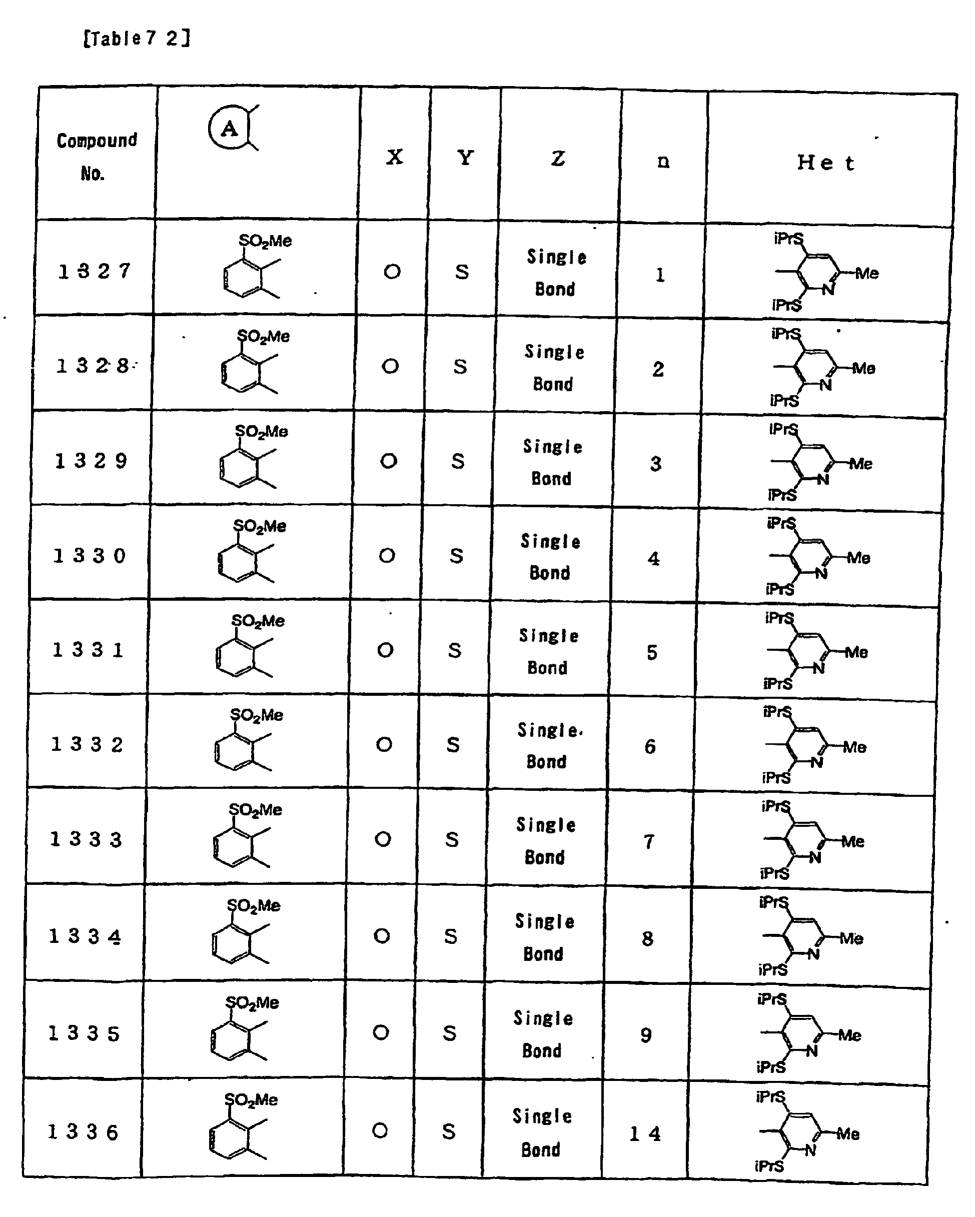

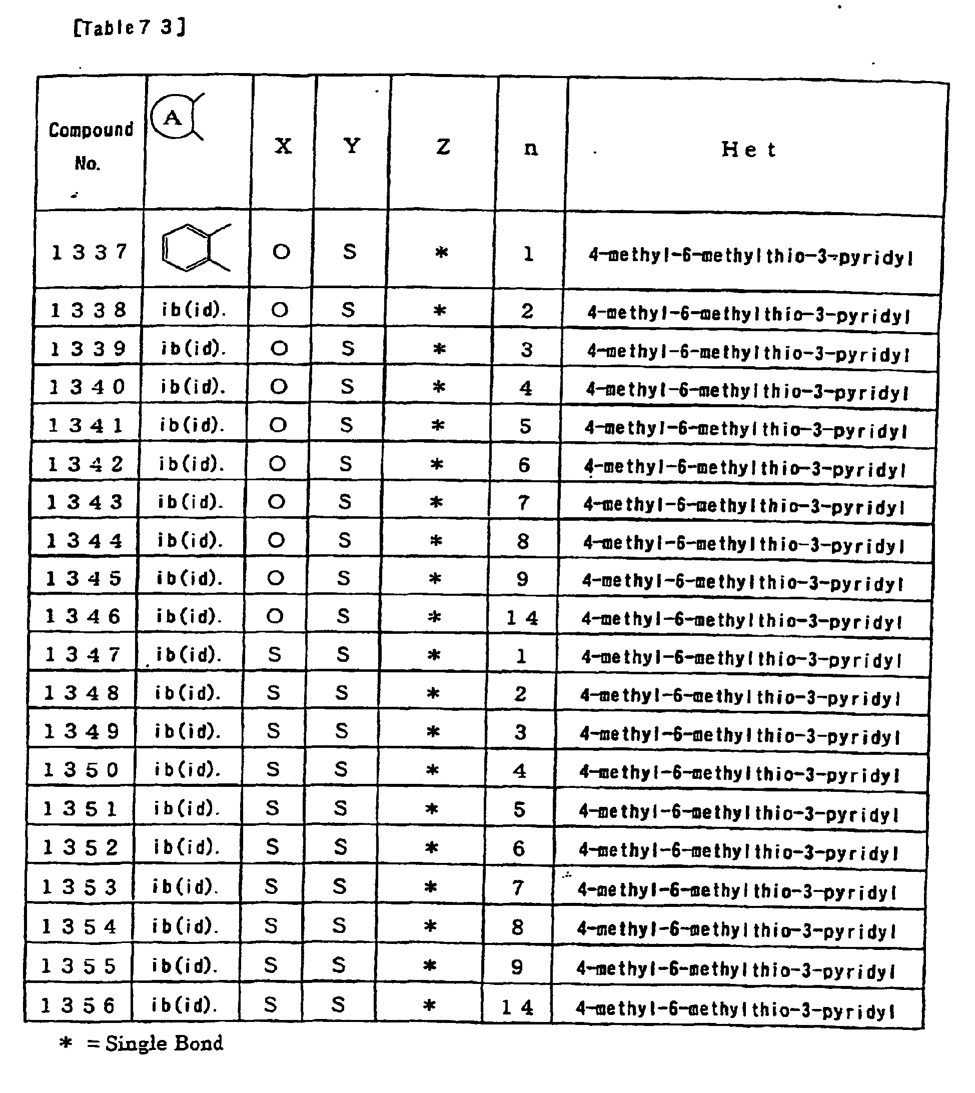

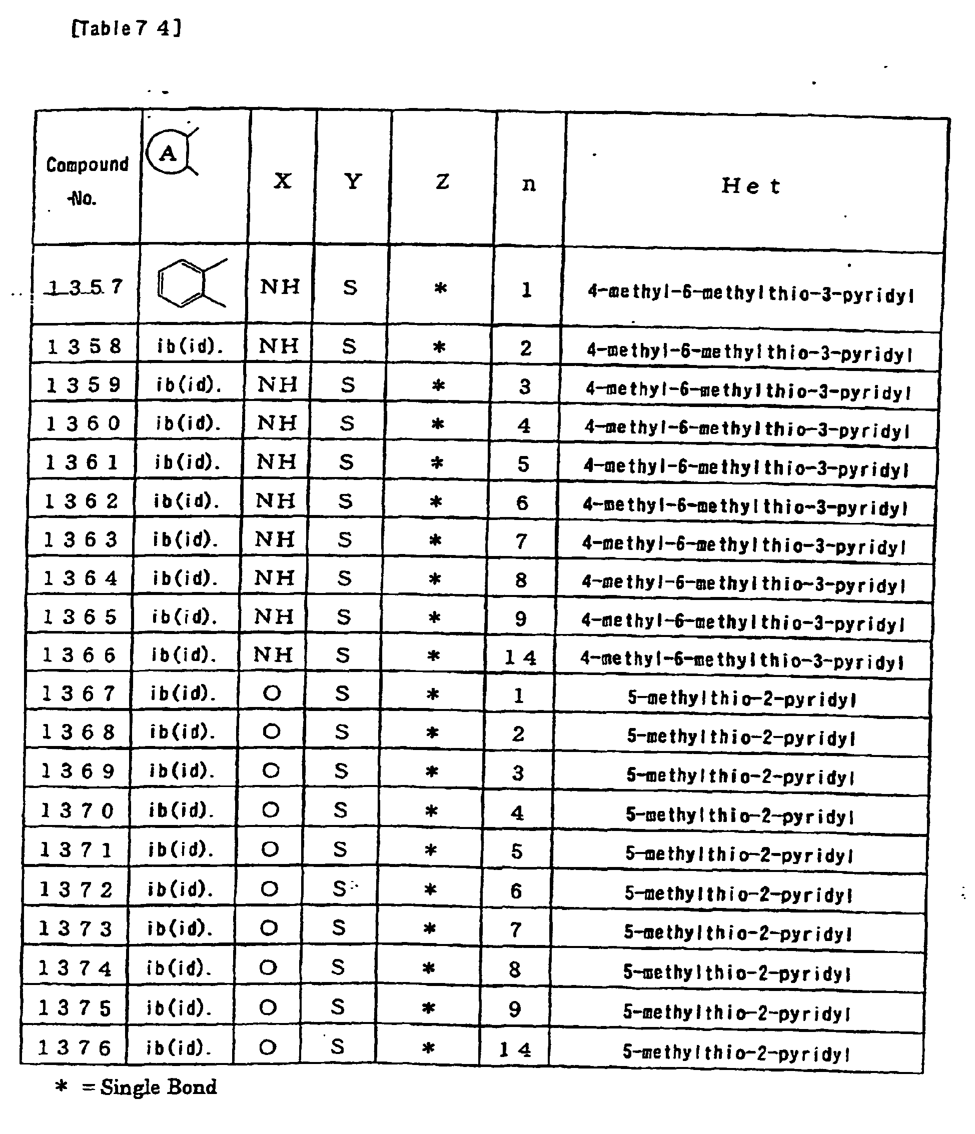

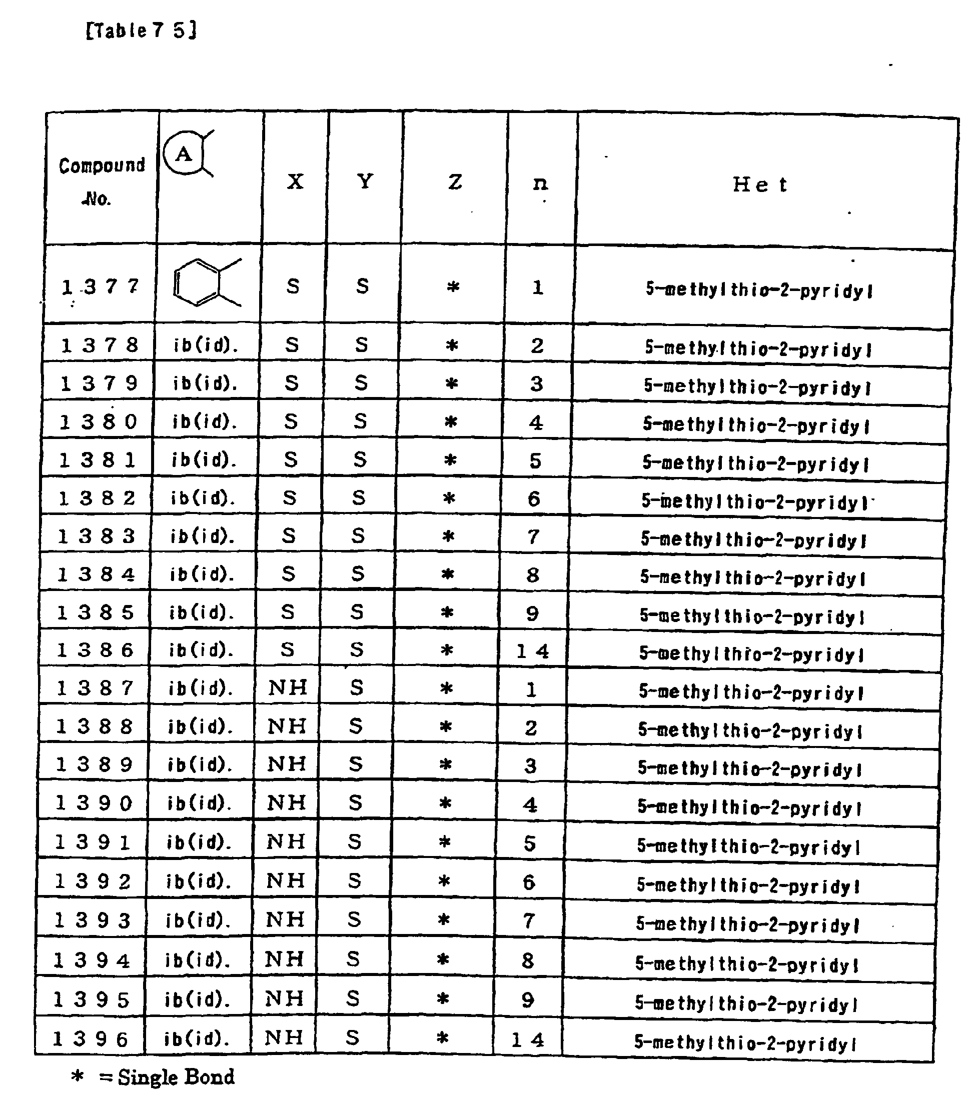

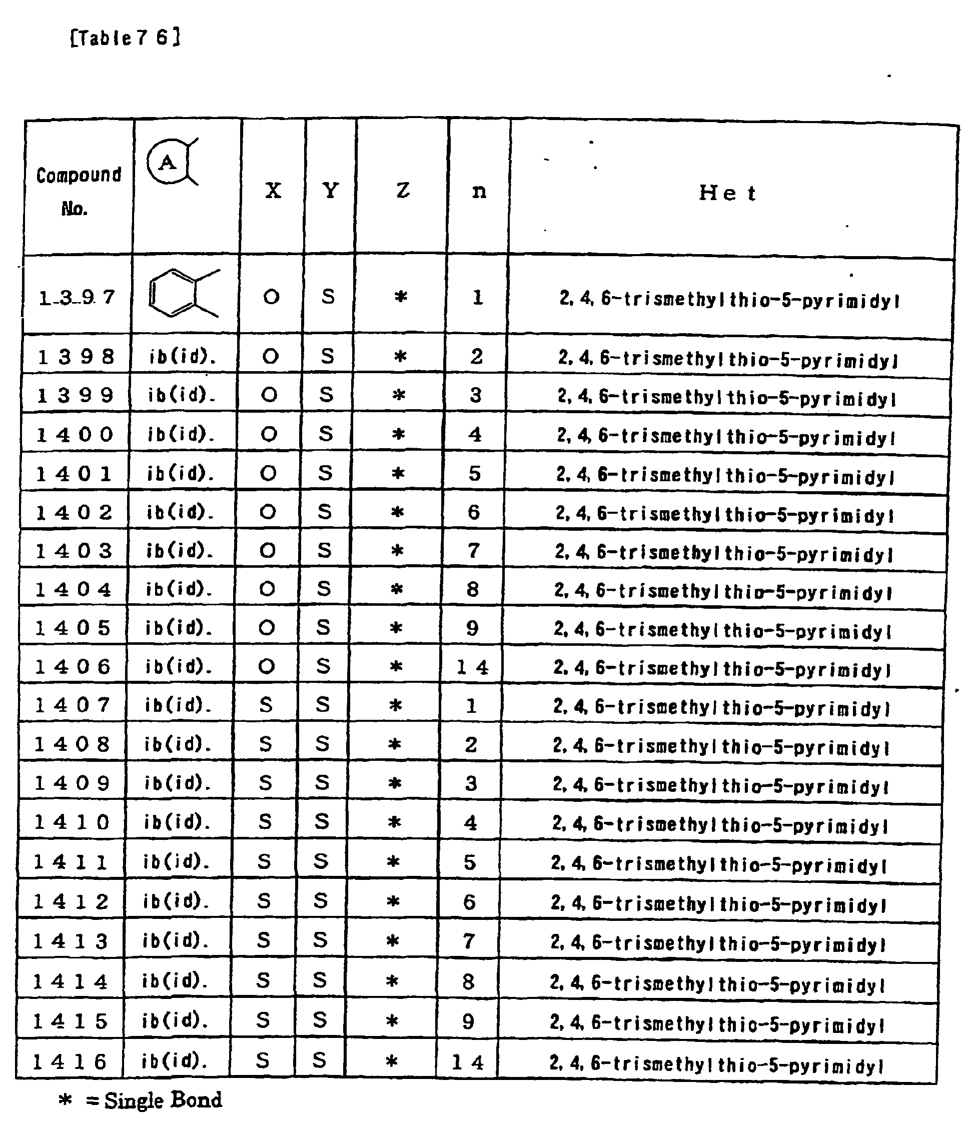

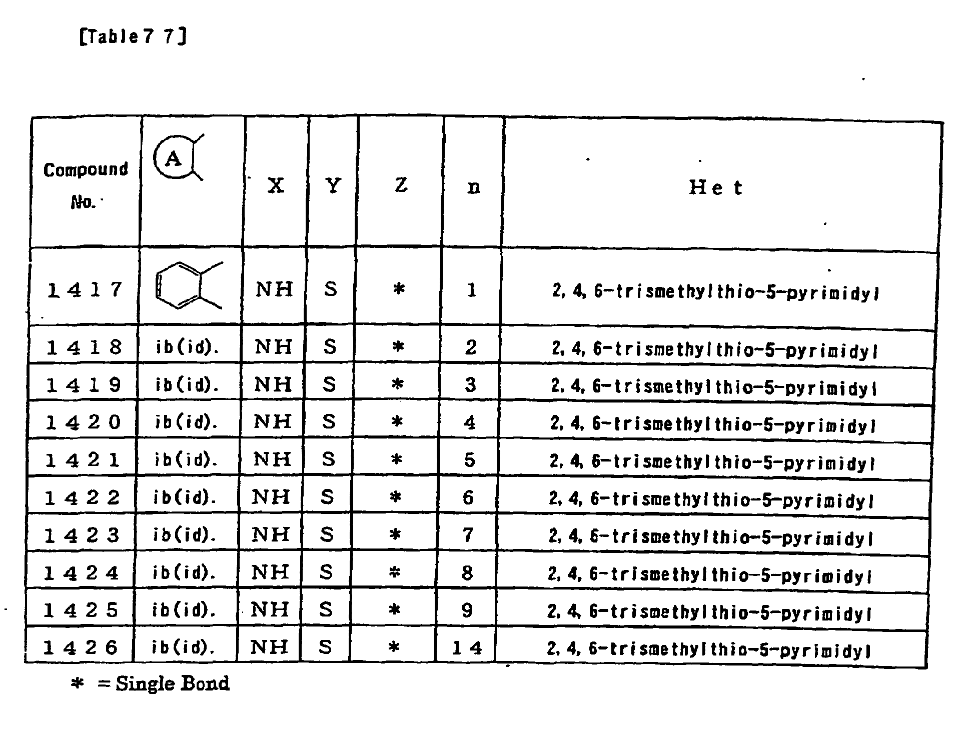

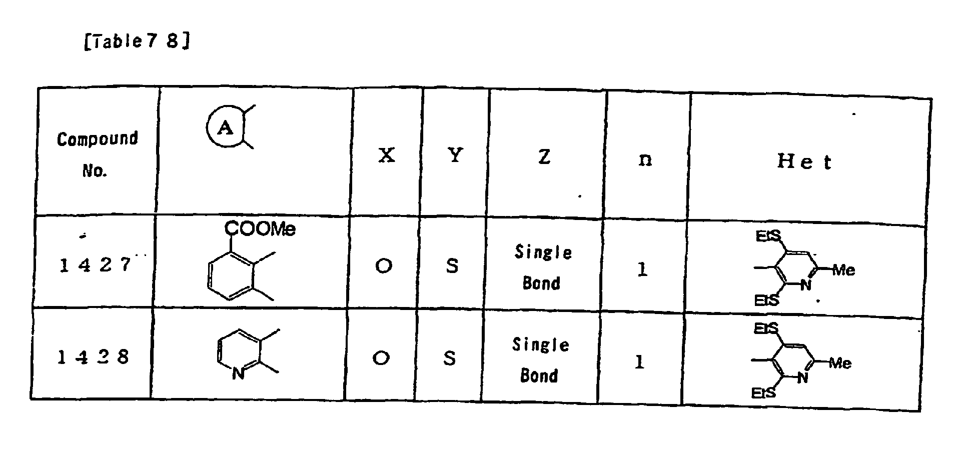

The compounds represented by the formula (I) in the present invention has an ACAT inhibitory activity and/or an intracellular cholesterol transfer inhibitory activity, and is useful in the medical field as medications for treating hyperlipemia or arteriosclerosis. Especially, the compounds of the present invention exhibit an activity of selectively inhibiting an ACAT enzyme which is present in the blood vessel wall. Accordingly, it is expected to have a less side effect than a non-selective ACAT inhibitor, and is preferable as an active ingredient of a drug.

The pharmaceutical composition of the present invention contains the compounds represented by the formula (I) or acid addition salts or solvates thereof as active ingredients. It comprises at least one type of the active ingredients in a therapeutically effective amount, and a pharmaceutically acceptable carrier.

The pharmaceutical composition of the present invention contains the compounds represented by the formula (I), or the acid addition salts or the solvates thereof as active ingredients. At least one type of the active ingredients is used singly, or can be shaped into an administrable preparation such as a tablet, a capsule, a granule, a powder, an injection or a suppository using a pharmaceutically acceptable carrier well-known to those skilled in the art, such as a excipient, a binder, a support or a diluent. These preparations can be produced by a known method. For example, an orally administrable preparation can be produced by mixing the compound represented by the formula (I) with an excipient such as starch, mannitol or lactose, a binder such as carboxymethylcellulose sodium or hydroxypropyl cellulose, a disintegrant such as crystalline cellulose or carboxymethyl cellulose calcium, a lubricant such as talc or magnesium stearate, and a fluidity improving agent such as light silicic anhydride, which are combined as required.

The pharmaceutical composition of the present invention can be administered either orally or parenterally.

The dose of the pharmaceutical composition of the present invention varies depending on the weight, the age, the sex, the progression of disease and the like of patients. Generally, it is preferably administered to an adult person at a dose of from 1 to 100 mg, preferably from 5 to 200 mg a day, from one to three times a day.

The ACAT inhibitory activity of the compounds represented by the formula (I) in the present invention was tested in the following Experiment Examples.

### Experiment Example 1 (ACAT inhibitory activity)

A microsome was prepared from the breast aorta of a rabbit which had been fed with 1% cholesterol food for 8 weeks in a usual manner, and suspended in a 0.15 M phosphate buffer solution (pH 7.4) to form an enzyme solution. An enzyme solution derived from the small intestine was prepared from the small intestine of a rabbit that had eaten a normal food.

The ACAT inhibitory activity was measured by modifying the method of J. G. Heider (J. Lipid Res., 24, 1127 - 1134, 1983). That is, 2 µl of a test compound dissolved in dimethyl sulfoxide (DMSO) were added to 88 µl of a 0.15 M phosphate buffer solution (pH 7,4 containing ¹⁴C-Oleoyl-CoA (40 µM , 60,000 dpm) and bovine serum albumin (2.4 mg/ml), and the mixture was incubated at 37 °C for 5 minutes.

To this solution were added 10 µl of the enzyme solution, and the mixture was reacted at 37°C for 5 minutes (for 3 minutes in the case of the small intestine). Then, 3 ml of a chloroform/methanol (2/1) mixture and 0.5 ml of 0.04 N hydrochloric acid were added thereto to stop the reaction. The lipid was then extracted. The solvent layer was concentrated to dryness, and dissolved in hexane. The solution was spotted on a TLC plate (supplied by Merck Co.). The elution was conducted with a hexane:ether:acetic acid (75:25:1) mixture.

The radioactivity of the resulting cholesterol ester fraction was measured using BAS 2000 (supplied by Fuji Photo Film Co., Ltd.). An IC₅₀ value was obtained from the calculation in contrast with a control containing only DMSO. The results are shown in Table 79.

**[Table 79]**

| Test Compound No. | Enzyme from A‡ IC₅₀ (µM) | Enzyme from B‡ IC₅₀ (*µ*M) | IC₅₀ (B‡) / IC₅₀ (A‡) |
|---|---|---|---|
| 795 | 0.028 | 0.016 | 0.6 |
| 811 | 0.014 | 0.38 | 27.1 |
| 815 | 0.014 | 0.017 | 1.2 |
| 818 | 0.0056 | 0.016 | 2.9 |
| 831 | 0.63 | 0.61 | 1.0 |
| Control 1 | 0.45 | 0.87 | 1.9 |
| Control 2 | 0.047 | 0.13 | 2.8 |
| Control 3 | 0.034 | 0.056 | 1.7 |
| Control 4 | 0.026 | 0.037 | 1.4 |
| Control 5 | 0.01 | 0.065 | 6.5 |
| Control 16 | 0.11 | 0.51 | 4.6 |

| | | | |
|---|---|---|---|
| A‡: the blood vessel wall | | | |
| B‡: the small intestine | | | |

### Experiment Example 2

### (ACAT inhibitory activity (anti-foamation activity) in J744 cells and HepG2 cells) .

J774 cells or HepG2 cells were spread on a 24-well plate. The cells were incubated in a 5% CO₂ incubator at 37°C for 24 hours using DMEM in the case of the J774 cells and a MEM culture solution in the case of the HepG2 cells (both containing 10% fetal calf serum).

The medium was replaced with 0.5 ml of each culture solution containing 10 µg/ml of 25-OH cholesterol and a test piece, and the cells were further incubated for 18 hours.

The medium was removed, and the residue was washed twice with PBS, then extracted with 1.5 ml of a hexane:isopropanol (3:2) mixture, and concentrated to dryness. The extract was dissolved in 0.2 ml of isopropanol containing 10% Triton X-100. Total cholesterol (TC) and free cholesterol (FC) were measured using Cholesterol E Test Wako (supplied by Wako Pure Chemical Industries, Ltd.) and Free Cholesterol E Test Wako (supplied by Wako Pure Chemical Industries, Ltd.).

The cell extract residue was solubilized in 0.25 ml of 2N NaOH at 37°C for 30 minutes, and the protein amount was measured using BCA Protein Assay Reagent (Pierce).

The amount of cholesterol based on the protein was calculated from the difference between TC and FC, and an IC₅₀ value was obtained from the calculation in contrast with the control. The results are shown in Table 80.

**[Table 80]**

| Test Compound No. | Enzyme (J774) IC₅₀ (*µ*M) | Enzyme (HepG2) IC₅₀ (*µ*M) | IC₅₀ (HepG2) / IC₅₀ (J774) |
|---|---|---|---|
| 795 | 0.050 | 0.35 | 7.0 |
| 797 | 0.0036 | 0.029 | 8.1 |
| 811 | 0.050 | 1.8 | 36.0 |
| 815 | 0.12 | 2.6 | 21.7 |
| 818 | 0.062 | 0.063 | 1.0 |
| 831 | 0.057 | 5.4 | 94.7 |
| 1253 | 0.0041 | 0.0044 | 1.1 |
| 1282 | 0.0032 | 0.0062 | 1.9 |
| 1292 | 0.0027 | 0.030 | 11.1 |
| 1294 | 0.0042 | 0.0024 | 0.6 |
| 1302 | 0.0021 | 0.015 | 7.1 |
| Control 1 | 0.56 | 5.3 | 9.5 |
| Control 2 | 0.58 | 1.1 | 1.9 |
| Control 3 | 0.32 | 1.3 | 4.3 |
| Control 4 | 0.12 | 0.75 | 6.3 |
| Control 5 | 1.9 | 1.6 | 0.8 |
| Control 6 | 0.28 | 9.1 | 32.8 |

As control compounds, the following control compounds (1) to (6) were subjected to the same test, and the results are also shown in Tables 64 and 65. Control Compounds (1) to (6) are as follows.

### Control compound (1):

### 5-[2-(2-(4-fluorophenyl)ethyl)-3-(1-methyl-1H-imidazol-2--yl)-2H-1-benzopyran-6-yl]oxy-2,2-dimethyl-N-(2,6-diisopropylphenyl)pentanamide (WO 92/09582)

### Control compound (2):

### (+)-(S)-2-[5-(3,5-dimethylpyrazol-1-yl)pentasulfinyl]-4,5-diphenylimidazole (EP 523941)

### Control compound (3):

### N-(2,2,5,5-tetramethyl-1,3-dioxan-4-ylcarbonyl)-β-alanine 2 (S)-[N'-(2,2-dimethylpropyl)-N'-nonylureido]-1(S)-cyclohexyl ester (EP 421441)

### Control compound (4):

### [5-(4,5-diphenyl-1H-imidazol-2-ylthio)pentyl]-N-heptyl-2-benzoxazolamie (WO 93/23392)

### Control compound (5):

### 6-(benzoxazol-2-ylthio)-N-(2,6-diisopropylphenyl)hexanamide (compound of Japanese Patent Application No. 88,660/1997)

### Contol compound (6):

### 2-[4-[2-(benzimidazol-2-ylthio)ethyl]piperazin-1-yl]-N-(2,6-diisopropylphenyl)acetamide (compound of Japanese Patent Application No. 149,892/1997)

### Examples

The present invention is illustrated more specifically by referring to the following Examples. However, the present invention is not limited to these Examples.

### Example 1 (Compound No. 5 in Table)

### Production of 6-(benzoxazol-2-ylthio)-N-(2-methylthio-3-pyridyl)hexanamide:

A methanol (50 ml) solution of 2-chloro-3-nitropyridine (4.30 g, 27.1 mmol) was added dropwise to a methanol (30 ml) solution of sodium thiomethoxide (2.10 g, 28.5 mmol) while being cooled with ice, and the mixed solution was stirred for 17 hours. Water was then added to the reaction mixture, and the resulting mixture was extracted with ethyl acetate. The organic layer was washed with a saturated aqueous solution of sodium chloride, and dried over sodium sulfate. Subsequently, the solvent was distilled off, and the resulting crystals were recrystallized from a mixture of an ethyl acetate-hexane mixture to obtain 2.93 g (yield 64%) of 2-methylthio-3-nitropyridine as a yellow needle crystal.

This nitropyridine (851 mg, 5.0 mmol) was dissolved in a mixed solvent of acetic acid (35 ml) and conc. hydrochloric acid (1.4 ml), and zinc (3.92 g, 60 mmol) was added thereto in small portions while being cooled with ice. After the mixture was stirred for 30 minutes, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Subsequently, the solvent was distilled off to obtain 600 mg (yield 86%) of 3-amino-2-methylthiopyridine as a pale yellow oil.

Triethylamine (520 mg, 5.14 mmol) was added to a THF (7 ml) solution of this aminopyridine (600 mg, 4.28 mmol). Subsequently, 6-bromohexanoyl chloride (1.10 g, 5.14 mmol) was slowly added dropwise thereto while being cooled with ice, and the mixture was stirred at room temperature for 3 hours. The reaction mixture was diluted with water, and extracted with ethyl acetate. The organic layer was washed with water and then with a saturated aqueous solution of sodium chloride, and dried over sodium sulfate. Thereafter, the solvent was distilled off, and the resulting crude product was purified through silica gel chromatography (silica gel 125 g, eluent - hexane : ethyl acetate = 6:1 **→** 3:1 → 2:1) to obtain 1.08 g (yield 79%) of 6-bromo-N-(2-methylthio-3-pyridyl)hexanamide as a colorless needle crystal (melting point: 66 to 67°C).

To a DMF (2 ml) solution of this amide (159 mg, 0.5 mmol) and 2-mercaptobenzoxazole (83 mg, 0.55 mmol) were added 18-crown-6 (13 mg, 0.05 mmol) and potassium carbonate (83 mg, 0.6 mmol), and the mixture was stirred at 80°C for 3 hours. The reaction mixture was diluted with water, and extracted with ethyl acetate. The organic layer was washed with water and then with a saturated aqueous solution of sodium chloride, and dried over sodium sulfate. Subsequently, the solvent was distilled off, and the resulting crude product was purified through silica gel chromatography (silica gel 20 g, eluent - hexane : ethyl acetate = 5 : 2 → 2:1) to obtain 156 g (yield 81%) of a desired compound as a colorless needle crystal.
Melting point : 127 - 128°C
IR (KBr) cm⁻¹ : 3447, 3265, 1654, 1522, 1508.
¹H-NMR (CDCl₃) δ :
1.58 - 1.65 (2H, m), 1.83 (2H, quint, J = 7.4 Hz), 1.92 (2H, quint, J = 7.4 Hz), 2.46 (2H, t, J = 7.4 Hz), 2.62 (3H, s), 3.34 (2H, t, J = 7.4 Hz),
7.06 (1H, dd, J = 8.1, 4.6 Hz), 7.21 - 7.30 (3H, m), 7.44 (1H, m), 7.59 (1H, m), 8.26 (1H, d, J = 4.6 Hz), 8.28 (1H. d, J = 8.1 Hz).
EIMS m/z (relative intensity) ; 387 (M⁺), 165 (100).

| Elemental analysis: as C₁₉H₂₁N₃O₂S₂ | | | | |
|---|---|---|---|---|
| calculated: | C, 58.89; | H, 5.46; | N, 10.84; | S, 16.55. |
| found: | C, 58.92; | H, 5.43; | N, 10.78: | S, 16.55. |

### Example 2 (Compound No. 8 in Table)

### Production of 9-(benzoxazol-2-ylthio)-N-(2-methylthio-3-pyridyl) nonanamide:

The reaction and the treatment were conducted in the same manner as in Example 1 except that 9-bromononanoyl chloride was used instead of 6-bromohexanoyl chloride to obtain 9-bromo-N-(2-methylthio-3-pyridyl)nonanamide.

To a DMF (5 ml) solution of this amide (90 mg, 0.25 mmol) and 2-mercaptobenzoxazole (38 mg, 0.25 mmol) were added potassium carbonate (42 mg, 0.30 mmol) and 18-crown-6 (7 mg, 0.03 mmol), and the mixture was stirred at 80°C for 3 hours. The reaction mixture was allowed to cool, and then extracted with ethyl acetate. The organic layer was washed with water and then with a saturated aqueous solution of sodium chloride, and dried over sodium sulfate. Subsequently, the solvent was distilled off, and the resulting residue was recrystallized from a mixture of ethyl acetate-hexane to obtain 83 mg (yield 77%) of the desired compound as a colorless powdery crystal.
Melting point: 84 - 85°C
IR (KBr) cm⁻¹ :3465, 3276, 2926, 1664, 1505.
¹H-NMR (CDCl₃) δ :
1.35 - 1.53 (8H, m), 1.72 - 1.77 (2H, m), 1.80 - 1.87 (2H, m), 2.42 (2H, t, J = 7.3 Hz), 2.63 (3H, s), 3.31 (2H, t, J = 7.4 Hz), 7.06 (1H, dd, J = 8.0 , 4.7 Hz), 7.21 - 7.30 (3H, m). 7.43 (1H, dd, J = 7.0 , 0.6 Hz), 7.59 (1H, dd, J = 7.6 , 0.6 Hz), 8.25 (1H, d, J = 4.7 Hz), 8.31 (1H, d, J = 7.8 Hz).
EIMS m/z (relative intensity) : 429 (M⁺), 297 (100).

| Elemental analysis: as C₂₂H₂₇N₃O₂S₂ | | | | |
|---|---|---|---|---|
| calculated: | C, 61.51; | H, 6.33; | N, 9.78; | S, 14.93. |
| found: | C, 61.51; | H, 6.28; | N, 9.64; | S, 14.99. |

### Example 3 (Compound No. 15 in Table) .

### Production of 6-(benzothiazol-2-ylthio)-N-(2-methylthio-3-pyridyl)hexanamide:

The reaction and the treatment were conducted in the same manner as in Example 1 except that 2-mercaptobenzothiazole was used instead of 2-mercaptobenzoxazole to obtain the desired compound as a colorless needle crystal.
Melting point: 118 - 119°C
IR (KBr) cm⁻¹ : 3429, 3265, 1654, 1522, 1508.
¹H-NMR (CDCl₃) δ :
1.57 - 1.65 (2H, m), 1.83 (2H, quint, J = 7.4 Hz), 1.91 (2H, quint, J = 7.4 Hz), 2.46 (2H, t, J = 7.4 Hz), 2.61 (3H, s), 3.38 (2H, t, J = 7.4 Hz),
7.06 (1H, dd, J = 8.1, 4.9 Hz), 7.25 (1H, br s), 7.29 (1H, m), 7.41 (1H, m), 7.75 (1H, m), 7.86 (1H, m), 8.25 (1H, d, J = 4.9 Hz), 8.29 (1H, d, J = 8.1 Hz).
EIMS m/z (relative intensity): 403 (M⁺), 223 (100).

| Elemental analysis: as C₁₉H₂₁N₃OS₃ | | | | |
|---|---|---|---|---|
| calculated: | C, 56.55; | H, 5.24; | N, 10.41; | S, 23.83. |
| found: | C, 56.69; | H, 5.30; | N, 10.24; | S, 23.77. |

### Example 4 (Compound No. 18 in Table)

### Production of 9-(benzothiazol-2-ylthio)-N-(2-methylthio-3-pyridyl)nonanamide:

The reaction and the treatment were conducted in the same manner as in Example 2 except that 2-mercaptobenzothiazole was used instead of 2-mercaptobenzoxazole to obtain the desired compound as a colorless needle crystal.
Melting point: 107 - 108°C
IR (KBr) cm⁻¹ : 3448, 3256, 2923, 1656, 1525.
¹H-NMR (d6-DMSO) δ :
1.24 - 1.34 (6H, m), 1.36 - 1.43 (2H, m), 1.54 - 1.59 (2H, m), 1.69 - 1.77 (2H, m), 2.26 (2H, t, J = 7.4 Hz), 2.40 (3H, s), 3.28 (2H, t, J = 7.2 Hz), 7.01 (1H, dd, J = 7.8, 4.6 Hz), 7.26 (1H, dt, J = 8.1, 1.2 Hz), 7.36 (1H, dt, J = 7.3, 1.2 Hz), 7.58 (1H, dd, J = 7.8, 1.5 Hz), 7.74 (1H, d, J = 8.1 Hz), 7.85 (1H, dd, J = 7.3, 1.2 Hz), 8.21 (1H, dd, J = 4.6, 1.5 Hz), 8.73 (1H, br s).
EIMS m/z (relative intensity): 445 (M⁺), 297 (100).

| Elemental analysis: as C₂₂H₂₇N₃OS₃ | | | | |
|---|---|---|---|---|
| calculated: | C, 59.29; | H, 6.11: | N, 9.43; | S, 21.58. |
| found: | C, 59.12; | H, 6.02: | N, 9.25; | S, 21.62. |

### Example 5 (Compound No. 25 in Table)

### Production of 6-(benzimidazol-2-ylthio)-N-(2-methylthio-3-pyridyl)hexanamide:

The reaction and the treatment were conducted in the same manner as in Example 1 except that 2-mercaptobenzimidazole was used instead of 2-mercaptobenzoxazole to obtain the desired compound as a pale yellow needle crystal.
Melting point: 121 - 123°C
IR (KBr) cm⁻¹ : 3386, 3276, 1658, 1511, 1398.
¹H-NMR (CDCl₃) δ :
1.52 - 1.60 (2H, m), 1.74 - 1.86 (4H, m), 2.42 (2H, t, J = 7.2 Hz), 2.60 (3H, s), 3.32 (2H, t, J = 7.2 Hz), 7.05 (1H, dd, J = 8.1, 4.9 Hz), 7.18 - 7.19 (2H, m), 7.32 (1H, br s), 7.36 (1H, br s), 7.66 (1H, br s), 8.23 - 8.26 (2H, m), 9.84 (1H, br s).
EIMS m/z (relative intensity): 386 (M⁺), 205 (100).

| Elemental analysis: as C₁₉H₂₂N₄OS₂ | | | | |
|---|---|---|---|---|
| calculated: | C, 59.04; | H, 5.74: | N, 14.49; | S, 16.59. |
| found: | C, 59.06; | H, 5.76: | N, 14.35: | S, 16.57. |

### Example 6 (Compound No. 28 in Table)

### Production of 9-(benzimidazol-2-ylthio)-N-(2-methylthio-3-pyridyl)nonanarnide:

The reaction and the treatment were conducted in the same manner as in Example 2 except that 2-mercaptobenzimidazole was used instead of 2-mercaptobenzoxazole to obtain the desired compound as a colorless powdery crystal.
IR (KBr) cm⁻¹ : 3260, 2929, 2851, 1664, 1519, 1394.
¹H-NMR (CDCl₃) δ :
1.31 - 1.47 (6H, m), 1.57 - 1.61 (2H, m), 1.69 - 1.79 (4H, m), 2.42 (2H, t, J = 7.2 Hz), 2.63 (3H, s), 3.32 (2H, t, J = 7.4 Hz), 7.06 (1H, dd, J = 8.1 , 4.6 Hz), 7.18 - 7.23 (4H, m), 7.67 (1H, br s), 8.26 (1H, d, J = 4.6 Hz), 8.30 (1H, d, J = 7.8 Hz), 9.31 (1H, br s).
EIMS m/z (relative intensity): 428 (M⁺), 164 (100).

### Example 7 (Compound No. 158 in Table)

### Production of 9-(benzoxazol-2-ylthio)-N-(4-methyl-2-methylthio-3-pyridyl)nonanamide:

The reaction and the treatment were conducted in the same manner as in Example 1 except that 2-chloro-4-methyl-3-nitropyridine was used instead of 2-chloro-3-nitropyridine to obtain 4-methyl-2-methylthio-3-nitropyridine. This nitropyridine (474 mg, 2.57 mmol) was dissolved in a mixed solvent of acetic acid (18 ml) and conc. hydrochloric acid (0.7 ml), and zinc (2.02 g, 30.88 mmol) was added thereto in small portions while being cooled with ice. After the mixture was stirred for 30 minutes, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Subsequently, the solvent was distilled off to obtain 307 mg (yield 77%) of 3-amino-4-methyl-2-methylthiopyridine as a colorless crystal.

Triethylamine (302 mg, 2.99 mmol) was added to a chloroform (4 ml) solution of this aminopyridine (307 mg, 1.99 mmol), and a chloroform (4 ml) solution of 9-bromononanyl chloride (2.99 mmol) was then slowly added thereto dropwise while being cooled with ice. The mixture was stirred at room temperature for 3 hours. The reaction mixture was diluted with water, and extracted with ethyl acetate. The organic layer was washed with water and then with a saturated aqueous solution of sodium chloride, and dried over sodium sulfate. Thereafter, the solvent was distilled off, and the resulting crude product was purified through silica gel chromatography (silica gel 125 g, eluent - hexane : ethyl acetate = 3:1 → 2:1) to obtain 261 mg (yield 35%) of 9-bromo-N-(4-methyl-2-methylthio-3-pyridyl)nonanamide as a colorless powdery crystal (melting point: 77 to 78°C). To a DMF (5 ml) solution of this amide (114 mg, 0.31 mmol) and 2-mercaptobenzoxazole (46 mg, 0.31 mmol) were added 18-crown-6 (8 mg, 0.03 mmol) and potassium carbonate (51 mg, 0.37 mmol), and the mixture was stirred at 80°C for 2 hours. The reaction mixture was diluted with water, and then extracted with ethyl acetate. The organic layer was washed with water and then with a saturated aqueous solution of sodium chloride, and dried over sodium sulfate. Subsequently, the solvent was distilled off, and the resulting crude product was purified through preparative thin-layer chromatography (eluent - chloroform : methanol = 20:1) to obtain 89 mg (yield 66%) of the desired compound as a colorless powdery crystal.
Melting point : 91 - 92°C
IR (KBr) cm⁻¹ 3433, 3268, 2924, 1518, 1496.
¹H-NMR (CDCl₃) δ :
1.36 - 1.53 (8H, m), 1.74 - 1.88 (4H, m), 2.21 (3H, s), 2.43 (2H, t, J = 7.6 Hz), 2.53 (3H, s), 3.32 (2H, t, J = 7.3 Hz), 6.63 (1H, br s), 6.90 (1H, d, J = 5.1 Hz), 7.22 - 7.30 (1H, m), 7.43 (1H, dd, J = 7.2 , 1.4 Hz), 7.60 (1H, dd, J = 7.6 , 1.4 Hz). 8.24 (1H. d, J = 4.9 Hz).
EIMS m/z (relative intensity): 443 (M⁺, 100).

| Elemental analysis: as C₂₃H₂₉N₃O₂S₂ | | | | |
|---|---|---|---|---|
| calculated: | C, 62.27; | H, 6.59: | N, 9.47; | S, 14.45. |
| found: | C, 62.34; | H, 6.58: | N, 9.33; | S, 14.44. |

### Example 8 (Compound No. 168 in Table)

### Production of 9-(benzothiazol-2-ylthio)-N-(4-methyl-2-methylthio-3-pyridyl)nonanamide: .

The reaction and the treatment were conducted in the same manner as in Example 7 except that 2-mercaptobenzothiazole was used instead of 2-mercaptobenzoxazole to obtain the desired compound as a colorless powdery crystal.
Melting point: 88 - 90°C
IR (KBr) cm⁻¹ : 3449, 3271, 2925, 1657, 1425, 997.
¹H-NMR (CDCl₃) δ :
1.37 - 1.53 (8H, m), 1.73 - 1.87 (4H, m), 2.21 (3H, s), 2.43 (2H, t, J = 7.6 Hz), 2.53 (3H, s), 3.35 (2H, t, J = 7.3 Hz), 6.62 (1H, br s), 6.90 (1H, d, J = 5.1 Hz), 7.23 - 7.31 (1H, m), 7.39 - 7.43 (1H, m), 7.75 (1H, dd, J = 8.1 , 0.5 Hz), 7.86 (1H, dd, J = 8.1 , 0.5 Hz), 8.24 (1H, d, J = 5.1 Hz).

| Elemental analysis: as C₂₃H₂₉N₃OS₃ | | | |
|---|---|---|---|
| calculated: | C, 60.10; | H, 6.36: | N, 9.14. |
| found: | C, 59.99; | H, 6.36: | N, 9.00. |

### Example 9 (Compound No. 275 in Table)

### Production of 6-(benzoxazol-2-ylthio)-N-[2,6-bis(methylthio)-3-pyridyl]hexanamide:

The reaction and the treatment were conducted in the same manner as in Example 1 except that 2,6-dichloro-3-nitropyridine was used instead of 2-chloro-3-nitropyridine. This nitropyridine (800 mg, 3.70 mmol) was dissolved in a mixed solvent of acetic acid (100 ml) and conc. hydrochloric acid (5.6 ml), and zinc (2.90 g, 44.39 mmol) was added thereto in small portions while being cooled with ice. After the mixture was stirred for 30 minutes, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Subsequently, the solvent was distilled off, and the resulting crude product was purified through silica gel chromatography (eluent : hexane : ethyl acetate = 4:1) to obtain 301 mg (yield 44%) of 3-amino-2,6-bis(methylthio)pyridine as a pale yellow powdery crystal.

Triethylamine (196 mg, 1.94 mmol) was added to a THF (3 ml) solution of this aminopyridine (301 mg, 1.62 mmol), and a THF (1 ml) solution of 6-bromohexanoyl chloride (345 mg, 1.62 mmol) was then slowly added thereto dropwise while being cooled with ice, and the mixture was stirred at 0°C for 3 hours. The reaction mixture was diluted with water, and extracted with ethyl acetate. The organic layer was washed with water and then with a saturated aqueous solution of sodium chloride, and dried over magnesium sulfate. Subsequently, the solvent was distilled off and the resulting crude product was purified through silica gel chromatography (eluent - hexane : ethyl acetate = 4:1) to obtain 453 mg (yield 77%) of 6-bromo-N-[2,6-bis(methylthio)-3-pyridyl]hexanamide as a colorless powdery crystal (melting point: 117 to 119°C). To a DMF (4 ml) solution of this amide (100 mg, 0.28 mmol) and 2-mercaptobenzoxazole (42 mg, 0.28 mmol) were added 18-crown-6 (7 mg, 0.03 mmol) and potassium carbonate (46 mg, 0.33 mmol), and the mixture was stirred at 80°C for 3 hours. The reaction mixture was diluted with water, and then extracted with ethyl acetate. The organic layer was washed with water and then with a saturated aqueous solution of sodium chloride, and dried over magnesium sulfate- Subsequently, the solvent was distilled off, and the resulting crude product was recrystallized from a mixture of ethyl acetate and hexane to obtain 83 mg (yield 70%) of the desired compound as a colorless powdery crystal.
Melting point: 125 - 126°C
IR (KBr) cm⁻¹ : 3436, 3253, 2937, 1653, 1519, 1505.
¹H-NMR (CDCl₃) δ :
1.57 - 1.65 (2H, m), 1.78 - 1.86 (2H, m), 1.88 - 1.95 (2H, m), 2.44 (2H, t, J = 7.4 Hz), 2.57 (3H, s), 2.62 (3H, s), 3.33 (2H, t, J = 7.3 Hz), 6.93 (1H, d, J = 8.4 Hz),7.02 (1H, br s), 7.21 - 7.30 (2H, m), 7.43 (1H, dd, J = 7.4 , 1.7 Hz), 7.59 (1H, dd, J = 7.4 , 1.7 Hz), 8.01 (1H, d, J = 8.4 Hz),

| Elemental analysis: as C₂₀H₂₃N₃O₂S₃ | | | |
|---|---|---|---|
| calculated: | C, 55.40; | H, 5.35: | N, 9.69. |
| found: | C, 55.53; | H, 5.38: | N, 9.68. |

### Example 10 (Compound No. 455 in Table)

### Production of 6-(benzoxazol-2-ylthio)-N-(6-methyl-2-methylthio-3-pyridyl)hexanamide:

The reaction and the treatment were conducted in the same manner as in Example 1 except that 2-chloro-6-methyl-3-nitropyridine was used instead of 2-chloro-3-nitropyridine to obtain 6-methyl-2-methylthio-3-nitropyrldime. This nitropyridine (921 mg, 5.0 mmol.) was dissolved in a mixed solvent of acetic acid (40 ml) and conc. hydrochloric acid (1.75 ml), and zinc (3.81 g, 60 mmol) was added thereto in small portions while being cooled with ice. After the mixture was stirred for 30 minutes, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Subsequently, the solvent was distilled off to obtain 685 mg (yield 88%) of 3-amino-6-methyl-2-methylthiopyridine as a yellow oil.

Triethylamine (475 mg, 4.7 mmol) was added to a chloroform (10 ml) solution of this aminopyridine (601 mg, 3.9 mmol), and 6-bromohexanoyl chloride (944 mg, 4.29 mmol) was then slowly added thereto dropwise while being cooled with ice, and the mixture was stirred at room temperature for 12 hours. The reaction mixture was diluted with water, and extracted with methylene chloride. The organic layer was washed with water and then with a saturated aqueous solution of sodium chloride, and dried over magnesium sulfate. Subsequently, the solvent was distilled off, and the resulting crude product was purified through silica gel chromatography (silica gel 50 g, eluent - hexane : ethyl acetate = 10:1 → 5:1) to obtain 773 mg (yield 59%) of 6-bromo-N-(6-methyl-2-methylthio-3-pyridyl)hexanamide as a colorless crystal (melting point: 98 to 99°C). To a DMF (2 ml) solution of this amide (133 mg, 0.4 mmol) and 2-mercaptobenzoxazole (67 mg, 0.44 mmol) were added 18-crown-6 (11 mg, 0.04 mmol) and potassium carbonate (67 mg, 0.44 mmol), and the mixture was stirred at 80°C for 90 minutes. The reaction mixture was diluted with water, and extracted with ethyl acetate. The organic layer was washed with water and then with a saturated aqueous solution of sodium chloride, and dried over sodium sulfate. Subsequently, the solvent was distilled off, and the resulting crude product was purified through silica gel chromatography (silica gel 20 g, eluent - hexane : acetone = 5:1 → 5:3) to obtain 125 mg (yield 78%) of the desired compound as a colorless needle crystal.
Melting point: 140 - 141°C
IR (KBr) cm⁻¹ : 3437, 3267, 1654, 1528, 1506.
¹H-NMR (CDCl₃) δ :
1.57- 1.65 (2H, m), 1.82 (2H, quint, J = 7.4 Hz), 1.91 (2H, quint, J = 7.4 Hz), 2.44 (2H, t, J = 7.4 Hz), 2.48 (3H, s), 2.60 (3H, s), 3.33 (2H, t, J = 7.4 Hz), 6.90 (1H, d, J = 8.1 Hz), 7.21 - 7.30 (2H, m), 7.43 (1H, m), 7.59 (1H, m), 8.13 (1H, d, J = 8.1 Hz).
EIMS m/z (relative intensity): 401 (M⁺), 203 (100).

| Elemental analysis: as C₂₀H₂₃N₃O₂S₂ | | | |
|---|---|---|---|
| calculated: | C, 59.82; | H, 5.77: | N, 10.46. |
| found: | C, 59.90; | H, 5.84: | N, 10.32. |

### Example 11 (Compound No. 458 in Table)

### Production of 9-(benzoxazol-2-ylthio)-N-(6-methyl-2-methylthio-3-pyridyl)nonanamide:

Triethylamine (607 mg, 6.0 mmol) was added to a chloroform (10 ml) solution of 3-amino-6-methyl-2-methylthiopyridine (685 mg, 4.44 mmol), and a chloroform (3 ml) solution of 9-bromononanyl chloride (1,281 mg, 5 mmol) was then slowly added thereto dropwise while being cooled with ice. The mixture was stirred at room temperature for 17 hours. The reaction mixture was diluted with water, and then extracted with ethyl acetate. The organic layer was washed with water and then with a saturated aqueous solution of sodium chloride, and dried over sodium sulfate. Subsequently, the solvent was distilled off, and the resulting crude product was purified through silica gel chromatography (silica gel 75 g, eluent - hexane : ethyl acetate =10:1 → 4:1) to obtain 433 mg (yield 27%) of 9-bromo-N-(6-methyl-2-methylthio-3-pyrldyl)nonanamide as a colorless crystal (melting point: 80 to 82°C).

To a DMF (1.5 ml) solution of this amide (131 mg, 0.35 mmol) and 2-mercaptobenzoxazole (58 mg, 0.385 mmol) were added 18-crown-6 (9·mg, 0.035 mmol) and potassium carbonate (58 mg, 0.42 mmol), and the mixture was stirred at 80°C for 3 hours. The reaction mixture was diluted with water, and then extracted with ethyl acetate. The organic layer was washed with water and then with a saturated aqueous solution of sodium chloride, and dried over sodium sulfate. Subsequently, the solvent was distilled off, and the resulting crude product was purified through silica gel chromatography (silica gel 30 g, eluent - hexane : ethyl acetate = 4:1 → 3:1) to obtain 123 mg (yield 79%) of the desired compound as a colorless needle crystal. Melting point: 99 - 100°C
IR (KBr) cm⁻¹ : 3421, 3235, 2924, 1655, 1528, 1497, 1455.
¹H-NMR (CDCl₃) δ :
1.32-1.42 (6H, m), 1.43-1.51 (2H, m), 1.70-1.78 (2H, m), 1.83 (2H, quint, J = 7.4 Hz), 2.40 (2H, t, J = 7.4 Hz), 2.48 (3H, s), 2.61 (3H, s), 3.31 (2H, t, J = 7.4 Hz), 6.90 (1H, d, J = 8.1 Hz), 7.21-7.30 (3H, m), 7.43 (1H, m), 7.60 (1H, m), 8.15 (1H, d, J = 8.1 Hz).
EIMS m/z (relative intensity): 443 (M⁺), 311 (100).

### Example 12 (Compound No. 465 in Table)

### Production of 6-(benzothiazol-2-ylthio)-N-(6-methyl-2-methylthio-3-pyridyl)hexanamide:

The reaction and the treatment were conducted in the same manner as in Example 10 except that 2-mercaptobenzothiazole was used instead of 2-mercaptobenzoxazole to obtain the desired compound as a colorless needle crystal.
Melting point: 122 - 123°C
IR (KBr) cm⁻¹ : 3438, 3290, 1656, 1515, 1431.
¹H-NMR (CDCl₃) δ :
1.57 - 1.65 (2H, m), 1.82 (2H, quint, J = 7.4 Hz), 1.90 (2H, quint, J = 7.4 Hz), 2.44 (2H, t, J = 7.4 Hz), 2.48 (3H, s), 2.60 (3H, s), 3.37 (2H, t, J = 7.4 Hz), 6.90 (1H, d, J = 8.3 Hz), 7.22(1H, br s) 7.29 (1H, m), 7.41 (1H, m), 7.75 (1H, m), 7.86 (1H, m), 8.13 (1H, J = 8.3 Hz).
EIMS m/z (relative intensity): 417 (M⁺), 168 (100).

| Elemental analysis: as C₂₀H₂₃N₃OS₃ | | | | |
|---|---|---|---|---|
| | calculated: | C, 57.52; | H, 5.55: | N, 10.06. |
| | found: | C, 57.65; | H, 5.63: | N, 9.97. |

### Example 13 (Compound No. 468 in Table)

### Production of 9-(benzothiazol-2-ylthio)-N-(6-methyl-2-methylthio-3-pyridyl)nonanamide:

The reaction and the treatment were conducted in the same manner as in Example 11 except that 2-mercaptobenzothiazole was used instead of 2-mercaptobenzoxazole to obtain the desired compound as a colorless needle crystal.
Melting point: 104 - 105°C
IR (KBr) cm⁻¹: 3280, 2924, 1662, 1527, 1428.
¹H-NMR (CDCl₃) δ :
1.32-1.41 (6H, m), 1.43-1.51 (2H, m), 1.70-1.77 (2H, m), 1.82 (2H, quint. J = 7.4 Hz), 2.40 (2H, t, J =7.4 Hz), 2.48 (3H, s), 2.61 (3H, s), 3.34 (2H, t, J = 7.4 Hz), 6.90 (1H, d, J = 8.1 Hz), 7.22 (1H, br s) 7.29 (1H, m), 7.41 (1H, m), 7.76 (1H, m), 7.86 (1H, m), 8.15 (1H, d, J = 8.1 Hz),
EIMS m/z (relative intensity): 459 (M⁺). 293 (100).

| Elemental analysis: as C₂₃H₂₉N₃OS₃ | | | | |
|---|---|---|---|---|
| | calculated: | C, 60.10; | H, 6.36: | N, 9.14. |
| | found: | C, 60.17; | H, 6.40: | N, 9.11. |

### Example 14 (Compound No. 475 in Table)

### Production of 6-(benzimidazol-2-ylthio)-N-(6-methyl-2-methylthio-3-pyridyl)hexanamide:

The reaction and the treatment were conducted in the same manner as in Example 10 except that 2-mercaptobenzimidazole was used instead of 2-mercaptobenzoxazole to obtain the desired compound as a colorless crystal.
Melting point: 138 - 140°C
IR (KBr) cm⁻¹ 3385, 3244, 1668, 1509, 1440.
¹H-NMR (CDCl₃ ) δ:
1.53 - 1.61 (2H, m), 1.78 (2H, quint, J = 7.6 Hz), 1.82 (2H, quint, J = 7.6 Hz), 2.41 (2H, t, J = 7.6 Hz), 2.48 (3H, s). 2.59 (3H, s), 3.31 (2H, t, J = 7.6 Hz), 6.88 (1H, d, J = 8.3 Hz), 7.16 - 7.23 (2H, m), 7.31-7.32 (2H, m), 7.67 (1H, m), 8.08 (1H, d, J = 8.3 Hz), 9.72 (1H, br s).
EIMS m/z (relative intensity): 400 (M⁺), 164 (100).

| Elemental analysis: as C₂₀H₂₄N₄OS₂ | | | | |
|---|---|---|---|---|
| | calculated: | C, 59.97: | H, 6.04: | N, 13.99. |
| | found: | C, 60.08: | H, 6.08: | N, 13.94. |

### Example 15 (Compound No. 478 in Table)

### Production of 9-(benzimidazol-2-ylthio)-N-(6-methyl-2-methylthio-3-pyridyl)nonanamide:

The reaction and the treatment were conducted in the same manner as in Example 11 except that 2-mercaptobenzimidazole was used instead of 2-mercaptobenzoxazole to obtain the desired compound as a colorless needle crystal.
Melting point: 73 - 75°C
IR (KBr) cm⁻¹ : 3254, 2926, 1663, 1515, 1438.
¹H-NMR (CDCl₃) δ :
1.27-1.43 (8H, m), 1.68-1.78 (4H, m), 2.40 (2H, t, J = 7.4 Hz), 2.48 (3H, s), 2.60 (3H, s), 3.31 (2H, t, J = 7.4 Hz), 6.89 (1H, d, J = 8.1 Hz), 7.17-7.20 (2H, m), 7.31-7.33 (2H, m), 7.67 (1H, m), 8.13 (1H, d, J = 8.1 Hz), 9.69 (1H, br s).

### Example 16. (Compound No. 781 in Table)

### Production of 2-(benzoxazol-2-ylthio)-N-[2,4-bis(methylthio)-6-methyl-3-pyridyl]acetamide:

Triethylamine (274 mg, 2.71 mmol) was added to a chloroform (10 ml) solution of 3-amino-2,4-bis(methylthio)-6-methylpyridine (492 mg, 2.46 mmol), and bromoacetyl bromide (521 mg, 2.58 mmol) was then slowly added thereto dropwise while being cooled with ice. The mixture was stirred at room temperature for 2 hours. The reaction mixture was diluted with water, and then extracted with methylene chloride. The organic layer was washed with 1N hydrochloric acid, water, an aqueous solution of sodium hydrogencarbonate, water and a saturated aqueous solution of sodium chloride in this order, and dried over sodium sulfate. Subsequently, the solvent was distilled off, and the resulting crude product was purified through silica gel chromatography (silica gel 25 g, eluent - hexane : acetone = 7:1 → 5:1 → 3:1) to obtain 100 mg (yield 13%) of 2-bromo-N-[2,4-bis(methylthio)-6-methyl-3-pyridyl]acetamide as a colorless crystal (melting point: 171 to 172°C).

Potassium carbonate (46 mg, 0.33 mmol) was added to an acetonitrile (5 ml) solution of this amide (96 mg, 0.3 mmol) and 2-mercaptobenzoxazole (45 mg, 0.3 mmol), and the mixture was stirred at room temperature for 90 minutes. The reaction mixture was diluted with water, and then extracted with ethyl acetate. The organic layer was washed with water and then with a saturated aqueous solution of sodium chloride, and dried over sodium sulfate. Subsequently, the solvent was distilled off, and the resulting crude product was purified through silica gel chromatography (silica gel 10 g, eluent - hexane : acetone = 5:2) to obtain 88 mg (yield 75%) of the desired compound as a colorless crystal.
Melting point: 203 - 205°C
IR (KBr) cm⁻¹ : 3437, 3238, 1669, 1509, 1454,
¹H-NMR (CDCl₃) δ :
2.31 (3H, s), 2.41 (3H, s), 2.46 (3H, s), 4.10 (2H, s), 6.61 (1H, s), 7.28 - 7.33 (2H, m), 7.49 (1H, m), 7.60 (1H, m), 8.77 (1H, br s).
EIMS m/z (relative intensity): 391 (M⁺), 227 (100).

| Elemental analysis: as C₁₇Hl₁₇N₃O₂S₃ | | | | |
|---|---|---|---|---|
| | calculated: | C, 52.15; | H, 4.38; | N, 10.73. |
| | found: | C, 52.14; | H, 4.44; | N, 10.57. |

### Example 17 (Compound No. 783 in Table)

### Production of 4-(benzoxazol-2-ylthio)-N-[2,4-bis(methylthio)-6-methyl-3-pyridyl]butanamide:

Triethylamine (206 mg, 2.04 mmol) was added to a THF (6 ml) solution of 3-amino-2,4-bis(methylthio)-6-methylpyridine (341 mg, 1.70 mmol), and 4-bromobutanoyl chloride (379 mg, 2.04 mmol) was then slowly added thereto dropwise while being cooled with ice. The mixture was stirred at room temperature for 2 hours The reaction mixture was diluted with water, and then extracted with ethyl acetate. The organic layer was washed with water and then with a saturated aqueous solution of sodium chloride, and dried over sodium sulfate. Subsequently, the solvent was distilled off, and the resulting crude product was purified through silica gel chromatography (silica gel 75 g, eluent - hexane : acetone = 5:1 → 3:1) to obtain 390 mg (yield 66%) of 4-bromo-N-[2.4.-bis(methylthio)-6-methyl-3-pyridyl]butanamide as a colorless crystal (melting point: 139 to 140°C).

To a DMF (2 ml) solution of this amide (105 mg, 0.3 mmol) and 2-mercaptobenzoxazole (50 mg, 0.33 mmol) were added 18-crown-6 (8 mg, 0.03 mmol) and potassium carbonate (50 mg, 0.36 mmol), and the mixture was stirred at 80°C for 3 hours. The reaction mixture was diluted with water, and then extracted with ethyl acetate. The organic layer was washed with water and then with a saturated aqueous solution of sodium chloride, and dried over sodium sulfate. Subsequently, the solvent was distilled off, and the resulting crude product was purified through preparative thin-layer chromatography (eluent - hexane : ethyl acetate = 3:2, eluted twice) to obtain 67 mg (yield 53%) of the desired compound as a colorless needle crystal.
Melting point: 149 - 150°C
IR (KBr) cm⁻¹: 3437, 3248, 1667, 1503, 1455.
¹H-NMR (d6-DMSO) δ :
2.13 (2H, quint, J = 7.2 Hz), 2.37 (3H, s), 2.38 (3H, s), 2.44 (3H, s), 2.49 (2H, t, J = 7.2 Hz), 3.43 (2H, t, J = 7.2 Hz), 6.88 (1H, s), 7.30 - 7.37 (2H, m), 7.64 - 7.68 (2H, m), 9.45 (1H, br s).
EIMS m/z (relative intensity): 419 (M⁺, 100).

| Elemental analysis: as C₁₉H₂₁N₃O₂S₃ | | | | |
|---|---|---|---|---|
| | calculated: | C, 54.39; | H, 5.04; | N, 10.01. |
| | found: | C, 54.58; | H, 5.08; | N, 9.98. |

### Example 18 (Compound No. 785 in Table)

### Production of 6-(benzoxazol-2-ylthio)-N-[2,4-bis(methylthio)-6-methyl-3-pyridyl]hexanamide:

The reaction and the treatment were conducted in the same manner as in Example 17 except that 6-bromohexanoyl chloride was used instead of 4-bromobutanoyl chloride to obtain the desired compound as a colorless powdery crystal.
Melting point: 120 - 121°C
IR (KBr) cm⁻¹ : 3433, 3235, 1662, 1502, 1455.
¹H-NMR (d6-DMSO) δ :
1.44 - 1.54 (2H, m), 1.58 - 1.68 (2H, m), 1.72 - 1.82 (2H, m), 2.18 - 2.27 (2H, m), 2.32 (3H, s), 2.34 (3H, s), 2.37 (3H, s), 3.27 (2H, t, J = 7.2 Hz), 6.78 (1H, s), 7.19 - 7.26 (2H, m), 7.47 - 7.53 (2H, m), 8.74 (1H, br s).
EIMS m/z (relative intensity): 446 (M⁺-1), 200 (100).

| Elemental analysis: as C₂₁H₂₅N₃O₂S₃ | | | | | |
|---|---|---|---|---|---|
| | calculated: | C, 56.35; | H, 5.63: | N, 9.39; | S, 21.49. |
| | found: | C, 56.42; | H, 5.62: | N, 9.26; | S, 21.39. |

### Example 19 (Compound No. 788 in Table)

### Production of 9-(benzoxazol-2-ylthio)-N-[2,4-bis(methylthio)-6-methyl-3-pyridyl]nonanamide:

The reaction and the treatment were conducted in the same manner as in Example 17 except that 9-bromononanoyl chloride was used instead of 4-bromobutanoyl chloride to obtain the desired compound as a colorless powdery crystal.
Melting point: 123 - 124°C
IR (KBr) cm⁻¹ : 3461, 3246, 1671, 1504, 1454.
¹H-NMR (d6-DMSO) δ :
1.26 - 1.46 (8H, m), 1.53 - 1.63 (2H, m), 1.72 - 1.83 (2H, m), 2.24 (2H, t, J = 7.3 Hz), 2.37 (3H, s), 2.38 (3H, s), 2.43 (3H, s), 3.31 - 3.41 (2H, m), 6.86 (1H, s), 7.27 - 7.34 (2H, m), 7.58 - 7.66 (2H, m), 9.26 (1H, br s).
EIMS m/z (relative intensity): 489 (M⁺, 100).

| Elemental analysis: as C₂₄H₃₁N₃O₂S₃ | | | | | |
|---|---|---|---|---|---|
| | calculated: | C, 58.86; | H, 6.38: | N, 8.58; | S, 19.64. |
| | found: | C, 58.94; | H, 6.37: | N, 8.44; | S, 19.55. |

### Example 20 (Compound No. 793 in Table)

### Production of 4-(benzothiazol-2-ylthio)-N-[2,4-bis(methylthio)-6-methyl-3-pyridyl]butanamide:

The reaction and the treatment were conducted in the same manner as in Example 17 except that 2-mercaptobenzothiazole was used instead of 2-mercaptobenzoxazole to obtain the desired compound as a colorless crystal.
Melting point: 131 - 133°C
IR (KBr) cm⁻¹ : 3435, 3250, 1665, 1509, 1428.
¹H-NMR (d6-DMSO) δ :
2.11 (2H, quint, J = 7.2 Hz), 2.37 (3H, s), 2.38 (3H, s), 2.44 (3H, s), 2.49 (2H, t, J = 7.2 Hz), 3.46 (2H, t, J = 7.2 Hz), 6.88 (1H, s), 7.37 (1H, m), 7.47 (1H, m), 7.87 (1H, m), 8.02 (1H, m), 9.45 (1H, s).
EIMS m/z (relative intensity): 435 (M⁺), 168 (100).

| Elemental analysis: as C₁₉H₂₁N₃OS₄ | | | |
|---|---|---|---|
| calculated: | C, 52.39; | H, 4.86: | N, 9.65. |
| found: | C, 52.39; | H, 4.84: | N, 9.56. |

### Example 21 (Compound No. 795 in Table)

### Production of 6-(benzothiazol-2-ylthio)-N-[2,4-bis(methylthio)-6-methyl-3-pyridyl]hexanamide:

The reaction and the treatment were conducted in the same manner as in Example 18 except that 2-mercaptobenzothiazole was used instead of 2-mercaptobenzoxazole to obtain the desired compound as a pale yellow crystal.
Melting point: 123 - 125°C
IR (KBr) cm⁻¹: 3433, 3258, 2923, 1661, 1429
¹H-NMR (d6-DMSO) δ :
1.49 - 1.58 (6H, m), 1.67 (2H, quint, J = 7.2 Hz), 1.83 (2H, quint, J = 7.2 Hz), 2.29 (2H, t, J = 7.2 Hz), 2.38 (3H, s), 2.39 (3H, s), 2.45 (3H, s), 3.38 (2H, t, J = 7.2 Hz), 6.68 (1H, s), 7.36 (1H, td, J = 8.0, 1.0 Hz), 7.46 (1H, td, J = 8.0, 1.0 Hz), 7.86 (1H, dd, J = 8.0, 1.0 Hz), 8.01 (1H, br d, J = 8.0 Hz), 9.31 (1H, s).
EIMS m/z (relative intensity): 463 (M⁺), 201 (100).

| Elemental analysis: as C₂₁H₂₅N₃OS₄ | | | | | |
|---|---|---|---|---|---|
| | calculated: | C, 54.40; | H, 5.43: | N, 9.06; | S, 27.66. |
| | found: | C, 54.42; | H, 5.45: | N, 8.79; | S, 27.68. |

### Example 22 (Compound No. 798 in Table)

### Production of 9-(benzothiazol-2-ylthio)-NT-[2,4-bis(methylthio)-6-methyl-3-pyridyl]nonanamide:

The reaction and the treatment were conducted in the same manner as in Example 19 except that 2-mercaptobenzothiazole was used instead of 2-mercaptobenzoxazole to obtain the desired compound as a colorless powdery crystal.
Melting point: 126 - 127°C
IR (KBr) cm⁻¹: 3440, 3252, 2924, 1661, 1430.
¹H-NMR (d6-DMSO) δ :
1.31 - 1.52 (8H, m), 1.59 - 1.68 (2H, m), 1.77 - 1.85 (2H, m), 2.23 - 2.33 (2H, m), 2.40 (3H, s), 2.42 (3H, s), 2.45 (3H, s), 3.36 (2H, t, J = 7.2 Hz), 6.86 (1H, s), 7.34 (1H, dt. J = 7.8, 1.2 Hz), 7.44 (1H, dt, J = 7.8 , 1.2 Hz), 7.83 (1H, d, J = 8.3 Hz), 7.93 (1H. dt, J = 7.8, 0.6 Hz), 8.78 (1H, br s).
EIMS m/z (relative intensity): 504 (M+-1), 200 (100).

| Elemental analysis: as C₂₄H₃₁N₃OS₄ | | | | | |
|---|---|---|---|---|---|
| | calculated: | C, 57.00; | H, 6.18: | N, 8.31; | S, 25.36. |
| | found: | C, 57.08; | H, 6.17: | N, 8.15; | S, 25.41. |

### Example 23 (Compound No. 803 in Table)

### Production of 4-(benzimidazol-2-ylthio)-N-[2,4-bis(methylthio)-6-methyl-3-pyridyl]butanamide:

The reaction and the treatment were conducted in the same manner as in Example 17 except that 2-mercaptobenzimidazole was used instead of 2-mercaptobenzoxazole to obtain the desired compound as a pale yellow needle crystal.
Melting point: 177 - 179°C
IR (KBr) cm⁻¹ 3421, 3147, 1659, 1645, 1438.
¹H-NMR (d6-DMSO) δ :
2.06 (2H, quint, J = 7.2 Hz), 2.38 (3H, s), 2.39 (3H, s), 2.44 (3H, s), 2.46 (2H, t, J = 7.2 Hz), 3.36 (2H, t, J = 7.2 Hz), 6.88 (1H, s), 7.09 - 7.13 (2H, m), 7.34 - 7.52 (2H, m), 9.48 (1H, s), 12.54 (1H, br s).
EIMS m/z (relative intensity): 418 (M⁺), 150 (100).

### Example 24 (Compound No. 805 in Table)

### Production of 6-(benzimidazol-2-ylthio)-N-[2,4-bis(methylthio)-6-methyl-3-pyridyl]hexanamide:

The reaction and the treatment were conducted in the same manner as in Example 18 except that 2-mercaptobenzimidazole was used instead of 2-mercaptobenzoxazole to obtain the desired compound as a colorless crystal.
Melting point: 139 - 141°C
IR (KBr) cm⁻¹: 3433, 3244, 2924, 1659, 1437.
¹H-NMR (d6-DMSO) δ :
1.47 - 1.56 (2H, m), 1.65 (2H, quint, J = 7.2 Hz), 1.76 (2H, quint, J = 7.2 Hz), 2.28 (2H, t, J = 7.2 Hz), 2.38 (3H, s), 2.39 (3H, s), 2.44 (3H, s), 3.29 (2H, t, J = 7.2 Hz), 6.68 (1H, s), 7.08 - 7.13 (2H, m), 7.36 (1H, m), 7.50 (1H, m), 9.30 (1H, s), 12.50 (1H, br s)
EIMS m/z (relative intensity): 446 (M⁺), 200 (100).

### Example 25 (Compound No. 808 in Table)

### Production of 9-(benzimidazol-2-ylthio)-N-[2,4-bis(methylthio)-6-methyl-3-pyridyl]nonanamide:

The reaction and the treatment were conducted in the same manner as in Example 19 except that 2-mercaptobenzimidazole was used instead of 2-mercaptobenzoxazole to obtain the desired compound as a colorless powdery crystal.
IR (KBr) cm⁻¹ : 3146, 2925, 2854, 1660, 1523, 1437.
¹H-NMR (d6-DMSO) δ :
1.25 - 1.44 (8H, m), 1.53 - 1.61 (2H, m), 1.65 - 1.74 (2H, m), 2.24 (2H, t, J = 7.3 Hz), 2.37 (3H, s), 2.38 (3H, s), 2.43 (3H, s), 3.26 (2H, t, J = 7.1 Hz), 6.86 (1H, s), 7.07 - 7.12 (2H, m), 7.32 - 7.37 (1H, m), 7.46 - 7.54 (1H, m), 9.26 (1H, s).
EIMS m/z (relative intensity): 488 (M⁺), 150 (100).

### Example 26 (Compound No. 811 in Table)

### Production of 2-(benzoxazol-2-ylthio)-N-[2,4-bis(ethylthio)-6-methyl-3-pyridyl]acetamide:

Ethanethiol (1.55 g, 25 mmol) was added dropwise to an ethanol (50 ml) solution of sodium ethoxide (1.27 g, 25 mmol) while being cooled with ice, and the mixture was stirred for 30 minutes. While being cooled with ice, a DMF (40 ml) solution of 2,4-dichloro-6-methyl-3-nitropyridine (2.1 g, 10 mmol) was slowly added thereto dropwise. After the mixture was stirred for 2 hours, the reaction mixture was extracted with ethyl acetate. The organic layer was washed with water and then with a saturated aqueous solution of sodium chloride, and dried over sodium sulfate. Subsequently, the solvent was distilled off to obtain 2.45 g (yield 95%) of 2,4-bis(ethylthio)-6-methyl-3-nitropyridine as a yellow needle crystal.

This nitropyridine (775 mg, 3 mmol) was dissolved in a mixed solvent of acetic acid (30 ml) and conc. hydrochloric acid (1.5 ml), and zinc (4g, 60 mmol) was added thereto in small portions while being cooled with ice. After the mixture was stirred for 10 minutes, the reaction mixture was filtered, and the filtrate was neutralized with a sodium hydroxide aqueous solution, and extracted with ethyl acetate. The organic layer was washed with water and then with a saturated aqueous solution of sodium chloride, and dried over sodium sulfate. Subsequently, the solvent was distilled off to obtain 590 mg (yield 86%) of 3-amino-2,6-bis(ethylthio)-6-methylpyridine as a yellow oil. Triethylamine (304 mg, 3 mmol) was added to a THF (10 ml) solution of this aminopyridine (590 mg, 2.6 mmol), and bromoacetyl bromide (606 mg, 3 mmol) was then slowly added thereto dropwise while being cooled with ice. The mixture was stirred at room temperature for 1 hour. The reaction mixture was filtered, and the filtrate was concentrated. Then, the residue was purified through silica gel chromatography (silica gel 60 g, eluent - hexane : acetone = 10:1 → 5:1) to obtain 410 mg (yield 45%) of 2-bromo-N-[2,4-bis(ethylthio)-6-methyl-3-pyridyl]acetamide as a light brown needle crystal. Potassium carbonate (46 mg, 0.33 mmol) was added to an acetonitrile (3 ml) solution of this amide (105 mg. 0.3 mmol) and 2-mercaptobenzoxazole (45 mg, 0.3 mmol), and the mixture was stirred at room temperature for 2 hours The reaction mixture was extracted with ethyl acetate. The organic layer was washed with water and then with a saturated aqueous solution of sodium chloride, and dried over magnesium sulfate. Subsequently, the solvent was distilled off, and the resulting crude product was purified through preparative thin-layer chromatography (eluent - hexane : ethyl acetate = 3:1) to obtain 70 mg (yield 56%) of the desired compound as a colorless needle crystal.
Melting point: 143 - 145°C
IR (KBr) cm⁻¹: 3429, 3224, 1673, 1509, 1454.
¹H-NMR (CDCl₃) δ :
1.17 (3H, t, J = 7.3 Hz), 1.20 (3H, t, J = 7.5 Hz), 2.43 (3H, s), 2.81 (2H, q, J = 7.3 Hz), 3.04 (2H, q, J = 7.5 Hz), 4.11 (2H, s), 6.63 (1H, s), 7.25 - 7.33 (2H, m), 7.48 (1H, m), 7.61 (1H, m), 8.63 (1H, br s).
EIMS m/z (relative intensity): 419 (M⁺), 268 (100).

| Elemental analysis: as C₁₉H₂₁N₃O₂S₃ | | | | |
|---|---|---|---|---|
| | calculated: | C, 54.39; | H, 5.04: | N, 10.01. |
| | found: | C, 54.39; | H, 5.05: | N, 10.00. |

### Example 27 (Compound No. 815 in Table)

### Production of 6-(benzoxazol-2-ylthio)-N-[2,4-bis(ethylthio)-6-methyl-3-pyridyl]hexanamide:

The reaction and the treatment were conducted in the same manner as in Example 26 except that 6-bromohexanoyl chloride was used instead of bromoacetyl bromide to obtain 6-bromo-N-[2,4-bis(ethylthio)-6-methyl-3-pyridyl]hexanamide. To a DMF (2 ml) solution of this amide (122 mg, 0.3 mmol) and 2-mercaptobenzoxazole (45 mg, 0.3 mmol) were added potassium carbonate (46 mg, 0.33 mmol) and 18-crown-6 (8 mg, 0.03 mmol), and the mixture was stirred at 80°C for 1.5 hours. The reaction mixture was allowed to cool, and then extracted with ethyl acetate. The organic layer was washed with water and then with a saturated aqueous solution of sodium chloride, and dried over sodium sulfate. Subsequently, the solvent was distilled off, and the resulting residue was purified through preparative thin-layer chromatography (eluent - hexane : acetone = 5:2) to obtain 65 mg (yield 46%) of the desired compound as a light brown needle crystal.
Melting point: 100 - 103°C
IR (KBr) cm⁻¹ : 3233, 2928, 1668, 1504, 1455.
¹H-NMR (d6-DMSO) δ :
1.26 (3H, t, J = 7.3 Hz), 1.27 (3H, t, J = 7.3 Hz). 1.58 (2H, m), 1.70 (2H, m), 1.85 (2H, m), 2.32 (2H, m), 2.43 (3H, s), 2.94 (2H, q, J = 7.3 Hz),
3.07 (2H, q, J = 7.3 Hz), 3.35 (2H, t, J = 7.3 Hz), 6.89 (1H, s), 7.26 - 7.34 (2H, m), 7.54 - 7.62 (2H, m), 8.77 (1H, br s).
EIMS m/z (relative intensity): 475 (M⁺, 100).

| Elemental analysis: as C₂₃H₂₉N₃O₂S₃ | | | | | |
|---|---|---|---|---|---|
| | calculated: | C, 58.08; | H, 6.14: | N, 8.83; | S, 20.22. |
| | found: | C, 58.07; | H, 6.13; | N, 8.66; | S, 20.27. |

### Example 28 (Compound No. 818 in Table)

### Production of 9-(benzoxazol-2-ylthio)-N-[2.4-bis(ethylthio)-6-methyl-3-pyridyl]nonanamide:

The reaction and the treatment were conducted in the same manner as in Example 27 except that 9-bromononanoyl chloride was used instead of 6-bromohexanoyl bromide to obtain the desired compound as a colorless needle crystal.
Melting point: 84 - 87°C
IR (KBr) cm⁻¹ : 3252, 2923, 1665, 1501, 1455.
¹H-NMR (d6-DMSO) δ :
1.26 (3H, t, J = 7.3 Hz), 1.27 (3H, t, J = 7.3 Hz), 1.28 - 1.52 (8H, m), 1.63 (2H, m), 1.82 (2H, quint, J = 7.2 Hz), 2.26 (2H, m), 2.43 (3H, s), 2.94 (2H, q, J = 7.3 Hz), 3.07 (2H, q, J = 7.3 Hz), 3.34 (2H, t, J = 7.2 Hz), 6.88 (1H. s), 7.26 - 7.34 (2H, m), 7.54 - 7.62 (2H, m), 8.72 (1H, br s).
EIMS m/z (relative intensity): 517 (M⁺), 367 (100).

| Elemental analysis: as C₂₆H₃₅N₃O₂S₃ | | | | |
|---|---|---|---|---|
| | calculated: | C, 60.31; | H, 6.81; | N, 8.12. |
| | found: | C, 60.52; | H, 6.85; | N, 7.85. |

### Example 29 (Compound No. 821 in Table)

### Production of 2-(benzothiazol-2-ylthio)-N-[2,4-bis(ethylthio)-6-methyl-3-pyridyl]acetamide:

The reaction and the treatment were conducted in the same manner as in Example 26 except that 2-mercaptobenzothiazole was used instead of 2-mercaptobenzoxazole to obtain the desired compound as a colorless needle crystal.
Melting point: 119 - 120°C
IR (KBr) cm⁻¹ : 3453, 3254, 1672, 1510, 1428.
¹H-NMR (CDCl₃) δ :
1.20 (3H, t, J = 7.4 Hz), 1.22 (3H, t, J = 7.4 Hz), 2.42 (3H, s), 2.82 (2H, q, J = 7.4 Hz), 3.06 (2H, q, J = 7.4 Hz), 4.18 (2H, s), 6.63 (1H, s), 7.33 (1H, m), 7.42 (1H, m), 7.77 (1H, m), 7.91 (1H, m), 8.95 (1H, br s).
EIMS m/z (relative intensity): 435 (M⁺), 148 (100).

| Elemental analysis: as C₁₉H₂₁N₃OS₄ | | | | |
|---|---|---|---|---|
| | calculated: | C, 52.39; | H, 4.86; | N, 9.65. |
| | found: | C, 52.40; | H, 4.86; | N, 9.53. |

### Example 30 (Compound No. 825 in Table)

### Production of 6-(benzothiazol-2-ylthio)-N-[2.4-bis(ethylthio)-6-methyl-3-pyridyl]hexanamide:

The reaction and the treatment were conducted in the same manner as in Example 27 except that 2-mercaptobenzothiazole was used instead of 2-mercaptobenzoxazole to obtain the desired compound as a colorless needle crystal.
Melting point: 81 - 83°C
IR (KBr) cm⁻¹ : 3150, 2927, 1647, 1524, 1428.
¹H-NMR (d6-DMSO) δ :
1.25 (3H, t, J = 7.3 Hz), 1.26 (3H, t, J = 7.3 Hz), 1.57 (2H, m), 1.69 (2H, m), 1.84 (2H, m), 2.29 (2H, m), 2.42 (3H, s), 2.93 (2H, q, J = 7.3 Hz), 3.05 (2H, q, J = 7.3 Hz), 3.36 (2H, t, J = 7.3 Hz), 6.87 (1H, s), 7.33 (1H, m), 7.43 (1H, m), 7.82 (1H, m), 7.92 (1H, m), 8.77 (1H, br s).
EIMS m/z (relative intensity): 491 (M⁺) , 168 (100).

| Elemental analysis: as C₂₃H₂₉N₃OS₄ | | | | | |
|---|---|---|---|---|---|
| | calculated: | C, 56.18; | H, 5.94; | N, 8.55; | S. 26.08. |
| | found: | C, 56.19; | H, 5.91; | N, 8.43; | S, 26.06. |

### Example 31 (Compound No. 828 in Table)

### Production of 9-(benzothiazol-2-ylthio)-N-[2,4-bis(ethylthio)-6-methyl-3-pyridyl]nonanamide:

The reaction and the treatment were conducted in the same manner as in Example 28 except that 2-mercaptobenzothiazole was used instead of 2-mercaptobenzoxazole to obtain the desired compound as a colorless needle crystal.
Melting point: 88 - 92°C
IR (KBr) cm⁻¹: 3433, 3241, 2928, 1668, 1510.
¹H-NMR (d6-DMSO) δ :
1.25 (3H, t, J = 7.3 Hz), 1.26 (3H, t, J = 7.3 Hz), 1.28 - 1.54 (8H, m), 1.62 (2H, m), 1.80 (2H, quint, J = 7.2 Hz), 2.24 (2H, m), 2.42 (3H, s), 2.93 (2H, q, J = 7.3 Hz), 3.05 (2H, q, J = 7.3 Hz), 3.35 (2H, t, J = 7.2 Hz), 6.87 (1H. s), 7.33 (1H, m), 7.43 (1H, m), 7.81 (1H, m), 7.92 (1H, m), 8.72 (1H, br s).

### Example 32 (Compound No. 831 in Table)

### Production of 2-(benzimidazol-2-ylthio)-N-[2,4-bis(ethylthio)-6-methyl-3-pyridyl]acetamide:

The reaction and the treatment were conducted in the same manner as in Example 26 except that 2-mercaptobenzimidazole was used instead of 2-mercaptobenzoxazole to obtain the desired compound as a colorless needle crystal.
Melting point: 182 - 183°C
IR (KBr) cm⁻¹ : 3148, 2928, 1674, 1524, 1412.
¹H-NMR (d6-DMSO) δ :
1.21 (3H, t, J = 7.3 Hz), 1.21 (3H, t, J = 7.3 Hz), 2.41 (3H, s), 2.90 (2H, q, J = 7.3 Hz), 3.03 (2H, q, J = 7.3 Hz), 4.15 (2H, br s), 6.87 (1H, s), 7.08 - 7.12 (2H, m), 7.39 - 7.44 (2H, m).
EIMS m/z (relative intensity): 418 (M⁺), 357 (100).

| Elemental analysis: as C₁₉H₂₂N₄OS₃ | | | | |
|---|---|---|---|---|
| | calculated: | C, 54.52; | H, 5.30; | N, 13.38. |
| | found: | C, 54.44: | H, 5.30; | N, 13.16. |

### Example 33 (Compound No. 835 in Table)

### Production of 6-(benzimidazol-2-ylthio)-M-[2,4-bis(ethylthio)-6-methyl-3-pyridyl]hexanamide:

The reaction and the treatment were conducted in the same manner as in Example 27 except that 2-mercaptobenzimidazole was used instead of 2-mercaptobenzoxazole to obtain the desired compound as a colorless needle crystal.
Melting point: 139 - 142°C
IR (KBr) cm⁻¹ 3433, 3143, 2928, 1660, 1510.
¹H-NMR (CDCl₃) δ :
1.25 (3H, t, J = 7.3 Hz), 1.26 (3H, t, J = 7.3 Hz), 1.54 (2H, m), 1.68 (2H, m), 1.77 (2H, m), 2.28 (2H, m), 2.42 (3H, s), 2.92 (2H, q, J = 7.3 Hz), 3.05 (2H, q, J = 7.3 Hz), 3.27 (2H, t, J = 7.2 Hz), 6.87 (1H, s), 7.05 - 7.11 (2H, m), 7. 27 - 7.52 (2H, m), 8.75 (1H, br s), 12.05 (1H, br s).

### Example 34 (Compound No. 838 in Table)

### Production of 9-(benzimidazol-2-ylthio)-N-[2,4-bis(ethylthio)-6-methyl-1-3-pyrldyl]nonanamide:

The reaction and the treatment were conducted in the same manner as in Example 28 except that 2-mercaptobenzimidazole was used instead of 2-mercaptobenzoxazole to obtain the desired compound as a colorless needle crystal.
Melting point: 76 - 78°C
IR (KBr) cm⁻¹ : 3104, 2928, 2854, 1658, 1526.
¹H-NMR (d6-DMSO) δ :
1.25 (3H, t, J = 7.3 Hz), 1.26 (3H, t, J = 7.3 Hz), 1.28 - 1.49 (8H, m), 1.61 (2H, m), 1.73 (2H, quint, J = 7.2 Hz), 2.24 (2H, m), 2.42 (3H, s), 2.92 (2H, q, J = 7.3 Hz), 3.05 (2H, q, J = 7.3 Hz), 3.26 (2H, t, J = 7.2 Hz), 6.87 (1H, s), 7.05 - 7.10 (2H, m), 7.24 - 7.54 (2H, m), 8.71 (1H, br s), 12.05 (1H, br s).

### Example 35 (Compound No. 841 in Table)

### Production of 2-(benzoxazol-2-ylthio)-N-[2,4-bis(isopropylthio)-6-methyl-3-pyridyl]acetamide:

To a 2-propanol (50 ml) solution of sodium isopropoxide (2.05 g, 25 mmol) was added dropwise 2-propanethiol (1.90, 25 mmol) while being cooled with ice, and the mixtrue was stirred for 30 minutes. While being cooled with ice, a DMF (40 ml) solution of 2,4-dichloro-6-methyl-3-nitropyridine (2.07 g, 10 mmol) was slowly added thereto dropwise. After the mixture was stirred for 2 hours, the reaction mixture was extracted with ethyl acetate. The organic layer was washed with water and then with a saturated aqueous solution of sodium chloride, and dried over sodium sulfate. Subsequently, the solvent was distilled off to obtain 2.77 g (yield 97%) of 2,4-bis(isopropylthio)-6-methyl-3-nitropyridine as a yellow needle crystal.

This nitropyridine (1.08 g, 3.77 mmol) was dissolved in a mixed solvent of acetic acid (35 ml) and conc. hydrochloric acid (1.6 ml), and zinc (2.96 g, 45.25 mmol) was added thereto in small portions while being cooled with ice. After the mixture was stirred for 1 hour, the reaction mixture was filtered, and the filtrate was neutralized with an aqueous solution of sodium hydrogencarbonate, and extracted with chloroform. The organic layer was washed with water and then with a saturated aqueous solution of sodium chloride, and dried over sodium sulfate. Subsequently, the solvent was distilled off, and the resulting residue was purified through silica gel column chromatography (eluent - hexane : ethyl acetate = 30:1 → 10:1) to obtain 774 mg (yield 80%) of 3-amino-2,4-bis(isopropylthio)-6-methylpyridine as a yellow oil. Triethylamine (336 mg, 3.32 mmol) was added to a THF (10 ml) solution of this aminopyridine (774 mg, 3.02 mmol), and bromoacetyl bromide (732 mg, 3.62 mmol) was then slowly added thereto dropwise while being cooled with ice, and the mixture was stirred for 17 hours. The reaction mixture was filtered, and the filtrate was concentrated. Then, the residue was purified through silica gel chromatography (eluent - hexane : ethyl acetate = 10:1) to obtain 595 mg (yield 52%) of 2-bromo-N-[2,4-bis(isopropylthio)-6-methyl-3-pyridyl]acetamide as a colorless powdery crystal. sodium hydrogencarbonate (29 mg, 0.35 mmol) was added to an acetonitrile (5 ml) solution of this amide (132 mg, 0.35 mmol) and 2-mercaptobenzoxazole (53 mg, 0.35 mmol), and the mixture was stirred at room temperature for 28 hours. The reaction mixture was extracted with ethyl acetate. The organic layer was washed with water and then with a saturated aqueous solution of sodium chloride, and dried over magnesium sulfate. Subsequently, the solvent was distilled off, and the resulting crude product was purified through preparative thin-layer chromatography (eluent - hexane : benzen =6:1) to obtain 69 mg (yield 44%) of the desired compound as a colorless powdery crystal.
Melting point: 151 - 152°C
IR (KBr) cm⁻¹ : 3404, 2967, 1743, 1637, 1360.
¹H-NMR (CDCl₃) δ :
1.37 - 1.40 (12H, m), 2.52 (3H, s), 3.58 (1H, sept, J = 6.8 Hz), 4.06 (2H, s), 4.11 (1H, sept, J = 6.8 Hz), 6.01 (1H, s), 6.81 - 6.86 (2H, m), 6.92 (1H, dd, J = 8.1, 1.3 Hz), 7.00 - 7.07 (2H, m).

### Example 36 (Compound No. 845 in Table)

### Production of 6-(benzoxazol-2-ylthio)-N-[2,4-bis(isopropylthio)-6-methyl-3-pyridyl]hexanamide:

The reaction and the treatment were conducted in the same manner as in Example 35 except that 6-bromohexanoyl chloride was used instead of bromoacetyl bromide to obtain 6-bromo-N-[2,4-bis(isopropylthio)-6-methyl-3-pyridyl]hexanamide. To a DMF (4 ml) solution of this amide (100 mg, 0.23 mmol) and 2-mercaptobenzoxazole (35 mg, 0.23 mmol) were added potassium carbonate (38 mg, 0.28 mmol) and 18-crowa-6 (6 mg, 0.02 mmol), and the mixture was stirred at 80°C for 2.5 hours. The reaction mixture was allowed to cool, and then extracted with ethyl acetate . The organic layer was washed with water and then with a saturated aqueous solution of sodium chloride, and dried over sodium sulfate. Subsequently, the solvent was distilled off, and the resulting residue was purified through preparative thin-layer chromatography (eluent - hexane : ethyl acetate = 3:1) to obtain 92 mg (yield 79%) of the desired compound as a colorless powdery crystal.
Melting point: 98 - 100°C
IR (KBr) cm⁻¹: 3135, 2961, 1648, 1498, 1454, 1133.
¹H-NMR (d6-DMSO) δ :
1.32 (6H, d, J = 6.8 Hz), 1.35 (6H, d, J = 6.8 Hz), 1.55 - 1.64 (2H, m), 1.65 - 1.75 (2H, m), 1.82 - 1.92 (2H, m), 2.23 - 2.36 (2H, m), 2.46 (3H, s), 3.38 (2H, t, J = 7.1 Hz), 3.59 (1H, sept, J = 6.8 Hz), 3.93 (1H, sept, J = 6.8 Hz), 6.96 (1H, s), 7.29 - 7.37 (2H, m), 7.57 - 7.64 (2H, m), 8.95 (1H, br s).

### Example 37 (Compound No. 1237 in Table)

### Production of 6-(oxazolo[4,5-b]pyridin-2-ylthio)-N-[2,4-bis(methylthio)-6-methyl-3-pyridyl]hexanamide:

To a DMF (4 ml) solution of 6-bromo-N-[2,4-bis(methylthio)-6-methyl-3-pyridyl]hexanamide (100 mg, 0.27 mmol) and 2-mercaptoxazolo[4,5-b]pyridine (40 mg, 0.27 mmol) were added 18-crown-6 (7 mg, 0.03 mmol) and potassium carbonate (40 mg, 0.29 mmol), and the mixture was stirred at 80°C for 4 hours. The reaction mixture was diluted with water, and then extracted with ethyl acetate. The organic layer was washed with water and then with a saturated aqueous solution of sodium chloride, and dried over magnesium sulfate. Subsequently, the solvent was distilled off, and the resulting crude product was purified through preparative thin-layer chromatography (eluent - hexane : acetone = 2:1) to obtain 85 mg (yield 72%) of the desired compound as a colorless powdery crystal.
Melting point: 132 - 133°C
IR (KBr) cm⁻¹ : 3435, 3243, 2923, 1655, 1493, 1404.
¹H-NMR (d6-DMSO) δ :
1.53-1.63(2H,m), 1.65-1.76(2H,m), 1.83-1.93(2H,m), 2.27-2.35(2H,m), 2.40(3H,s),2.42(3H,s),2.45(3H,s), 3.40(2H,t,J=7.3Hz), 6.86(1H,S), 7.30(1H,dd,J=8.3,4.9Hz), 7.97(1H,dd,J=8.1,1.3Hz), 8.42(1H,dd,J=4.9,1.3Hz), 8.83(1H,br s).
EIMS m/z (relative intensity) : 447 (M⁺-1), 400(100).

| Elemental analysis: as C₂₀H₂₄N₄O₂S₃ | | | | | |
|---|---|---|---|---|---|
| | calculated: | C, 53.55; | H, 5.39; | N, 12.59; | S, 21.44. |
| | found: | C, 53.72; | H, 5.39; | N, 12.41; | S, 21.51. |

### Example 38 (Compound No. 1238 in Table)

### Production of 6-(7-methoxycarbonylbenzoxazol-2-ylthio)-N-[2,4-bis(methylthio)-6-methyl-3-pyridyl]hexanamide:

The reaction and the treatment were conducted in the same manner as in Example 37 except that 7-methoxycarbonyl-2-mercaptobenzoxazole was used instead of 2-mercaptoxazolo[4,5-b]pyridine to obtain the desired compound as a colorless powdery crystal.
Melting point: 141 - 142°C
IR (KBr) cm⁻¹ : 3425, 3236, 2923, 1726, 1667, 1509.
¹H-NMR (d6-DMSO) δ :
1.54-1.63(2H,m), 1.67-1.76(2H,m), 1.84-1.93(2H,m), 2.28-2.35(2H,m). 2.40(3H,s), 2.42(3H,s), 2.45(3H,s), 3.39(2H,t,J=7.1Hz), 3.95(3H,s), 6.86(1H,s), 7.44(1H,t,J=7.8Hz), 7.81(1H,dd,J=7.8,1.2Hz), 7.85(1H,dd,J=7.8,1.2Hz), 8.82(1H,br s).
EIMS m/z (relative intensity) : 504 (M⁺-1), 167(100).

| Elemental analysis: as C₂₃H₂₇N₃O₄S₃ | | | | | |
|---|---|---|---|---|---|
| | calculated: | C, 54.63;H, | 5.38; N, | 8.31: S, | 19.02. |
| | found: | C, 54.70; H, | 5.37; N, | 8.27: S, | 19.15. |

### Example 39 (Compound No. 1240 in Table)

### Production of 9-(7-methoxycarbonylbenzoxazol-2-ylthio)-N-[2,4-bis(methylthio)-6-methyl-3-pyridyl]nonanamide:

To a DMF (4 ml) solution of 9-bromo-N-[2,4-bis(methylthio)-6-methyl-3-pyridyl]nonanamide (90 mg, 0.22 mmol) and 7-methoxycarbonyl-2-mercaptobenzoxazole (45 mg, 0.22 mmol) were added 18-crown-6 (6 mg, 0.02 mmol) and potassium carbonate (36 mg, 0.26 mmol), and the mixture was stirred at 80 °C for 4 hours. The reaction mixture was diluted with water, and then extracted with ethyl acetate. The organic layer was washed with water and then with a saturated aqueous solution of sodium chloride, and dried over magnesium sulfate. Subsequently, the solvent was distilled off, and the resulting crude product was recrystallized from a mixture of ethyl acetate and hexane to obtain 84 mg (yield 72%) of the desired compound as a colorless powdery crystal.
Melting point: 126 - 128°C .
IR (KBr) cm⁻¹ : 3231, 2924, 1720, 1657, 1508, 1297
¹H-NMR (d6-DMSO) δ :
1.27-1.47(8H,m), 1.54-1.62(2H,m), 1.74-1.85(2H,m), 2.24(2H,t,J=7.3Hz), 2.37(3H,s),2.38(3H,s),2.43(3H,s), 3.31-3.41(2H,m), 3.91(3H,s), 6.86(1H,s), 7.45(1H,t,J=7.8Hz), 7.81(1H,dd,J=7.8,1.0Hz), 7.91(1H,dd,J=7.8,1.0Hz), 9.26(1H,s).
EIMS m/z (relative intensity) : 546(M⁺-1), 500(100).

| Elemental analysis: as C₂₆H₃₃N₃O₄S₃ | | | | | |
|---|---|---|---|---|---|
| | calculated: | C, 57.01; | H, 6.07; | N, 7.67; | S, 17.56. |
| | found: | C, 57.10; | H, 5.95; | N, 7.67; | S, 17.60. |

### Examples 40 (Compound No. 151 in Table)

### Production of 2-(benzoxazol-2-ylthio)-N-(4-methyl-2-methylthio-3-pyridyl)acetamide:

The reaction and the treatment were conducted in the same manner as in Example 16 except that 3-amino-4-methyl-2-methylthiopyridine was used instead of 3-amino-2,4-bis(methylthio)-6-methylpyridine to obtain the desired compound as a colorless needle crystal.
Melting point : 146 - 148°C
IR (KBr) cm⁻¹: 3437, 3245, 1671, 1659, 1507, 1454.
¹H-NMR (CDCl₃) δ:
2.17 (3H, s), 2.42 (3H, s), 4.11 (2H, s), 6.87 (1H, d, J = 4.9 Hz), 7.28 - 7.34 (2H, m). 7.50 (1H, m), 7.61 (1H, m), 8.23 (1H, d, J = 4.9 Hz), 8.88 (1H, br s).
EIMS m/z (relative intensity): 345 (M⁺, 100).

| Elemental analysis: as C₁₆H₁₅N₃O₂S₂ | | | | | |
|---|---|---|---|---|---|
| | calculated: | C, 55.63; | H, 4.38; | N, 12.16; | S, 18.56. |
| | found: | C, 55.66; | H, 4.46; | N, 12.02; | S, 18.55. |

### Example 41 (Compound No. 155 in Table)

### Production of 6-(benzoxazol-2-ylthio)-N-(4-methyl-2-methylthio-3-pyridyl)hexanamide:

The reaction and the treatment were conducted in the same manner as in Example 18 except that 3-amino-4-methyl-2-methylthiopyridine was used instead of 3-amino-2.4-bis(methylthio)-6-methylpyridine to obtain the desired compound as a colorless needle crystal.
Melting point: 122 - 124°C
IR (KBr) cm⁻¹: 3437, 3245, 1660, 1521, 1507, 1133.
¹H-NMR (d₆-DMSO) δ:
1.49 - 1.56 (2H, m), 1.68 (2H, quint. J = 7.4 Hz), 1.84 (2H, quint, J = 7.4 Hz), 2.09 (3H, s), 2.33 (2H, t, J = 7.4 Hz), 2.40 (3H, s), 3.36 (2H, t, J = 7.4 Hz), 7.02 (1H, d, J = 4.9 Hz), 7.29 - 7.36 (2H, m), 7.61 - 7.66 (2H, m),8.24 (1H, d, J = 4.9 Hz), 9.40 (1H, br s).
EIMS m/z (relative intensity): 401 (M⁺, 100).

| Elemental analysis: as C₂₀H₂₃N₃O₂S₂ | | | | | |
|---|---|---|---|---|---|
| | calculated: | C, 59.82; | H, 5.77; | N, 10.46; | S, 15.97. |
| | found: | C, 59.93; | H, 5.89; | N. 10. 34; | S, 15.99. |

### Example 42 (Compound No. 365 in Table)

### Production of 6-(benzoxasole-2-ylthio)-N-(6-methoxy-2-methylthio-3-pyridyl)hexanamide:

A methanol (100 ml) solution of 2-chloro-6-methoxy-3-nitropyridine (2.0 g, 10.4 mmol) was added dropwise to a methanol (20 ml) solution of sodium thiomethoxide (805 mg, 10.9 mmol) while being cooled with ice, and the temperature thereof was raised to the room temperature and the mixed solution was stirred for 17 hours and the precipitated crystal was filtered to obtain 1.26 g (yield 59%) of 6-methoxy-2-methylthio-3-nitropyridine as a yellow powdery crystal.

This nitropyridine (400 mg, 2.0 mmol) was dissolved in a mixed solvent of acetic acid (20 ml) and conc. hydrochloric acid (0.5 ml), and zinc (1.57 g, 24.0 mmol) was added thereto in small portions while being cooled with ice for 5 minutes. After the mixture was stirred for 40 minutes at the room temperature, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Thereafter, the solvent was distilled off, and the resulting crude product was purified through silica gel chromatography (eluent - hexane: ethyl acetate = 6:1→ 4:1) to obtain 264 mg (yield 78%) of 3-amino-6-methoxy-2-methylthiopyridine as a pale brown powdery crystal.

And then the reaction and the treatment were conducted in the same manner as in Example 18 except that 3-amino-6-methoxy-2-methylthiopyridine was used instead of 3-amino-2,4-bis(methlthio)-6-methylpyridine to obtain the desired compound as a colorless powdery crystal.
Melting point: 102 - 104°C
IR (KBr) cm⁻¹: 3430. 3224, 2940, 1652, 1591.
¹H-NMR (CDCl₃) δ :
1.61 (2H, quint, J = 7.4 Hz), 1.82 (2H, quint, J = 7.4 Hz), 1.92 (2H, quint, J = 7.4 Hz), 2.42 (2H, t, J = 7.4 Hz), 2.59 (3H, s), 3.34 (2H, t, J = 7.4 Hz), 3.94 (3H, s), 6.47 (1H, d, J = 8.5 Hz). 6.91 (1H. brs), 7.23 (1H, td, J = 7.7 , 1.5 Hz), 7.27 (1H, td, J = 7.7 ,1.5 Hz), 7.43 (1H, dd, J = 7.7 , 1.5 Hz), 7.58 (1H, dd, J=7.7, 1.5 Hz), 7.93 (1H, d, J = 8.5 Hz).
EIMS m/z (relative intensity): 417 (M⁺). 171 (100).

### Example 43 (Compound No. 451 in Table)

### Production of 2-(benzoxazol-2-ylthio)-N-(6-methylthio-3-pyridyl) acetamide:

The reaction and the treatment were conducted in the same manner as in Example 16 except that 3-amino-6-methyl-2-methylthiopyridine was used instead of 3-amino-2,4-bis(methylthio)-6-methylpyridine to obtain the desired compound as a colorless needle crystal.
Melting point: 180 - 181°C
IR (KBr) cm⁻¹: 3437, 3254, 1661, 1534, 1509, 1135.
¹H-NMR (CDCl₃) δ :
2.46 (3H, s), 2.50 (3H, s), 4.10 (2H, s), 6.87 (2H, d, J = 8.1 Hz), 7.26 - 7.34 (2H, m), 7.48 (1H, m), 7.62 (1H, m), 8.12 (2H, d, J = 8.1 Hz), 9.27 (1H, br s).
EIMS m/z (relative intensity): 345 (M⁺), 298 (100).

| Elemental analysis: as C₁₆H₁₅N₃O₂S₂ | | | | | |
|---|---|---|---|---|---|
| | calculated: | C, 55.63; | H, 4.38; | N, 12.16; | S, 18.56. |
| | found: | C, 55.62; | H, 4.40; | N, 12. 10; | S, 18.50. |

### Example 44 (Compound No. 461 in Table)

### Production of 2-(benzothiazol-2-ylthio)-N-(6-methyl-2-methylthio-3-pyridyl)acetamide:

The reaction and the treatment were conducted in the same manner as in Example 43 except that 2-mercaptobenzothiazole was used instead of 2-mercaptobenzoxazole to obtain the desired compound as a colorless needle crystal.
Melting Point : 175 - 176°C
IR (KBr) cm⁻¹: 3437, 3248, 1656, 1532, 1430.
¹H-NMR (CDCl₃) δ:
2.45 (3H, s), 2.47 (3H, s), 4.18 (2H, s), 6.87 (1H, d, J = 8.1 Hz), 7.34 (1H, m), 7.44 (1H, m), 7.77 (1H, m), 8.01 (1H, m), 8.07 (1H, d, J = 8.1 Hz), 9.31 (1H, br s).
EIMS m/z (relative intensity): 361 (M⁺), 210 (100).

| Elemental analysis : as C₁₆H₁₅N₃OS₃ | | | | | |
|---|---|---|---|---|---|
| | calculated: | C, 53.16: | H, 4.18; | N, 11.62; | S, 26.61. |
| | found: | C, 53.23; | H, 4.25; | N, 11.55; | S, 26.67. |

### Example 45 (Compound No. 471 in Table)

### Production of 2-(benzimidazol-2-ylthio)-N-(6-methyl-2-methylthio-3-pyridyl)acetamide:

The reaction and the treatment were conducted in the same manner as in Example 43 except that 2-2-mercaptobenzimidazole was used instead of 2-mercaptobenzoxazole to obtain the desired compound as a colorless needle crystal.
Melting point : 192 - 193°C (d.)
IR (KBr) cm⁻¹: 3420, 3249, 1667, 1550, 1438, 744.
¹H-NMR (CDCl₃) δ:
2.45 (3H, s), 2.50 (3H, s), 4.08 (2H, s), 6.84 (1H, d, J = 8.1 Hz), 7.19 - 7.25 (2H, m), 7.35 (1H, m), 7.73 (1H m), 8.00 (1H, d, J = 8.1 Hz), 9.95 (1H, br s), 10.00 (1H, br s).
EIMS m/z (relative intensity): 344 (M⁺), 118 (100) .

| Elemental analysis: as C₁₆H₁₆N₄OS₂ | | | | | |
|---|---|---|---|---|---|
| | calculated: | C, 55.79; | H, 4.68; | N, 16.27; | S, 18.62. |
| | found: | C, 55.80; | H, 4. 68; | N, 16.16; | S, 18. 65. |

### Example 46 (Compound No. 784 in Table)

### Production of 5-(benzoxazol-2-ylthio)-N-(2,4-bis(methylthio)-6-methyl-3-pyridyl)pentanamide:

The reaction and the treatment were conducted in the same manner as in Example 17 except that 5-bromopentqnoic acid chloride was used instead of 4-bromobutanoyl chloride to obtain the desired compound as a colorless needles crystal.
Melting point: 147 - 150°C
IR (KBr) cm⁻¹: 3230, 1664, 1501, 1455, 1136.
¹H-NMR (d₆-DMSO) δ :
1.72 - 1.96 (4H, m), 2.36 (3H, s), 2.26 - 2.42 (2H, m), 2.39 (3H, s), '2.43 (3H, s), 3.36 (2H, t, J = 7.2 Hz), 6.83 (1H, s),
7.23 - 7.33 (2H, m), 7.52 - 7.59 (2H, m), 8.74 (1H, br s).
EIMS m/z (relative intensity): 433 (M⁺), 201 (100).

### Example 47 (Compound No. 786 in Table)

### Production of 7-(benzoxazol-2-ylthio)-N-[2,4-bis(methylthio)-6-methyl-3-pyridyl]heptanamide:

The reaction and the treatment were conducted in the same manner as in Example 17 except that 7-bromoheptanonyl chloride was used instead of 4-bromobutanoyl chloride to obtain the desired compound as a colorless powdery crystal.
Melting point: 137 - 139°C
IR (KBr) cm⁻¹: 3437, 3242, 2922, 2857, 1660, 1500, 1455, 1132.
¹H-NMR (d₆-DMSO) δ :
1.41 - 1.54 (4H, m), 1.60 - 1.70 (2H, m), 1.81 (2H, quint, J = 7.1 Hz), 2.26 - 2.32 (2H, m), 2.38 (3H, s), 2.40 (3H, s), 2.43 (3H, s), 3.33 (2H, t, J = 7.1 Hz),
6.81 (1H, s), 7.27 (1H, td, J = 7.6 , 1.7 Hz), 7.30 (1H, td, J = 7.6 , 1.7 Hz), 7.54 - 7.60 (2H, m), 8.79 (1H, br s).
EIMS m/z (relative intensity): 461 (M⁺), 200 (100).

### Example 48 (Compound No. 787 in Table)

### Production of 8-(benzoxazol-2-ylthio)-N-[2,4-bis(methylthio)-6-methyl-3-pyridyl]octanamide:

The reaction and the treatment were conducted in the same manner as in Example 17 except that 8-bromooctanoyl chloride was used instead of 4-bromobutanonyl chloride to obtain the desired compound as a colorless prism crystal.
Melting point: 119 - 122°C
IR (KBr) cm⁻¹: 3435, 3248, 2923, 2856, 1660, 1501, 1454, 1131.
¹H-NMR (d₆-DMSO) δ:
1.33 - 1.52 (6H, m), 1.58 - 1.69 (2H, m), 1.81 (2H, quint, J = 7.1 Hz), 2.26 - 2.32 (2H, m), 2.38 (3H, s),
2.41 (3H, s), 2.44 (3H, s), 3.33 (2H, t, J = 7.1 Hz), 6.84 (1H, s), 7.27 (1H, td, J = 7.6, 1.7 Hz), 7.30 (1H, td, J = 7.6 , 1.7 Hz), 7.54 - 7.60 (2H, m), 8.77 (1H, br s).
EIMS m/z (relative intensity): 475 (M⁺), 200 (100).

### Example 49 (Compound No. 791 in Table)

### Production of 2-(benzothiazol-2-ylthio)-N-[2,4-bis(methylthio)-6-methyl-3-pyridyl)acetamide:

An acetonitrile solution (6 ml) of 2-bromo-N-[2,4-bis(methylthio)-3-pyridyl]acetamide (64 mg, 0.2 mmol) was added to an acetonitrile solution (1 ml) of sodium hydrogencarbonate (17 mg, 0.2 mmol) and 2-mercaptobenzothiazole (34 mg, 0.2 mmol), and the mixed solution was stirred for 48 hours at the room temperature. And the solution of reaction mixture was concentrated under reduced pressure, and the residue was extraxted with ethyl acetate after dilluting with water. The organic layer was washed with water and then with a saturated aqueous solution of sodium chloride, and dried over sodium sulfate. Thereafter, the solvent was distilled off, and the resulting crude product was purified through preparative thin layer chromatography (eluent - chloroform:methanol = 20:1) to obtain 46 mg (yield 33%) as a colorless needle crystal.
Melting point: 178 - 179°C
IR (KBr) cm⁻¹: 3437, 3246, 1665, 1564, 1497, 1430.
¹H-NMR (CDCl₃) δ :
2.33 (3H, s), 2.44 (3H, s), 2.46 (3H, s), 4.17 (2H, s), 6.61 (1H, s), 7.33 (1H, m), 7.43 (1H, m), 7.78 (1H, m), 7.90 (1H, m), 9.11 (1H, br s).
EIMS m/z (relative intensity): 407 (M⁺), 209 (100).

| Elemental analysis: as C₁₇H₁₇N₃OS₄ | | | | | |
|---|---|---|---|---|---|
| | calculated: | C, 50.10; | H, 4.20; | N, 10.31; | S, 31.46. |
| | found: | C, 50.18; | H, 4.29; | N, 10.23; | S, 31.49. |

### Example 50 (Compound No. 794 in Table)

### Production of 5-(benzothiazol-2-ylthio)-N-[2,4-bis(methylthio)-6-methyl-3-pyridyl)pentanamide:

The reaction and the treatment were conducted in the same manner as in Example 46 except that 2-mercaptobenzothiazole was used instead of 2-mercaptobenzoxazole to obtain the desired compound as a colorless needle crystal.
Melting point: 121 - 123°C
IR (KBr) cm⁻¹: 3437, 3240, 2923, 1664, 1515, 1456, 1428, 995.
¹H-NMR (d₆―DMSO) δ :
1.78 - 1.87 (2H, m), 1.88 - 1.96 (2H, m). 2.30 - 2.40 (2H, m), 2.38 (3H, s), 2.41 (3H, s), 2.45 (3H, s), 3.41 (2H, t, J = 7.1 Hz), 6.85 (1H, s), 7.34 (1H, t, J = 7.6 Hz), 7.45 (1H, t, J = 7.6 Hz), 7.84 (1H, d, J = 7.6 Hz), 7.94 (1H, d, J = 7.6 Hz), 8.87 (1H, br s).
EIMS m/z (relative intensity): 449 (M⁺), 201 (100).

### Example 51 (Compound No. 796 in Table)

### Production of 7-(benzothiazol-2-ylthio)-N-[2,4-bis(methylthio)-6-methyl-3-pyridyl)heptamamide:

The reaction and the treatment were conducted in the same manner as in Example 47 except that 2-mercaptobenzothiazole was used instead of 2-mercaptobenzoxazole to obtain the desired compound as a colorless needle crystal.
Melting point: 129 - 130°C
IR (KBr) cm⁻¹: 3436, 3245, 2922, 1661, 1506, 1428.
¹H-MM (d₆―DMSO) δ :
1.44 - 1.54 (4H, m), 1.62 - 1.71 (2H, m), 1.83 (2H, quint, J = 7.2 Hz), 2.13 - 2.33 (2H, m), 2.39 (3H, s), 2.42 (3H, s), 2.45 (3H. s), 3.37 (2H, t. J = 7.2 Hz), 6.86 (1H, s), 7.34 (1H, td, J = 7.8, 1.2 Hz), 7.45 (1H, td, J = 7.8, 1.2 Hz), 7.84 (1H, dd, J = 7.8, 1.2 Hz), 7.94 (1H, dd, J = 7.8, 1.2 Hz), 8.81 (1H, br s).
EIMS m/z (relative intensity): 477 (M⁺), 200 (100).

| Elemental analysis: as C₂₂H₂₇N₃OS₄ | | | | |
|---|---|---|---|---|
| | calculated: | C, 55.31; | H, 5.70; | N, 8.80. |
| | found: | C, 55.41: | H, 5.71; | N, 8.64. |

### Example 52 (Compound No. 797 in Table)

### Production of 8-(benzthiazol-2-ylthio)-N-[2,4-bis(methylthio)-6-methyl-3-pyridyl)octanamide:

The reaction and the treatment were conducted in the same manner as in Example 48 except that 2-mercaptobenzothiazole was used instead of 2-mercaptobenzoxazole to obtain the desired compound as a colorless needle crystal.
Melting point: 104 - 108°C
IR (KBr) cm⁻¹: 3242, 2925, 1665, 1508, 1459, 1428.
¹H-NMR (d₆-DMSO) δ :
1.30 - 1.51 (6H, m), 1.55 -1.69 (2H, m), 1.81 (2H, quint, J = 7.1 Hz), 2.23 - 2.29 (2H, m), 2.38 (3H, s), 2.41 (3H, s), 2.44 (3H, s), 3.35 (2H, t, J = 7.2 Hz) 6.83 (1H, s), 7.32 (1H, m), 7.43 (1H, m), 7.81 (1H, m), 7.91 (1H, m), 8.76 (1H, br s).
EIMS m/z (relative intensity): 491 (M⁺), 200 (100).

### Example 53 (Compound No. 801 in Table)

### Production of 2-(benzimidazol-2-ylthio)-N-[2,4-bis(methylthio)-6-methyl-3-pyridyl)pentanamide:

The reaction and the treatment were conducted in the same manner as in Example 49 except that 2-mercaptobenzimidazole was used instead of 2-mercaptobenothiazole to obtain the desired compound as a colorless needle crystal.
Melting point: 235 - 237°C (d.)
IR (KBr) cm⁻¹: 3429, 3243, 2978, 2923, 1661, 1505, 1439.
¹H-NMR (CDCl₃) δ :
2.35 (3H, s), 2.46 (3H, s), 2.47 (3H, s), 4.03 (2H, s), 6.63 (1H, s), 7.21 (1H, t, J =6.1 Hz), 7.22 (1H, t, J = 6.1 Hz), 7.43 - 7.60 (2H, m), 9.43 (1H, br s).
EIMS m/z (relative intensity): 390 (M⁺), 344 (100).

### Example 54 (Compound No. 804 in Table)

### Production of 5-(benzimidazol-2-ylthio)-N-[2,4-bis(methylthio)-6-methyl-3-pyridyl]pentanamide:

The reaction and the treatment were conducted in the same manner as in Example 46 except that 2-mercaptobenzimdazole was used instead of 2-mercaptobenoxazole to obtain the desired compound as a colorless needle crystal.
Melting point: 176 - 177°C
¹H-NMR (d₆-DMSO) δ :
1.74 - 1.84 (4H, m), 2.26 - 2.35 (2H, m), 2.36 (3H, s), 2.39 (3H, s), 2.43 (3H, s), 3.26 - 3.36 (2H, m), 6.84 (1H, s), 7.04 - 7.13 (2H, m), 7.34 - 7.45 (2H, m), 8.84 (1H, br s), 12.06 (1H, br s).
EIMS m/z (relative intensity): 432 (M⁺), 200 (100).

### Example 55 (Compound No. 806 in Table)

### Production of 7-(benzimidazol-2-ylthio)-N-[2,4-bis(methylthio)-6-methyl-3-pyridyl]heptanamide:

The reaction and the treatment were conducted in the same manner as in Example 47 except that 2-mercaptobenzimidazole was used instead of 2-mercaptobenzoxazole to obtain the desired compound as a colorless prism crystal.
Melting point: 189 - 192°C
IR (KBr) cm⁻¹: 3139, 2925, 2854, 1668, 1561, 1523, 1435, 1401.
¹H-NMR (d₆―DMSO) δ :
1.39 - 1.52 (4H, m), 1.56 - 1.70 (2H, m), 1.75 (2H. quint, J = 7.1 Hz), 2.28 - 2.34 (2H, m), 2.38 (3H, s), 2.40 (3H, s), 2.43 (3H, s), 3.27 (2H, t, J = 7.1 Hz), 6.84 (1H, s), 7.07 (1H, t, J = 7.1 Hz), 7.08(1H, t, J = 7.1 Hz), 7.32 (1H, d, J = 7.1 Hz), 7.46 (1H, d, J = 7.1 Hz), 8.79 (1H, br s).
EIMS m/z (relative intensity): 460 (M⁺), 150 (100).

### Example 56 (Compound No. 807 in Table)

### Production of 8-(benzimidazol-2-ylthio)-N-[2,4-bis(methylthio)-6-methyl-3-pyridyl]octanamide:

The reaction and the treatment were conducted in the same manner as in Example 48 except that 2-mercaptobenzimidazole was used instead of 2-mercaptobenzoxazole to obtain the desired compound as a colorless crystal.
Melting point: 186 - 187°C
IR (KBr) cm⁻¹ : 3430, 3222, 2925, 1661, 1564, 1522, 1437, 808.
¹H-NMR (d₆-DMSO) δ:
1.35 - 1.43 (4H, m), 1.47 (2H, quint, J = 7.2 Hz), 1.60 - 1.68 (2H, m), 1.76 (2H, quint, J = 7.2 Hz), 2.23 - 2.32 (2H, m), 2.40 (3H, s), 2.42 (3H, s), 2.45 (3H,s), 3.28 (2H, t, J = 7.2 Hz), 6.89 (1H, s), 7.09 (1H, t, J = 5.9 Hz), 7.09 (1H, t, J = 5.9 Hz), 7.40 (1H, d, J = 5.9 Hz), 7.41 (1H, d, J = 5.9 Hz), 8.80 (1H, br s). 12.09 (1H, br s).
EIMS m/z (relative intensity): 474 (M⁺), 150 (100).

### Example 57 (Compound No. 813 in Table)

### Production of 4-(benzoxazol-2-ylthio)-N-[2,4-bis(ethylthio)-6-methyl-3-pyridyl]butanamide;

The reaction and the treatment were conducted in the same manner as in Example 27 except that 4-bromobutanoyl chloride was used instead of 6-bromohexanoyl chloride to obtain the desired compound as a colorless crystal.
Melting point: 123 - 125°C
IR (KBr) cm⁻¹: 3436, 3239, 2974, 2929, 1656, 1502, 1454, 1130.
¹H-NMR (d₆-DMSO) δ:
1.23 - 1.28 (6H, m),2.12 - 2.19 (2H, m), 2.43 (3H, s), 2.48 - 2.50 (2H,m), 2.93 (2H, q, J = 7.1 Hz), 3.06 (2H, q, J = 7.1 Hz), 3.41 - 3.48 (2H, m), 6.89 (3H, s), 7.29 - 7.34 (2H, m), 7.56 - 7.62 (2H, m), 8.96 (1H, br s).
EIMS m/z (relative intensity) : 447 (M⁺), 227 (100).

### Example 58 (Compound No. 814 in Table)

### Production of 5-(benzoxazol-2-ylthio)-N-[2,4-bis(ethylthio)-6-methyl-3-pyridyl]pentanamide:

The reaction and the treatment were conducted in the same manner as in Example 27 except that 5-bromopentanoyl chloride was used instead of 6-bromohexanoyl chloride to obtain the desired compound as a colorless needle crystal.
Melting point: 122 - 123°C.
¹H-NMR (d₆―DMSO) δ :
1.25 (3H, t, J = 7.3 Hz), 1.26 (3H, t, J = 7,3 Hz), 1,76 - 1.87 (2H, m), 1.87 - 1.97 (2H, m), 2.29 - 2.40 (2H, m), 2.43 (3H, s), 2.92 (2H, q, J = 7.3 Hz), 3.05 (2H, q, J = 7.3 Hz), 3.38 (2H, t, J = 7.2 Hz), 6.88 (1H, s), 7,26 - 7.35 (2H, m), 7.55 - 7.60 (2H, m), 8.82 (1H, br s).
EIMS m/z (relative intensity): 461 (M⁺), 227 (100).

### Example 59 (Compound No. 816 in Table)

### Production of 7-(benzoxazol-2-ylthio)-M-[2,4-bis(ethylthio)-6-methyl-3-pyridyl]heptanamide:

The reaction and the treatment were conducted in the same manner as in Example 27 except that 7-bromoheptanoyl chloride was used instead of 6-bromohexanoyl chloride to obtain the desired compound as a colorless needle crystal.
Melting point: 103 - 105°C.
IR (KBr) cm⁻¹: 3247. 1663, 1501, 1455.
¹H-NMR (d₆-DMSO) δ :
1.24 (3H, t, J = 7.3 Hz), 1.25 (3H, t, J = 7.3 Hz), 1.38 - 1.54 (4H, m), 1.57 - 1.72 (2H, m), 1.73 - 1. 89 (2H, m), 2 .19 - 2.32 (2H, m), 2.41 (3H, s), 2.92 (2H, q, J = 7.3 Hz), 3.05 (2H, q, J = 7.3 Hz), 3.33 (2H, t, J = 7.1 Hz), 6.86 (1H, s), 7.24 - 7.32 (2H, m), 7.52 - 7.60 (2H, m), 8.65 (1H, br s).
EIMS m/z (relative intensity): 489 (M⁺), 228 (100).

### Example 60 (Compound No. 817 in Table)

### Production of 8-(benzoxazol-2-ylthio)-N-[2,4-bis(ethylthio)-6-methyl-3-pyridyl]octanamide:

The reaction and the treatment were conducted in the same manner as in Example 27 except that 8-bromooctanoyl chloride was used instead of 6-bromohexanoyl chloride to obtain the desired compound as a colorless needle crystal.
Melting point: 82 - 84°C
IR (KBr) cm⁻¹: 3449, 3245, 2932, 1669, 1500, 1455, 1132.
¹H-NMR (d₆ ―DMSO) δ :
1.26 (3H, t, J = 7.3 Hz), 1.27 (3H, t, J = 7.3 Hz), 1.37 - 1.42 (4H, m), 1.48 (2H, quint. J = 7.2 Hz), 1.60 - 1.67 (2H, m), 1.82 (2H, quint. J = 7.2 Hz), 2.24 - 2.30 (2H, m), 2.43 (3H, s), 2.94 (2H, q, J = 7.3 Hz), 3.07 (2H, q, J = 7.3 Hz), 3.34 (2H, t, J = 7.2 Hz), 6.88 (1H, s), 7.27 - 7.33 (2H, m), 7.56 - 7.61 (2H, m), 8.73 (1H, or s).
EIMS m/z (relative intensity): 503 (M⁺), 229 (100).

### Example 61 (Compound No. 823 in Table)

### Production of 4-(benzothiazol-2-ylthio)-N-[2,4-bis(ethylthio)-6-methyl-3-pyridyl]butanamide:

The reaction and the treatment were conducted in the same manner as in Example 57 except that 2-mercaptobenzothiazole was used instead of 2-mercaptobenzoxazole to obtain the desired compound as a colorless powdery crystal.
Melting point: 119 - 120°C
¹H-NMR (d₆-DMSO) δ :
1.25 (3H, t, J = 7.4 Hz), 1.26 (3H, t, J = 7.4 Hz), 2.07 - 2.23 (2H, m), 2.43 (3H, s), 2.45 - 2.55 (2H, m,), 2.93 (2H, q, J = 7.4 Hz), 3.06 (2H, q, J = 7.4 Hz), 3.41-3.54 (2H, m), 6.89 (1H, s), 7.35 (1H, t, J=8.1Hz), 7.45 (1H, t, J = 8.1 Hz), 7.83 (1H, d, J = 8.1 Hz). 7.94 (1H, d, J = 8.1 Hz), 8.95 (1H, br s).
EIMS m/z (relative intensity): 463 (M⁺), 229 (100).

### Example 62 (Compound No. 824 in Table)

### Production of 5-(benzothiazol-2-ylthio)-N-[2,4-bis(ethylthio)-6-methyl-3-pyridyl]pentanamide:

The reaction and the treatment were conducted in the same manner as in Example 58 except that 2-mercaptobenzothiazole was used instead of 2-mercaptobenzoxazole to obtain the desired compound as a colorless needle crystal.
Melting point: 102 - 104°C
¹H-NMR (d₆-DMSO) δ :
1.25 (3H, t, J = 7.3 Hz), 1.26 (3H, t, J = 7.3 Hz), 1.77 - 1.88 (2H, m), 1.88 - 2.00 (2H, m), 2.29 - 2.41 (2H, m), 2.43 (3H, s), 2.93 (2H, q, J = 7.3 Hz), 3.06 (2H, q, J = 7.3 Hz), 3.41 (2H, t, J = 7.0 Hz), 6.89 (1H, s), 7.35 (1H, ddd, J = 8.2 , 7.2 , 1.2 Hz), 7.45 (1H, ddd, J = 8.2 , 7.2 , 1.2 Hz), 7.84 (1H, dd. J - 8.2 , 1.2 Hz), 7.94 (1H, dd, J = 8.2 , 1.2 Hz), 8.84 (1H, br s).
EIMS m/z (relative intensity): 477 (M⁺), 229 (100).

### Example 63 (Compound No. 826 in Table)

### Production of 7-(benzothiazol-2-ylthio)-N-[2,4-bis(ethylthio)-6-methyl-3-pyridyl]heptanamide:

The reaction and the treatment were conducted in the same manner as in Example 59 except that 2-mercaptobenzothiazole was used instead of 2-mercaptobenzoxazole to obtain the desired compound as a colorless needle crystal.
Melting Point : 114 - 116°C
IR (KBr) cm⁻¹: 3245, 1665, 1536, 1509, 1426.
¹H-NMR (d₆―DMSO) δ :
1.24 (3H, t, J = 7.3 Hz), 1.25 (3H, t, J = 7.3 Hz), 1.39 - 1.56 (4H, m), 1.58 - 1.71 (2H, m), 1.75 - 1.88 (2H, m), 2.19 - 2.31 (2H, m), 2.42 (3H, s), 2.92 (2H, q, J = 7.3 Hz), 3.05 (2H, q, J = 7.3 Hz), 3.35 (2H, t, J = 7.2 Hz), 6.86 (1H, s), 7.32 (1H, td, J = 7.6 , 1.2 Hz), 7.42 (1H. td. J = 7.6 , 1.2 Hz). 7.81 (1H, dd, J = 7.6 , 1.2 Hz), 7.91 (1H, dd, J = 7.6 , 1.2 Hz), 8.67 (1H, br s).
EIMS m/z (relative intensity): 505 (M⁺), 227 (100).

### Example 64 (Compound No. 827 in Table)

### Production of 8-(benzothiazol-2-ylthio)-N-[2,4-bis(ethylthio)-6-methyl-3-pyridyl]octanamide:

The reaction and the treatment were conducted in the same manner as in Example 60 except that 2-mercaptobenzothiazole was used instead of 2-mercaptobenzoxazole to obtain the desired compound as a colorless needle crystal.
Melting point: 94 - 96°C
IR (KBr) cm⁻¹: 3433, 3243, 2929, 1669, 1511, 1428.
¹H-NMR (d₆-DMSO) δ :
1.26 (3H, t, J = 7.3 Hz), 1.27 (3H, t, J = 7.3 Hz), 1.37 - 1.43 (4H, m), 1.45 - 1.52 (2H, m), 1.57 - 1.68 (2H, m), 1.82 (2H, quint, J = 7.2 Hz), 2.20 - 2.32 (2H, m), 2.43 (3H, s), 2.94 (2H, q, J = 7.3 Hz), 3.07 (2H, q, J = 7.3 Hz), 3.37 (2H, t, J = 7.2 Hz), 6.88 (1H, s), 7.34 (1H, td, J = 7.6, 1.1 Hz), 7.44 (1H, td, J = 7.6, 1.1 Hz), 7.83 (1H, dd, J = 7.6 , 1.1 Hz), 7.93 (1H, dd, J = 7.6, 1.1 Hz), 8.73 (1H, br s).
EIMS m/z (relative intensity): 519 (M⁺), 227 (100).

### Example 65 (Compound No. 833 in Table)

### Production of 4-(benzimidazol-2-ylthio)-N-[2,4-bis(ethylthio)-6-methyl-3-pyridyl]butanamide:

The reaction and the treatment were conducted in the same manner as in Example 57 except that 2-mercaptobehzimidazole was used instead of 2-mercaptobenzoxazole to obtain the desired compound as a pale-yellow powdery crystal.
Melting point: 160 - 161°C
¹H-NMR (d₆-DMSO) δ :
1.25 (3H, t, J = 7.3 Hz), 1.26 (3H, t, J = 7.3 Hz), 2.27 - 2.37 (2H, m), 2.44 (3H, s), 2.48 - 2.50 (2H, m), 2.93 (2H, q, J = 7.3 Hz), 3.06 (2H, q, J = 7.3 Hz), 3.34 - 3.46 (2H, m), 6.89 (1H, s), 7.05 - 7.14 (2H, m), 7.33 (1H, m), 7.46 (1H, m), 8.95 (1H, br s).
EIMS m/z (relative intensity): 446 (M⁺), 195 (100).

### Example 66 (Compound No. 834 in Table)

### Production of 5-(benzimidazol-2-ylthio)-N-[2,4-bis(ethylthio)-6-methyl-3-pyridyl]pentanamide:

The reaction and the treatment were conducted in the same manner as in Example 58 except that 2-mercaptobenzimidazole was used instead of 2-mercaptobenzoxazole to obtain the desired compound as a colorless needle crystal.
Melting point: 163 - 165°C
¹H-NMR (d₆―DMSO) δ :
1.23 (3H, t, J = 7.3 Hz), 1.24 (3H, t, J = 7.3 Hz), 1.74 - 1.88 (4H, m), 2.27 - 2.38 (2H, m), 2.41 (3H, s), 2.90 (2H, q, J = 7.3 Hz), 3.03 (2H, q, J = 7.3 Hz), 3.26 - 3.34 (2H, m), 6.86 (1H, s), 7.04 - 7.11 (2H, m), 7.32 (1H, m), 7.46 (1H, m), 8.79 (1H, br s).
EIMS m/z (relative intensity): 460 (M⁺), 195 (100).

### Example 67 (Compound No. 836 in Table)

### Production of 7-(benzimidazol-2-ylthio)-N-[2,4-bis(ethylthio)-6-methyl-3-pyridyl]heptanamide:

The reaction and the treatment were conducted in the same manner as in Example 59 except that 2-mercaptobenzimidazole was used instead of 2-mercaptobenzoxazole to obtain the desired compound as a colorless needle crystal.
Melting point: 151 - 156°C
IR (KBr) cm⁻¹: 3136, 3106, 1656, 1518, 1438, 1401, 1337, 1268.
¹H-NMR (d₆-DMSO) δ :
1.24 (3H, t, J = 7.3 Hz), 1.25 (3H, t, J = 7.3 Hz), 1.36 - 1.54 (4H, m), 1.55 - 1.82 (4H, m), 2.15 - 2.32 (2H, m),
2.41 (3H, s), 2.92 (2H, q, J = 7.3 Hz), 3.05 (2H, g, J = 7.3 Hz),
3.26 (2H, t, J = 7.3 Hz), 6.86 (1H, s), 7.03 - 7.11 (2H, m), 7.34 - 7.44 (2H, m), 8.67 (1H, br s).
EIMS m/z (relative intensity): 488 (M⁺), 151 (100).

### Example 68 (Compound No. 837 in Table)

### Production of 8-(benzoimidazol-2-ylthio)-N-[2,4-bis(ethylthio)-6-methyl-3-pyridyl]octanamide:

The reaction and the treatment were conducted in the same manner as in Example 60 except that 2-mercaptobenzoimidazole was used instead of 2-mercaptobenzoxazole to obtain the desired compound as a colorless crystal.
Melting point: 166 - 168°C
IR (KBr) cm⁻¹: 3427, 3147, 2928, 1660, 1560, 1526, 1437.
¹H-NMR (d₆-DMSO) δ :
1.26 (3H, t, J = 7.3 Hz), 1.27 (3H, t, J = 7.3 Hz), 1.36 - 1.41 (4H, m), 1.47 (2H, quint, J = 7.2 Hz), 1.60 - 1.67 (2H, m), 1.75 (2H, quint, J = 7.2 Hz), 2.22 - 2.32 (2H, m), 2.43 (3H, s), 2.94 (2H, q, J = 7.3 Hz), 3.07 (2H, q, J = 7.3 Hz), 3.28 (2H, t, J = 7.2 Hz), 6.88 (1H, s), 7.08 (1H, t, J = 5.9 Hz), 7.09 (1H, t, J = 5.9 Hz), 7.40 (1H, d, J = 5.9 Hz), 7.41 (1H, d. J = 5.9 Hz), 8.73 (1H, br s).
EIMS m/z (relative intensity): 502 (M⁺), 151 (100).

### Example 69 (Compound No. 843 in Table)

### Production of 4-(benzoxazol-2-ylthio)-N-[2,4-bis(isopropylthio)-6-methyl-3-pyridyl]butanamide:

The reaction and the treatment were conducted in the same manner as in Example 36 except that 4-bromobutanoyl chloride was used instead of 6-bromohexanoyl chloride to obtain the desired compound as a colorless needle crystal.
Melting point: 128 - 129°C
IR (KBr) cm⁻¹: 3448, 3235, 2962, 1683, 1657, 1555, 1515, 1500, 1456, 1131.
¹H-NMR (d₆-DMSO), δ :
1.27 (6H, d, J = 6.6 Hz), 1.30 (6H, d, J = 6.8 Hz), 2.10 - 2.17 (2H, m), 2.42 (3H, s), 2.47 - 2.50 (2H, m), 3.39 - 3.47 (2H, m), 3.55 (1H, sept, J = 6.6 Hz), 3.89 (1H, sept, J = 6.8 Hz), 6.92 (1H, s), 7.28 (1H, td, J = 7.3 , 1.7 Hz), 7.30 (1H, td, J = 7.3 , 1.7 Hz), 7.56 (1H, dd, J = 7.3, 1.7 Hz), 7.58 (1H, dd, J = 7.3 , 1.7 Hz), 8.90 (1H, br s).
EIMS m/z (relative intensity): 475 (M⁺), 207 (100).

### Example 70 (Compound No. 844 in Table)

### Production of 5-(benzoxazol-2-ylthio)-N-[2,4-bis(isopropylthio)-6-methyl-3-pyridyl}pentanamide:

The reaction and the treatment were conducted in the same manner as in Example 36 except that 5-bromopentanoyl chloride was used instead of 6-bromohexanoyl chloride to obtain the desired compound as a colorless prism crystal.
Melting point: 129 - 130°C
IR (KBr) cm⁻¹: 3448, 3215, 3167, 2965, 1654, 1555, 1525, 1500, 1454, 1128.
¹H-NMR (d₆-DMSO) δ :
1.27 (6H, d, J = 6.8 Hz), 1.30 (6H, d, J = 6.8 Hz), 1.75 - 1.85 (2H, m), 1.86 - 1.96 (2H, m), 2.26 - 2.40 (2H, m), 2.42 (3H, s), 3.37 (2H, t, J = 7.1 Hz), 3.54 (1H, sept, J = 6.8 Hz), 3.88 (1H, sept, J = 6.8 Hz), 6.91 (1H, s), 7.27 (1H, td, J = 7.6 , 1.7 Hz), 7.30 (1H, td, J = 7.6 , 1.7 Hz), 7.55 (1H, dd, J = 7.6 , 1.7 Hz), 7.58 (1H, dd, J = 7.6 , 1.7 Hz), 8.75 (1H, br s).
EIMS m/z (relative intensity): 489 (M⁺), 221 (100).

### Example 71 (Compound No. 846 in Table)

### Production of 7-(benzoxazol-2-ylthio)-N-[2,4-bis(isopropylthio)-6-methyl-3-pyridyl]heptanamide:

The reaction and the treatment were conducted in the same manner as in Example 36 except that 7-bromoheptanoyl chloride was used instead of 6-bromohexanoyl chloride to obtain the desired compound as a colorless needle crystal.
Melting point: 76 - 78°C
IR (KBr) cm⁻¹: 3436, 3265, 2929, 1663, 1503, 1455.
¹H-NMR (d₄-DMSO) δ:
1.29 (6H, d, J = 6.8 Hz), 1.32 (6H, d, J = 6.8 Hz), 1.43 - 1.54 (4H. m), 1.65 (2H, quint, J = 7.2 Hz), 1.83 (2H, quint, J = 7.2 Hz), 2.20 - 2.33 (2H, m), 2.43 (3H, s), 3.35 (2H, t, J = 7.2 Hz), 3.56 (1H, sept, J = 6.8 Hz), 3.90 (1H, sept, J = 6.8 Hz), 6.93 (1H, s), 7.27 - 7.34 (2H, m), 7.56 - 7.61 (2H, m), 8.72 (1H, br s).
EIMS m/z (relative intensity): 517 (M⁺), 249 (100).

### Example 72 (Compound No. 847 in Table)

### Production of 8-(benzoxazol-2-ylthio)-N-[2,4-bis(isopropylthio)-6-methyl-3-pyridyl]octanamide:

The reaction and the treatment were conducted in the same manner as in Example 36 except that 8-bromooctanoyl chloride was used instead of 6-bromohexanoyl chloride to obtain the desired compound as a colorless oil.
IR (KBr) cm⁻¹: 3241, 1664, 1559, 1526, 1501, 1454.
¹H-NMR (d₆-DMSO) 6 :
1.29 (6H, d, J = 6.8 Hz), 1.31 (6H, d, J = 6.8 Hz), 1.34 - 1.54 (6H, m), 1.55 - 1.69 (2H, m), 1.73 - 1.89 (2H, m), 2.15 - 2.28 (2H, m), 2.42 (3H, s), 3.27 (2H, t, J = 7.3 Hz), 3.54 (1H, sept, J = 6.8 Hz), 3.89 (1H, sept, J = 6.8 Hz), 6.90 (1H, s), 7.24 - 7.32 (2H, m), 7.51 - 7.60 (2H, m), 8.59 (1H, br s).
EIMS m/z (relative intensity): 531 (M⁺), 263 (100).

### Example 73 (Compound No. 848 in Table)

### Production of 9- (benzoxazol-2-ylthio)-N-[2,4-bis(isopropylthio)-6-methyl-3-pyridyl]nonanamide:

The reaction and the treatment were conducted in the same manner as in Example 36 except that 9-bromononanoyl chloride was used instead of 6-bromohexanoyl chloride to obtain the desired compound as a pale yellow oil.
IR (Cap) cm⁻¹ : 3243, 2962, 2927, 1668, 1558, 1505, 1455, 1130.
¹H-NMR (d₆-DMSO) δ :
1.28 (6H, d, J = 6.8 Hz) 1.31 (6H, d, J = 6.8 Hz) 1.28 - 1.50 (8H, m), 1.55 - 1.65 (2H, m), 1.80 (2H, quint, J = 7.3 Hz), 2.17 - 2.27 (2H, m), 2.42 (3H, s), 3.32 (2H, t, J = 7.3 Hz), 3.55 (1H, sept, J = 6.8 Hz), 3.89 (1H, sept, J = 6.8 Hz), 6.91 (1H, s), 7.27 (1H, td, J = 7.3 , 1.7 Hz), 7.30 (1H, td, J = 7.3 , 1.7 Hz), 7.54 - 7.60 (2H, m), 8.65 (1H, br s).
EIMS m/z (relative intensity): 545 (M⁺), 277 (100).

### Example 74 (Compound No. 851 in Table)

### Production of 2-(benzothiazol-2-ylthio)-N-[2,4-bis(isopropylthio)-6-methyl-3-pyridyl]acetamide:

The reaction and the treatment were conducted in the same manner as in Example 49 except that 2-bromo-N-[2,4-bis(isopropylthio)-6-methy-3-pyridyl]acetamide was used instead of 2-bromo-N-2,4-bis(methylthio)-6-methyl-3-pyridyl]acetamide to obtain the desired compound as a colorless needle crystal.
Melting point: 117 - 118°C
IR (KBr) cm⁻¹: 3431, 3179, 2967, 1660, 1559, 1526, 1428.
¹H-NMR (CDCl₃) δ:
1.19 (6H, d, J = 6.7 Hz), 1.21 (6H, d, J = 6.7 Hz), 2.41 (3H, s), 3.39 (1H, sept, J = 6.7 Hz), 3.92 (1H, sept, J = 6.7 Hz), 4.18 (2H, s), 6.68 (1H, s), 7.32 (1H, td, J = 7.7 , 1.2 Hz), 7.41 (1H, td, J = 7.7 1.2 Hz), 7.77 (1H, d, J = 7.7 Hz), 7.91 (1H, d, J = 7.7 Hz), 8.80 (1H, br s).
EIMS m/z (relative intensity): 463 (M⁺), 180 (100).

| Elemental Analysis : as C₂₁H₂₅N₃OS₄ | | | | | |
|---|---|---|---|---|---|
| | Calculated : | C, 54.39; | H, 5.43; | N, 9.06; | S, 27.66. |
| | Found : | C, 54-28; | H, 5.45; | N, 8.93; | S, 27.73. |

### Example 75 (Compound No. 853 in Table)

### Production of 4-(benzothiazol-2-ylthio)-N-[2,4-bis(isopropylthio)-6-methyl-3-pyridyl]butanamide:

The reaction and the treatment were conducted in the same manner as in Example 69 except that 2-mercaptobenzothiazole was used instead of 2-mercaptobenzoxazole to obtain the desired compound as a colorless powdery crystal.
Melting point: 116 - 117°C
IR (KBr) cm⁻¹: 3450, 3257, 2962, 1667, 1557, 1510, 1457, 1429, 987.
¹H-NMR (d₆-DMSO) δ :
1.27 (6H, d, J = 6.8 Hz), 1.30 (6H, d, J = 6.8 Hz), 2.08 - 2.17 (2H. m), 2.42 (3H, s), 2.43 - 2.47 (2H, m), 3.45 (2H, t, J = 7.1 Hz), 3.55 (1H, sept, J = 6.8 Hz), 3.89 (1H, sept, J = 6.8 Hz), 6.92 (1H, s), 7.33 (1H, t, J = 7.8 Hz), 7.43 (1H, t, J = 7.8 Hz), 7.81 (1H, d, J = 7.8 Hz), 7.92 (1H, d, J = 7.8 Hz), 8.90 (1H, br s).
EIMS m/z (relative intensity): 491 (M⁺), 69 (100).

### Example 76 (Compound No. 854 in Table)

### Production of 5-(benzotbLiazol-2-ylthio)-N-[2,4-bis(isopropylthio)-6-methyl-3-pyriglyl]pentanamide:

The reaction and the treatment were conducted in the same manner as in Example 70 except that 2-mercaptobenzothiazole was used instead of 2-mercaptobenzoxazole to obtain the desired compound as a colorless powdery crystal.
Melting point: 107 - 109°C
IR (KBr) cm⁻¹: 3441, 3215, 2963, 1656, 1557, 1523, 1460, 1429, 996.
¹H-NMR (d₆-DMSO) δ:
1.27 (6H, d, J = 6.8 Hz), 1.30 (6H, d, J = 6.8 Hz), 1.76 - 1.85 (2H, m), 1.86 - 1.96 (2H, m), 2.26 - 2.40 (2H, m), 2.42 (3H, s), 3.39 (2H, t, J = 7.1 Hz), 3.54 (1H, sept, J = 6.8 Hz), 3.89 (1H, sept, J = 6.8 Hz), 6.91 (1H, s), 7.33 (1H, td, J = 8.1 , 1.2 Hz), 7.43 (1H, td, J = 8.1, 1.2 Hz), 7.82 (1H, dd, J = 8.1, 1.2 Hz), 7.92 (1H, dd, J = 8.1, 1.2 Hz), 8.75 (1H, br s).
EIMS m/z (relative intensity): 505 (M⁺), 221 (100).

### Example 77 (Compound No. 855 in Table)

### Production of 6-(benzothiazol-2-ylthio)-N-[2,4-bis(isopropylthio)-6-methyl-3-pyridyl]hexanamide:

The reaction and the treatment were conducted in the same manner as in Example 36 except that 2-mercaptobenzothiazole was used instead of 2-mercaptobenzoxazole to obtain the desired compound as a colorless crystal.
Melting point: 84 - 86°C
IR (KBr) cm⁻¹: 3436, 3212, 2961, 2925, 1655, 1555, 1522, 1428.
¹H-NMR (d₆―DMSO) δ:
1.30 (6H, d, J = 6.6 Hz), 1.33 (6H, d, J = 6.8 Hz), 1.54 - 1.62 (2H, m), 1.65 - 1.73 (2H, m), . 1.85 (2H, quint, J = 7.0 Hz), 2.22 - 2.33 (2H, m), 2.43 (3H, s),
3.38 (2H, t, J = 7.0 Hz), 3.57 (1H, sept, J = 6.6 Hz), 3.91 (1H, sept, J = 6.8 Hz), 6.93 (1H, s), 7.34 (1H, t, J = 7.8 Hz), 7.44 (1H, t, J = 7.8 Hz), 7.83 (1H, d, J = 7.8 Hz), 7.93 (1H, d, J = 7.8 Hz), 8.73 (1H, br s).
EIMS m/z (relative intensity): 519 (M⁺), 235 (100).

### Example 78 (Compound No. 856 in Table)

### Production of 7-(benzothiazol-2-ylthio)-N-[2,4-bis(isopropylthio)-6-methyl-3-pyridyl]heptanamide:

The reaction and the treatment were conducted in the same manner as in Example 71 except that 2-mercaptobenzothiazole was used instead of 2-mercaptobenzoxazole to obtain the desired compound as a colorless crystal.
Melting point: 74 - 76°C
IR (KBr) cm⁻¹: 3436, 3200, 3158, 2961, 2928, 1654, 1525, 1427.
¹H-NMR (d₆―DMSO) δ:
1.29 (6H, d, J = 6.6 Hz), 1.32 (6H, d, J = 6.8 Hz), 1.43 - 1.55 (4H, m), 1.65 (2H, quint, J = 7.2 Hz), 1.83 (2H, quint, J = 7.2 Hz), 2.22 - 2.33 (2H, m), 2.43 (3H, s), 3.37 (2H, t, J = 7.2 Hz), 3.56 (1H, sept, J = 6.6 Hz), 3.90 (1H, sept, J = 6.8 Hz), 6.93 (1H, s), 7.34 (1H, td, J = 7.7, 1.2 Hz), 7.44 (1H, td, J = 7.7, 1.2 Hz), 7.83 (1H, dd, J = 7.7, 1.2 Hz), 7.94 (1H, dd, J = 7.7, 1.2 Hz), 8.68 (1H, br s).
EIMS m/z (relative intensity): 533 (M⁺), 249 (100),

### Example 79 (Compound No. 857 in Table)

### Production of 8-(benzothiazol-2-ylthio)-N-[2,4-bis(isopropylthio)-6-methyl-3-pyridyl]octanamide:

The reaction and the treatment were conducted in the same manner as in Example 72 except that 2-mercaptobenzothiazole was used instead of 2-mercaptobenzoxazole to obtain the desired compound as a colorless neddle crystal.
Melting point: 107 - 108°C
IR (KBr) cm⁻¹: 3239, 1664, 1559, 1526, 1456, 1428.
¹H-NMR (d₆-DMSO) **δ** :
1.29 (6H, d, J = 6.8 Hz), 1.31 (6H, d, J = 6.8 Hz), 1.34 - 1.54 (6H, m), 1.55 - 1.70 (2H, m), 1.73 - 1.88 (2H, m), 2.15 - 2.29 (2H, m), 2.42 (3H, s), 3.35 (2H, t, J = 7.3 Hz), 3.54 (1H. sept. J = 6.8 Hz), 3.89 (1H, sept, J = 6.8 Hz), 6.90 (1H, s), 7.31 (1H, t, J = 7.8 Hz), 7.42 (1H, t, J = 7.8 Hz), 7.81 (1H, d, J = 7.8 Hz), 7.90 (1H, d, J = 7.8 Hz), 8.59 (1H, br s).
EIMS m/z (relative intensity): 547 (M⁺), 263 (100).

### Example 80 (Compound No. 858 in Table)

### Production of 9-(benzothiazol-2-ylthio)-N-[2,4-bis (isopropylthio)-6-methyl-3-pyridyl]nonanamide:

The reaction and the treatment were conducted in the same manner as in Example 73 except that 2-mercaptobenzothiazole was used instead of 2-mercaptobenzoxazole to obtain the desired compound as a pale yellow oil.
IR (Cap) cm⁻¹: 3243, 2962, 2927, 1668, 1559, 1526, 1456.
¹H-NMR (d₆-DMSO) δ :
1.28 (6H, d, J = 6.8 Hz), 1.31 (6H, d, J = 6.8 Hz), 1.28 - 1.50 (8H, m), 1.55 - 1.65 (2H, m), 1.80 (2H, quint, J = 7.0 Hz), 2.17 - 2.27 (2H, m), 2.42 (3H, s), 3.34 (2H, t, J = 7.0 Hz), 3.55 (1H, sept, J = 6.8 Hz), 3.89 (1H, sept, J = 6.8 Hz), 6.91 (1H, s), 7.32 (1H, td, J = 7.1 , 1.2 Hz), 7.43 (1H, td, J = 7.1, 1.2 Hz), 7.81 (1H, dd, J = 7.1, 1.2 Hz), 7.91 (1H, dd, J = 7.1, 1.2 Hz), 8.65 (1H, br s).
EIMS m/z (relative intensity): 561 (M⁺), 277 (100).

### Example 81 (Compound No. 861 in Table)

### Production of 2-(benzimidazol-2-ylthio)-N-[2,4-bis(isopropylthio)-6-methyl-3-pyridyl]acetamide:

The reaction and the treatment were conducted in the same manner as in Example 53 except that 2-bromo-N-[2,4-bis(isopropylthio)-6-methylpyridyl]acetamide was used instead of 2-bromo-N-[2,4-bis(methylthio)-6-methylpyridyl]acetamide to obtain the desired compound as a colorless needle crystal.
Melting point: 223 - 224°C
IR (KBr) cm⁻¹**:** 3437, 3138, 3106, 2960, 1668, 1534, 1414.
¹H-NMR (CDCl₃) δ :
1.22 (6H, d, J = 6.8 Hz), 1.25 (6H, d, J = 6.8 Hz), 2.42 (3H, s), 3.41 (1H, sept, J = 6.8 Hz), 3.95 (1H, sept, J = 6.8 Hz), 4.05 (2H, s), 6.69 (1H, s), 7.18 (1H, t, J = 6.1 Hz), 7.19 (1H, t, J = 6.1 Hz), 7.34 (1H, br s), 7.62 (1H, br s), 9.33 (1H, br s), 10.61 (1H, br s).
EIMS m/z (relative intensity): 446 (M⁺), 371 (100).

| Elemental analysis: as C₂₁H₂₆N₄OS₃ | | | | |
|---|---|---|---|---|
| | calculated: | C, 56.47; | H, 5.87; | N, 12.54. |
| | found: | C, 56.42; | H, 5.87; | N, 12.56. |

### Example 82 (Compound No. 863 in Table)

### Production of 4-(benzomidazol-2-ylthio)-N-[2,4-bis(isopropylthio)-6-methyl-3-pyridyl]butanamide:

The reaction and the treatment were conducted in the same manner as in Example 69 except that 2-mercaptobenzimidazole was used instead of 2-mercaptobenzoxazole to obtain the desired compound as a pale-yellow powdery crystal.
Melting point: 209 - 211°C
IR (KBr) cm⁻¹: 3480, 3196, 2963, 1664, 1557, 1529, 1428.
¹H-NMR (d₆―DMSO) δ:
1.25 (6H, d, J = 6.8 Hz), 1.28 (6H, d, J = 6.8 Hz), 2.04 (2H, quint, J = 7.1 Hz), 2.43 (3H, s), 2.44 (2H, t, J = 7.1 Hz), 3.36 (2H, t, J = 7.1 Hz), 3.61 (1H, sept, J = 6.8 Hz), 3.86 (1H, sept, J = 6.8 Hz), 6.96 (1H, s), 7.09 (1H, dd, J = 7.3 , 5.4 Hz), 7.12 (1H, dd, J = 7.3 , 5.4 Hz), 7.35 (1H, m), 7.49 (1H, m), 9.38 (1H, s), 12.53 (1H, s).
EIMS m/z (relative intensity): 474 (M⁺), 207 (100).

### Example 83 (Compound No. 864 in Table)

### Production of 5-(benzimidazol-2-ylthio)-N-[2,4-bis (Isopropylthio)-6-methyl-3-pyridyl]pentanamide:

The reaction and the treatment were conducted in the same manner as in Example 70 except that 2-mercaptobenimidazole was used instead of 2-mercaptobenzoxazole to obtain the desired compound as a colorless powdery crystal.
Melting point: 175 - 176°C
IR (KBr) cm⁻¹: 3447, 3195, 2965, 1663, 1557, 1526, 1428, 1400.
¹H-NMR (d₆-DMSO) δ :
1.28 (6H, d, J = 6.8 Hz), 1.30 (6H, d, J = 6.8 Hz), 1.75 - 1.90 (4H, m), 2.26 - 2.38 (2H, m), 2.42 (3H, s), 3.30 (2H, t, J = 7.1 Hz), 3.54 (1H, sept, J = 6.8 Hz), 3.88 (1H, sept, J = 6.8 Hz), 6.91 (1H, s), 7.07 (1H, t, J = 6.1 Hz), 7.08 (1H, t, J = 6.1 Hz), 7.32 (1H, d, J = 6.1 Hz), 7.46 (1H, d, J = 6.1 Hz), 8.72 (1H, br s).
EIMS m/z (relative intensity): 488 (M⁺), 221 (100).

### Example 84 (Compound No. 865 in Table)

### Production of 6-(benzimidazol-2-ylthio)-N-[2,4-bis(isopropylthio)-6-methyl-3-pyridyl]hexanamide:

The reaction and the treatment were conducted in the same manner as in Example 36 except that 2-mercaptobenzimidazole was used instead of 2-mercaptobenzoxazole to obtain the desired compound as a colorless powdery crystal.
Melting point: 175 - 176°C
¹H-NMR (d₆-DMSO) δ :
1.30 (6H, d, J = 6.7 Hz), 1.32 (6H. d, J = 6.7 Hz), 1.47 - 1.61 (2H, m), 1.62 - 1.72 (2H, m), 1.73 - 1.84 (2H, m), 2.18 - 2.35 (2H, m), 2.43 (3H, s), 3.21 - 3.33 (2H, m), 3.55 (1H, sept, J = 6.7 Hz), 3.90 (1H, sept, J = 6.7 Hz), 6.92 (1H, s), 7.03 - 7.12 (2H, m), 7.33 (1H, m), 7.47 (1H, m), 8.75 (1H, br s), 12.05 (1H, br s).
EIMS m/z (relative intensity): 502 (M⁺), 235 (100).

### Example 85 (Compound No. 866 in Table)

### Production of 7-(benzoimidazol-2-ylthio)-N-[2,4-bis(isopropylthio)-6-methyl-3-pyridyl]heptanamide:

The reaction and the treatment were conducted in the same manner as in Example 71 except that 2-mercaptobenzoimidazole was used instead of 2-mercaptobenzoxazole to obtain the desired compound as a pale-yellow needle crystal.
Melting point: 118 - 121°C
IR (KBr) cm⁻¹: 3393, 3219, 2963, 2928, 1663, 1559, 1526, 1439.
¹H-NMR (d₆―DMSO ) δ :
1.29 (6H, d, J = 6.6 Hz), 1.32 (6H, d, J = 6.8 Hz), 1.41 - 1.53 (4H, m), 1.64 (2H, quint, J = 7.2 Hz), 1.76 (2H, quint, J = 7.2 Hz), 2.18 - 2.33 (2H, m), 2.43 (3H, s), 3.28 (2H, t, J = 7.2 Hz), 3.56 (1H, sept, J = 6.6 Hz). 3.90 (1H, sept, J = 6.8 Hz), 6.93 (1H, s), 7.08 (1H, t, J = 5.9 Hz), 7.09 (1H, t, J = 5.9 Hz), 7.40 (1H, d, J = 5.9 Hz). 7.41 (1H, d, J = 5.9 Hz), 8.86 (1H, br s).
EIMS m/z (relative intensity): 516 (M⁺), 399 (100).

### Example 86 (Compound No. 867 in Table)

### Production of 8-(benzimidazol-2-ylthio)-N-[2,4-bis(isopropylthio)-6-methyl-3-pyridyl]octanamide:

The reaction and the treatment were conducted in the same manner as in Example 72 except that 2-mercaptobenzimidazole was used instead of 2-mercaptobenzoxazole to obtain the desired compound as a colorless powdery crystal.
Melting point: 170 - 171°C
IR (KBr) cm⁻¹ : 3158, 2963, 2930, 1665, 1559, 1526, 1508, 1429.
¹H-NNR (d₆-DMSO) δ :
1.28 (6H, d, J = 6.8 Hz). 1.31 (6H. d, J = 6.8 Hz) 1.32 - 1.50 (6H, m), 1.56 - 1.66 (2H, m), 1.74 (2H, quint, J = 7.3 Hz), 2.17 - 2.27 (2H, m), 2.42 (3H, s), 3.26 (2B. t, J = 7.3 Hz), 3.54 (1H, sept, J = 6.8 Hz), 3.89 (1H, sept, J = 6.8 Hz), 6.91 (1H, s), 7.05 - 7.10 (2H, m), 7.32 (1H, m), 7.45 (1H, m), 8.65 (1H, br s).
EIMS m/z (relative intensity): 530 (M⁺). 413 (100).

### Example 87 (Compound No. 868 in Table)

### Production of 9-(benzimidazol-2-ylthio)-N-[2,4-bis(isopropylthio)-6-methyl-3-pyridyl]nonanamide:

The reaction and the treatment were conducted in the same manner as in Example 73 except that 2-mercaptobenzimidazole was used instead of 2-mercaptobenzoxazole to obtain the desired compound as a pale brown powdery crystal.
Melting point: 112 - 114°C
IR (KBr) cm⁻¹ : 3435, 3185, 2927, 1660, 1558, 1526, 1437.
¹H-NMR (d₆―DMSO) δ:
1.28 (6H, d, J = 6.8 Hz) 1.31 (6H, d, J = 6.8 Hz) 1.28 - 1.48 (8H, m), 1.52 - 1.65 (2H, m), 1.73 (2H, quint, J = 7.1 Hz), 2.18 - 2.28 (2H, m), 2.42 (3H, s), 3.25 (2H, t, J = 7.1 Hz), 3.55(1H, sept, J = 6.8 Hz), 3.89 (1H, sept, J = 6. 8Hz), 6.91 (1H, s), 7.07 (1H, t, J = 6.1 Hz), 7.08 (1H, t, J= 6.1 Hz), 7.32 (1H, d, J = 6.1 Hz), 7.46 (1H, d, J = 6.1 Hz), 8.80 (1H, br s), 12.05 (1H, br s).
EIMS m/z (relative intensity): 544 (M⁺), 151 (100).

### Example 88 (Compound No. 1145 in Table)

### Production of 6-(benzoxazole-2-ylthio)-N-[2-methyl-4,6-bis(methylthio)-5-pyrimidyl)hexanamide:

4,6-Dihydroxy-2-methylpyrimidine (1.0 g, 7.9 mmol) was added gradualy to ice-cooled fuming nitric acid (3 ml) stirring. The mixture was stirred for 2 hours cooling with ice and for 1 hour at the room temperature, and then the precipitated crystal was filtered and dried to obtain 207 mg (yield 15%) of 4,6-dihydroxy-2-methy-5-nitropyrimidine.

This nitropyrimidine (205 mg, 1.2 mmol) was dissolved in phosphoryl chloride (1ml) and diethylaniline (0.3 ml, 1.9 mmol) was added thereto, and the mixture was stirred for 1 hour at 100°C and for 1 hour at 120 °C. The reaction solution was added to ice and then extracted with ethyl acetate. The organic layer was washed with water and then with a saturated aqueous solution of sodium chloride, and dried over magnesium sulfate. Thereafter, the solvent was distilled off, and the resulting crude product was purified through silica gel chromatography (eluent-hexane: ethyl acetate = 20:1) to obtain 194 mg (yield 77%) of 4,6-dichloro-2-methyl-5-nitropyrimidine as a colorless needle crystal.

And then a methanol (10 mml) solution of 4,6-dichloro-2-methyl-5-nitropyrimidine (1.0 g. 4.81 mmol) was added dropwise to a methanol (10 ml) solution of sodium thiomethoxide (780 mg, 10.6 mmol) while being cooled with ice, and after the mixture was stirred for 1 hour while being cooled with ice, water added to the reaction mixture and the mixture was extracted with ethyl acetate. The organic layer was washed with water and then with a saturated aqueous solution of sodium chloride, and dried over magnesium sulfate. Thereafter, the solvent was distilled off, and the resulting crude product was recrystalized with ethyl acetate-hexan to obtain 609 mg (yield 55%) of 4,6-bis(methylthio)-2-methyl-5-nitropyrimidine.

Potassium carbonate (119 mg, 0.865 mmol) and pratinum dioxide (40 mg, 0.18 mmol) were added to ethanol (100 ml) solution of this nitropyrimidine (100 mg, 0.43 mmol) and stirred in hydrogen. After 1. 5 hours, the reaction mixture was filtered, the fltrate was distilled off, and the resulting crude product was purified through silica gel chromatography (eluent - hexane:ethyl acetate = 6:1) to obtain 66 mg. (yield 76%) of 5-amino-4,6-bis(methylthio)-2-methylpyrimidine.

And then the reaction and the treatment were conducted in the same manner as in Example 18 except that 5-amino-4,6-bis(methylthio)-2-methylthiopyrimidine was used instead of 3-amino-2,4-bis(methlthio)-6-methylpyridine to obtain the desired compound as a colorless powdery crystal.
Melting point: 148 - 151°C
IR (KBr) cm⁻¹ : 3440, 3245, 2929, 1660, 1530.
¹H-NMR (CDCl₃) δ :
1.43 - 1.55 (2H, m), 1.57 - 1.69 (2H, m), 1.72 - 1.84 (2H,m), 2.14 - 2.29 (2H, m), 2.38 (6H, s), 2.48 (3H, m), 3.28 (2H, t, J = 7.3 Hz), 7.21 (1H, td, J = 7.4 , 1.7Hz), 7.24 (1H, td, J=7.4 , 1.7Hz), 7.49 (1H, dd, J=7.4Hz), 7.51 (1H, dd, J = 7.4 , 1.7 Hz), 8.91 (1H, br s).
EIMS m/z (relative intensity): 448 (M⁺, 100).

### Example 89 (Compound No. 1247 in Table)

### Production of 2-(7-trifluoromethylbenzoxazol-2-ylthio)-N-[2,4-bis(methylthio)-6-methyl-3-pyridyl]acetamide:

The reaction and the treatment were conducted in the same manner as in Example 49 except that 2-mercapto-7-trifluoromethylbenzoxazole was used instead of 2-mercaptobenzoxazole to obtain the desired compound as a colorless needle crystal.
Melting point: 207 - 209°C
IR (KBr) cm⁻¹: 3435, 3235, 1673, 1509, 1433, 1329, 1130.
¹H-NMR (CDCl₃) δ:
2.32 (3H, s), 2.41 (3H, s), 2.48 (3H, s), 4.14 (2H,s), 6.81 (1H, s), 7.41 (1H, t, J = 7.8 Hz), 7.52 (1H. d, J = 7.8 Hz), 7.79 (1H, d. J = 7.8 Hz), 8.46 (1H, br s).
EIMS m/z (relative intensity): 459 (M⁺), 227 (100).

| Elemental analysis: as C₁₈H₁₆F₃N₃O₂S₃ | | | | |
|---|---|---|---|---|
| | Calculated | : C, 47.05; | H, 3.51; | N, 9.14. |
| | Found | : C, 46. 84; | H, 3.66 ; | N, 9. 03. |

### Example 90 (Compound No. 1250 in Table)

### Production of 5-(7-trifluoromethylbenzoxazol-2-ylthio)-N-[2,4-bis(methylthio)-6-methyl-3-pyridyl]pentanamide:

The reaction and the treatment were conducted in the same manner as in Example 46 except that 2-mercapto-7-trifluoromethylbenzoxazole was used instead of 2-mercaptobenzoxazole to obtain the desired compound as a colorless crystal.
Melting point: 179 - 180°C.
¹H-NMR (d₆-DMSO) δ :
1.75 - 1.87 (2H, m), 1.87 - 2.00 (2H, m), 2.37 (3H, s), 2.39 (3H, s), 2.30 - 2.39 (2H, m), 2.43 (3H, s), 3.36 - 3.46 (2H, m), 6.84 (1H, s), 7.50 (1H, t, J = 7.9 Hz), 7.59 (1H, d, J = 7.9 Hz), 7.89 (1H, d, J = 7.9 Hz), 8.85 (1H, br s).
EIMS m/z (relative intensity): 501 (M⁺), 200 (100).

### Example 91 (Compound No. 1252 in Table)

### Production of 7-(7-trifluoromethylbenzoxazol-2-ylthio)-N-[2,4-bis(methylthio)-6-methyl-3-pyridyl]heptanamide:

The reaction and the treatment were conducted in the same manner as in Example 47 except that 2-mercapto-7-trifluoromethylbenzoxazole was used instead of 2-mercaptobenzoxazole to obtain the desired compound as a colorless needle crystal.
Melting point: 129 - 131°C
IR (KBr) cm⁻¹ : 3247, 1662, 1505, 1435, 1337, 1128.
¹H-NMR (d₆―DMSO) δ:
1.40 - 1.55 (4H, m), 1.60 - 1.71 (2H, m), 1.80 - 1.89 (2H,m), 2.20 - 2.34 (2H, m), 2.38 (3H, s), 2.40 (3H, s), 2.44 (3H, s), 3.37 (2H, t, J = 7.1 Hz), 6.84 (1H, s), 7.49 (1H, t, J = 7.8 Hz), 7.58 (1H, d, J = 7.8 Hz), 7.88 (1H, d, J = 7.8 Hz), 8.78 (1H, br s).
EIMS m/z (relative intensity): 529 (M⁺), 200 (100).

### Example 92 (Compound No. 1253 in Table)

### Production of 8-(7-trifluoromethylbenzoxazol-2-ylthio)-N-[2,4-bis(methylthio)-6-methyl-3-pyridyl]octanamide:

The reaction and the treatment were conducted in the same manner as in Example 48 except that 2-mercapto-7-trifluoromethylbenzoxazole was used instead of 2-mercaptobenzoxazole to obtain the desired compound as a colorless powdery crystal.
Melting point: 115 - 116°C
¹H-NMR (d₆-DMSO) δ :
1.40 - 1.54 (6H, m), 1.56 - 1.72 (2H, m), 1.85 (2H, quint, J = 7.0 Hz), 2.18 - 2.36 (2H, m), 2.40 (3H, s), 2.43 (3H, s), 2.46 (3H, s), 3.38 (2H, t, J = 7.3 Hz), 6.86 (1H, s), 7.51 (1H, t, J = 7.5 Hz), 7.60 (1H, d, J = 7.5 Hz), 7.90 (1H, d, J = 7.5 Hz), 8.16 (1H, br s).
EIMS m/z (relative intensity): 543 (M⁺), 200 (100).

### Example 93 (Compound No. 1260 in Table)

### Production of 5-(5-chloro-7-isopropyl-4-methylbenzoxazol-2-ylthio)-N-[2,4-bis(methylthio)-6-methyl-3-pyridyl]pentanamide:

The reaction and the treatment were conducted in the same manner as in Example 46 except that 5-chloro-7-isopropyl -2-mercapto-4-metylbenzoxazole was used instead of 2-mercaptobenzoxazole to obtain the desired compound as a colorless needle crystal.
Melting point: 155 - 156°C.
¹H-NMR (d₆-DMSO) δ :
1.31 (6H, d. J = 7.1 Hz), 1.72 - 1.85 (2H, m), 1.85-1.98 (2H, m),
2.36 (3H, s);, 2.39 (3H, s), 2.32 - 2.40 (2H, m),
2.43 (3H, s), 2.46 (3H, s), 3.22 (1H, sept, J = 7.1Hz),
3.31 - 3.42 (2H, m), 6.84 (1H, s), 7.13 (1H, s), 8.73 (1H, br s).
EIMS m/z (relative intensity): 525 (M⁺:³⁷Cl) , 523 (M⁺:³⁵Cl), 200 (100).

### Example 94 (Compound No. 1262 in Table)

### Production of 7-(5-chloro-7-isopropyl-4-methylbenzoxazol-2-ylthio)-N-[2,4-bis(methylthio)-6-methyl-3-pyridyl]heptanamide:

The reaction and the treatment were conducted in the same manner as in Example 47 except that 5-chloro-7-isopropyl-2-mercapto-4-metylbenzoxazole was used instead of 2-mercaptobenzoxazole to obtain the desired compound as a colorless prism crystal.
Melting point: 129 - 131°C
IR (KBr) cm⁻¹ : 3413, 3241, 2964, 2924, 1655, 1567, 1505, 1490, 1435, 1149.
¹H-NMR (d₆-DMSO) δ:
1.31 (6H, d, J = 7.1 Hz), 1.40 - 1.55 (4H. m), 1.56 - 1.70 (2H, m), 1.83 (2H, quint, J = 7.1 Hz), 2.30 (2H, t, J = 7.1 Hz), 2.38 (3H, s), 2.40 (3H, s), 2.41 (3H, s), 2.46 (3H,s), 3.21 (1H, sept, J = 7.1 Hz), 3.34 (2H, t, J = 7.1 Hz), 6.84 (1H, s), 7.14 (1H, s), 8.51 (1H, br s).
EIMS m/z (relative intensity) : 553 (M⁺:³⁷Cl), 551 (M⁺:³⁵Cl), 200 (100).

### Example 95 (Compound No. 1260 in Table)

### Production of 8-(5-chloro-7-isopropyl-4-methylbenzoxazol-2-ylthio)-N-[2,4-bis(methylthio)-6-methyl-3-pyridyl]octanamide:

The reaction and the treatment were conducted in the same manner as in Example 48 except that 5-chloro-7-isopropyl-2-mercapto-4-methylbenzoxazole was used instead of 2-mercaptobenzoxazole to obtain the desired compound as a colorless needle crystal.
Melting point: 128 - 131 °C
IR (KBr) cm⁻¹ : 3423, 3231, 2929, 1662, 1504, 1489.
¹H-NMR (d₆-DMSO) δ :
1.32 (6H, d, J = 7.0 Hz), 1.38 - 1.43 (4H, m), 1.49 (2H, quint, J = 7.2 Hz), 1.60 - 1.69 (2H, m), 1.84 (2H, quint, J = 7.2 Hz), 2.23 - 2.33 (2H, m), 2.40 (3H, s),
2.42 (3H, s), 2.45 (3H, s), 2.47 (3H, s), 3.23 (1H, sept, J = 7.0 Hz), 3.35 (1H, t, J = 7.2 Hz), 6.86 (1H. s), 7.15 (1H, s), 8.78 (1H, br s).
EIMS m/z (relative intensity) : 567 (M⁺; ³⁷Cl) , 565 (M⁺;³⁵Cl), 200 (100).

### Example 96 (Compound No. 1267 in Table)

### Production of 2-(7-trifluoromethylbenzoxazol-2-ylthio)-N-[2,4-bis(ethylthio)-6-methyl-3-pyridyl]acetamide:

The reaction and the treatment were conducted in the same manner as in Example 89 except that 3-amino-2,4-bis(ethylthio)-6-methylpyridine was used instead of 3-amino-2,4-bis(methylthio)-6-methylpyridine to obtain the desired compound as a colorless prism crystal.
Melting point: 182 - 183°C
IR (KBr) cm⁻¹ : 3435, 3244, 1663, 1508, 1432, 1332.
¹H-NMR (CDCl₃) δ :
1.16 (3H, t, J = 7.4 Hz), 1.20 (3H, t, J = 7.4 Hz), 2.42 (3H, s), 2.81 (2H, q, J = 7.4 Hz), 3.03 (2H, q, J = 7.4 Hz), 4.14(2H,s), 6.63 (1H, s), 7.40 (1H, t, J = 7.8 Hz), 7.52 (1H, d, J = 7.8Hz),
7.68 (1H, d, J = 7.8 Hz), 8.34 (1H, br s).
EIMS m/z (relative intensity): 487 (M⁺), 235 (100).

| Elemental Analysis : C₂₀H₂₀F₃N₃O₂S₃ | | | | | |
|---|---|---|---|---|---|
| | Calculated : | C, 49.27; | H, 4.13; | N, 8.62; | F, 11.69. |
| | Found : | C, 49.41; | H, 4.20; | N, 8.62; | F, 11.59. |

### Example 97 (Compound No. 1269 in Table)

### Production of 4-(7-trifluoromethylbenzoxazol-2-ylthio)-N-[2,4-bis(ethylthio)-6-methyl-3-pyridyl]butanamide:

The reaction and the treatment were conducted in the same manner as in Example 57 except that 2-mercapto-7-trifluoromethylbenzoxazole was used instead of, 2-mercaptobenzoxazole to obtain the desired compound as a colorless powdery crystal.
Melting point: 148 - 150°C
IR (KBr) cm⁻¹ : 3439, 3256, 2975, 2929, 1656, 1509, 1433, 1332, 1125.
¹H-NMR (d₆-DMSO) δ :
1.23 (3H, t, J = 7.3 Hz), 1.24 (3H, t, J = 7.3 Hz), 2.04 - 2.22 (2H, m), 2.42 (3H, s), 2.47- 2.48 (2H, m), 2.92 (2H, q, J = 7.3 Hz), 3.04 (2H, q, J = 7.3 Hz), 3.42 - 3.51 (2H, m), 6.87(1H,s),
7.51 (1H, t, J = 7.8 Hz) 7.59 (1H, d, J = 7.8 Hz), 7.89 (1H, d, J = 7.8 Hz), 8.95 (1H, br s).
EIMS m/z (relative intensity): 515 (M⁺), 227 (100).

### Example 98 (Compound No. 1270 in Table)

### Production of 5-(7-trifluoromethylbenzoxazol-2-ylthio)-N-[2,4-bis(ethylthio)-6-methyl-3-pyridyl] pentanamide:

The reaction and the treatment were conducted in the same manner as in Example 58 except that 2-mercapto-7-trifluoromethylbenzoxazole was used instead of 2-mercaptobenzoxazole to obtain the desired compound as a colorless powdery crystal.
Melting point: 155 - 156°C
¹H-NMR (d₆-DMSO) δ :
1.20 - 1.30 (6H, m), 1.73 - 2.05 (4H, m), 2.30 - 2.41 (2H, m), 2.42 (3H, s), 2.85 - 3.00 (2H, m), 3.01- 3.09 (2H, m), 3.37 - 3.48 (2H; m), 6.88 (1H, s), 7.51 (1H, t, J = 7.5 Hz), 7.60 (1H, d, J = 7.5 Hz), 7.90 (1H, d, J = 7.5 Hz), 8.75 (1H, br s).
EIMS m/z (relative intensity) : 529 (M⁺), 227 (100).

### Example 99 (Compound No. 1272 in Table)

### Production of 7-(7-trifluoromethylbenzoxazol-2-ylthio)-N-[2,4-bis(ethylthio)-6-methyl-3-pyridyl] heptanamide:

The reaction and the treatment were conducted in the same manner as in Example 59 except that 2-mercapto-7-trifluoromethylbenzoxazole was used instead of 2-mercaptobenzoxazole to obtain the desired compound as a colorless needle crystal.
Melting point: 127 - 128°C
IR (KBr) cm⁻¹ : 3448, 1659, 1506, 1336, 1128, 1116.
¹H-NMR (d₆―DMSO) δ:
1.24 (3H, t, J = 7.3 Hz), 1.25 (3H, t, J = 7.3 Hz), 1.39 - 1.56 (4H, m), 1.56 - 1.72 (2H, m), 1.78 - 1.91 (2H, m), 2.19 - 2.33 (2H, m), 2.42 (3H, s), 2.92 (2H, q, J = 7.3 Hz), 3.05 (2H, q, J = 7.3 Hz), 3.37 (2H, t, J = 7.2 Hz), 6.86 (1H, s), 7.49 (1H, t, J = 7.9 Hz), 7.58 (1H, d, J = 7.9 Hz), 7.88 (1H. d, J = 7.9 Hz). 8.67 (1H. br s).
EIMS m/z (relative intensity): 557 (M⁺), 227 (100).

### Example 100 (Compound No. 1273 in Table)

### Production of 8-(7-trifluoromethylbenzoxazol-2-ylthio)-N-[2,4-bis(ethylthio)-6-methyl-3-pyridyl]octanamide:

The reaction and the treatment were conducted in the same manner as in Example 60 except that 2-mercapto-7-trifluoromethylbenzoxazole was used instead of 2-mercaptobenzoxazole to obtain the desired compound as a colorless crystal.
Melting point: 99 - 100°C
IR (KBr) cm⁻¹ : 3425, 3245, 2923, 1655, 1509, 1433, 1332, 1125.
¹H-NMR (d₆―DMSO) δ :
1.26 (3H, t, J = 7.3 Hz), 1.27 (3H, t, J = 7.3 Hz), 1.38 - 1.43 (4H, m), 1.49 (2H, quint, J = 7.2 Hz), 1.60 - 1.68 (2H, m), 1.85 (2H, quint, J = 7.2 Hz), 2.20 - 2.30 (2H, m), 2.43 (3H, s), 2.94 (2H, q, J = 7.3 Hz), 3.06 (2H, q, J = 7.3 Hz), 3.38 (2H, t, J = 7.2 Hz), 6.88 (1H, s), 7.51 (1H, t, J = 7.8 Hz), 7.60 (1H, d, J = 7.8 Hz), 7.90 (1H, d, J = 7.8 Hz), 8.73 (1H, br s).
EIMS m/z (relative intensity): 571 (M⁺), 227 (100).

### Example 101 (Compound No. 1274 in Table)

### Production of 9-(7-trifluoromethylbenzoxazol-2-ylthio)-N-[2,4-bis(ethylthio)-6-methyl-3-pyridyl]nonanamide:

The reaction and the treatment were conducted in the same manner as in Example 28 except that 2-mercapto-7-trifluoromethylbenzoxazole was used instead of 2-mercaptobenzoxazole to obtain the desired compound as a colorless powdery crystal.
Melting point: 115 - 116°C
¹H-NMR (d6-DMSO) δ :
1.26 (3H, t, J = 7.2 Hz), 1.27 (3H, t, J = 7.2 Hz), 1.31 - 1.55 (8H, m), 1.57 - 1.69 (2H, m), 1.84 (2H, quint, J = 6.9 Hz), 2.18 - 2.34 (2H, m), 2.43 (3H, s), 2.94 (2H, q, J = 7.2 Hz), 3.06 (2H, q, J = 7.2 Hz), 3.37 (2H, t, J = 7.3 Hz), 6.88 (1H, s), 7.51 (1H, t, J = 8.4 Hz), 7.61 (1H, d, J = 8.4 Hz), 7.90 (1H, d, J = 8.4 Hz), 8.73 (1H, br s).
EIMS m/z (relative intensity): 585 (M⁺), 227 (100).

### Example 102 (Compound No. 1279 in Table)

### Production of 4-(5-chloro-7-isopropyl-4-methylbenzoxazol-2-ylthlo)-N-[2,4-bis(ethylthio)-6-methyl-3-pyridyl]butanamide:

The reaction and the treatment were conducted in the same manner as in Example 57 except that 5-chloro-7-isopropyl-2-mercapto-4-methylbenzoxazole was used instead of 2-mercaptobenzoxazole to obtain the desired compound as a colorless needle crystal.
Melting point: 122 - 123°C.
IR (KBr) cm⁻¹ : 3258, 1665, 1502, 1145.
¹H-NMR (d₆-DMSO) δ :
1.23 (3H, t, J = 7.3 Hz), 1.24 (3H, t, J = 7.3 Hz), 1.31 (6H, d, J = 6.8 Hz). 2.15 (2H, t, J = 7.0 Hz), 2.42 (3H, s), 2.46 (3H, s), 2.47 - 2.50 (2H, m), 2.92 (2H, q, J = 7.3 Hz), 3.04 (2H, q, J =7.3 Hz), 3.22 (1H, sept, J = 6.8 Hz), 3.43 (2H, t, J = 7.0 Hz), 6.87 (1H, s), 7.14(1H, s), 8.83 (1H, br s).
EIMS m/z (relative intensity): 559 (M⁺:³⁷Cl), 557 (M⁺:³⁵Cl) , 227 (100).

### Example 103 (Compound No. 1280 in Table)

### Production of 5-(5-chloro-7-isopropyl-4-methylbenzoxazol-2-ylthio)-N-[2,4-bis(ethylthio)-6-methyl-3-pyridyl]pentanamide:

The reaction and the treatment were conducted in the same manner as in Example 58 except that 5-chloro-7-isopropyl-2-mercapto-4-methylbenzoxazole was used instead of 2-mercaptobenzoxazole to obtain the desired compound as a colorless powdery crystal.
Melting point: 141 - 142°C
¹H-NMR (d6-DMSO) δ:
1.25(3H, t, J = 7.4 Hz), 1.26 (3H, t, J = 7.4 Hz), 1.32 (6H, d, J = 6.9 Hz), 1.75 - 1.86 (2H, m), 1.87 - 2.00 (2H, m), 2.30 - 2.40 (2H, m), 2.43 (3H,s), 2.45 - 2.52 (3H, s), 2.92 (2H, q, J = 7.4 Hz), 3.04 (2H, q, J = 7.4 Hz), 3.23 (1H, sept, J = 6.9 Hz), 3.33 - 3.43 (2H, m), 6.88 (1H, s), 7.15 (1H, s), 8.82 (1H, br s).
EIMS m/z (relative intensity): 553 (M⁺;³⁷Cl), 551 (M⁺;³⁵Cl), 227 (100).

### Example 104 (Compound No. 1282 in Table)

### Production of 7-(5-chloro-7-isopropyl-4-methylbenzoxazol-2-ylthio)-N-[2,4-bis(ethylthio)-6-methyl-3-pyridyl]heptanamide:

The reaction and the treatment were conducted in the same -manner as in Example 59 except that 5-chloro-7-isopropyl-2--mercapto-4-methylbenzoxazole was used instead of 2-mercaptobenzoxazole to obtain the desired compound as a colorless prism crystal.
Melting point: 117 - 120°C.
IR (KBr) cm⁻¹ : 3320, 1668, 1506, 1482, 1150.
¹H-NMR (d₆―DMSO) δ :
1.24 (3H, t, J = 7.3 Hz), 1.25 (3H, t, J = 7.3 Hz), 1.31 (6H, d, J = 6.8 Hz), 1.39 - 1.57 (4H, m), 1.57- 1.71 (2H, m),
1.77- 1.89 (2H, m), 2.19 - 2.30 (2H, m), 2.42 (3H,s), 2.46 (3H, s), 2.92 (2H, q, J = 7.3 Hz), 3.05 (2H, q, J = 7.3 Hz), 3.21 (1H, sept, J = 6.8 Hz), 3.33 (2H, t, J = 7.2 Hz), 6.86 (1H, s), 7.13 (1H, s), 8.66 (1H, br s).
EIMS m/z (relative intensity): 581 (M⁺:³⁷Cl), 579 (M⁺:³⁵Cl), 227 (100).

### Example 105 (Compound No. 1283 in Table)

### Production of 8-(5-chloro-7-isopropyl-4-methylbenzoxazol-2-ylthio)-N-[2,4-bis(ethylthio)-6-methyl-3-pyridyl]octanamide:

The reaction and the treatment were conducted in the same manner as in Example 60 except that 5-chloro-7-isopropyl-2-mercapto-4-methylbenzoxazole was used instead of 2-mercaptobenzoxazole to obtain the desired compound as a colorless needle crystal.
Melting point: 82 - 84°C
IR (KBr) cm⁻¹ : 3435, 3259, 2929, 1655, 1504, 1490.
¹H-NMR (d₆-DMSO) δ :
1.26 (3H, t, J = 7.3 Hz), 1.27 (3H, t, J = 7.3 Hz), 1.32 (6H, d, J = 6.8 Hz), 1.39 - 1.43 (4H, m), 1.49 (2H, quint, J = 7.2 Hz), 1.60 - 1.68 (2H, m), 1.84 (2H, quint, J = 7.2 Hz), 2.22 - 2.32 (2H, m), 2.43 (3H, s),
2.47 (3H, s), 2.94 (2H, q, J = 7.3 Hz), 3.06 (2H, q, J = 7.3 Hz),
3.22 (1H, sept, J = 6.8 Hz), 3.35 (2H, t, J = 7.2 Hz), 6.88 (1H, s), 7.15 (1H, s), 8.73 (1H, br s).
EIMS m/z (relative intensity) : 595 (M⁺;³⁷Cl) 593 (M⁺;³⁵Cl),

### Example 106 (Compound No. 1284 in Table)

### Production of 9-(5-chloro-7-isopropyl-4-methylbenzoxazol-2-ylthio)-N-[2,4-bis(ethylthio)-6-methyl-3-pyridyl]nonanamide:

The reaction and the treatment were conducted in the same manner as in Example 28 except that 5-chloro-7-isopropyl-2-mercapto-4-methylbenzoxazole was used instead of 2-mercaptobenzoxazole to obtain the desired compound as a colorless powdery crystal.
Melting point: 93 - 94°C
¹H-NMR (d₅-DMSO) δ :
1.27 (3H, t, J = 7.3 Hz), 1.28 (3H, t, J = 7.3 Hz), 1.32 (6H, d, J = 7.0 Hz), 1.29 - 1.55 (8H, m), 1.56 - 1.69 (2H, m), 1.83 (2H, quint, J = 6.9 Hz), 2.07 - 2.17 (2H, m), 2.43 (3H, s), 2.45 - 2.49 (3H, m), 2.94 (2H, q, J = 7.3 Hz), 3.07 (2H, q, J=7.3 Hz), 3.22 (1H, sept, J = 7.0 Hz), 3.34 (2H, t, J = 7.3 Hz), 6.88 (1H, s), 7.15 (1H, s), 8.73 (1H, br s).
EIMS m/z (relative intensity): 609 (M⁺;³⁷Cl) , 607 (M⁺;³⁵Cl), 229 (100).

### Example 107 (Compound No. 1287 in Table)

### Production of 2-(7- triffluoromethylbenzoxazol-2-ylthio)-N-[2,4-bis(isopropylthio)-6-methyl-3-pyridyl] acetamide:

The reaction and the treatment were conducted in the same manner as in Example 89 except that 2-bromo-N-[2,4-bis(isopropylthio)-6-methylpyridyl]amide was used instead of 2-bromo-[2,4-bis(methylthio)-6-methylpyridyl]acetamide to obtain the desired compound as a colorless needle crystal.
Melting point: 121 - 122°C
IR (KBr) cm⁻¹ : 3426, 3210, 2967, 1655, 1507, 1431, 1329.
¹H-NMR (CDCl₃) δ :
1.17 (6H, d, J = 6.8 Hz), 1.19 (6H, d, J = 6.8 Hz), 2.42 (3H, s),
3.39 (1H, sept, J = 6.8 Hz), 3.90 (1H, sept, J = 6.8Hz), 4.13 (2H, s), 6.68 (1H, s), 7.41 (1H, t, J = 7.9 Hz), 7.52 (1H, d, J = 7.9 Hz), 7.80 (1H, d, J = 7.9 Hz), 8.30 (1H, br s).
EIMS m/z (relative intensity): 515 (M⁺), 181 (100).

| Elemental analysis: as C₂₂H₂₄F₃N₃O₂S₃ | | | | | |
|---|---|---|---|---|---|
| | Calculated : | C, 51-25; | H, 4.69; | N, 8.15; | F, 11.05. |
| | Found | : C, 51.28; | H, 4.73; | N, 8.07: | F, 11.02. |

### Example 108 (Compound No. 1289 in Table)

### Production of 4-(7-trifluoromethylbenzoxazol-2-ylthio)-N-[2,4-bis(isopropylthio)-6-methyl-3-pyridyl] butanamide:

The reaction and the treatment were conducted in the same manner as in Example 69 except that 2-mercapto-7-trifluoromethylbenzoxazole was used instead of 2-mercaptobenzoxazole to obtain the desired compound as a colorless prism crystal.
Melting point: 135 - 136°C
IR (KBr) cm⁻¹ : 3446, 3255, 2968, 1660, 1559, 1531, 1504, 1491, 1433, 1139.
¹H-NMR (d₆-DMSO) δ :
1.27 (6H, d, J = 6.8 Hz), 1.29 (6H, d, J = 6.8 Hz), 2.13 - 2.21 (2H, m), 2.42 (3H, s), 2.47 - 2.50 (2H, m) 3.44 - 3.50 (2H, m), 3.55 (1H, sept, J = 6.8 Hz), 3.88 (1H, sept, J = 6.8Hz), 6.92 (1H, s) 7.51 (1H, t, J = 7.8 Hz), 7.59 (1H, d, J = 7.8 Hz), 7.88 (1H, d, J = 7.8 Hz), 8.91 (1H, br s).
EIMS m/z (relative intensity): 543 (M⁺), 207 (100).

### Example 109 (Compound No. 1290 in Table)

### Production of 5-(7-trifluoromethylbenzoxazol-2-ylthio)-N-[2,4-bis(isopropylthio)-6-methyl-3-pyridyl] pentanamide:

The reaction and the treatment were conducted in the same manner as in Example 70 except that 2-mercapto-7-trifluoromethylbenzoxazole was used instead of 2-mercaptobenzoxazole to obtain the desired compound as a colorless needle crystal.
Melting point: 118 - 120°C
IR (KBr) cm⁻¹ : 3208, 3163, 1663, 1506, 1431, 1328, 1139.
¹H-NMR (d₆-DMSO) δ :
1.27 (6H, d, J = 6.8 Hz), 1.30 (6H, d, J = 6.8 Hz), 1.73 - 1.87 (2H, m), 1.87 - 2.01 (2H, m), 2.23 - 2.38 (2H, m), 2.41 (3H, s), 3.41 (2H, t, J = 7.0 Hz), 3.54 (1H, sept, J = 6.8 Hz), 3.88 (1H. sept. J = 6.8 Hz), 6.91 (1H, s), 7.49 (1H, t, J = 7.9 Hz), 7.58 (1H, d, J = 7.9 Hz), 7.88 (1H, d, J = 7.9 Hz), 8.67 (1H, br s).
EIMS m/z (relative intensity): 557 (M⁺), 221 (100).

### Example 110 (Compound No. 1291 in Table)

### Production of 6-(7-trifluoromethylbenzoxazol-2-ylthio)-N-[2,4-bis(isopropylthio)-6-methyl-3-pyridyl] hexanamide:

The reaction and the treatment were conducted in the same manner as in Example 36 except that 2-mercapto-7-trifluoromethylbenzoxazole was used instead of 2-mercaptobenzoxazole to obtain the desired compound as a colorless needle crystal.
Melting point: 102 - 103°C
IR (KBr) cm⁻¹ : 3136, 1648, 1507, 1431, 1332, 1129.
¹H-NMR (d₆-DMSO) δ :
1.28 (6H, d, J = 6.8 Hz), 1.31 (6H, d, J = 6.8 Hz), 1.49 - 1.76 (4H, m), 1.77 - 1.94 (2H, m), 2.19 2.32 (2H, m), 2.42 (3H, s), 3.38 (2H, t, J = 7.3 Hz),
3.55 (1H, sept, J = 6.8 Hz), 3.89 (1H, sept, J = 6.8Hz), 6.91 (1H, s), 7.49 (1H, t, J = 7.8 Hz), 7.58 (1H, d, J = 7.8 Hz),
7.87 (1H, d, J = 7.8 Hz), 8.62 (1H, br s).
EIMS m/z (relative intensity): 571 (M⁺), 235 (100).

### Example 111(Compound No. 1292 in Table)

### Production of 7-(7-trifluoromethylbenzoxazol-2-ylthio)-N-[2,4-bis(isopropylthio)-6-methyl-3-pyridyl] heptanamide:

The reaction and the treatment were conducted in the same manner as in Example 71 except that 2-mercapto-7-trifluoromethylbenzoxazole was used instead of 2-mercaptobenzothiazole to obtain the desired compound as a colorless crystal.
Melting point: 76 - 78°C
IR (KBr) cm⁻¹ : 3423, 3268, 2931, 1660, 1506, 1433, 1334.
¹H-NMR (d₆-DMEO) δ :
1.29 (6H, d, J = 6.8 Hz), 1.31 (6H, d, J = 6.8 Hz), 1.43 - 1.54 (4H, m), 1.61 - 1.69 (2H, m), 1.86 (2H, quint, J = 7.2 Hz), 2.18 - 2.32 (2H, m), 2.43 (3H, s), 3.39 (2H, t, J = 7.2 Hz), 3.56 (1H, sept, J = 6.8 Hz), 3.90 (1H, sept, J = 6.8 Hz), 6.93 (1H, s), 7.51 (1H. dd, J = 8.1, 7.8 Hz), 7.60 (1H, d, J = 7.8Hz), 7.90 (1H, d, J = 8.1 Hz), 8.68 (1H, br s).
EIMS m/z (relative intensity): 585 (M⁺), 249 (100).

### Example 112 (Compound No. 1293 in Table)

### Production of 8-(7-trifluoromethylbenzoxazol-2-ylthio)-N-[2,4-bis(isopropylthio)-6-methyl-3-pyridyl] octanamide:

The reaction and the treatment were conducted in the same manner as in Example 72 except that 2-mercapto-7-trifluoromethylbenzoxazole was used instead of 2-mercaptobenzoxazole to obtain the desired compound as a pale yellow oil.
IR (Cap) cm⁻¹: 3246, 2964, 2930, 1664, 1559, 1506, 1432.
¹H-NMR (d₆―DMSO) δ :
1.28 (6H, d, J = 6.8 Hz), 1.30 (6H, d, J = 6.8 Hz), 1.32 - 1.50 (6H, m), 1.56 - 1.66 (2H, m), 1.83 (2H, quint, J = 7.1 Hz), 2.17 - 2.27 (2H, m), 2.42 (3H, s), 3.36 (2B, t, J = 7.1 Hz), 3.55 (1H, sept, J = 6.8 Hz), 3.89 (1H, sept, J = 6.8 Hz), 6.91 (1H, s), 7.50 (1H, t, J = 7.8 Hz), 7.59 (1H, d, J = 7.8 Hz), 7.88 (1H, d, J = 7.8 Hz), 8.65 (1H, br s).
EIMS m/z (relative intensity): 599 (M⁺), 263 (100)

### Example 113 (Compound, No. 1294 in Table)

### Production of 9-(7-trifluoromethylbenzoxazol-2-ylthio)-N-[2,4-bis(isopropylthio)-6-methyl-3-pyridyl] nonanamide:

The reaction and the treatment were conducted in the same manner as in Example 73 except that 2-mercapto-7-trifluoromethylbenzoxazole was used instead of 2-mercaptobenzoxazole to obtain the desired compound as a pale-yellow powdery crystal.
Melting point : 97 - 98°C
IR (KBr) cm⁻¹ : 3446, 3266, 2928, 1661, 1560, 1506, 1335, 1127.
¹H-NMR (d₆-DMSO) δ :
1.28 (6H, d, J = 6.6 Hz), 1.30 (6H, d, J = 6.8 Hz) 1.28 - 1.51 (8H, m), 1.55 - 1.64 (2H, m), 1.83 (2H, quint, J = 7.3 Hz), 2.20 - 2.30 (2H, m), 2.42 (3H, s), 3.36 (2H, t, J = 7.3 Hz), 3.55 (1H, sept, J = 6.6 Hz), 3.89 (1H, sept, J = 6.8Hz), 6.91 (1H, s), 7.50 (1H, t, J = 7.8 Hz), 7.59 (1H, d, J = 7.8 Hz), 7.89 (1H, d, J = 7.8 Hz), 8.71 (1H, br s).
EIMS m/z (relative intensity): 613 (M⁺), 277 (100).

### Example 114 (Compound No. 1299 in Table)

### Production of 4-(5-chloro-7-isopropyl-4-methylbenzoxazol-2-ylthio)-N-[2,4-bis(isopropylthio)-6-methyl-3-pyridyl]butanamide:

The reaction and the treatment were conducted in the same manner as in Example 69 except that 5-chloro-2-mercapto-4-methylbenzoxazole was used instead of 2-mercaptobenzoxazole to obtain the desired compound as a colorless needle crystal.
Melting point: 141 - 143°C.
¹H-NMR (d₆-DMSO) δ :
1.27 (6H, d, J = 6.8 Hz), 1.29 (6H, d, J = 6.8 Hz), 1.31 (6H, d, J = 6.8 Hz), 2.03 - 2.21 (2H, m), 2.42 (3H, s), 2.43 - 2.50 (5H, m), 3.22 (1H, sept, J = 6.8 Hz), 3.38 - 3.48 (2H, m), 3.55 (1H, sept, J = 6.8 Hz), 3.88 (1H, sept, J = 6.8 Hz), 6.92 (1H, s), 7.14 (1H,s), 8.87 (1H, br s).
EIMS m/z (relative intensity): 567 (M⁺:³⁷Cl) , 565 (M⁺:³⁵Cl), 207 (100).

### Example 115 (Compound No. 1300 in Table)

### Production of 5-(5-chloro-7-isopropyl-4-methylbenzoxazol-2-ylthio)-N-[2,4-bis(isopropylthio)-6-methyl-3-pyridyl]pentanamide:

The reaction and the treatment were conducted in the same manner as in Example 70 except that 5-chloro-7-isopropyl-2-mercapto-4-methylbenzoxazole was used instead of 2-mercaptobenzoxazole to obtain the desired compound as a colorless needle crystal.
Melting point: 143 - 145°C.
IR (KBr) cm⁻¹ : 3231, 2924, 1720, 1657, 1508, 1297
¹H-NMR (d6-DMSO) δ :
1.27 (6H, d, J = 6.8 Hz), 1.29 (6H, d, J = 6.8 Hz), 1.31 (6H, d, J = 6.8 Hz), 1.73 - 1.85 (2H, m), 1.85 - 1.98 (2H, m), 2.25 - 2.37 (2H, m), 2.41 (3H, s), 2.43 - 2.50 (3H, s), 3.21 (1H, sept, J = 6.8 Hz), 3.37 (2H, t, J=7.2 Hz), 3.54 (1H, sept, J = 6.8 Hz), 3.88 (1H, sept, J = 6.8 Hz), 6.92 (1H, s), 7.14 (1H,s), 8.76 (1H, br s).
EIMS m/z (relative intensity): 581 (M⁺:³⁷Cl), 579 (M⁺:³⁵Cl, 100).

### Example 116 (Compound No. 1301 in Table)

### Production of 6-(5-chloro-7-isopropyl-4-methylbenzoxazol-2-ylthio)-N-[2,4-bis(isopropylthio)-6-methyl-3-pyridyl]hexanamide: .

The reaction and the treatment were conducted in the same manner as in Example 36 except that 5-chloro-7-isopropyl-2-mercapto-4-methylbenzoxazole was used instead of 2-mercaptobenzoxazole to obtain the desired compound as a colorless needle crystal.
Melting point: 99 - 101°C
IR (KBr) cm⁻¹ : 3413, 3224, 2964, 1663, 1506, 1148.
¹H-NMR (d₆-DMSO) δ :
1.29 (6H, d, J = 6.8 Hz), 1.32 (12H, d, J = 6.8 Hz), 1.54 - 1.62 (2H, m), 1.70 (2H, quint, J = 7.1 Hz), 1.87 (2H, quint, J = 7.1 Hz), 2.22 - 2.33 (2H, m), 2.43 (3H, s), 2.48 (3H, s), 3.23 (1H, sept, J = 6.8 Hz), 3.36 (2H, t, J = 7.1 Hz), 3.57 (1H, sept, J=6.8 Hz), 3.90 (1H, sept , J = 6.8Hz), 6.93 (1H, s), 7.15 (1H. s), 8.72 (1H, br s).
EIMS m/z (relative intensity) : 595 (M⁺;³⁷Cl) , 593 (M⁺;³⁵Cl), 518 (100)

### Example 117 (Compound No. 1302 in Table)

### Production of 7-(5-chloro-7-isopropyl-4-methylbenzoxazol-2-ylthio)-N-[2,4-bis(isopropylthio)-6-methyl-3-pyridyl]heptanamide:

The reaction and the treatment were conducted in the same manner as in Example 71 except that 5-chloro-7-isopropyl-2-mercapto-4-methylbenzoxazole was used instead of 2-mercaptobenzoxazole to obtain the desired compound as a colorless needle crystal.
Melting point: 91 - 93°C
IR (KBr) cm⁻¹: 3436, 3213, 3169, 2962, 2929, 1666, 1505, 1152.
¹H-NMR (d₆-DMSO) δ :
1.29 (6H, d, J = 6.8 Hz), 1.31 (6H, d, J = 6.8 Hz), 1.31 (6H, d, J = 6.8 Hz), 1.40 - 1.52 (4H, m), 1.60 - 1.68 (2H, m), 1.85 (2H, quint, J = 7.1 Hz), 2.17 - 2.32 (2H, m), 2.43 (3H, s), 2.47 (3H, s), 3.22 (1H, sept, J = 6.8 Hz), 3.35 (2H, t, J = 7.1 Hz), 3.56 (1H, sept, J = 6.8 Hz), 3.90 (1H, sept, J = 6.8Hz), 6.93 (1H, s), 7.15 (1H, s), 8.67 (1H, br s).
EIMS m/z (relative intensity) : 609 (M⁺;³⁷Cl), 607 (M⁺;³⁵Cl), 532 (100).

### Example 118 (Compound No. 1303 in Table)

### Production of 8-(5-chloro-7-isopropyl-4-methylbenzoxazol-2-ylthio)-N-[2,4-bis(isopropylthio)-6-methyl-3-pyridyl]octanamide:

The reaction and the treatment were conducted in the same manner as in Example 72 except that 5-chloro-7-isopropyl-2-mercapto-4-methylbenzoxazole was used instead of 2-mercaptobenzoxazole to obtain the desired compound as a pale yellow oil.
IR (Cap) cm⁻¹: 3242, 2964, 2928, 1668, 1559, 1506, 1148.
¹H-NMR (d₆-DMSO) δ:
1.28 (6H, d, J = 6.6 Hz), 1.31 (12H, d, J = 6.8 Hz), 1.32 - 1.50 (6H, m), 1.57 - 1.67 (2H, m). 1.82 (2H, quint, J = 7.1 Hz), 2.17 - 2.27 (2H, m), 2.42 (3H, s), 2.46 (3H, s), 3.21 (1H, sept, J = 6.8 Hz), 3.33 (2H, t, J = 7.1 Hz), 3.55 (1H, sept, J = 6.6 Hz), 3.89 (1H, sept, J = 6.8 Hz), 6.91 (1H, s), 7.14 (1H, s), 8.65 (1H, br s).
EIMS m/z (relative intensity): 623 (M⁺:³⁷Cl). 621 (M⁺:³⁵Cl), 546 (100).

### Example 119 (Compound No. 1304 in Table)

### Production of 9-(5-chloro-7-isopropyl-4-methylbenzoxazol-2-ylthio)-N-[2,4-bis(isopropylthio)-6-methyl-3-pyridyl]nonanamide:

The reaction and the treatment were conducted in the same manner as in Example 73 except that 5-chloro-7-isopropyl-2-mercapto-4-methylbenzoxazole was used instead of 2-mercaptobenzoxazole to obtain the desired compound as a pale yellow oil.
IR (Cap) cm⁻¹: 3249, 2961, 2926, 1667, 1563, 1505.
¹H-NMR (d₆-DMSO) δ :
1.28 (6H, d, J = 6.8 Hz), 1.30 (12H, d, J = 7.1 Hz) 1.28 - 1.50 (8H, m), 1.55 - 1.65 (2H, m), 1.81 (2H, quint, J = 7.1 Hz), 2.17 - 2.27 (2H, m), 2.41 (3H, s), 2.46 (3H, s), 3.21 (1H, sept, J=7.1 Hz), 3.32 (2H, t, J = 7.1 Hz), 3.54 (1H, sept, J = 6.8 Hz), 3.89 (1H, sept, J = 7.1 Hz), 6.91 (1H, s), 7.14 (1H, s), 8.65 (1H, br s).
EIMS m/z (relative intensity) : 637 (M⁺:³⁷Cl), 635 (M⁺:³⁵Cl), 560 (100).

### Example 120 (Compound No. 1317 in Table)

### Production of 2-(7-methansulfonylbenzoxazol-2-ylthio)-N-[2,4-bis(ethylthio)-6-methyl-3-pyridyl]acetamide:

The reaction and the treatment were conducted in the same manner as in Example 96 except that 2-mercapto-7-methansulfonylbenzoxazole was used instead of 2-mercapto-7-trifluoromethylbenzoxazole to obtain the desired compound as a colorless needle crystal.
Melting point: 159 - 162°C
IR (KBr) cm⁻¹ : 3449, 3271, 2966, 2928, 1678, 1508, 1315, 1118.
¹H-NMR (CDCl₃) δ :
1.14 (3H, t, J = 7.3 Hz), 1.20 (3H, t, J = 7.3 Hz), 2.43 (3H, s),
2.82 (2H, q, J = 7.3 Hz), 3.01 (2H, q, J = 7.3 Hz), 3.27 (2H, s),
4.15 (2H, s), 6.63 (1H, s), 7.49 (1H, t, J = 7.9 Hz), 7.83 (1H, dd, J = 7.9 , 1.2 Hz), 7.90 (1H, dd, J = 7.9 , 1.2 Hz),
8.17 (1H, br s).
EIMS m/z (relative intensity): 497 (M⁺), 311 (100).

| Elemental analysis: as C₂₀H₂₃N₃O₄S₄ | | | | | |
|---|---|---|---|---|---|
| | Calculated : | C, 48.27; | H, 4.66; | N, 8.44; | S, 25.77. |
| | Found : | C, 48.36; | H, 4.66; | N, 8.31; | S, 25.76. |

### Example 121 (Compound No. 1327 in Table)

### Production of 2-(7-methansulfonylbenzoxazol-2-ylthio)-N-[2,4-bis(isopropylthio)-6-methyl-3-pyridyl]acetamide:

The reaction and the treatment were conducted in the same manner as in Example 74 except that 2-mercapto-7-methansulfonylbenzoxazole was used instead of 2-mercaptobenzothiazole to obtain the desired compound as a pale yellow amorphous.
IR (KBr) cm⁻¹ : 3435, 3337, 2965, 2926, 1695, 1506, 1424, 1319, 1117.
¹H-NMR (CDCl₃) δ :
1.16 (6H, d, J = 6.8 Hz), 1.21 (6H, d, J = 6.8 Hz), 2.42 (3H, s),
3.26 (3H, s), 3.40 (1H, sept, J = 6.8 Hz), 3.90 (1H, sept, J = 6.8 Hz), 4.15 (2H, s), 6.68 (1H,s), 7.49 (1H, t, J = 7.9Hz), 7.83 (1H, dd, J = 7.9 , 1.0Hz), 7.90 (1H, dd, J = 7.9 , 1.0 Hz), 8.11 (1H, br s).
EIMS m/z (relative intensity): 525 (M⁺), 339 (100).

### Example 122 (Compound No. 1341 in Table)

### Production of 6-(benzoxasole-2-ylthio)-N-(4-methyl-2-(methylthio)-5-pyridyl)hexanamide:

A methanol (8 mml) solution of 2-dichloro-4-methyl-5-nitropyrimidine (2.0 g. 10.4 mmol) was added dropwise to a methanol (8 ml) solution of sodium thiomethoxide (436 mg, 5.9 mmol) while being cooled with ice, and after the mixture was stirred for 15 hours while raising its temperature to the room temperature, water added to the reaction mixture and the mixture was extracted with ethyl acetate. The organic layer was washed with water and then with a saturated aqueous solution of sodium chloride, and dried over sodium sulfate. Thereafter, the solvent was distilled off, and the resulting crude product was recrystalized with ethyl acetate-hexan to obtain 1.02 g (yield 98%) of 4-methyl-2-methylthio-5-nitropyridine as a pale-yellow needle crystal.

This nitropyridine (497 mg, 2.7 mmol) was dissolved in a mixed solvent of acetic acid (15 ml) and conc. hydrochloric acid (0.5 ml), and zinc (2.12 g, 32.4 mmol) was added thereto in small portions while being cooled with ice for 5 minutes. After the mixture was stirred for 30 minutes at the room temperature, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Thereafter, the solvent was distilled off, and the resulting crude product was purified through silica gel chromatography (eluent - hexane:ethyl acetate = 1:1) to obtain 352 mg (yield 85%) of 5-amino-4-methyl-2-methylthiopyridine as a pale-yellow powdery crystal.

And then the reaction and the treatment were conducted in the same manner as in Example 18 except that 5-amino-4-methyl-2-methylthiopyridine was used instead of 3-amino-2,4-bis(methlthio)-6-methylpyridine to obtain the desired compound as a colorless powdery crystal.
Melting point: 125 - 127°C
IR (KBr) cm⁻¹ : 3433, 3284, 2930, 1654, 1598.
¹H-NMR (CDCl3) δ :
1.61 (2H, quint, J = 7.4 Hz), 1.83 (2H, quint, J = 7.4Hz), 1.92 (2H, quint, J = 7.4 Hz), 2.19 (3H, s), 2.43 (2H, t, J = 7.4 Hz), 2.54 (3H, s), 3.33 (2H, t, J = 7.4 Hz), 6.92 (1H, br s), 7.03 (1H, s), 7.24 (1H, td, J = 7.7 , 1.7 Hz), 7.28 (1H, td, J = 7.7 , 1.7 Hz), 7.43 (1H. dd, J = 7.7 , 1.7 Hz), 7.57 (1H, dd, J = 7.7 , 1.7 Hz), 8.57 (1H. s).
EIMS m/z (relative intensity): 401 (M⁺), 69 (100).

### Example 123 (Compound No. 1371 in Table) .

### Production of 6-(benzoxasole-2-ylthio)-N-(5-methylthio-2-pyridyl)hexanamide:

After conc. sulfuric acid (50 ml) was cooled with ice, 30% aqueous solution of hydrogen peroxide (25 ml) was dropped thereto stirring, and then conc. sulfuric acid (50 ml) solution of 2-amino-5-chloropyridine (5.0 g, 38.9 mmol) was dropped thereto further and stirred for 48 hours at the room temperature. The reaction mixture was added into ice and filtered. The residue was recrystallized with ethanol to obtain 4.38 g (yield 71 %) of 5-chloro-2-nitoropyriine as a colorless powdery crystal.

A methanol (40 mml) solution of 5-chloro-2-nitropyridine (2.0 g. 12.6 mmol) was added dropwise to a methanol (20 ml) solution of sodium thiomethoxide (1.02 g, 13.9 mmol) while being cooled with ice, and after the mixture was stirred for 13 hours while raising its temperature to the room temperature, water added to the reaction mixture and the mixture was extracted with ethyl acetate. The organic layer was washed with water and then with a saturated aqueous solution of sodium chloride, and dried over magnesium sulfate. Thereafter, the solvent was distilled off , and the resulting crude product was recrystalized with ethyl acetate-hexane to obtain 972 mg (yield 45%) of 5-methylthio-2-nitropyridine.

This nitropyridine (300 mg, 1.8 mmol) was dissolved in a mixed solvent of acetic acid (7 ml) and conc. hydrochloric acid (0.5 ml), and zinc (692 g, 10.6 mmol) was added thereto in small portions while being cooled with ice for 5 minutes. After the mixture was stirred for 30 minutes at the room temperature, the reaction mixture was filtered, and the filtrate was neutralized with an aqueous solution of sodium hydrogencarbonate, and extracted with methylene chloride. The organic layer was washed with water and then with a saturated aqueous solution of sodium chloride, and dried over sodium sulfate. Thereafter, the solvent was distilled off, and the resulting crude product was purified through silica gel chromatography (eluent - hexane:ethyl acetate = 1:1 → chloroform:methanol = 20:1) to obtain 158 mg (yield 64%) of 2-amino-5-methylthiopyridine as a pale-yellow powdery crystal.

And then the reaction and the treatment were conducted in the same manner as in Example 18 except that 2-amino-5-methylthiopyridine was used instead of 3-amino-2,4-bis(methlthio)-6-methylpyridine to obtain the desired compound as a colorless powdery crystal.
Melting point: 83 - 85°C
IR (KBr) cm⁻¹ : 3246, 2930, 1684, 1576, 1522.
¹H-NMR (CDCl₃) δ :
1.59 (2H, quint, J = 7.4 Hz), 1.81 (2H, quint, J = 7.4Hz), 1.90 (2H, quint, J = 7.4 Hz), 2.42 (2H, t. J = 7.4 Hz), 2.48 (3H, s), 3.32 (2H, t, J = 7.4 Hz), 7.23 (1H, td, J = 7.4 , 1.4 Hz), 7.28 (1H, td, J = 7.4 , 1.4 Hz), 7.43 (1H, dd, J = 7.4 , 1.4 Hz), 7.59 (1H, dd, J = 7.4 , 1.4 Hz), 7.64 (1H, dd, J = 8.6 , 2.5 Hz), 7.82 (1H, br s), 8.15 (1H, d, J = 8.6 Hz), 8.18 (1H, d, J = 2.5 Hz).
EIMS m/z (relative intensity): 387 (M⁺, 100).

### Example 124 (Compound No. 1401 in Table)

### Production of 6-(benzoxazol-2-ylthio)-N-[2,4,6-tris(methylthio)-5-pyrimidyl]hexanamide:

The reaction and the treatment were conducted in the same manner as in Example 88 except that 4,6-dihydroxy-2-methylthiopyrimidine was used instead of 4,6-dihydroxy-2-methylpyrimidine to obtain the desired compound as a colorless powdery crystal.
Melting point: 149 - 153°C
IR (KBr) cm⁻¹ : 3448, 3247, 2926, 1667, 1496.
¹H-NMR (CDCl₃) δ :
1.46 - 1.62 (2H, m), 1.63 - 1.76 (2H, m), 1.77 - 1.91 (2H, m), 2.20 - 2.36 (2H, m), 2.46 (9H, s), 3.36 (2H, t, J = 7.1 Hz), 7.22 - 7.35 (2H, m), 7.51 - 7.62 (2H, m), 9.02 (1H, br s).
EIMS m/z (relative intensity): 480 (M⁺, 100).

### Example 125 (Compound No. 1427 in Table)

### Production of 2-(7-methoxycarbonylbenzoxazol-2-ylthio)-N-[2,4-bis(ethylthio)-6-methyl-3-pyridyl]acetamide:

The reaction and the treatment were conducted in the same manner as in Example 26 except that 2-mercapto-7-methoxycarbonylbenzoxazole was used instead of 2-mercaptobenzoxasole to obtain the desired compound as a colorless needle crystal.
Melting point: 168 - 169°C
IR (KBr) cm⁻¹ 3433, 3257, 1727, 1677, 1513, 1297, 1120.
¹H-NMR (CDCl₃) δ :
1.16 (3H, t, J = 7.4 Hz), 1.19 (3H, t, J = 7.4 Hz), 2.42 (3H, s), 2.80 (2H, q, J = 7.4 Hz), 3.03 (2H, q, J = 7.4 Hz), 4.00 (3H, s), 4.12 (2H, s), 6.63 (1H, s), 7.38 (1H, dd, J = 8.1,7.8Hz), 7.80 (1H, dd, J = 8.1 , 1.2 Hz), 7.92 (1H, dd, J = 7.8 , 1.2 Hz), 8.48 (1H, br s).
EIMS m/z (relative intensity): 477 (M⁺), 323 (100).

| Elemental analysis: as C₂₀H₂₃N₃O₄S₄ | | | | | |
|---|---|---|---|---|---|
| | Calculated : | C, 52.81; | H, 4.85; | N, 8.80; | S, 20.14. |
| | Found : | C, 52.90; | H, 4.91; | N, 8.73; | S, 20.12. |

### Example 126 (Compound No. 1428 in Table)

### Production of 2-(oxazolo[4,5-b]pyridine-2-ylthio)-N-[2,4-bis(ethylthio)-6-methyl-3-pyridyl]acetamide:

The reaction and the treatment were conducted in the same manner as in Example 49 except that 2-mercaptoxazolo[4,5-b]pyridine was used instead of 2-mercaptobenzoxasole to obtain the desired compound as a colorless crystal.
IR (KBr) cm⁻¹ : 3460, 3167, 2972, 1685, 1561.
¹H-NMR (CDCl₃) δ:
1.14 (3H, t, J = 7.4 Hz), 1.21 (3H, t, J = 7.4 Hz), 2.42 (3H, s), 2.82 (2H, q, J = 7.4 Hz), 3.02 (2H, q, J = 7.4 Hz), 4.16 (2H, s), 6.62 (1H, s), 7.25 (1H, dd, J = 8.3, 5.1 Hz), 7.78 (1H, dd, J = 8.3, 1.2 Hz), 8.40 (1H, br s), 8.49 (1H, dd, J = 5.1, 1.2 Hz).
EIMS m/z (relative intensity): 420 (M⁺, 100).

### Example 127 (Compound No. 1257 in Table)

### Production of 2-(5-chloro-7-isopropyl-4-methylbenzoxazol-2-ylthio)-N-[2,4-bis(methylthio)-6-methyl-3-pyridyl]acetamide:

The reaction and the treatment were conducted in the same manner as in Example 49 except that 5-chloro-7-isopropyl-2-mercapto4-methylbenzoxazole was used instead of 2-mercaptobenzothiazole to obtain the desired compound as a colorless powdery crystal.
EIMS m/z (relative intensity): 481 (M⁺), 210 (100).

### Example 128 (Compound No. 1277 in Table)

### Production of 2-(5-chloro-7-isopropyl-4-methylbenzoxazol-2-ylthio)-N-[2,4-bis(ethylthio)-6-methyl-3-pyridyl]acetamide:

The reaction and the treatment were conducted in the same manner as in Example 127 except that 3-amino-2,4-bis(isopropylthio)-6-methylpyridine was used instead of 3-amino-2.4-bis(methylthio)-6-methylpyridine to obtain the desired compound as a colorless powdery crystal.
EIMS m/z (relative intensity): 511 (M⁺;³⁷Cl), 509 (M⁺;³⁵Cl), 235 (100).

### Example 129 (Compound No. 1297 in Table)

### Production of 2-(5-chloro-7-isopropyl-4-methylbenzoxazol-2-ylthio)-N-[2,4-bis(isopropylthio)-6-methyl-3-pyridyl]acetamide:

The reaction and the treatment were conducted in the same manner as in Example 127 except that 3-amino-2,4-bis(isopropylthio)-6-methylpyridine was used instead of 3-amino-2,4-bis(methylthio)-6-methylpyridine to obtain the desired compound as a colorless powdery crystal.
EIMS m/z (relative intensity): 539 (M⁺;³⁷Cl), 537 (M⁺;³⁵Cl), 223 (100).

## Claims

1. An ACAT-inhibitor compound represented by the formula (I) 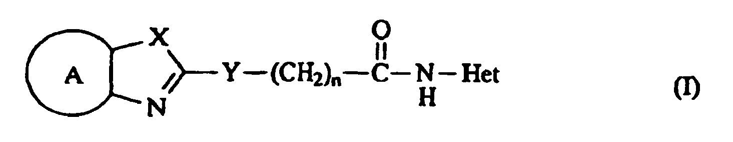 wherein 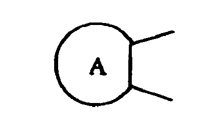 represents an optionally substituted benzene, pyridine, cyclohexane or naphthalene, X represents -NH-, an oxygen atom or a sulfur atom, Y represents -NR₄-, an oxygen atom, a sulfur atom, a sulfoxide or a sulfone, R₄ represents a hydrogen atom, a lower alkyl group, an aryl group or an optionally substituted silyl lower alkyl group, n is an integer of from 1 to 15, and wherein Het represents a substituted or unsubstituted pyridyl or pyrimidyl group, wherein any substituent on Het is selected from an amino group substituted with 1 or 2 lower alkyl groups each having from 1 to 10 carbon atoms, a lower alkoxy group having from 1 to 10 carbon atoms, a lower alkylthio group having from 1 to 10 carbon atoms, a lower alkylcarbonyl group having from 1 to 10 carbon atoms, a halogen atom, an amino group or a lower alkyl group having from 1 to 10 carbon atoms, a hydroxyl group, a phosphate group, a sulfonamide group, a lower alkyl sulphonyl group having 1 to 10 carbon atoms, or two substituents may be bound to form an alkylenedioxy group of 1 to 10 carbon atoms or a salt or solvate thereof.

2. A compound according to claim 1, represented by the formula (i)

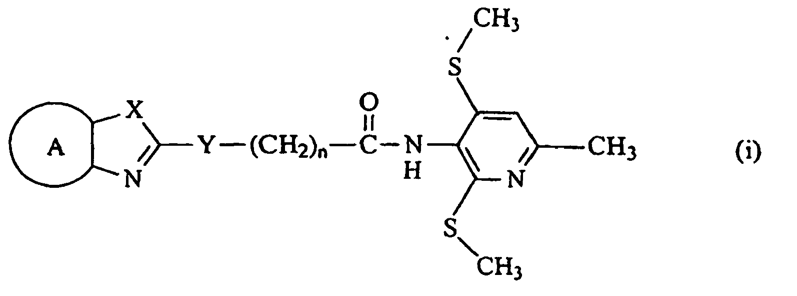

3. A compound according to claim 1, represented by the formula (ii)

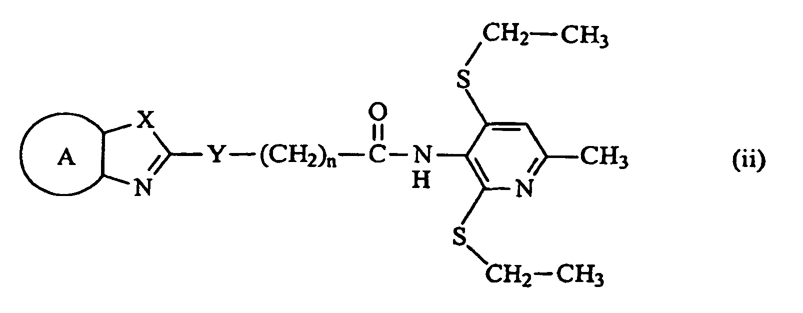

4. A compound according to claim 1, represented by the formula (iii)

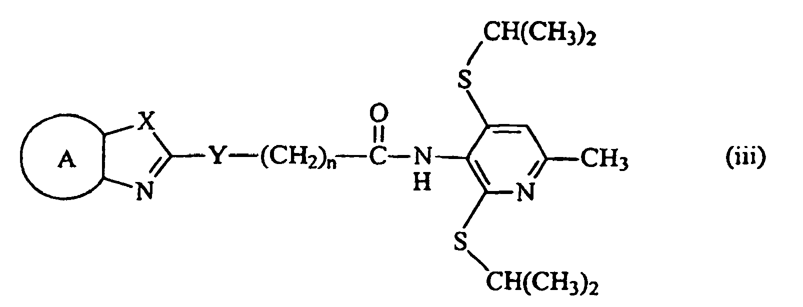

5. A compound according to claim 1, represented by the formula (iv)

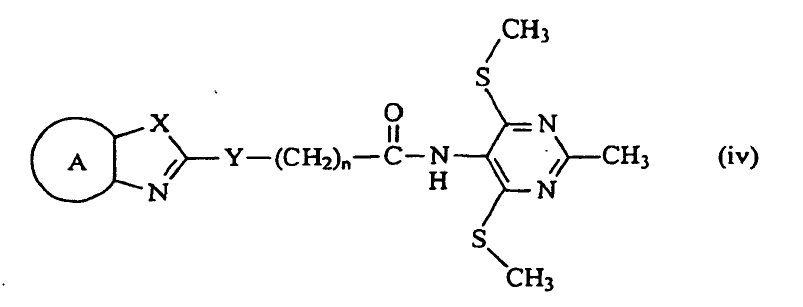

6. A compound according to claim 1, represented by the formula (v)

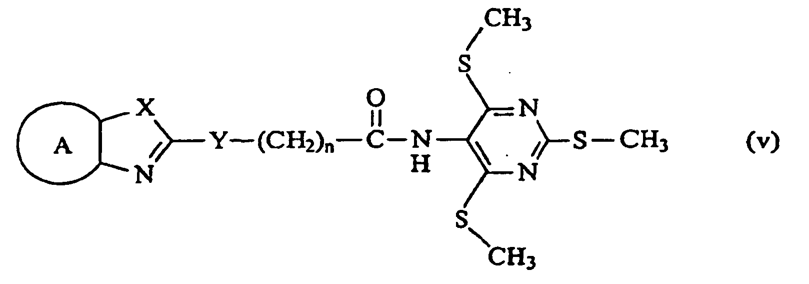

7. A compound according to claim 1, represented by the formula (IA) 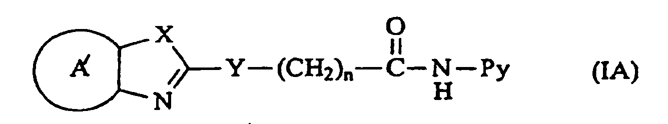 wherein 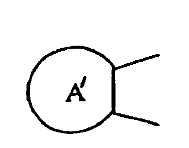 represents an optionally substituted benzene or pyridine, and Py represents an optionally substituted pyridyl or pyrimidyl group,
wherein any substituent on Py is selected from salt or solvate thereof. an amino group substituted with 1 or 2 lower alkyl groups each having from 1 to 10 carbon atoms, a lower alkoxy group having from 1 to 10 carbon atoms, a lower alkylthio group having from 1 to 10 carbon atoms, a lower alkylcarbonyl group having from 1 to 10 carbon atoms, a halogen atom, an amino group or a lower alkyl group having from 1 to 10 carbon atoms, a hydroxyl group, a phosphate group, a sulfonamide group, a lower alkyl sulphonyl group having 1 to 10 carbon atoms, or two substituents may be bound to form an alkylenedioxy group of to 10 carbon atoms, or a salt or solvate thereof.

8. A compound according to claim 1 or claim 7, represented by the formula (III) 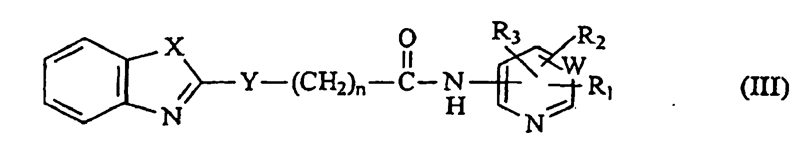 wherein
W represents =CH- or =N-,
R₁, R₂ and R₃ are the same or different, and each represents a hydrogen atom, a lower alkyl group having from 1 to 10 carbon atoms, a lower alkoxy group having from 1 to 10 carbon atoms, a halogen atom, a hydroxyl group, a phosphate group, a sulfonamide group, a lower alkylthio group having from 1 to 10 carbon atoms, an amino group or an amino group substituted with a lower alkyl group having from 1 to 10 carbon atoms, or two of R₁, R₂ and R₃ together form an alkylenedioxide group, or a salt or solvate thereof.

9. A pharmaceutical composition comprising at least one compound according to any one of claims 1 to 8 or a salt or solvate thereof, in combination with a pharmaceutically acceptable carrier.

10. A compound, salt or solvate as defined in any one of claims 1 to 8 for use in a method of treatment of the human or animal body by therapy.

11. A compound, salt or solvate as defined in anyone of claims 1 to 8 for use as an ACAT inhibitor, an intracellular cholesterol transfer inhibitor, a blood cholesterol depressant or a macrophage formation suppressant.

12. A compound, salt or solvate as defined in any one of claims 1 to 8 for use in preventing hyperlipemia, arteriosclerosis, cerebrovascular accidents, ischemic heart disease, ischemic intestinal disease and aortic aneurysm.

## Patentansprüche

1. ACAT-Inhibitorverbindung, angegeben durch die Formel (I) 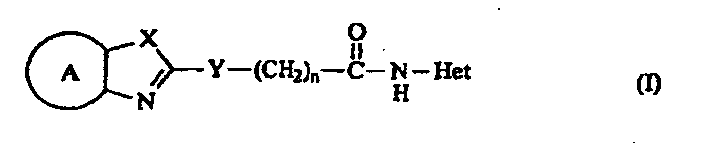 worin 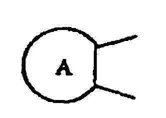 für ein wahlweise substituiertes Benzol, Pyridin, Cyclohexan oder Naphthalin steht, X für -NH-, ein Sauerstoffatom oder ein Schwefelatom steht, Y für -NR₄-, ein Sauerstoffatom, ein Schwefelatom, ein Sulfoxid oder ein Sulfon steht, R₄ für ein Wasserstoffatom, eine niedere Alkylgruppe, eine Arylgruppe oder eine wahlweise substituierte Silylniederalkylgruppe steht, n eine ganze Zahl von 1 bis 15 ist, und wobei Het für eine substituierte oder unsubstituierte Pyridyl- oder Pyrimidylgruppe steht, wobei jedweder Substituent auf Het gewählt ist aus einer Aminogruppe, die mit 1 oder 2 niederen Alkylgruppen jeweils mit 1 bis 10 Kohlenstoffatomen substituiert ist, einer niederen Alkoxygruppe mit 1 bis 10 Kohlenstoffatomen, einer niederen Alkylthiogruppe mit 1 bis 10 Kohlenstoffatomen, einer niederen Alkylcarbonylgruppe mit 1 bis 10 Kohlenstoffatomen, einem Halogenatom, einer Aminogruppe oder einer niederen Alkylgruppe mit 1 bis 10 Kohlenstoffatomen, einer Hydroxylgruppe, einer Phosphatgruppe, einer Sulfonamidgruppe, einer niederen Alkylsulfonylgruppe mit 1 bis 10 Kohlenstoffatomen, oder zwei Substituenten gebunden sein können unter Bildung einer Alkylendioxygruppe von 1 bis 10 Kohlenstoffatomen oder eines Salzes oder Solvats davon.

2. Verbindung nach Anspruch 1, angegeben durch die Formel (i)

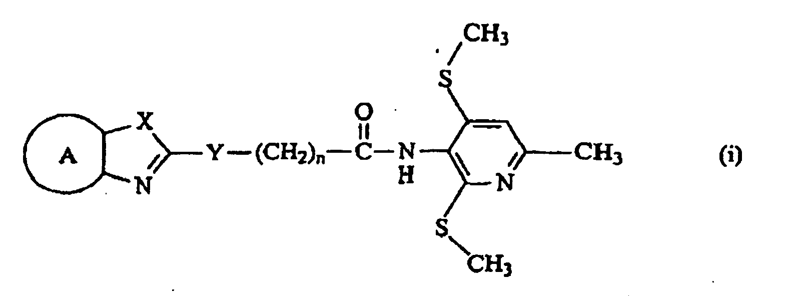

3. Verbindung nach Anspruch 1, angegeben durch die Formel (ii)

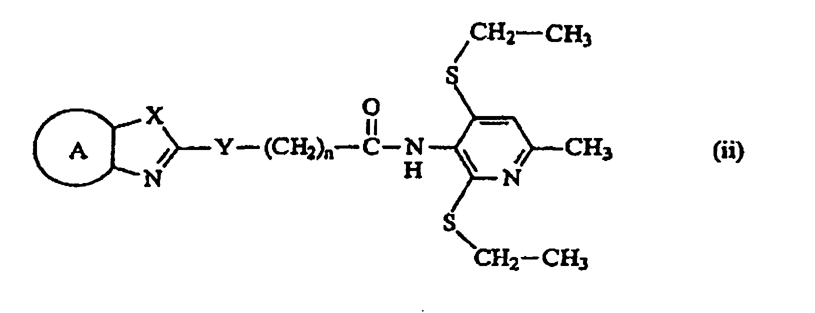

4. Verbindung nach Anspruch 1, angegeben durch die Formel (iii)

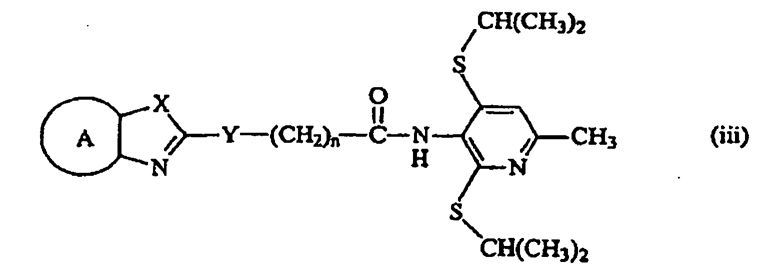

5. Verbindung nach Anspruch 1, angegeben durch die Formel (iv)

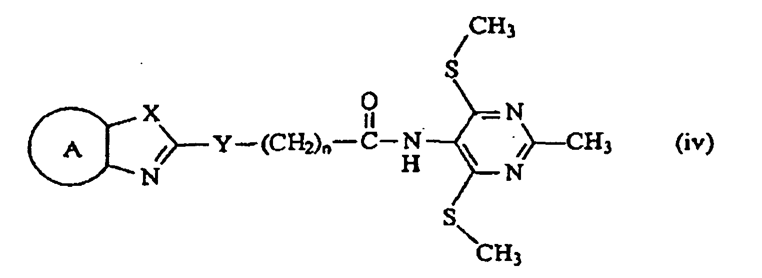

6. Verbindung nach Anspruch 1, angegeben durch die Formel (v)

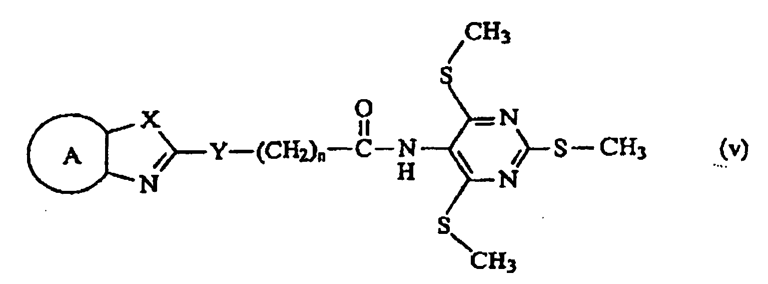

7. Verbindung nach Anspruch 1, angegeben durch die Formel (IA) 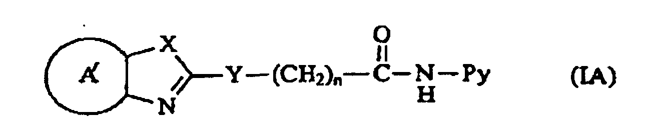 worin 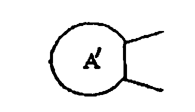 für wahlweise substituiertes Benzol oder Pyridin steht und Py für eine wahlweise substituierte Pyridyl- oder Pyrimidylgruppe steht, wobei jeder Substituent auf Py gewählt ist aus einer Aminogruppe, die mit 1 oder 2 niederen Alkylgruppen jeweils mit 1 bis 10 Kohlenstoffatomen substituiert ist, einer niederen Alkoxygruppe mit 1 bis 10 Kohlenstoffatomen, einer niederen Alkylthiogruppe mit 1 bis 10 Kohlenstoffatomen, einer niederen Alkylcarbonylgruppe mit 1 bis 10 Kohlenstoffatomen, einem Halogenatom, einer Aminogruppe oder einer niederen Alkylgruppe mit 1 bis 10 Kohlenstoffatomen, einer Hydroxylgruppe, einer Phosphatgruppe, einer Sulfonamidgruppe, einer niederen Alkylsulfonylgruppe mit 1 bis 10 Kohlenstoffatomen, oder zwei Substituenten gebunden sein können unter Bildung einer Alkylendioxygruppe von 1 bis 10 Kohlenstoffatomen oder eines Salzes oder Solvats davon.

8. Verbindung nach Anspruch 1 oder Anspruch 7, angegeben durch die Formel (III) 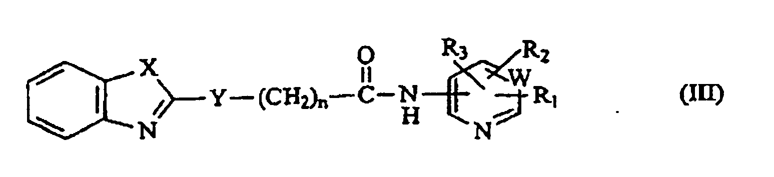 worin
W für =CH- oder =N- steht,
R₁, R₂ und R₃ gleich oder verschieden sind und jedes davon für ein Wasserstoffatom, eine niedere Alkylgruppe mit 1 bis 10 Kohlenstoffatomen, eine niedere Alkoxygruppe mit 1 bis 10 Kohlenstoffatomen, ein Halogenatom, eine Hydroxylgruppe, eine Phosphatgruppe, eine Sulfonamidgruppe, eine niedere Alkylthiogruppe mit 1 bis 10 Kohlenstoffatomen, eine Aminogruppe oder eine mit einer niederen Alkylgruppe mit 1 bis 10 Kohlenstoffatomen substituierte Aminogruppe steht, oder zwei aus R₁, R₂ und R₃ zusammen eine Alkylendioxidgruppe oder ein Salz oder Solvat davon bilden.

9. Pharmazeutische Zusammensetzung, umfassend mindestens eine Verbindung nach mindestens einem der Ansprüche 1 bis 8 oder ein Salz oder Solvat davon, in Kombination mit einem pharmazeutisch annehmbaren Träger.

10. Verbindung, Salz oder Solvat, wie in mindestens einem der Ansprüche 1 bis 8 definiert, für die Verwendung in einem Verfahren zur Behandlung des menschlichen oder tierischen Körpers durch eine Therapie.

11. Verbindung, Salz oder Solvat, wie in mindestens einem der Ansprüche 1 bis 8 definiert, für die Verwendung als ein ACAT-Inhibitor, ein intrazellulärer Cholesterol-Übertragungsinhibitor, ein Blutcholesterol-Absenkungsmittel oder ein die Makrophagenbildung unterdrückendes Mittel.

12. Verbindung, Salz oder Solvat, wie in mindestens einem der Ansprüche 1 bis 8 definiert, für die Verwendung zur Vorbeugung gegen Hyperlipämie, Arteriosklerose, zerebrovaskuläre Unfälle, ischämische Herzerkrankung, ischämische Darmerkrankung und aortisches Aneurysma.

## Revendications

1. Composé inhibiteur d'ACAT représenté par la formule (I) 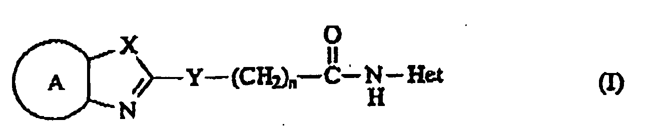 dans laquelle 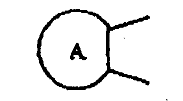 représente un benzène, une pyridine, un cyclohexane ou un naphtalène, facultativement substitués, X représente -NH-, un atome d'oxygène ou un atome de soufre, Y représente -NR₄-, un atome d'oxygène, un atome de soufre, un sulfoxyde ou une sulfone, R₄ représente un atome d'hydrogène, un groupe alkyle inférieur, un groupe aryle, ou un groupe silyl alkyle inférieur facultativement substitué, n est un entier de 1 à 15, et dans laquelle Het représente un groupe pyridyle ou pyrimidyle substitué ou non substitué, dans laquelle tout substituant sur Het est choisi parmi un groupe amino substitué par 1 ou 2 groupes alkyles inférieurs ayant chacun de 1 à 10 atomes de carbone, un groupe alcoxy inférieur ayant de 1 à 10 atomes de carbone, un groupe alkylthio inférieur ayant de 1 à 10 atomes de carbone, un groupe alkyl inférieur carbonyle ayant de 1 à 10 atomes de carbone, un atome d'halogène, un groupe amino ou un groupe alkyle inférieur ayant de 1 à 10 atomes de carbone, un groupe hydroxyle, un groupe phosphate, un groupe sulfonamide, un groupe alkyl inférieur sulfonyle ayant 1 à 10 atomes de carbone, ou deux substituants peuvent être liés pour former un groupe alkylènedioxy de 1 à 10 atomes de carbone,
ou un sel ou produit de solvatation de celui-ci.

2. Composé selon la revendication 1, représenté par la formule (i)

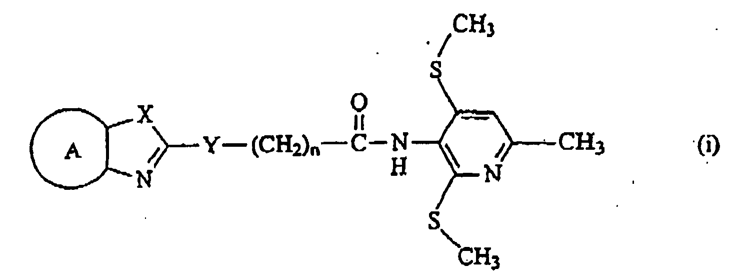

3. Composé selon la revendication 1, représenté par la formule (ii)

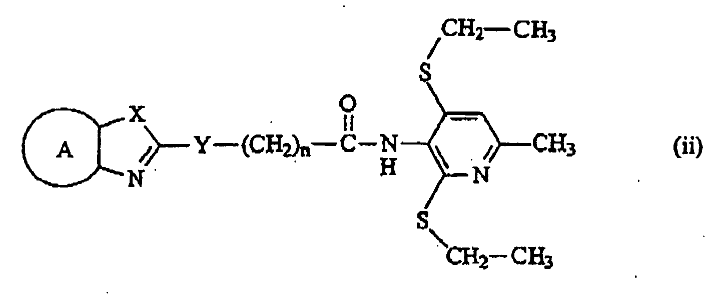

4. Composé selon la revendication 1, représenté par la formule (iii)

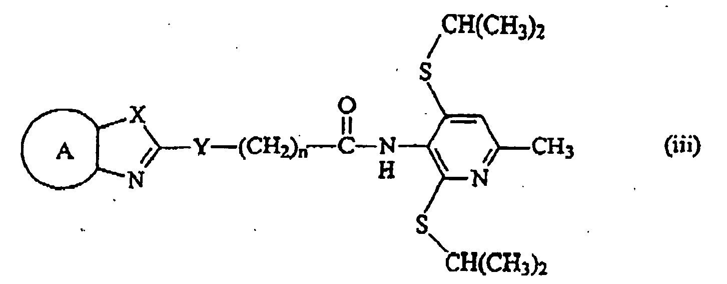

5. Composé selon la revendication 1, représenté par la formule (iv)

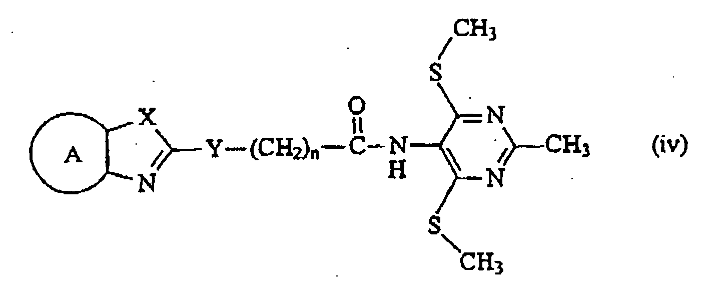

6. Composé selon la revendication 1, représenté par la formule (v)

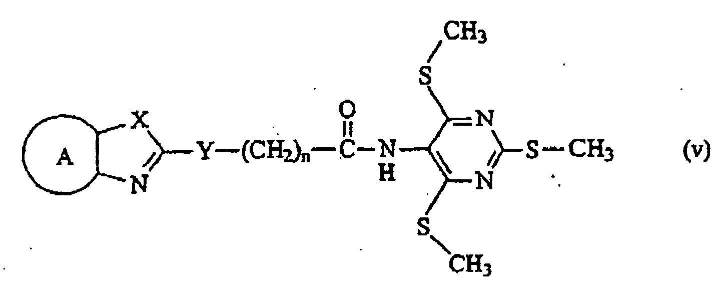

7. Composé selon la revendication 1, représenté par la formule (IA) 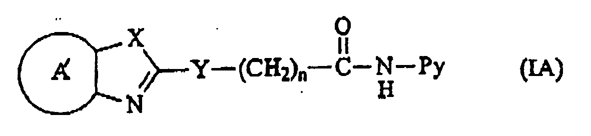 dans laquelle 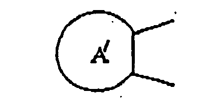 représente un benzène ou une pyridine facultativement substitués et Py représente un groupe pyridyle ou pyrimidyle facultativement substitué, dans laquelle tout substituant sur Py est choisi parmi un groupe amino substitué par 1 ou 2 groupes alkyles inférieurs ayant chacun de 1 à 10 atomes de carbone, un groupe alcoxy inférieur ayant de 1 à 10 atomes de carbone, un groupe alkylthio inférieur ayant de 1 à 10 atomes de carbone, un groupe alkyl inférieur carbonyle ayant de 1 à 10 atomes de carbone, un atome d'halogène, un groupe amino ou un groupe alkyle inférieur ayant de 1 à 10 atomes de carbone, un groupe hydroxyle, un groupe phosphate, un groupe sulfonamide, un groupe alkyl inférieur sulfonyle ayant 1 à 10 atomes de carbone, ou deux substituants peuvent être liés pour former un groupe alkylènedioxy de 1 à 10 atomes de carbone, ou un sel ou produit de solvatation de celui-ci.

8. Composé selon la revendication 1 ou la revendication 7, représenté par la formule (III) 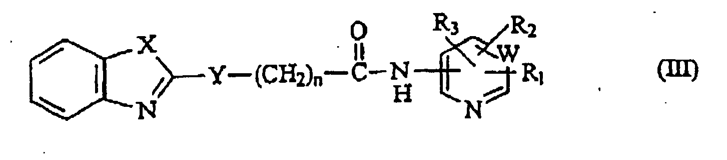 dans laquelle
W représente =CH- ou =N-,
R₁, R₂ et R₃ sont identiques ou différents, et chacun représente un atome d'hydrogène, un groupe alkyle inférieur ayant de 1 à 10 atomes de carbone, un groupe alcoxy inférieur ayant de 1 à 10 atomes de carbone, un atome d'halogène, un groupe hydroxyle, un groupe phosphate, un groupe sulfonamide, un groupe alkylthio inférieur ayant de 1 à 10 atomes de carbone, un groupe amino ou un groupe amino substitué par un groupe alkyle inférieur ayant de 1 à 10 atomes de carbone, ou deux parmi R₁, R₂ et R₃ forment ensemble un groupe alkylènedioxyde, ou un sel ou produit de solvatation de celui-ci.

9. Composition pharmaceutique comprenant au moins un composé selon l'une quelconque des revendications 1 à 8, ou un sel ou produit de solvatation de celui-ci, combiné avec un véhicule pharmaceutiquement acceptable.

10. Composé, sel ou produit de solvatation tel que défini dans l'une quelconque des revendications 1 à 8, pour l'utilisation dans un procédé de traitement du corps humain ou animal par thérapie.

11. Composé, sel ou produit de solvatation tel que défini dans l'une quelconque des revendications 1 à 8, pour l'utilisation en tant qu'inhibiteur d'ACAT, inhibiteur du transfert de cholestérol intracellulaire, dépresseur du cholestérol sanguin ou suppresseur de la formation des macrophages.

12. Composé, sel ou produit de solvatation tel que défini dans l'une quelconque des revendications 1 à 8, pour l'utilisation dans la prévention d'une hyperlipémie, d'une artériosclérose, d'accidents cérébrovasculaires, d'une cardiopathie ischémique, d'une maladie intestinale ischémique et d'un anévrisme aortique.
